(19) Europäisches Patentamt
European Patent Office
Office européen des brevets

(11) EP 4 567 032 A1

(12) **EUROPEAN PATENT APPLICATION**
published in accordance with Art. 153(4) EPC

(43) Date of publication:
**11.06.2025 Bulletin 2025/24**

(21) Application number: **23862449.8**

(22) Date of filing: **06.09.2023**

(51) International Patent Classification (IPC):
***C07D 401/12*** *(2006.01)* ***C07D 401/14*** *(2006.01)*
***A61K 31/438*** *(2006.01)* ***A61K 31/4427*** *(2006.01)*
***A61P 35/00*** *(2006.01)*

(52) Cooperative Patent Classification (CPC):
**A61K 31/438; A61K 31/4427; A61P 35/00; C07D 401/12; C07D 401/14**

(86) International application number:
**PCT/CN2023/117322**

(87) International publication number:
**WO 2024/051755 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.09.2022 CN 202211098225**
**16.11.2022 CN 202211436607**
**10.02.2023 CN 202310098701**
**30.08.2023 CN 202311107861**

(71) Applicant: **Changchun Genescience Pharmaceutical Co., Ltd.**
**Changchun, Jilin 130012 (CN)**

(72) Inventors:
- **LU, Biao**
  **Changchun, Jilin 130012 (CN)**
- **YANG, Fanglong**
  **Changchun, Jilin 130012 (CN)**
- **ZHANG, Chen**
  **Changchun, Jilin 130012 (CN)**
- **WANG, Siqin**
  **Changchun, Jilin 130012 (CN)**
- **JIN, Lei**
  **Changchun, Jilin 130012 (CN)**

(74) Representative: **Gille Hrabal Partnerschaftsgesellschaft mbB**
**Patentanwälte**
**Brucknerstraße 20**
**40593 Düsseldorf (DE)**

(54) **KIF18A INHIBITOR COMPOUND, AND PHARMACEUTICAL COMPOSITION AND PREPARATION METHOD THEREFOR AND USE THEREOF**

(57) The present invention provides a compound as represented by formula (I), and a pharmaceutical composition, and a preparation method therefor and a use thereof. The compound has a good inhibitory effect on KIF18A, can be used alone or in combination with microtubules to form a bonded complex to modulate a KIF18A protein for treatment of KIF18A-mediated conditions and/or diseases, such as tumors, and for preparation of a drug for such conditions or diseases.

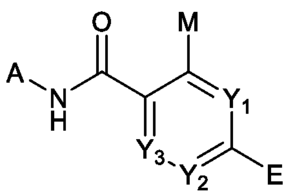

(I)

EP 4 567 032 A1

## Description

The invention claims:

priority to a prior application filed with the China National Intellectual Property Administration on September 8, 2022, having Patent Application No. 2022110982256 and entitled "KIF18A inhibitor compound, and pharmaceutical composition and preparation method therefor and use thereof";
priority to a prior application filed with the China National Intellectual Property Administration on November 16, 2022, having Patent Application No. 2022114366075 and entitled "KIF18A inhibitor compound, and pharmaceutical composition and preparation method therefor and use thereof";
priority to a prior application filed with the China National Intellectual Property Administration on February 10, 2023, having Patent Application No. 2023100987012 and entitled "KIF18A inhibitor compound, and pharmaceutical composition and preparation method therefor and use thereof";
priority to a prior application filed with the China National Intellectual Property Administration on August 30, 2023, having Patent Application No. 2023111078615 and entitled "KIF18A inhibitor compound, and pharmaceutical composition and preparation method therefor and use thereof";
and the above prior applications are incorporated herein by reference in their entirety.

## TECHNICAL FIELD

The present invention belongs to the field of medicine technology, and specifically relates to a KIF18A inhibitor compound, a pharmaceutical composition and a preparation method and use thereof.

## BACKGROUND ART

Cancer is one of the most serious diseases affecting human health, and its mortality and morbidity rates often rank among the highest among all diseases. Although the quality of life of some patients has been greatly improved with the continuous development and progress of medical technology and drug research and development, there are still more unmet clinical needs in the search for effective treatments or cures for different cancers, and more new targets will provide new possibilities for future cancer drug development.

Cancer cells experience unregulated cell proliferation due to damage or loss of one or more genes that regulate the cell cycle. Various kinases and kinesins have been identified to play a key role in cell cycle and mitotic regulation and progression in both normally dividing cells and cancer cells.

Kinesins are motor proteins, also called dyneins, that use intracellular microtubules as tracks. They can convert ATP energy into mechanical energy and are closely related to eukaryotic cells and cell mitosis and meiosis, the growth and development of tissues and organs, and the development and signal transduction of neurons. The kinesin members share a relatively conserved motor domain. Based on the location of the motor domain in the molecule, the kinesin family can be roughly divided into three types: N-type kinesins, which have a motor domain in the amino ($-NH_2$) terminal region of the protein polypeptide chain; M-type kinesins, which have a motor domain in the middle region; and C-type kinesins, which have a motor domain in the carboxyl (-COOH) terminal region.

Chromosomal instability is a hallmark of cancer and results from errors in chromosome segregation during mitosis. Targeting chromosomal instability is an emerging therapeutic strategy in drug development. KIF18A, a member of the N-type Kinesin-8 kinesin family, has been shown to play a role in maintaining the integrity of the bipolar spindle and promoting the viability of cancer cells with chromosomal instability. Mitosis is an effective point of intervention, and many anti-mitotic drugs are used clinically to treat human cancers. The most widely used tubulin inhibitor can both stabilize microtubules and prevent microtubule assembly. Current anti-mitotic drugs have the limitation of narrow therapeutic window, and these problems need to be solved by developing new targets.

Although tubulin inhibitors are widely used as standard treatments for a variety of human cancer types, these drugs are associated with collateral damage to normal cells, including myelosuppression and neurotoxicity. Since KIF18A may not be essential in normal diploid somatic cells (KIF18A knockout mice are viable but have reproductive defects, indicating that KIF18A is not an essential gene for normal somatic cell division), targeting KIF18A may significantly reduce its toxicity, which will help to improve the therapeutic safety window of drugs targeting tubulin in clinical practice.

KIF18A protein is highly expressed in a variety of tumors, including colon cancer, breast cancer, lung cancer, pancreatic cancer, prostate cancer, bladder cancer, head and neck cancer, cervical cancer, and ovarian cancer. KIF18A plays a key role in the occurrence, development and metastasis of breast cancer, and its high expression indicates poor prognosis of patients. KIF18A is necessary for the proliferation of cells with chromosomal instability derived from triple-negative breast cancer or colorectal cancer, but KIF18A is not required in diploid cells. Knockout of the KIF18A gene can cause infertility in male mice, but not in female mice. KIF18A mRNA expression is significantly associated with higher

tumor grade and larger tumor size in breast cancer patients, and KIF18A is an independent predictor of breast cancer lymph node metastasis with a risk coefficient of 3.2. In addition, inhibition of KIF18A expression not only affects the key function of KIF18A in mitosis, but also reduces cancer cell migration by stabilizing front microtubules, which eventually leads to inactivation of the PI3K-AKT signaling pathway and induces apoptosis.

Therefore, the development of KIF18A protein inhibitors may be a new breakthrough in cancer drugs.

## SUMMARY OF THE INVENTION

In order to improve the above technical problems, the present invention provides a compound represented by formula (I), a racemate, stereoisomer, tautomer, isotope-labeled counterpart, solvate, polymorph, pharmaceutically acceptable salt thereof or a prodrug compound thereof:

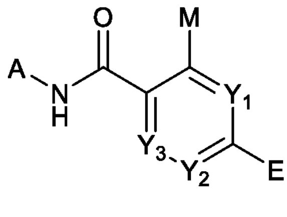

(I)

wherein, A is selected from fused ring groups which are unsubstituted or optionally substituted by one, two or more $R_a$ groups; the fused ring group comprises two, three or four rings independently selected from a saturated or partially unsaturated $C_{3-14}$carbon ring, a $C_{6-14}$aromatic ring, a 5 to 14-membered heteroaromatic ring, or a 3 to 14-membered heterocyclic ring;

the $R_a$ groups are each identical or different and are independently selected from H, OH, halogen, cyano, $NH_2$, $NO_2$, the following groups which are unsubstituted or optionally substituted by one, two or more $R_{a1}$ groups: $C_{1-12}$ alkyl, $C_{1-12}$ alkoxy, $C_{3-12}$ cycloalkyl; or two $R_a$ groups attached to the same ring carbon atom together with the carbon atom to which they are attached form a saturated or partially unsaturated $C_{3-14}$ carbon ring; or, two non-adjacent $R_a$ groups are linked at their end groups to form $C_{1-3}$ alkylene; the $R_{a1}$ groups are each identical or different and are independently selected from H, OH, halogen, cyano, $NH_2$, $NO_2$, $C_{1-12}$ alkyl, $C_{1-12}$ alkoxy, $C_{3-12}$ cycloalkyl;

E is selected from the following groups which are unsubstituted or optionally substituted by one, two or more $R_e$ groups: $-NH-S(=O)_2-R_{e1}$, $-S(=O)_2-NH-R_{e2}$, $-S(=O)(=NH)-R_{e3}$, $-N(R_{e4})(R_{e5})$, 3- to 14-membered heterocyclic groups; the $R_e$ groups are each identical or different and are independently selected from OH, halogen, cyano, $C_{1-12}$ alkyl, $C_{1-12}$ alkoxy, halo-$C_{1-12}$ alkyl, halo-$C_{1-12}$ alkoxy, cyano-$C_{1-12}$ alkyl, cyano-$C_{1-12}$ alkoxy, $-N(R_{e6})(R_{e7})$;

the $R_{e1}$, $R_{e2}$, $R_{e3}$, $R_{e4}$, $R_{e5}$, $R_{e6}$, $R_{e7}$ groups are each identical or different and are independently selected from H, $C_{1-12}$ alkyl, hydroxy-$C_{1-12}$ alkyl, halo-$C_{1-12}$ alkyl, halo-$C_{1-12}$ alkoxy, cyano-$C_{1-12}$ alkyl, cyano-$C_{1-12}$ alkoxy, $C_{3-12}$ cycloalkyl, 3- to 14-membered heterocyclic groups, $C_{1-12}$ alkoxy-$C_{1-12}$ alkyl;

M is selected from $C_{3-12}$ cycloalkyl, $C_{3-12}$ cycloalkenyl or nitrogen-containing 3- to 14-membered heterocyclic groups, which are unsubstituted or optionally substituted by one, two or more $R_m$ groups; the $R_m$ groups are each identical or different and are independently selected from H, halogen, cyano, $C_{1-12}$ alkyl, halo-$C_{1-12}$ alkyl, cyano-$C_{1-12}$ alkyl, $C_{1-12}$ alkoxy, cyano-$C_{1-12}$ alkoxy; or, two $R_m$ groups attached to the same ring carbon atom together with the carbon atom to which they are attached form a saturated or partially unsaturated $C_{3-14}$ carbon ring;

$Y_1$, $Y_2$, $Y_3$ are identical or different and are independently selected from N or CH.

According to some embodiments, A is selected from fused ring groups which are unsubstituted or optionally substituted by one, two or more $R_a$ groups; the fused ring group comprises two, three or four rings independently selected from a saturated or partially unsaturated $C_{3-8}$ carbon ring, a $C_{6-1O}$ aromatic ring, a 5- to 10-membered heteroaromatic ring, a 3- to 8-membered heterocyclic ring;

the $R_a$ groups are each identical or different and are independently selected from H, OH, halogen, cyano, $NH_2$, $NO_2$, $C_{1-6}$ alkyl, $C_{1-6}$ alkoxy, $C_{3-8}$ cycloalkyl, halo-$C_{1-6}$ alkyl, halo-$C_{1-6}$ alkoxy, cyano-$C_{1-6}$ alkyl, cyano-$C_{1-6}$ alkoxy, $C_{3-8}$ cycloalkyl-$C_{1-6}$ alkoxy; or, two $R_a$ groups attached to the same ring carbon atom together with the carbon atom to which they are attached form a saturated or partially unsaturated $C_{3-8}$ carbon ring; or, two non-adjacent $R_a$ groups are linked at their end groups to form $C_{1-3}$ alkylene;

According to some embodiments, A is selected from fused ring groups which are unsubstituted or optionally substituted by one, two, or more $R_a$ groups; the fused ring group is selected from

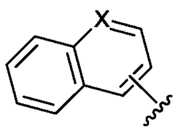, 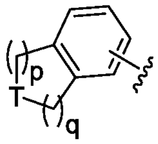, 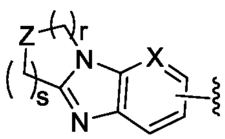;

wherein, T is selected from $CH_2$, CH, NH, $NR_a$ or O;
Z is selected from $CH_2$, CH, NH, $NR_a$ or O;
X is selected from N or CH;
p and q are independently selected from 0, 1, 2, and 3, and p and q are not 0 at the same time;
r and s are independently selected from 0, 1, 2, and 3, and r and s are not 0 at the same time.

According to some embodiments, A is selected from fused ring groups which are unsubstituted or optionally substituted by one, two, or more $R_a$ groups; the fused ring group is selected from

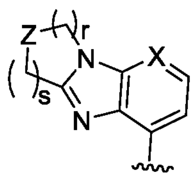, 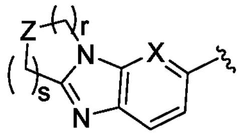 or 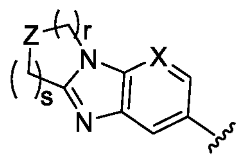;

wherein, Z is selected from $CH_2$, CH, NH, $NR_a$ or O;
X is selected from N or CH;
r and s are independently selected from 0, 1, 2, and 3, and r and s are not 0 at the same time.

According to some embodiments, A is selected from

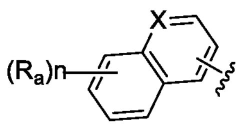, 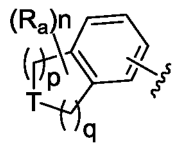,

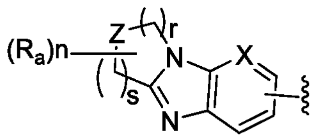;

wherein, T is selected from $CH_2$, CH, NH, $NR_a$ or O;
Z is selected from $CH_2$, CH, NH, $NR_a$ or O;
X is selected from N or CH;
n is selected from 0, 1, 2, 3, 4, or 5;
p and q are independently selected from 0, 1, 2, and 3, and p and q are not 0 at the same time;
r and s are independently selected from 0, 1, 2, and 3, and r and s are not 0 at the same time.

According to some embodiments, A is selected from fused ring groups which are unsubstituted or optionally substituted by one, two or more $R_a$ groups; the fused ring group is formed by fusing two, three, four or more rings selected from a benzene ring, a pyridine ring, an imidazole ring, a piperazine ring, a piperidine ring, a tetrahydropyrrole ring, a tetrahydropyran ring, a tetrahydrofuran ring, a furan ring, a morpholine ring, a cyclopropane ring, a cyclobutane ring, a cyclopentane ring, a cyclohexane ring, a cycloheptane ring, and a cycloheximide ring.

According to some embodiments, A is selected from

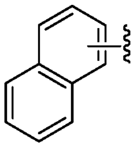, 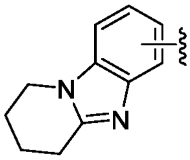,

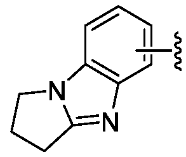, 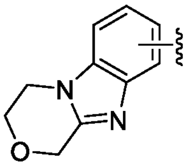, 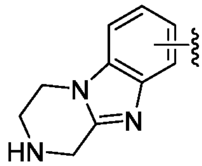, 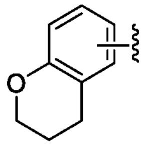, 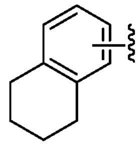, 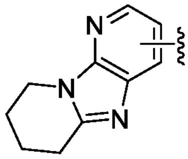,

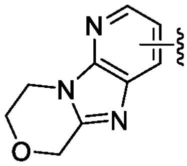, 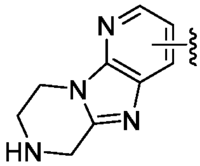, 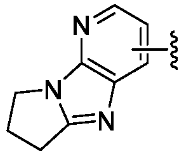, 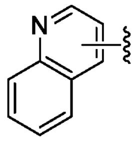, 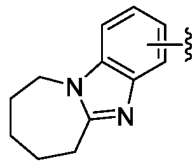, 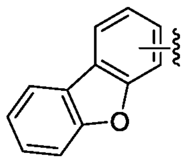,

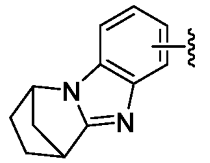

which are unsubstituted or optionally substituted by one, two, or more $R_a$ groups;

According to some embodiments, the $R_a$ groups are each identical or different and are independently selected from H, OH, F, Cl, cyano, methyl, ethyl, isopropyl, methoxy, ethoxy, trifluoromethyl, difluoromethoxy, trifluoromethoxy, cyclopropyl, cyclopropylmethoxy; or, two $R_a$ groups attached to the same ring carbon atom together with the carbon atom to which they are attached form a cyclopropane ring, a cyclobutane ring, a cyclopentane ring, a cyclohexane ring, a cycloheptane ring; or two non-adjacent $R_a$ groups are linked at their end groups to form methylene or ethylene.

According to some embodiments, A is selected from

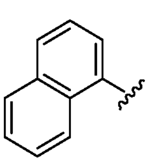, 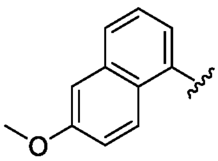,

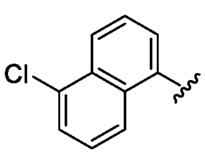, 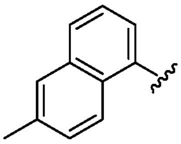, 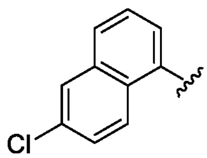, 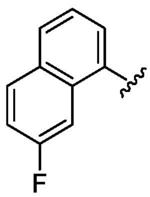, 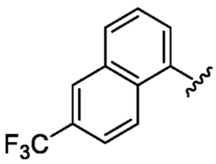, 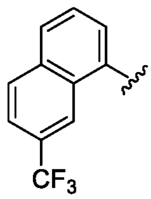,

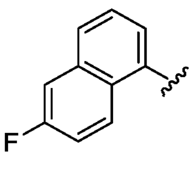, 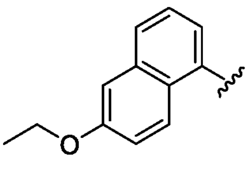, 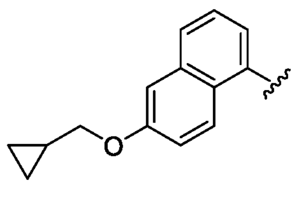, 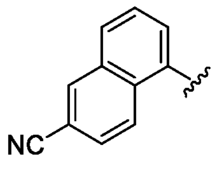,

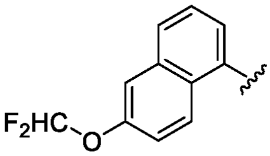, 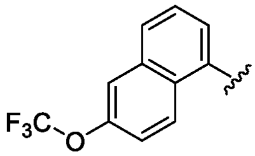, 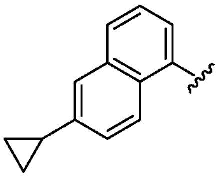, 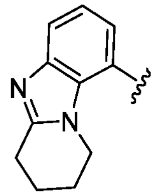, 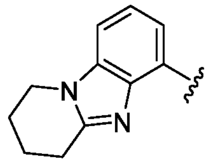,

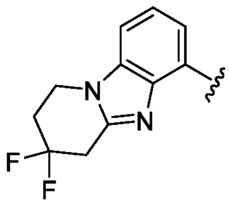, 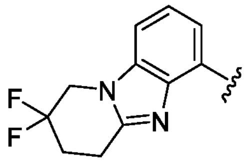, 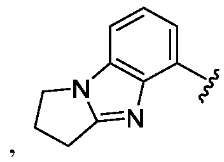, 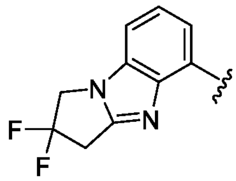, 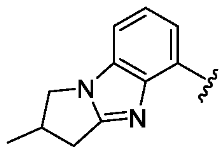,

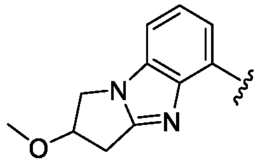, 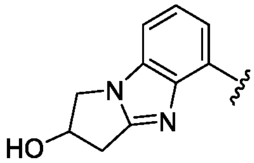, 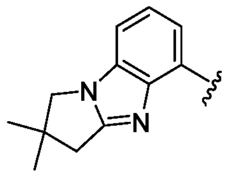, 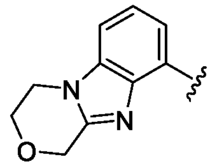,

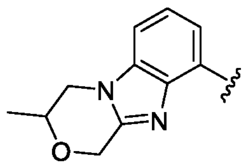, 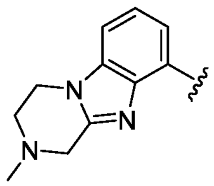, 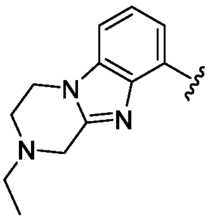, 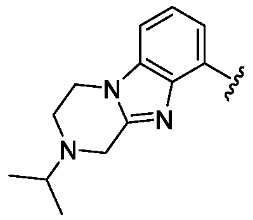, 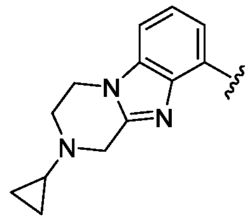,

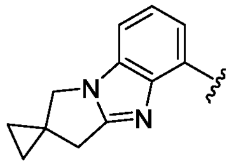, 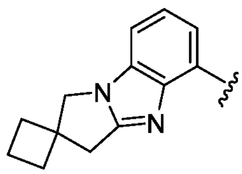, 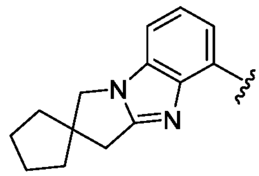, 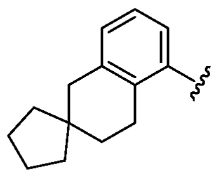, 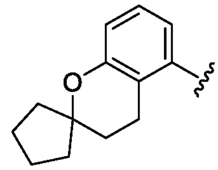,

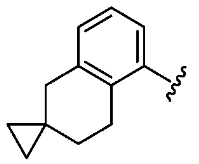, 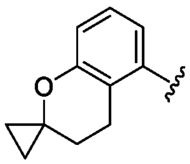, 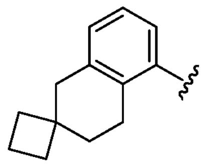, 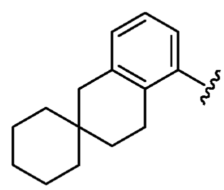, 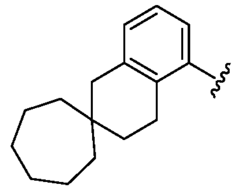,

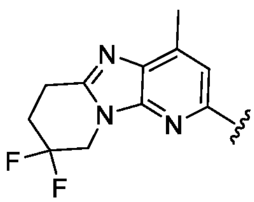, 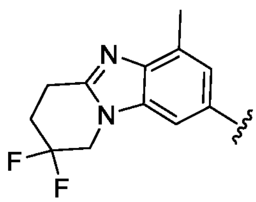, 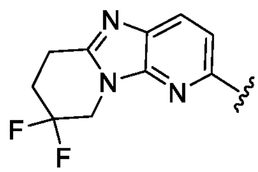, 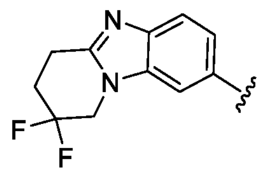,

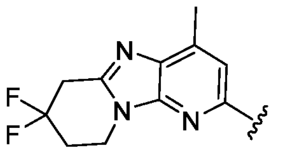 , 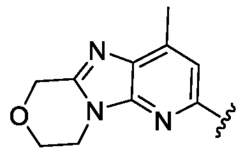 , 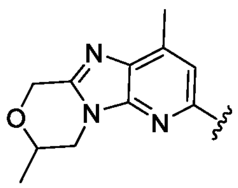 , 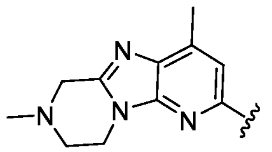 ,

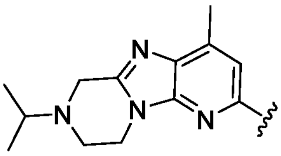 , 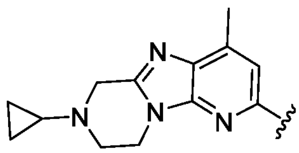 , 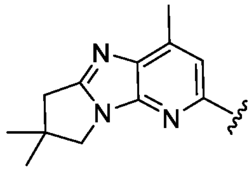 , 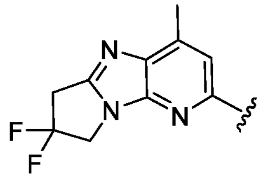 ,

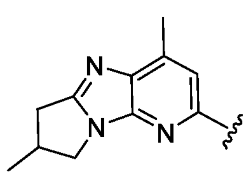 , 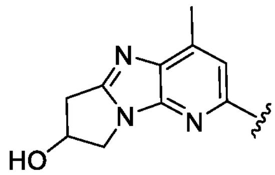 , 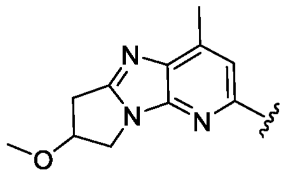 , 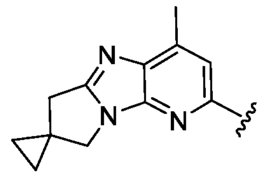 ,

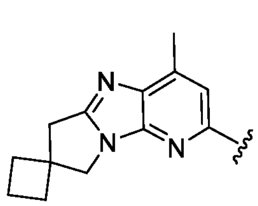 , 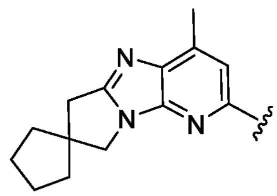 , 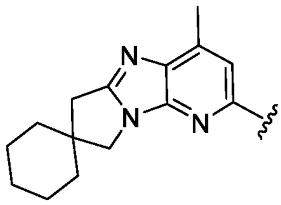 , 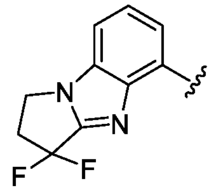 ,

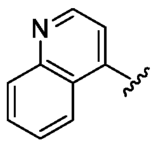 , 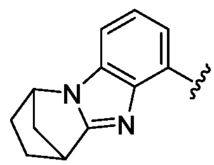 , 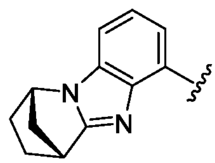 , 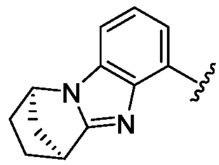 , 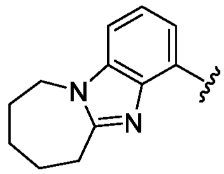 ,

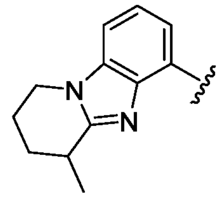 , 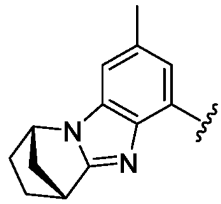 , 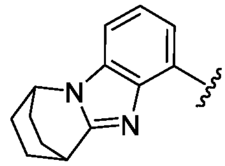 , 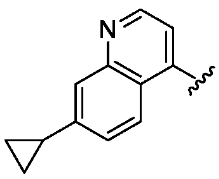 , 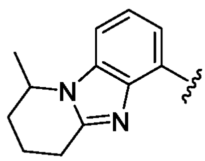 ,

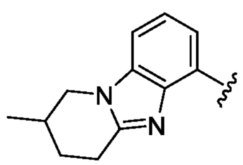 , 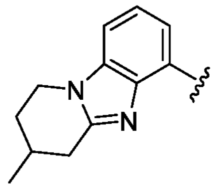 , 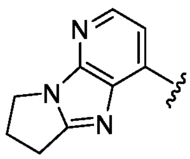 , 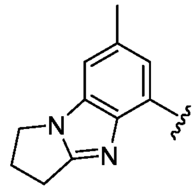 , 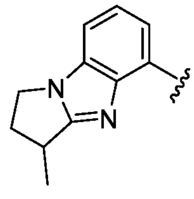 ,

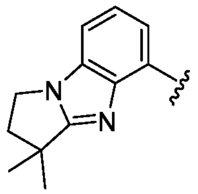, 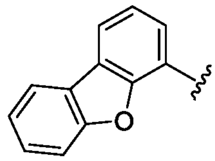, 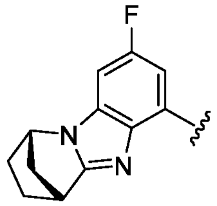.

According to some embodiments, E is selected from the following groups which are unsubstituted or optionally substituted by one or two $R_e$ groups: -NH-S(=O)$_2$-$R_{e1}$, -S(=O)$_2$-NH-$R_{e2}$, -S(=O)(=NH)-$R_{e3}$; the $R_{e1}$, $R_{e2}$, $R_{e3}$ groups are each identical or different and are independently selected from H, $C_{1-6}$ alkyl, hydroxy-$C_{1-6}$ alkyl, halo-$C_{1-6}$ alkyl, halo-$C_{1-6}$ alkoxy, cyano-$C_{1-6}$ alkyl, cyano-$C_{1-6}$ alkoxy, $C_{3-8}$ cycloalkyl, 3- to 8-membered heterocyclic groups, $C_{1-6}$ alkoxy-$C_{1-6}$ alkyl; the $R_e$ groups are each identical or different and are independently selected from OH, halogen, cyano, $C_{1-6}$ alkyl, $C_{1-6}$ alkoxy, halo-$C_{1-6}$ alkyl, halo-$C_{1-6}$ alkoxy, cyano-$C_{1-6}$ alkyl, cyano-$C_{1-6}$ alkoxy.

According to some embodiments, E is selected from

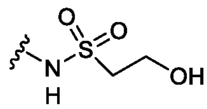.

According to some embodiments, M is selected from $C_{3-8}$ cycloalkyl, $C_{3-8}$ cycloalkenyl or nitrogen-containing 3- to 8-membered heterocyclic groups, which are unsubstituted or optionally substituted by one, two or more $R_m$ groups; the $R_m$ groups are each identical or different and are independently selected from H, halogen, cyano, $C_{1-6}$ alkyl, halo-$C_{1-6}$ alkyl, cyano-$C_{1-6}$ alkyl, $C_{1-6}$ alkoxy and cyano-$C_{1-6}$ alkoxy; or, two $R_m$ groups attached to the same ring carbon atom together with the carbon atom to which they are attached form a saturated or partially unsaturated $C_{3-8}$ carbon ring;

According to some embodiments, M is selected from

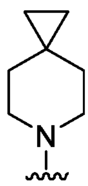, 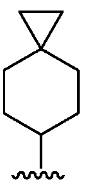 or 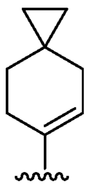.

According to some embodiments, $Y_1$, $Y_2$, $Y_3$ are not N at the same time;
According to some embodiments, when $Y_1$ is CH, $Y_2$ is CH, $Y_3$ is N or CH; when $Y_1$ is CH, $Y_2$ is N, $Y_3$ is N or CH; when $Y_1$ is N, $Y_2$ is CH, $Y_3$ is N or CH; when $Y_1$ is N, $Y_2$ is N, $Y_3$ is CH.

According to some embodiments, the compound represented by formula (I) has the following structure:

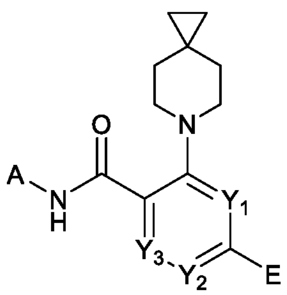
IA-1

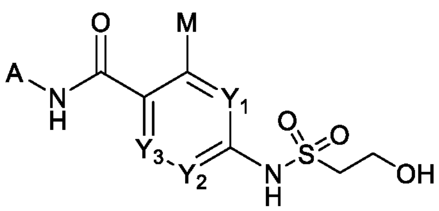
IA-2

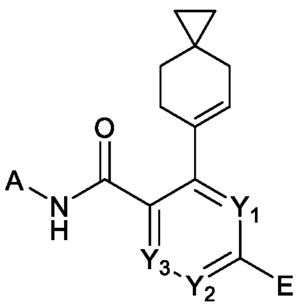
IA-3

wherein, A, M, E, $Y_1$, $Y_2$, and $Y_3$ are independently defined as described herein.

According to some embodiments, the compound represented by formula (I) has the following structure:

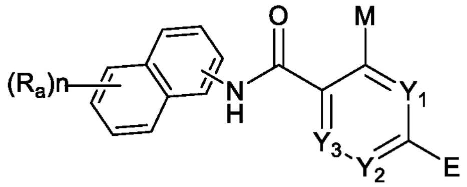

IB-1

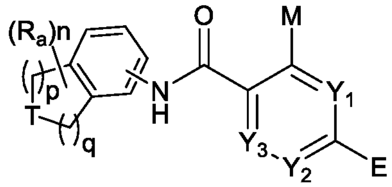

IB-2

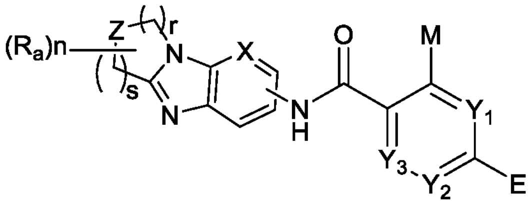

IB-3

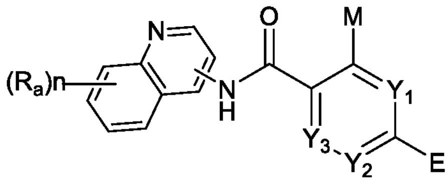

IB-4

wherein, M, E, $Y_1$, $Y_2$, $Y_3$, $R_a$, X, Z, T, n, p, q, r, s are independently defined as described herein.

According to some embodiments, the compound represented by formula (I) has the following structure:

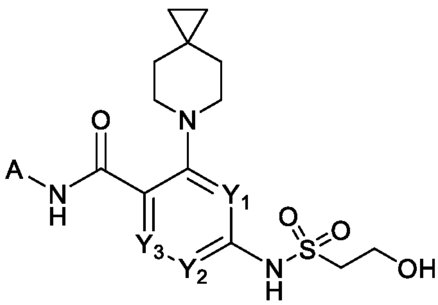

IC-1

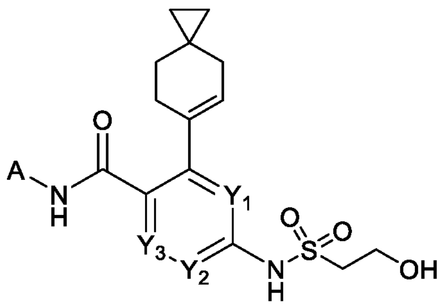

IC-2

wherein, A, $Y_1$, $Y_2$, and $Y_3$ are independently defined as described herein.

According to some embodiments, the compound represented by formula (I) has the following structure:

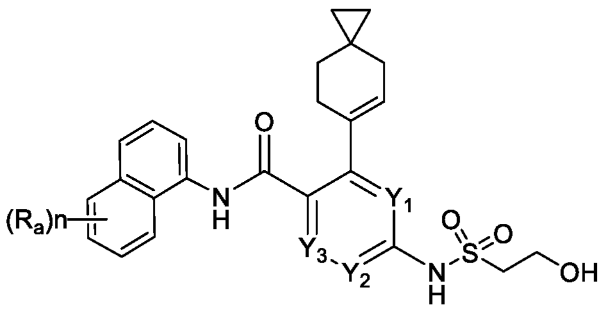

ID-1

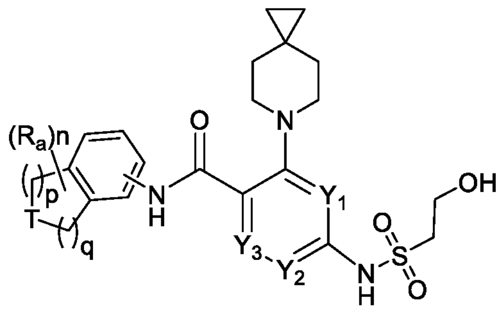

ID-2

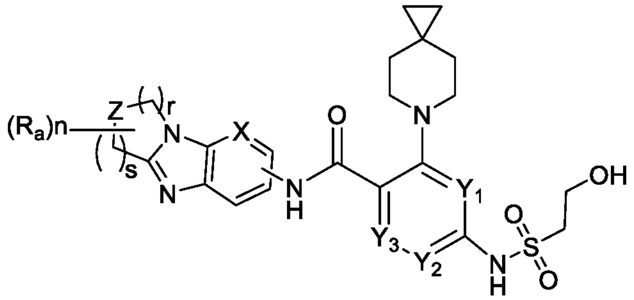

ID-3

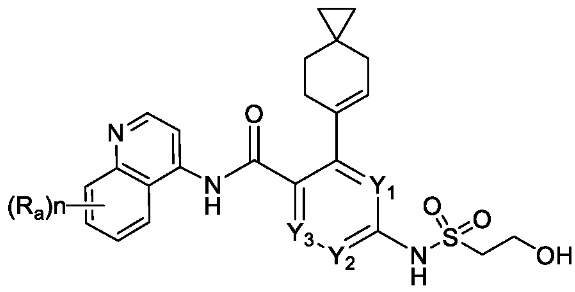

ID-4

wherein, $Y_1$, $Y_2$, $Y_3$, $R_a$, X, Z, T, n, p, q, r, s are independently defined as described herein.

According to some embodiments, the compound represented by formula (I) has the following structure:

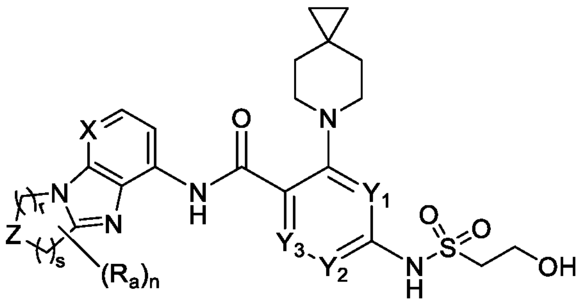

IE-1

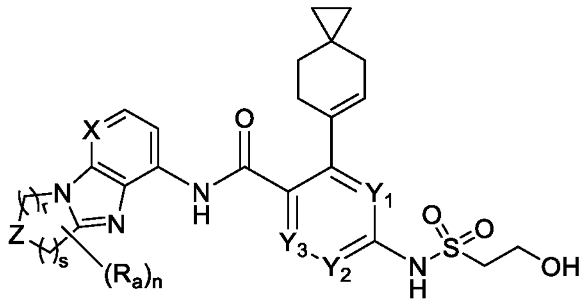

IE-2

wherein, $Y_1$, $Y_2$, $Y_3$, $R_a$, X, Z, n, r, s are independently defined as described herein.

According to some embodiments, the compound represented by formula (I) are selected from the following structures:

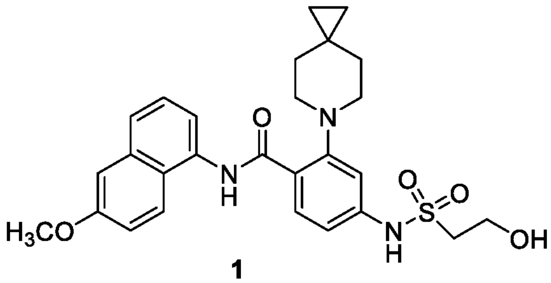

**1** ,

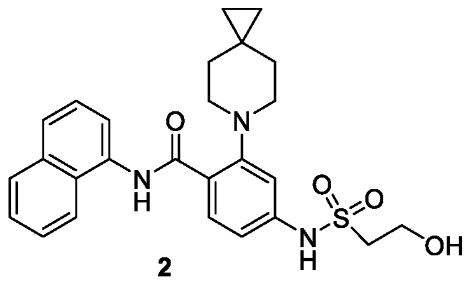

**2** ,

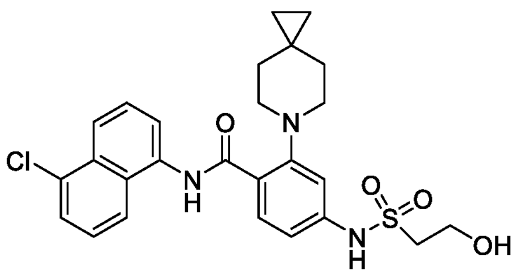

**3** ,

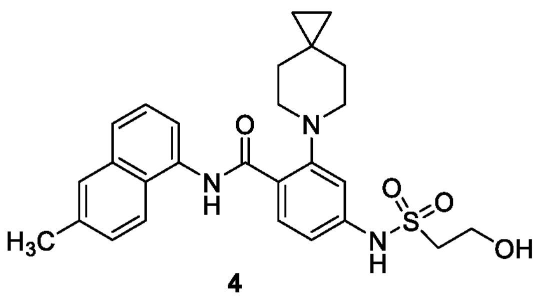

**4** ,

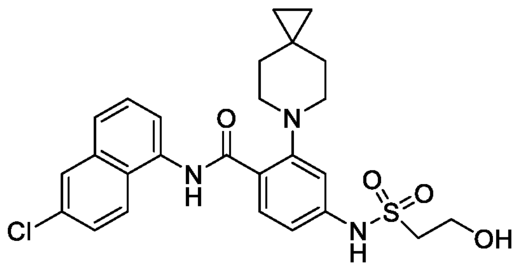

**5**

,

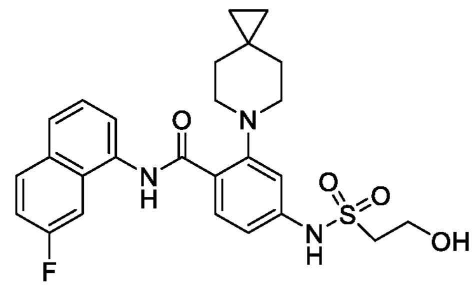

**6**

,

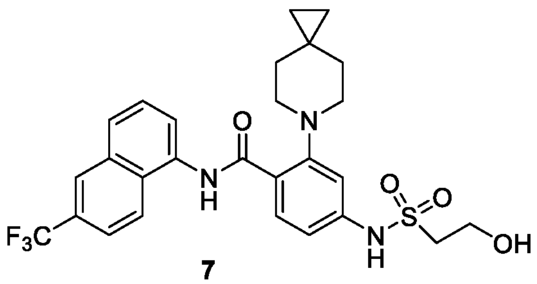

**7**

,

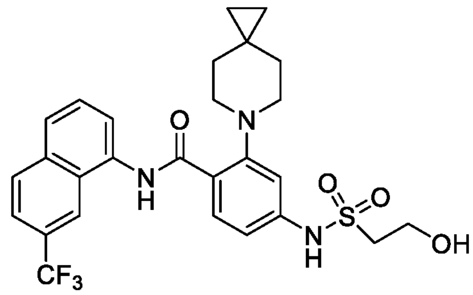

**8**

,

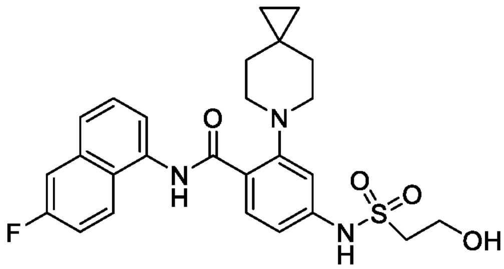

**9**

,

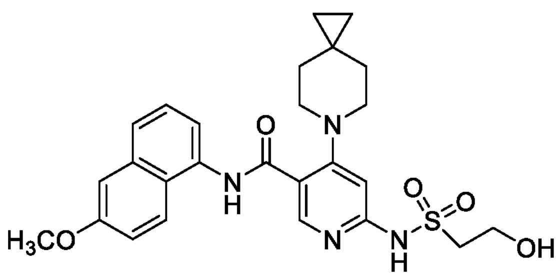

**10**

,

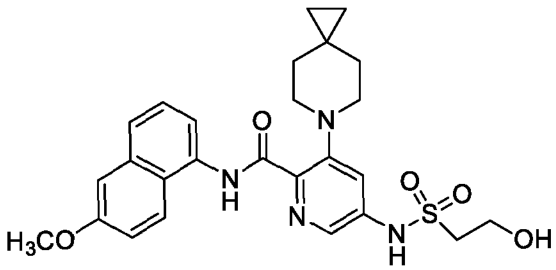

**11**

,

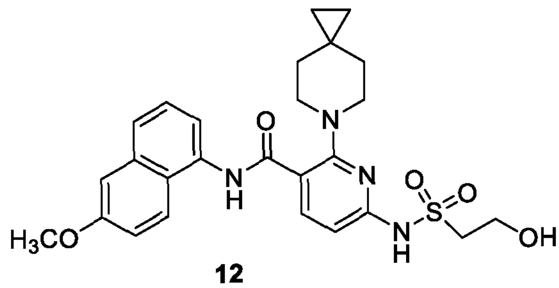

**12**

,

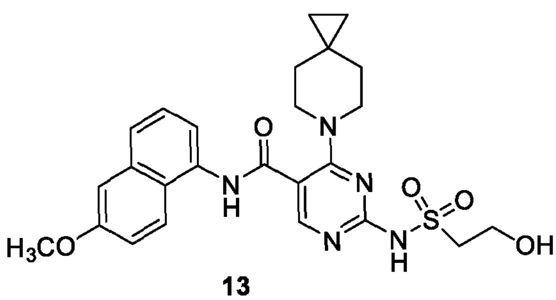

**13**

,

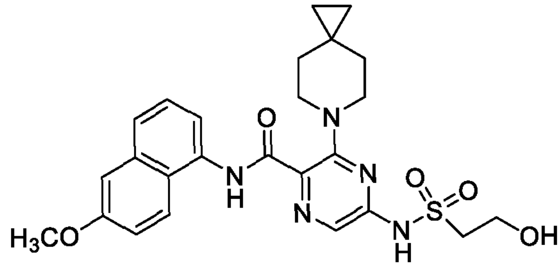

**14**

,

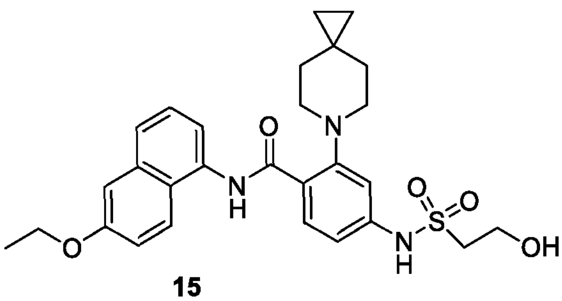

15

,

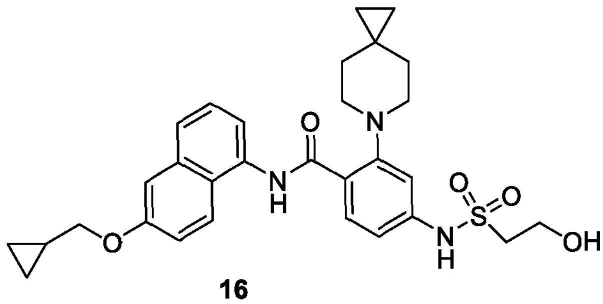

16

,

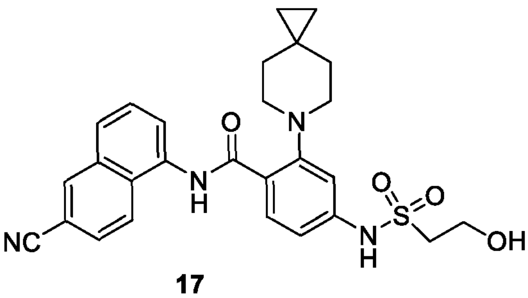

17

,

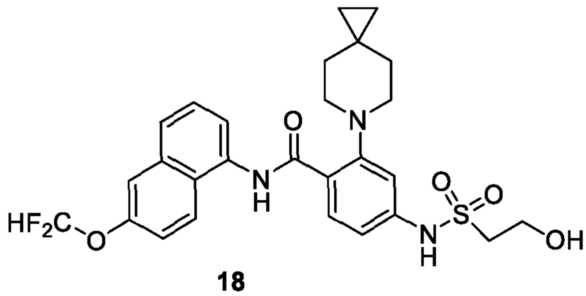

18

,

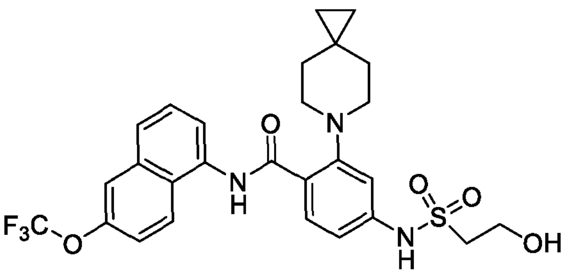

19

,

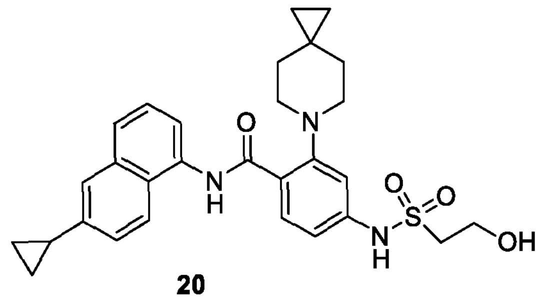

20

,

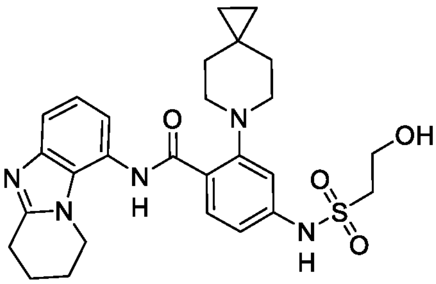

21

,

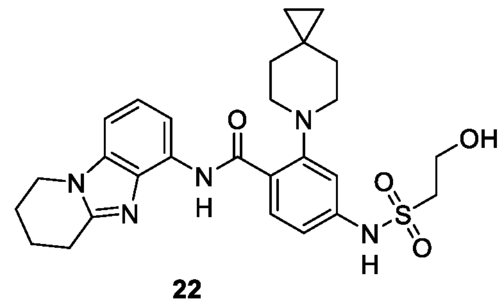

22

,

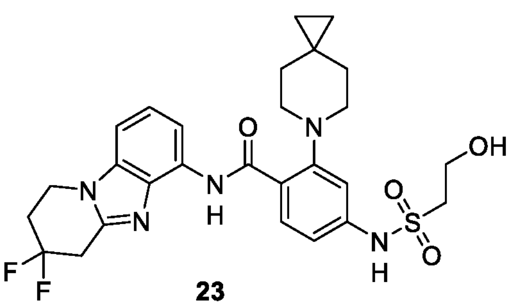

23

,

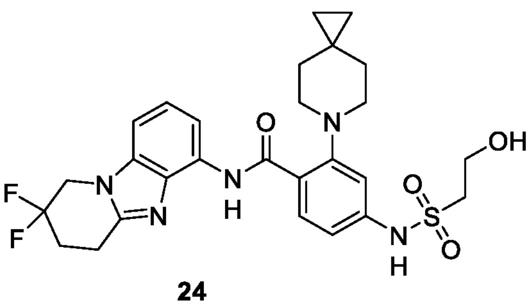

24

,

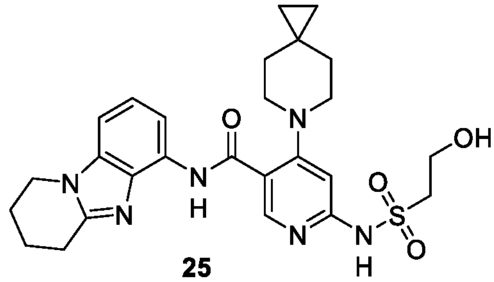

25

,

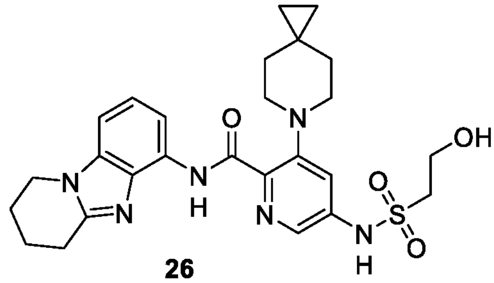

26

,

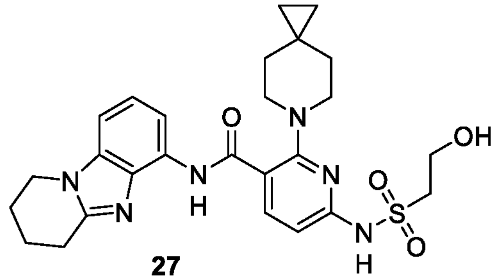

27

,

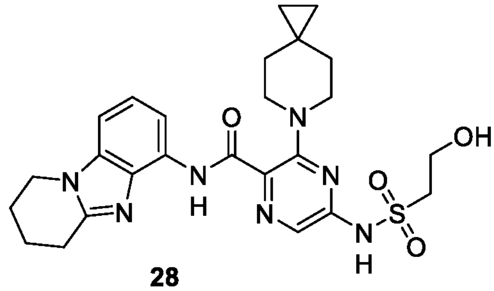

28

,

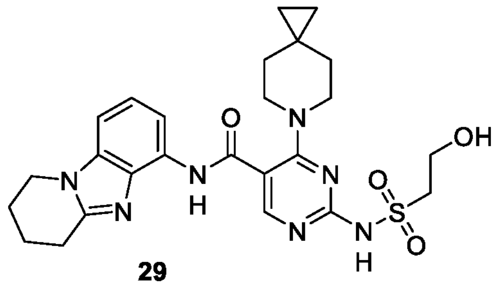

29

,

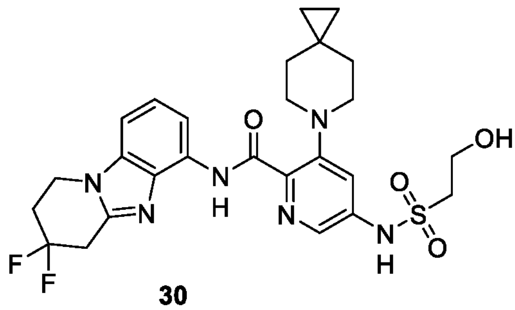

30

,

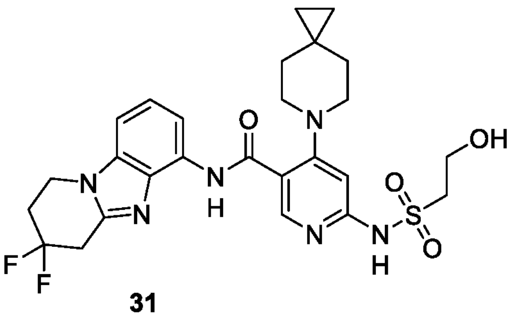

31

,

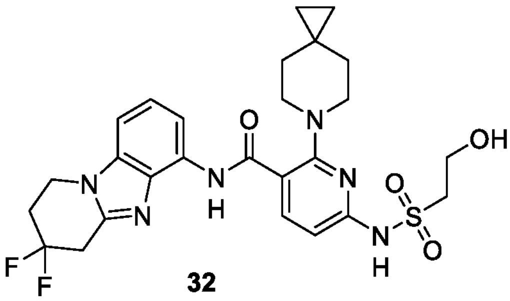

32

,

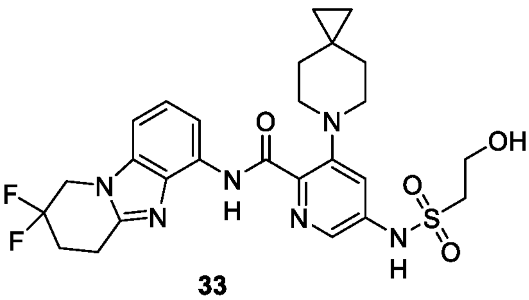

33

,

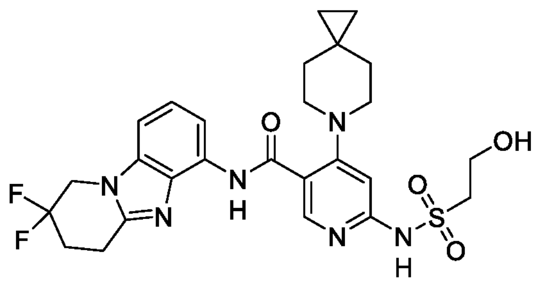

34

,

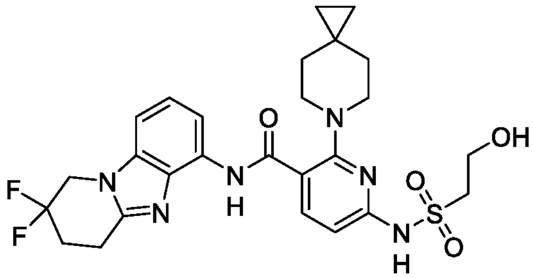

**35** ,

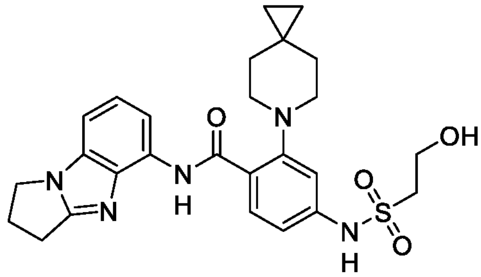

**36** ,

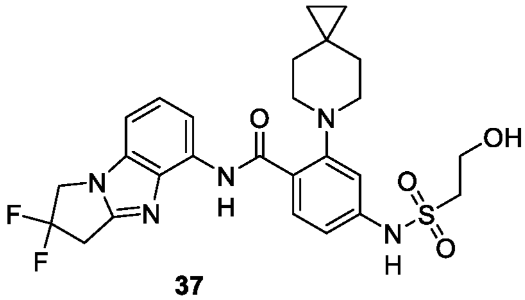

**37** ,

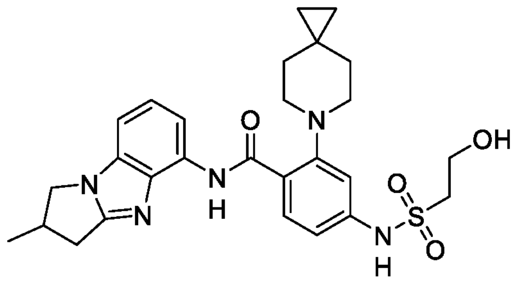

**38** ,

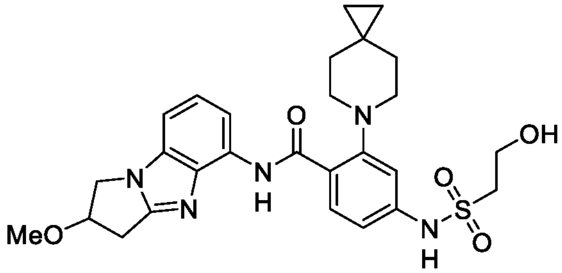

**39** ,

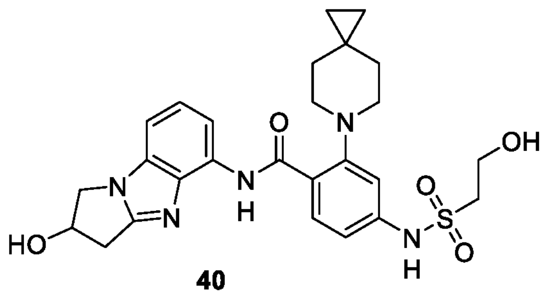

**40** ,

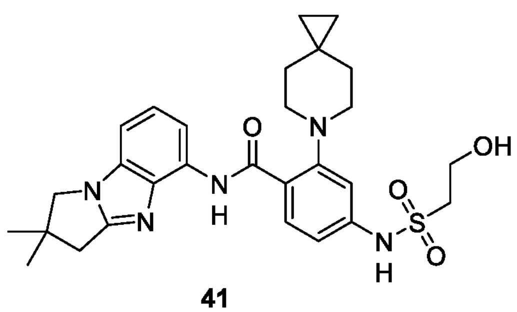

**41** ,

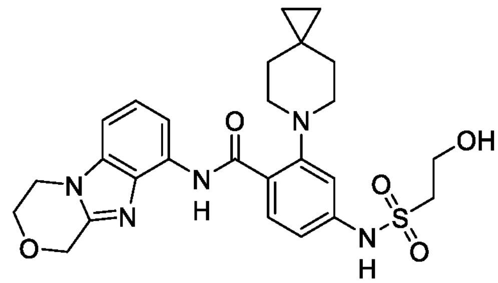

**42** ,

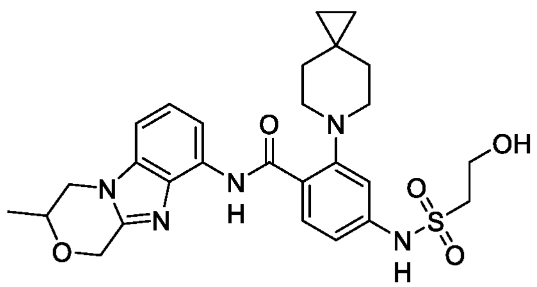

**43** ,

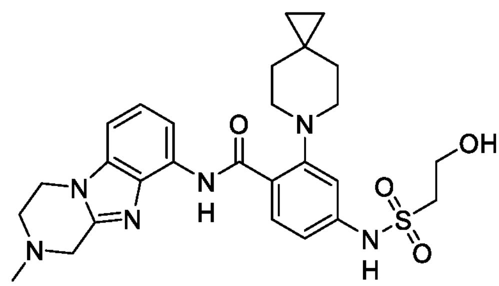

**44** ,

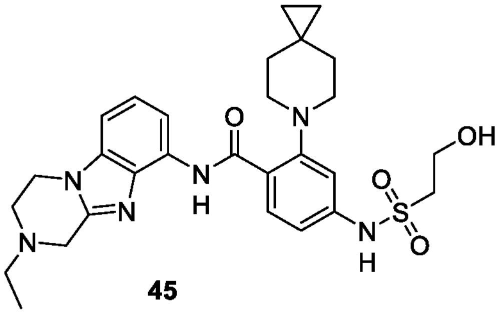
45
,

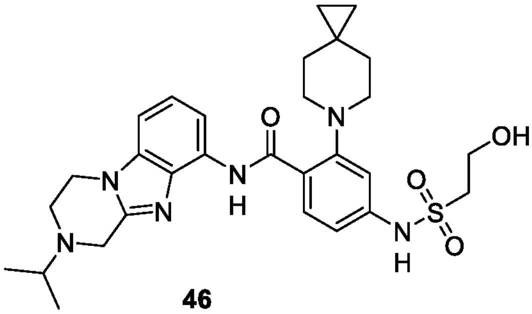
46
,

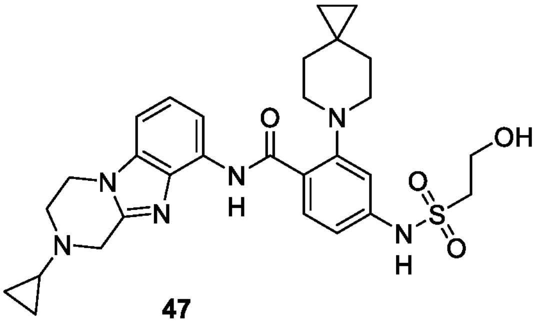
47
,

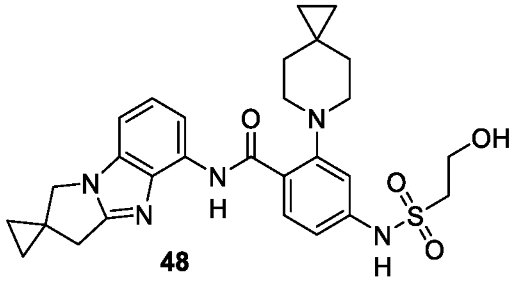
48
,

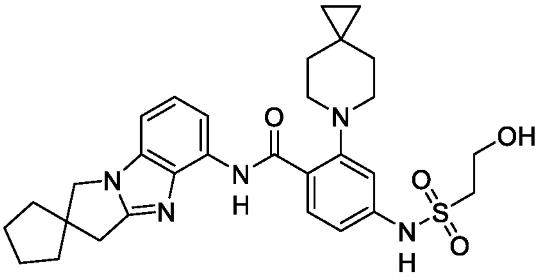
49
,

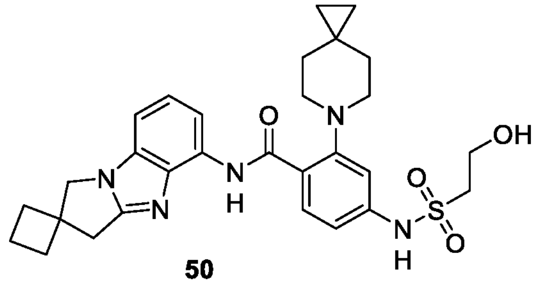
50
,

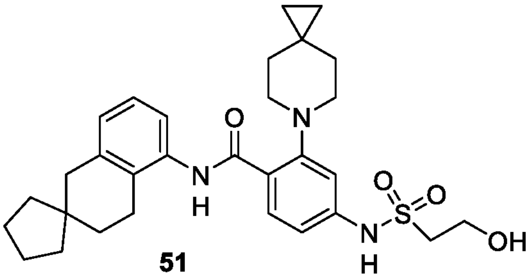
51
,

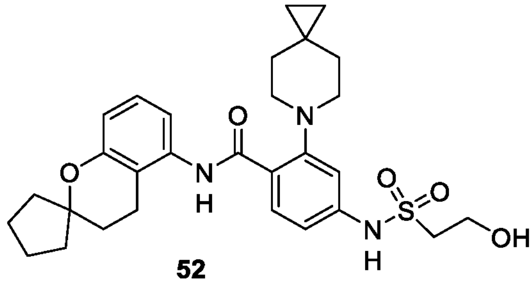
52
,

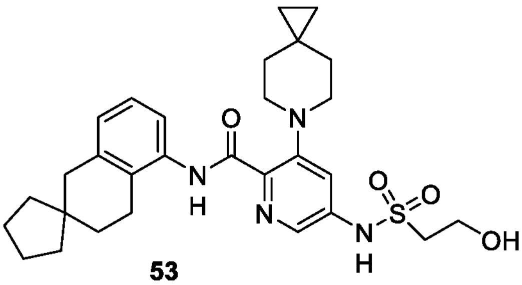
53
,

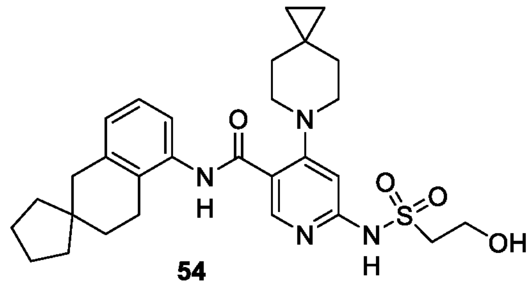
54
,

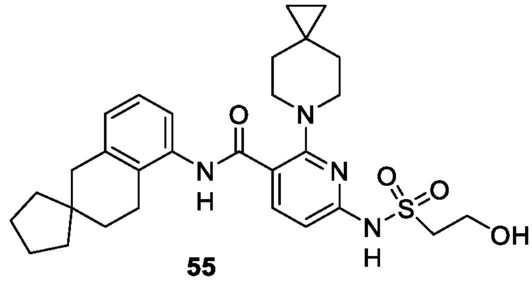

55

,

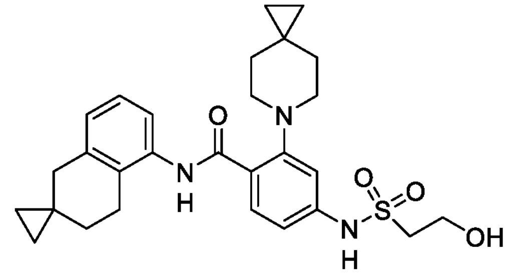

56

,

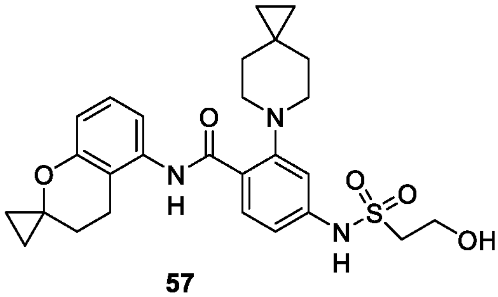

57

,

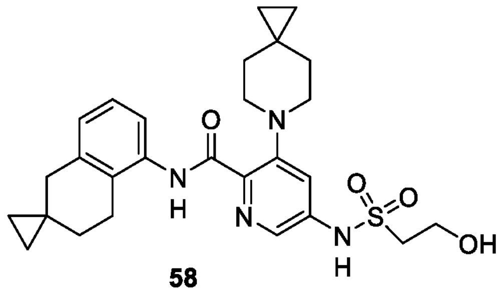

58

,

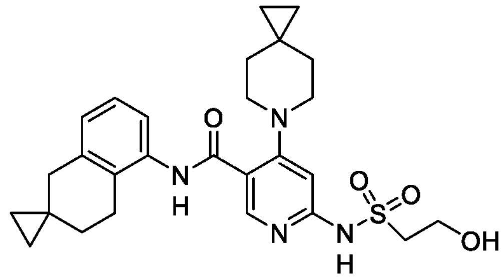

59

,

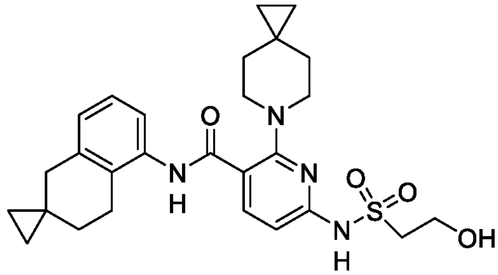

60

,

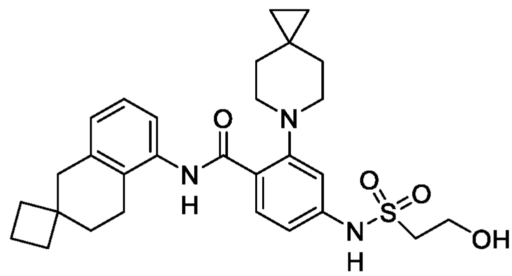

61

,

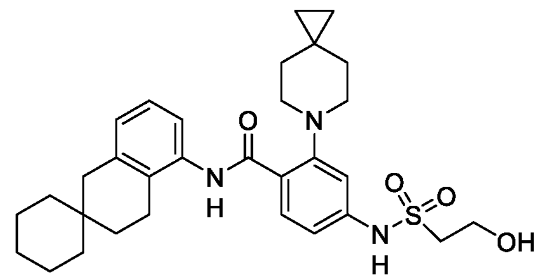

62

,

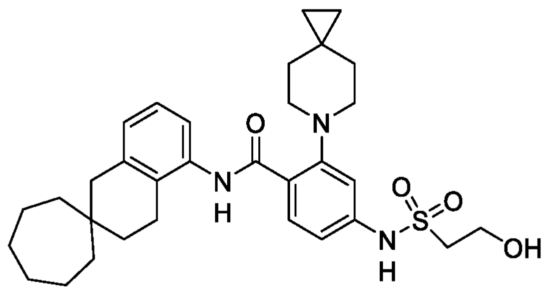

63

,

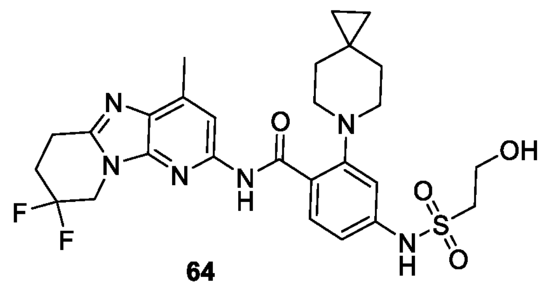

64

,

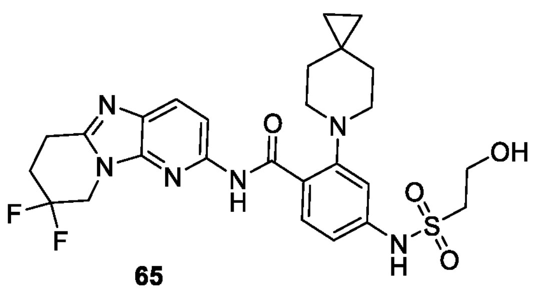

65

,

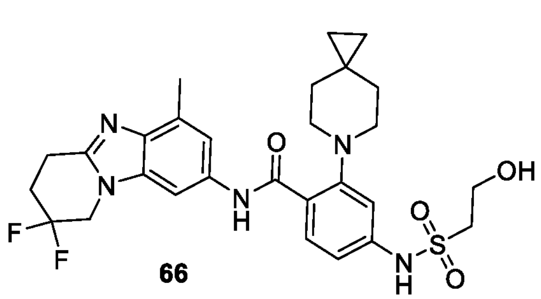

66

,

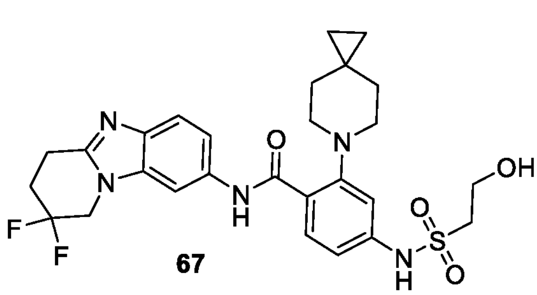

67

,

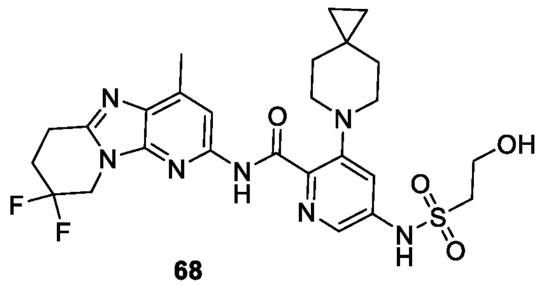

68

,

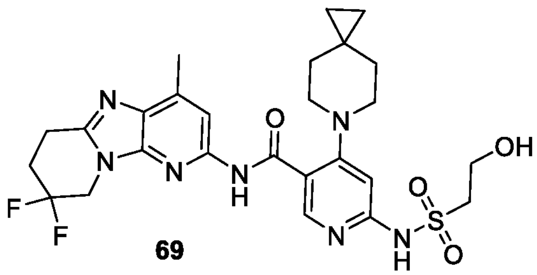

69

,

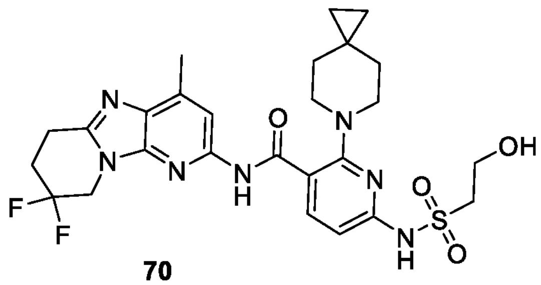

70

,

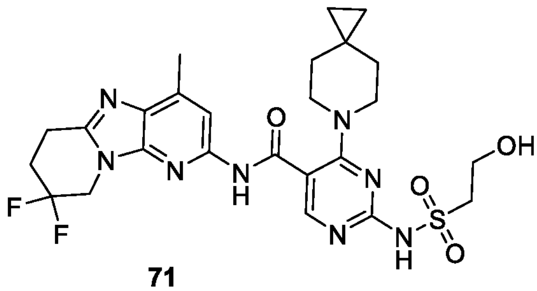

71

,

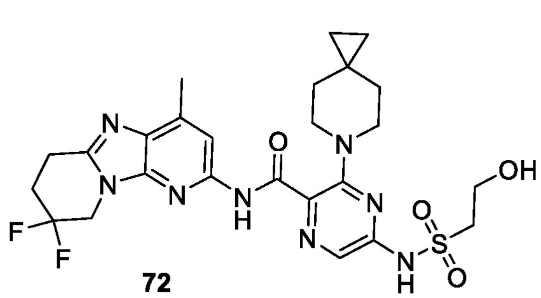

72

,

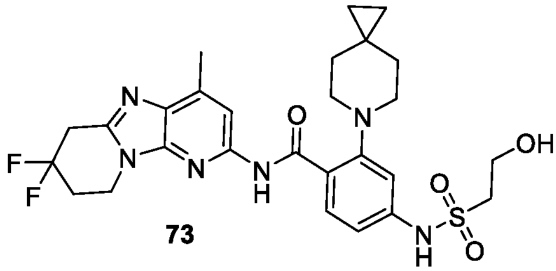
73
,

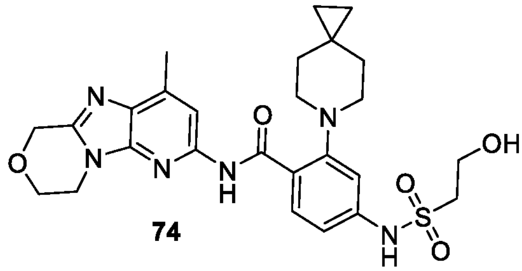
74
,

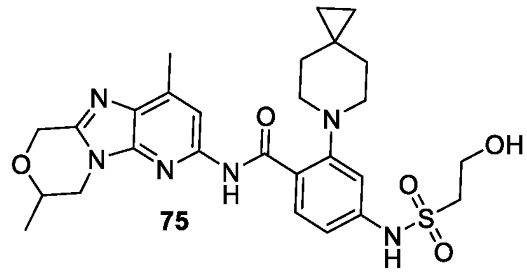
75
,

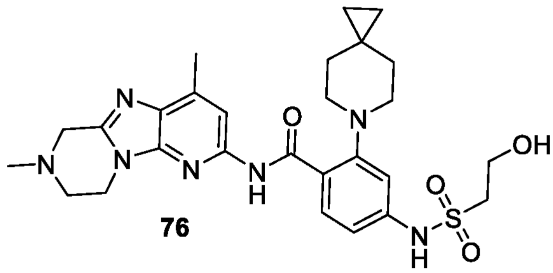
76
,

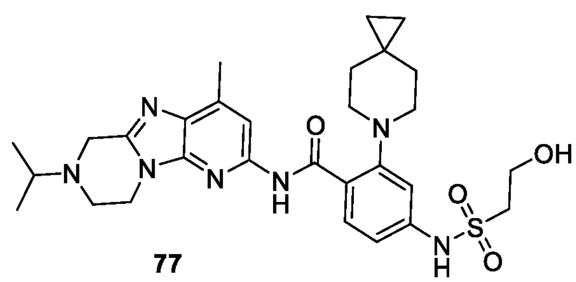
77
,

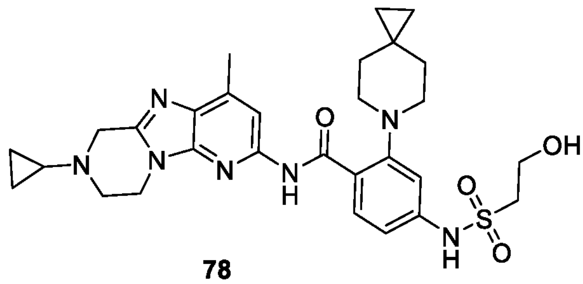
78
,

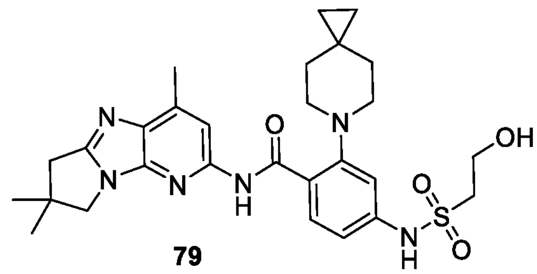
79
,

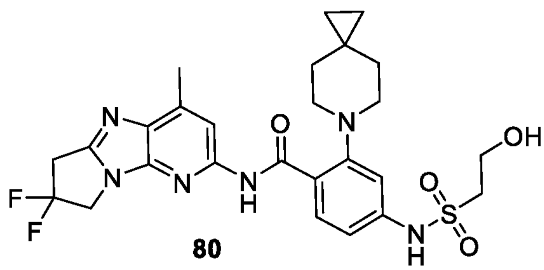
80
,

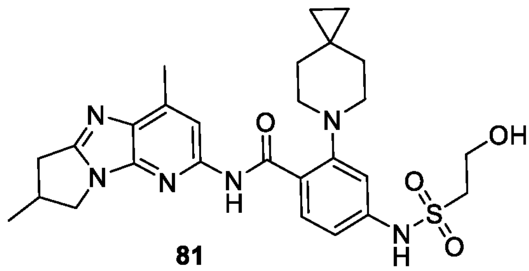
81
,

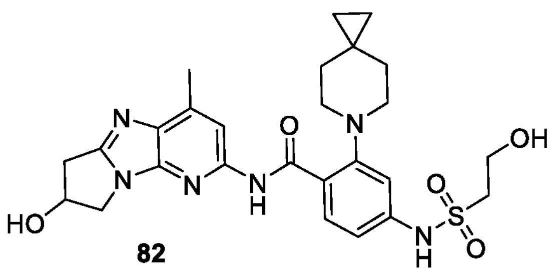
82
,

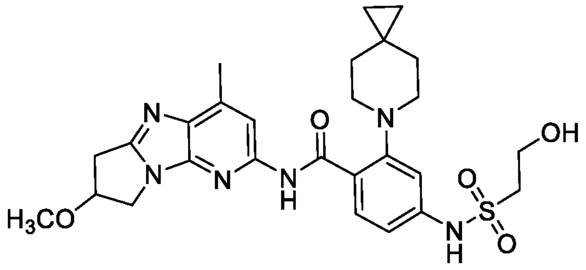

**83** ,

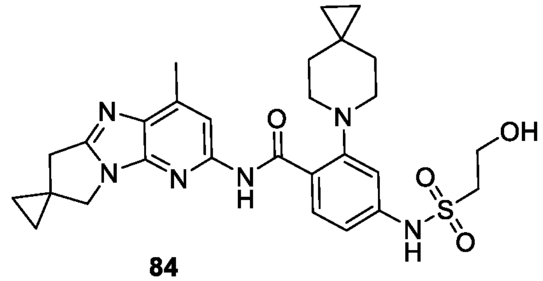

**84** ,

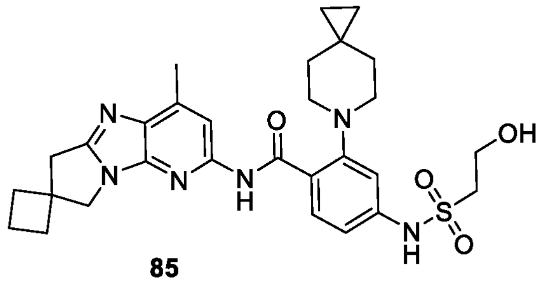

**85** ,

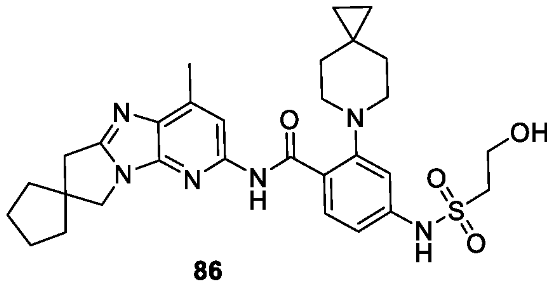

**86** ,

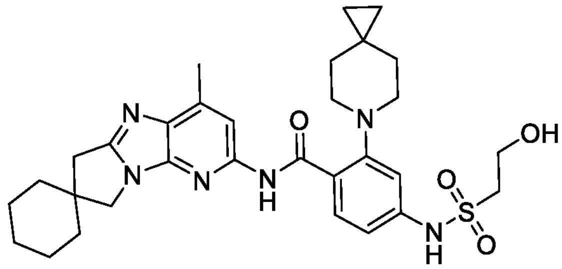

**87** ,

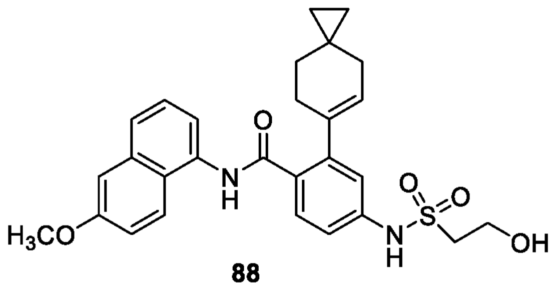

**88** ,

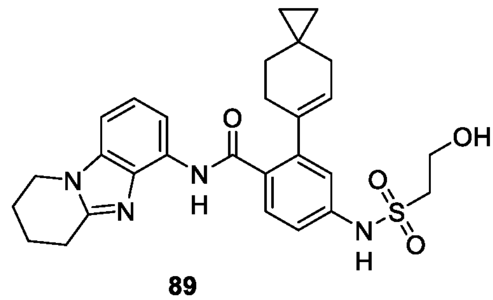

**89** ,

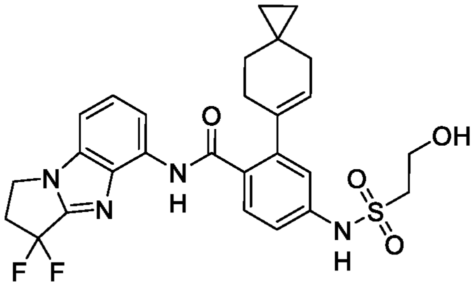

**90** ,

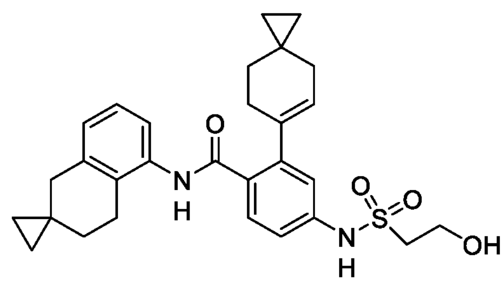

**91** ,

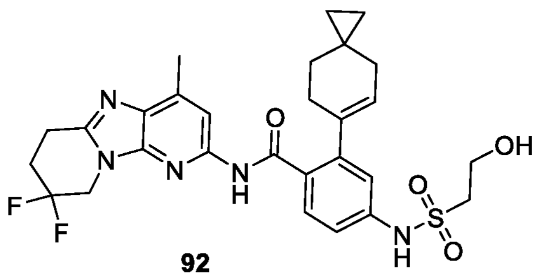

**92** ,

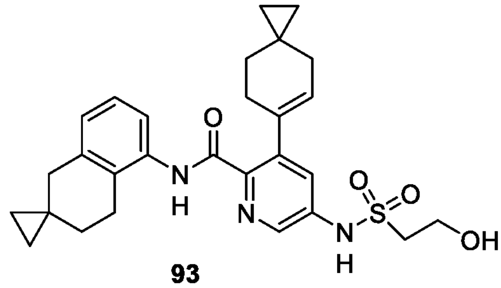

93

,

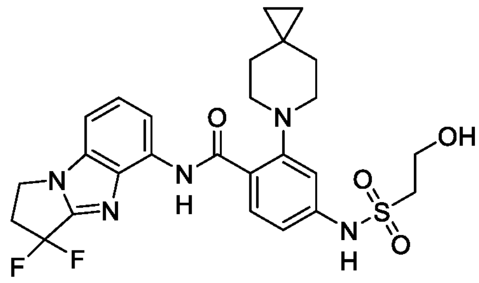

94

,

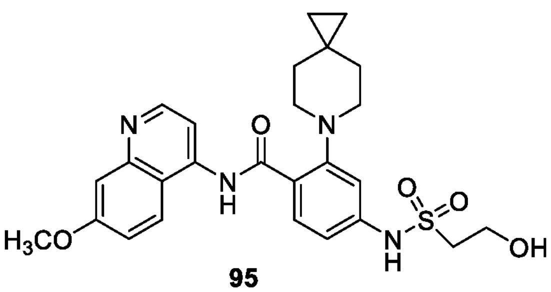

95

,

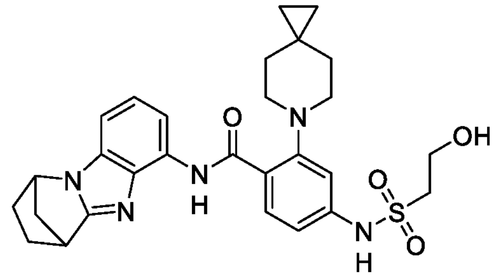

96

,

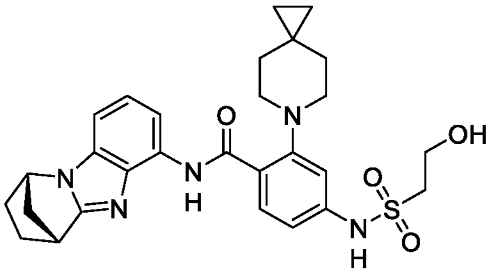

96a

,

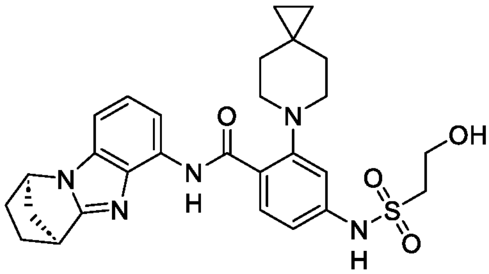

96b

,

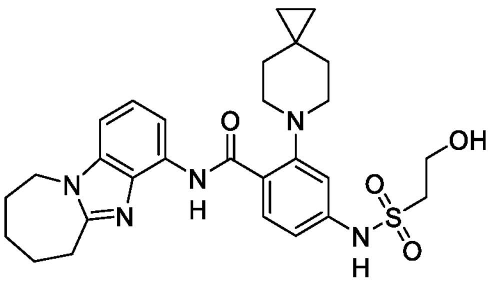

97

,

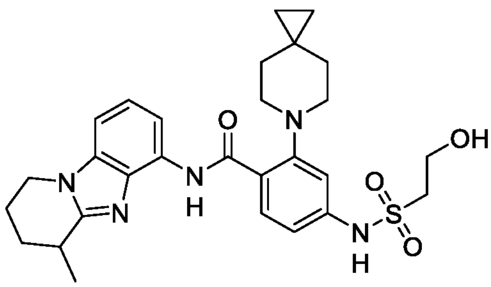

98

,

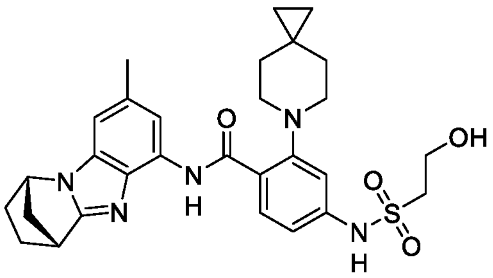

**99** ,

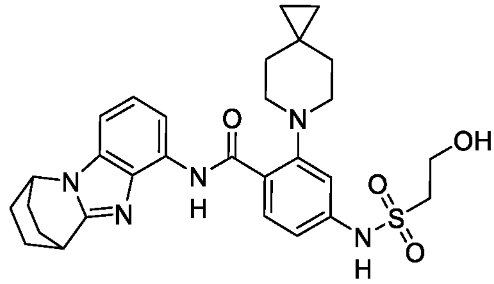

**100** ,

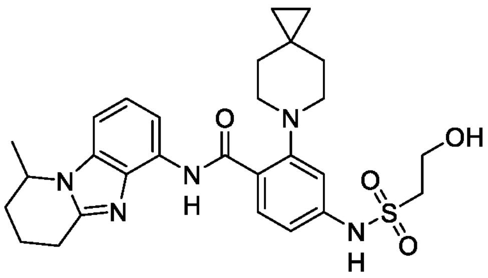

**101** ,

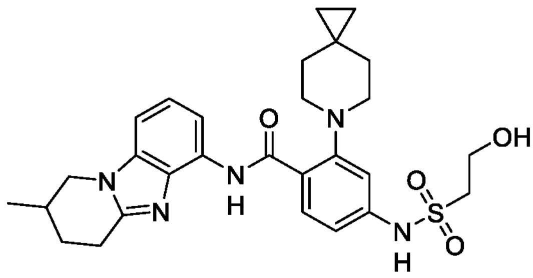

**102** ,

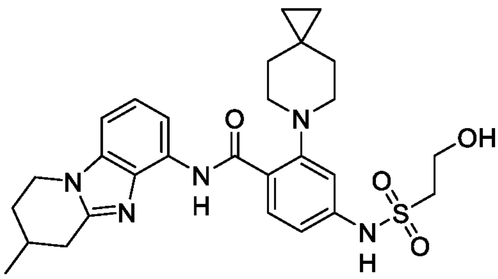

**103** ,

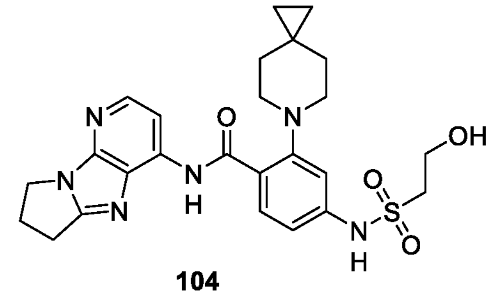

**104** ,

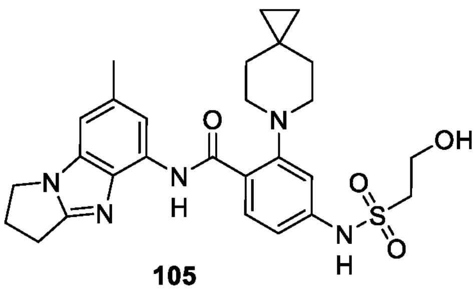

**105** ,

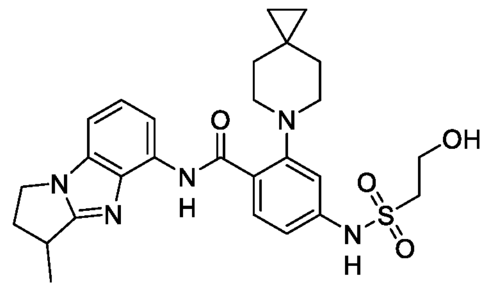

**106** ,

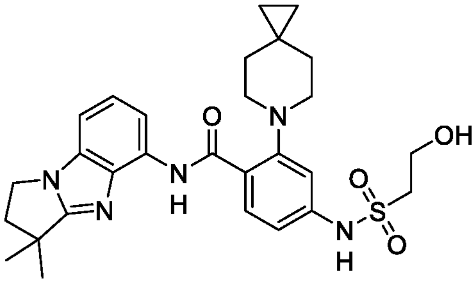

**107** ,

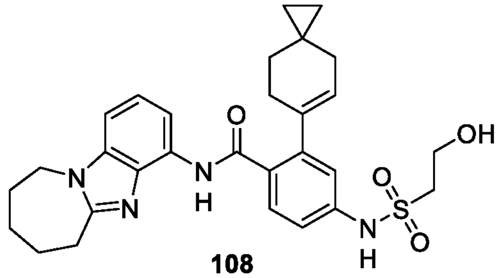

**108** ,

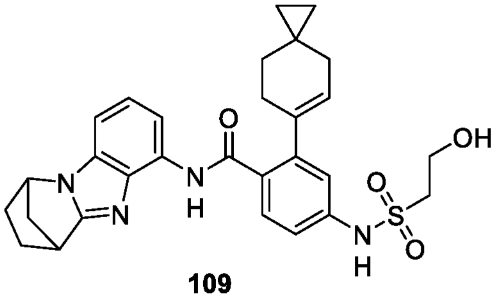

**109** ,

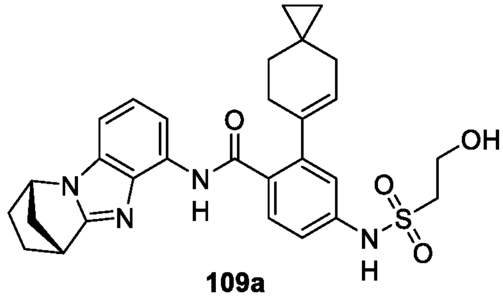

**109a** ,

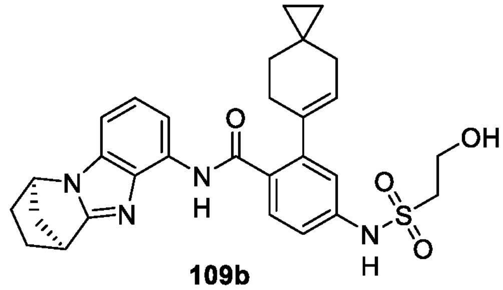

**109b** ,

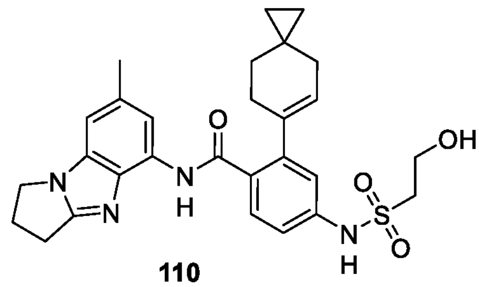

**110** ,

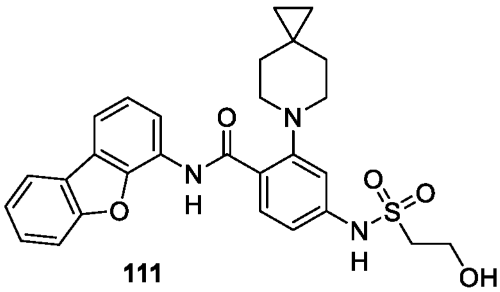

**111** ,

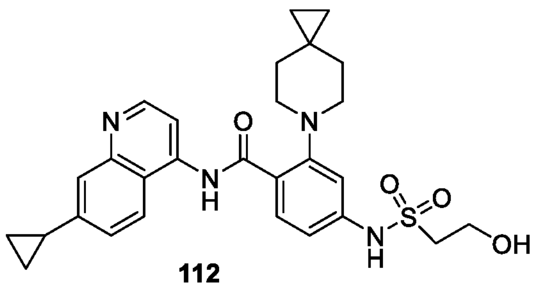

**112** ,

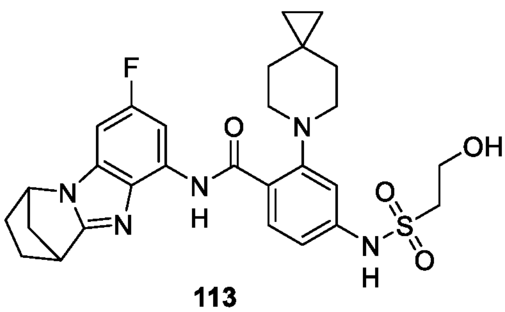

**113** ,

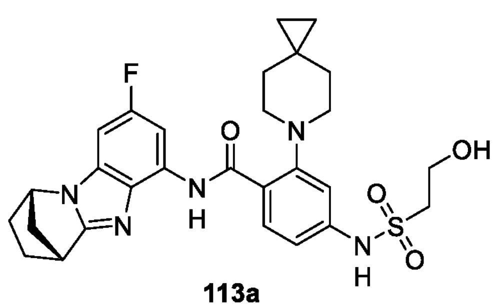

**113a** ,

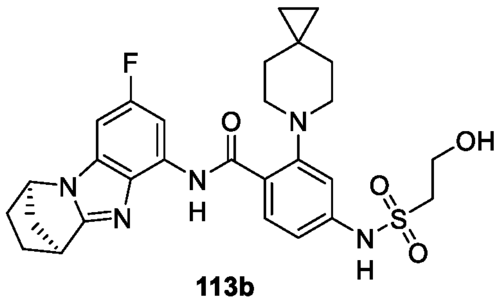

**113b**

The present invention further provides a preparation method of the compound represented by formula (I), which comprises the following steps:

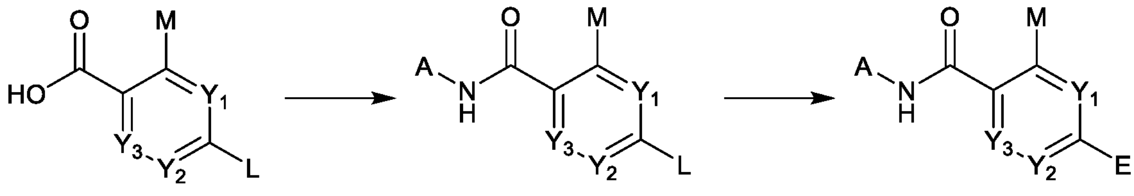

a b (I)

(1) reacting compound a with compound A-$NH_2$ to obtain compound b;

(2) reacting compound b with compound E-H to obtain the compound represented by formula (I);

wherein, A, E, M, $Y_1$, $Y_2$, $Y_3$ are independently defined as described above; L is selected from halogens, such as Cl, Br, I.

The present invention further provides a pharmaceutical composition comprising a therapeutically effective amount of at least one of a compound represented by formula (I), a racemate, stereoisomer, tautomer, isotope-labeled counterpart, solvate, polymorph, pharmaceutically acceptable salt thereof or a prodrug compound thereof.

According to embodiments of the present invention, the pharmaceutical composition further comprises one or more pharmaceutically acceptable excipients.

According to embodiments of the present invention, the pharmaceutical composition may further contain one or more additional therapeutic agents.

The present invention further provides a method for treating tumor diseases, which comprises administering to a patient a therapeutically effective amount of at least one of a compound represented by formula (I), a racemate, stereoisomer, tautomer, isotope-labeled counterpart, solvate, polymorph, pharmaceutically acceptable salt thereof or a prodrug compound thereof.

The present invention further provides a method for treating a tumor disease, which comprises administering to a patient a prophylactically or therapeutically effective amount of the aforementioned pharmaceutical composition.

The tumor disease includes intestinal cancer, breast cancer, lung cancer, pancreatic cancer, prostate cancer, bladder cancer, head and neck cancer, cervical cancer, and ovarian cancer.

In some embodiment, the patient includes a mammal, preferably a human.

The present invention further provides at least one of a compound represented by formula (I), a racemate, stereoisomer, tautomer, isotope-labeled counterpart, solvate, polymorph, pharmaceutically acceptable salt thereof or a prodrug compound thereof, or a pharmaceutical composition thereof for use in treating a tumor disease.

The present invention further provides use of at least one of a compound represented by formula (I), a racemate, stereoisomer, tautomer, isotope-labeled counterpart, solvate, polymorph, pharmaceutically acceptable salt thereof or a prodrug compound thereof in the preparation of a drug.

According to embodiments of the present invention, the use may be for use in the preparation of a drug for the treatment of a KIF18A mediated condition and/or disease, such as in the preparation of a KIF18A inhibitor drug.

According to embodiments of the invention, the disease is for example cancer, including intestinal cancer, breast cancer, lung cancer, pancreatic cancer, prostate cancer, bladder cancer, head and neck cancer, cervical cancer or ovarian cancer.

## Beneficial Effects

The present invention creatively obtains a compound with a novel structure through structure optimization, which

not only has good KIF18A inhibition effect and OVCAR-3 in vitro cell activity, but also has significantly improved physicochemical properties (solubility, permeability), significantly improved OVCAR-3 in vivo efficacy, and good safety. The compounds can be used to treat KIF18A-mediated conditions and/or diseases, such as tumor diseases, and to prepare drugs for treating such conditions or diseases.

**Definition and Explanation of Terms**

Unless otherwise indicated, the definitions of groups and terms recorded in the specification and claims of the present application, including their definitions as examples, exemplary definitions, preferred definitions, definitions recorded in tables, definitions of specific compounds in examples, etc., may be arbitrarily combined and associated with each other. Such combined and associated group definitions and compound structures should be understood to be within the scope of the specification and/or claims of the present application.

Unless otherwise stated, the numerical ranges recited in the specification and claims are equivalent to reciting at least each specific integer value therein. For example, the numerical range "1-14" is equivalent to recording each integer value in the numerical range "1-14", namely 1, 2, 3, 4, 5, 6, 7, 8, 9, 10, 11, 12, 13, 14.

The term "optional" (or "optionally") in the general formula definitions of the present application means the situation of being substituted by zero, one or more substituents, for example "optionally substituted by one, two or more Rs" means that it may not be substituted by R (unsubstituted) or may be selectively substituted by one, two or more Rs.

"More" means three or more.

The term "$C_{1-12}$ alkyl" is understood to mean linear and branched alkyl groups having 1 to 12 carbon atoms, "$C_{1-8}$ alkyl" means linear and branched alkyl groups having 1, 2, 3, 4, 5, 6, 7, or 8 carbon atoms, and "$C_{1-6}$ alkyl" means linear and branched alkyl groups having 1, 2, 3, 4, 5, or 6 carbon atoms. The alkyl is, for example, methyl, ethyl, propyl, butyl, amyl, hexyl, isopropyl, isobutyl, sec-butyl, tert-butyl, isoamyl, 2-methylbutyl, 1-methylbutyl, 1-ethylpropyl, 1,2-dimethylpropyl, neoamyl, 1,1-dimethylpropyl, 4-methylamyl, 3-methylamyl, 2-methylamyl, 1-methylamyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or isomers thereof.

The term "$C_{3-12}$ cycloalkyl" should be understood to mean a saturated monovalent monocyclic, bicyclic (such as fused, bridged, spiro) hydrocarbon ring or tricyclic alkane having 3 to 12 carbon atoms, preferably "$C_{3-10}$ cycloalkyl", more preferably "$C_{3-8}$ cycloalkyl". The term "$C_{3-12}$ cycloalkyl" should be understood to mean a saturated monovalent monocyclic, bicyclic (such as bridged, spiro) hydrocarbon ring or tricyclic alkane having 3, 4, 5, 6, 7, 8, 9, 10, 11 or 12 carbon atoms. The $C_{3-12}$ cycloalkyl may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group, such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonanyl, 2,6-diazaspiro[3,4]octanyl, or a tricyclic hydrocarbon group, such as adamantyl.

The term "$C_{3-12}$ cycloalkenyl" should be understood to mean a monovalent monocyclic, bicyclic (such as fused, bridged, spiro) or tricyclic alkene containing a carbon-carbon double bond, and having 3 to 12 carbon atoms, preferably "$C_{3-10}$ cycloalkenyl", more preferably "$C_{3-8}$ cycloalkenyl", which may have 3, 4, 5, 6, 7, 8, 9, 10, 11 or 12 carbon atoms. The $C_{3-12}$ cycloalkenyl may be a monocyclic hydrocarbon group, such as cyclopropenyl, cyclobutenyl, cyclopentenyl, cyclohexenyl, cycloheptenyl, cyclooctenyl, cyclononenyl or cyclodecenyl, or a bicyclic hydrocarbon group such as spiro[2.5]oct-5-enyl, spiro[3.5]non-6-enyl, spiro[4.5]dec-7-enyl.

The term "$C_{6-14}$ aryl" should be understood to preferably mean a monovalent aromatic or partially aromatic monocyclic, bicyclic or tricyclic hydrocarbon ring having 6 to 14 carbon atoms, which may be a single aromatic ring or a fused polyaromatic ring, preferably "$C_{6-10}$ aryl". The term "$C_{6-14}$ aryl" should be understood as preferably representing a monovalent aromatic or partially aromatic monocyclic, bicyclic or tricyclic hydrocarbon ring ("$C_{6-14}$ aryl") having 6, 7, 8, 9, 10, 11, 12, 13, or 14 carbon atoms, in particular a ring having 6 carbon atoms ("$C_6$ aryl"), such as a phenyl; or biphenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthracenyl. When the $C_{6-20}$ aryl is substituted, it can be monosubstituted or polysubstituted. Furthermore, there is no limitation on the substitution site, and for example, substitution may be at the ortho, para or meta position.

The term "5- to 14-membered heteroaryl" should be understood to include monovalent monocyclic, bicyclic (such as fused, bridged, spiro) or tricyclic aromatic ring systems which have 5 to 14 ring atoms and comprise 1-5 heteroatoms independently selected from N, O and S, for example "5- to 10-membered heteroaryl". The term "5- to 14-membered heteroaryl" should be understood to include monovalent monocyclic, bicyclic or tricyclic aromatic ring systems, which have 5, 6, 7, 8, 9, 10, 11, 12, 13 or 14 ring atoms, in particular 5 or 6 or 9 or 10 carbon atoms, and comprise 1 to 5, preferably 1 to 3 heteroatoms that are each independently selected from N, O and S, and which, in addition, in each case may be benzo-fused. "Heteroaryl" also means a group in which a heteroaromatic ring is fused with one or more aryl, alicyclic, or

heterocyclic rings, wherein the radical or site of attachment is on the heteroaromatic ring. Non-limiting examples include 1-, 2-, 3-, 5-, 6-, 7-, or 8-indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8-purinyl, 1-, 2-, 3-, 4-, 6-, 7-, 8-, or 9-quinolizinyl, 2-, 3-, 4-, 5-, 6-, 7- or 8-quinolinyl, 1-, 3-, 4-, 5-, 6-, 7- or 8-isoquinolinyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7-or 8-cinnolinyl, 2-, 4-, 6- or 7-pteridinyl, 1-, 2-, 3-, 4-, 5-, 6-, 7- or 8-4aH carbazolyl, 1-, 2-, 3-, 4-, 5-, 6-, 7- or 8-carbazolylcarbazolyl, 1-, 3-, 4-, 5-, 6-, 7-, 8- or 9-carbolinyl, 1-, 2-, 3-, 4-, 5-, 6-, 7-, 8- or 9-phenanthridinyl, 1-, 2-, 3-, 4-, 5-, 6-, 7-, 8- or 9-acridinyl, 1-, 2-, 4-, 5-, 6-, 7-, 8- or 9-pyridyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl, 2-, 3-, 4-, 5-, 6- or 1-, 3-, 4-, 5-, 6-, 7-, 8-, 9- or 10-benzoisoquinolyl, 2-, 3-, 4- or thieno[2,3-b]furanyl, 2-, 3-, 5-, 6-, 7-, 8-, 9-, 10- or 11-7H-pyrazino [2,3-c]carbazolyl, 2-, 3-, 5-, 6- or 7-2H-furo[3,2-b]pyranyl, 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d]thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9 -furo[3,4-c]cinnolinyl, 1-, 2-, 3-, 4-, 5-, 6-, 8-, 9-, 10 or 11-4H-pyrido[2,3-c] carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4-, 4-, 5-, 6-, or 7-benzothiazolyl, 1-, 2-, 4-, 5-, 6-, 7-, 8-, or 9-benzoxapinyl, 2-, 4-, 5-, 6-, 7-, or 8-benzoxazinyl, 1-, 2-, 3-, 5-, 6-, 7-, 8-, 9-, 10-, or 11-4H-pyrrolo[1,2-b][2]benzazepinyl. Typical fused heteroaryl groups include, but are not limited to, 2-, 3-, 4-, 5-, 6-, 7- or 8-quinolinyl, 1-, 3-, 4-, 5-, 6-, 7- or 8-isoquinolinyl, 2-, 3-, 4-, 5-, 6- or 7-indolyl, 2- 3-, 4-, 5-, 6- or 7-benzo[b]thienyl, 2-, 4-, 5-, 6- or 7-benzoxazolyl, 2-, 4-, 5-, 6- or 7-benzimidazolyl and 2-, 4-, 5-, 6- or 7-benzothiazolyl. When the 5-to 14-membered heteroaryl is linked to other groups to constitute the compound of the present invention, either the carbon atom on the 5- to 14-membered heteroaryl ring can be linked to other groups, or the heteroatom on the 5- to 14-membered heteroaryl ring can be linked to other groups. When the 5- to 14-membered heteroaryl is substituted, it can be monosubstituted or polysubstituted. Moreover, there is no restriction on the substitution sites, for example, hydrogen linked to a carbon atom on a heteroaryl ring may be substituted, or hydrogen linked to a heteroatom on a heteroaryl ring may be substituted.

Unless otherwise defined, the term "3- to 14-membered heterocyclic group" refers to a saturated or unsaturated non-aromatic ring or ring system, for example, a 4-, 5-, 6- or 7-membered monocyclic ring, a 7-, 8-, 9-, 10-, 11- or 12-membered bicyclic ring (e.g., fused, bridged, spiro) or a 10-, 11-, 12-, 13- or 14-membered tricyclic ring system, and contains at least one, for example 1, 2, 3, 4, 5 or more heteroatoms selected from O, S and N, wherein N and S may also be optionally oxidized to various oxidation states to form nitrogen oxides, -S(O)- or $-S(O)_2-$. Preferably, the heterocyclic group may be selected from "3- to 10-membered heterocyclic groups". The term "3-to 10-membered heterocyclic group" means a saturated or unsaturated non-aromatic ring or ring system and contains at least one heteroatom selected from O, S and N. The heterocyclic group may be attached to the rest of the molecule via any of the carbon atoms or the nitrogen atom, if present. The heterocyclic group may include a fused or bridged ring and a spirocyclic ring. In particular, the heterocyclic group may include, but is not limited to: a 4-membered ring, such as azetidinyl, oxetanyl; a 5-membered ring, such as tetrahydrofuranyl, dioxolyl, pyrrolidinyl, imidazolidinyl, pyrazolidinyl, pyrrolinyl; or a 6-membered ring, such as tetrahydropyranyl, piperidinyl, morpholinyl, dithianyl, thiomorpholinyl, piperazinyl or trithianyl; or a 7-membered ring, such as diazepanyl. Optionally, the heterocyclic group may be benzo-fused. The heterocyclic group may be bicyclic, such as but not limited to a 5,5-membered ring, such as a hexahydrocyclopenta[c]pyrrol-2(1H)-yl ring, or a 5,6-membered bicyclic ring, such as a hexahydropyrrolo[1,2-a]pyrazin-2(1H)-yl ring. The heterocyclic group may be partially unsaturated, i.e., it may contain one or more double bonds, such as but not limited to dihydrofuranyl, dihydropyranyl, 2,5-dihydro-1H-pyrrolyl, 4H-[1,3,4]thiadiazinyl, 1,2,3,5-tetrahydrooxazolyl or 4H-[1,4]thiazinyl, or it may be benzo-fused, such as but not limited to dihydroisoquinolinyl. When the 3- to 14-membered heterocyclic group is linked to other groups to constitute the compound of the present invention, either the carbon atom on the 3- to 14-membered heterocyclic group can be linked to other groups, or the heterocyclic atom on the 3- to 14-membered heterocyclic group can be linked to other groups. For example, when the 3- to 14-membered heterocyclic group is selected from piperazinyl, the nitrogen atom on the piperazinyl may be linked to other groups. Alternatively, when the 3- to 14-membered heterocyclic group is selected from piperidinyl, the nitrogen atom on the piperidinyl ring and the carbon atom at the para position thereof may be linked to other groups.

The term "spiro ring" refers to a ring system in which two rings share one ring atom.

The term "fused ring" refers to a ring system in which two rings share 2 ring atoms.

The term "bridged ring" refers to a ring system in which two rings share 3 or more ring atoms.

The term "halogen" refers to fluorine, chlorine, bromine and iodine.

"Halo" means substituted by one or more halogens.

Unless otherwise specified, $R_a$ described herein is a substituent on the fused ring group A, which means that $R_a$ can be optionally substituted at any position of the fused ring group A. For example, the structural formula

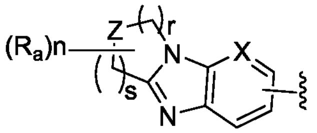

means that $R_a$ can be substituted on the N-containing heterocyclic ring on the left side of the ring

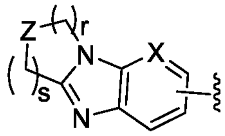
,

and also means that $R_a$ can be substituted on the X-containing ring on the right side of the ring

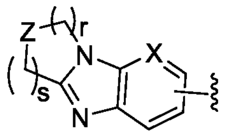
.

The total number of substitutions of $R_a$ on the fused ring group A is n.

Unless otherwise stated, the definition of a term herein also applies to a group containing the term, for example, the definition of $C_{1-12}$ alkyl also applies to $C_{1-12}$ alkyloxy (i.e., $C_{1-12}$ alkoxy).

The term "*ene" refers to a divalent group, and the group is as defined above. For example, the term "alkylene" refers to divalent alkyl, wherein the alkyl is as defined above. The alkylene is preferably alkylene having 1 to 12 carbon atoms (i.e., $C_{1-12}$ alkylene), and more preferably alkylene having 1 to 6 carbon atoms (i.e., $C_{1-6}$ alkylene).

The term "$C_{3-14}$ carbon ring" refers to a saturated or unsaturated aliphatic hydrocarbon ring having 3 to 14 carbon atoms, including a cycloalkane ring having 3 to 14 carbon atoms ($C_{3-14}$ cycloalkane ring), a cycloalkene ring having 3 to 14 carbon atoms ($C_{3-12}$ cycloalkene ring) or a cycloalkyne ring having 3 to 14 carbon atoms ($C_{3-12}$ cycloalkyne ring).

Those skilled in the art will appreciate that the compound represented by formula (I) may exist in the form of various pharmaceutically acceptable salts. If the compounds have a basic center, they can form acid addition salts; if they have an acidic center, they can form base addition salts; if the compounds contain both an acidic center (e.g., carboxyl) and a basic center (e.g., amino), they can also form inner salts.

The compounds of the present invention may be present in the form of solvates, such as hydrates, wherein the compounds of the present invention comprise polar solvents, such as water, methanol or ethanol, as structural elements of the lattice of the compounds. The polar solvent, especially water, may be present in a stoichiometric or non-stoichiometric amount.

Depending on their molecular structure, the compounds of the present invention may be chiral and may therefore exist in various enantiomeric forms. These compounds may therefore exist in racemic or optically active forms. The compounds of the present invention encompass isomers in which the chiral carbons are each in R or S configuration, or mixtures and racemates thereof. The compounds of the present invention or their intermediates can be separated into enantiomeric compounds by chemical or physical methods known to those skilled in the art, or used in synthesis in this form. In the case of racemic amines, diastereomers are prepared from the mixture by reaction with an optically active resolving reagent. Examples of suitable resolving reagents are optically active acids, such as the R and S forms of tartaric acid, diacetyltartaric acid, dibenzoyltartaric acid, mandelic acid, malic acid, lactic acid, appropriate N-protected amino acids (e.g., N-benzoylproline or N-phenylsulfonylproline) or various optically active camphorsulfonic acids. Chromatographic enantiomeric resolution can also be advantageously carried out by means of optically active resolving reagents, such as dinitrobenzoylphenylglycine, cellulose triacetate or other carbohydrate derivatives or chirally derivatized methacrylate polymers immobilized on silica gel. Suitable eluents for this purpose are aqueous or alcoholic solvent mixtures, for example, hexane/isopropanol/acetonitrile.

The corresponding stable isomers can be separated according to known methods, for example by extraction, filtration or column chromatography.

The term "patient" refers to any animal, including mammals, preferably mice, rats, other rodents, rabbits, dogs, cats, pigs, cows, sheep, horses or primates, and most preferably humans.

The term "therapeutically effective amount" refers to that amount of an active compound or drug which causes a biological or medical response sought by a researcher, veterinarian, physician or other clinician in a tissue, system, animal, individual or human, and which comprises one or more of the following: (1) prevention of disease: for example, prevention

of a disease, disorder or condition in an individual who is susceptible to the disease, disorder or condition but has not yet experienced or developed the pathology or symptoms of the disease. (2) Inhibition of disease: for example, inhibition of a disease, disorder or condition (i.e., prevention of further progression of pathology and/or symptoms) in an individual undergoing or exhibiting the pathology or symptoms of the disease, disorder or condition. (3) Alleviation of disease: for example, alleviation of a disease, disorder or condition (i.e., reversion of pathology and/or symptoms) in an individual undergoing or exhibiting the pathology or symptoms of the disease, disorder or condition.

**DETAILED DESCRIPTION OF THE INVENTION**

The technical solution of the present invention will be further described in detail below in conjunction with specific examples. It should be understood that the following examples are only intended to exemplify and explain the present invention and should not be construed as limiting the scope of protection of the present invention. All technologies realized based on the above contents of the present invention are included in the scope that the present invention intends to protect.

Unless otherwise indicated, the raw materials and reagents used in the following examples are commercially available or may be prepared by known methods.

Example 1

2-{6-azaspiro[2.5]octan-6-yl}-4-(2-hydroxyethanesulfonylamino)-N-(6-methoxynaphthalen-1-yl)benzamide (compound 1)

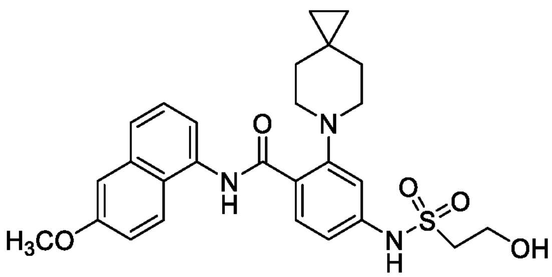

Compound 1

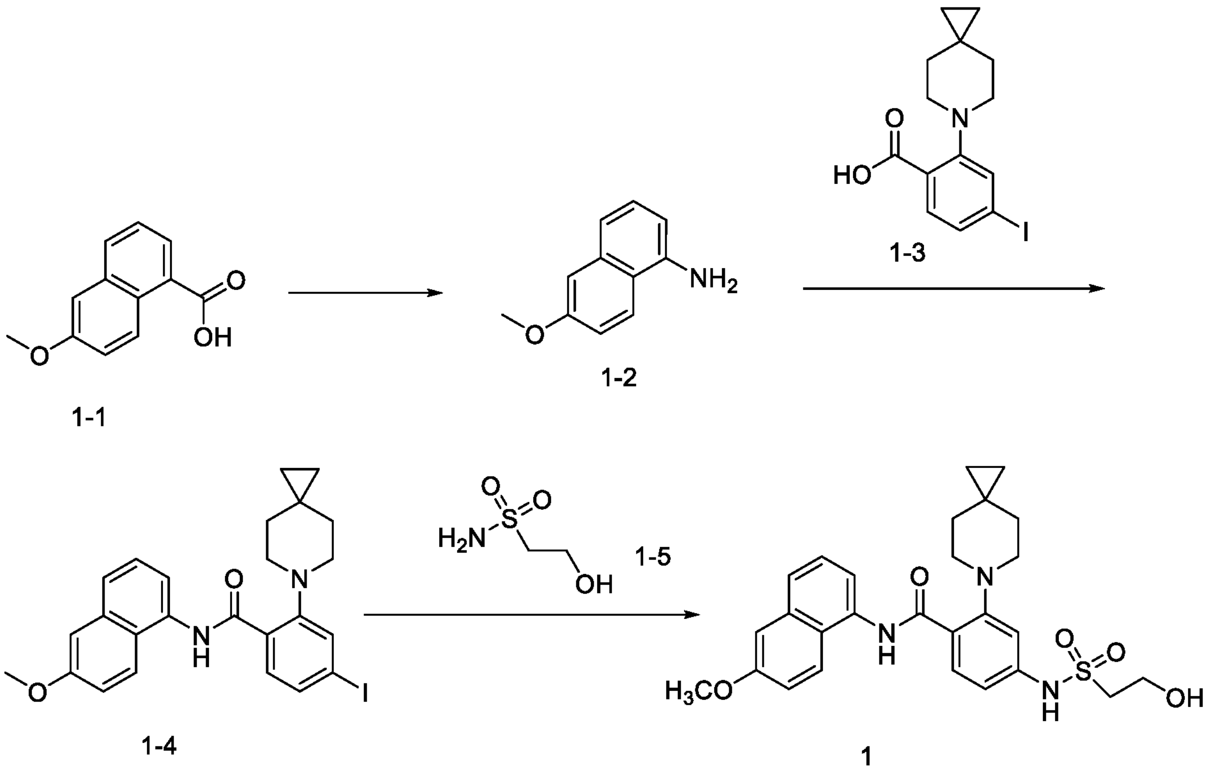

1-1 1-2 1-3 1-4 1-5 1

Step 1 Synthesis of 6-methoxynaphthalen-1-amine (compound 1-2):

Under nitrogen protection, hydroxylamine hydrochloride (206.2 mg, 2.967 mmol, 1.5 eq) was added to a solution of 6-methoxynaphthalen-1-carboxylic acid (400 mg, 1.978 mmol, 1 eq) in polyphosphoric acid (2 mL) at room temperature and stirred at 80°C for 2 h. The reaction mixture was quenched with ice water (30 mL) at 0°C. The aqueous phase was extracted with ethyl acetate (1 × 50 mL). The aqueous phase was then alkalinized to about pH 9 with solid sodium hydroxide and extracted with ethyl acetate (3 × 100 mL). The organic phases were combined, washed with saturated brine (1 × 50 mL), and dried over anhydrous sodium sulfate. The resulting mixture was filtered and the filtrate was concentrated under reduced pressure to obtain 6-methoxynaphthalen-1-amine (compound 1-2, 300 mg, 87.55%).
MS (ESI, *m/z*):174.35 $[M+H]^+$, RT(min):0.760

Step 2 Synthesis of 2-{6-azaspiro[2.5]octan-6-yl}-4-iodo-N-(6-methoxynaphthalen-1-yl)benzamide (compound 1-4):

Under nitrogen protection, 2-{6-azaspiro[2.5]octan-6-yl}-4-iodobenzoic acid (463.98 mg, 1.299 mmol, 1.5 eq), *N,N,N,N'*-tetramethylchloroformamidine hexafluorophosphate (971.9 mg, 3.464 mmol, 4 eq) and 1-methylimidazole (711.02 mg, 8.660 mmol, 10 eq) were added to a solution of 6-methoxynaphthalen-1-amine (150 mg, 0.866 mmol, 1 eq) in dichloromethane (3 mL) at room temperature, and the mixture was stirred for 2 h. The target product was found as detected by LC-MS. The reaction mixture was quenched with water (50 mL) at room temperature and extracted with ethyl acetate (3 × 50 mL). The organic phases were combined, washed with saturated brine (1 × 50 mL), and dried over anhydrous sodium sulfate. The resulting mixture was filtered, and then the filtrate was concentrated under reduced pressure. The resulting residue was purified by silica gel column chromatography with petroleum ether/ethyl acetate (5:1) to obtain 2-{6-azaspiro[2.5]octan-6-yl}-4-iodo-N-(6-methoxynaphthalen-1-yl)benzamide (compound 1-4, 300 mg, 67.61%).
MS (ESI, *m/z*):513.35 $[M+H]^+$, RT(min):1.499

Step 3 Synthesis of 2-{6-azaspiro[2.5]octan-6-yl}-4-(2-hydroxyethanesulfonylamino)-*N*-(6-methoxynaphthalen-1-yl)benzamide (compound 1)

To a solution of 2-{6-azaspiro[2.5]octan-6-yl}-4-iodo-*N*-(6-methoxynaphthalen-1-yl)benzamide (80 mg, 0.156 mmol, 1 eq) in *N,N*-dimethylformamide (0.5 mL) were added 2-hydroxyethanesulfonamide (23.45 mg, 0.187 mmol, 1.2 eq), cuprous iodide (7.43 mg, 0.039 mmol, 0.25 eq), potassium carbonate (64.73 mg, 0.468 mmol, 3 eq) and sarcosine (5.56 mg, 0.062 mmol, 0.4 eq) under nitrogen protection at 120°C, and the mixture was stirred overnight. The mixture was diluted with 10 mL of water and extracted with ethyl acetate (3 × 10 mL). The organic phases were combined, washed with

saturated brine (3 × 10 mL), and dried over anhydrous sodium sulfate. The resulting mixture was filtered and the filtrate was concentrated under reduced pressure to obtain a crude product. The crude product was purified by HPLC under the following conditions (chromatographic column specifications: XBridge Prep OBD C18 Column, 30X150 mm, 5μm; mobile phase A: water (10 mmol/L ammonium bicarbonate), mobile phase B: acetonitrile (0.1% diethylamine)--HPLC--merk; flow rate: 60 mL/min; elution gradient: 40% B to 58% B, 10 min, 58% B; detection wavelength: UV 220 nm; retention time (min): 8.23) to obtain 2-{6-azaspiro[2.5]octan-6-yl}-4-(2-hydroxyethanesulfonylamino)-*N*-(6-methoxynaphthalen-1-yl)benzamide (compound 1, 30.32 mg, 38.11 %).

MS (ESI, *m/z*):509.90 $[M+H]^+$, RT(min):1.572

$^1H$ NMR: (400 MHz, DMSO-$d_6$) δ 11.84 (s, 1H), 10.12 (s, 1H), 8.07 (d, 1H), 8.03 - 7.98 (m, 1H), 7.94 (d, 1H), 7.66 (d, 1H), 7.49 (t, 1H), 7.39 (d, 1H), 7.26 (d, 1H), 7.22 - 7.16 (m, 1H), 7.13 - 7.03 (m, 1H), 4.96 (s, 1H), 3.90 (s, 3H), 3.78 (t, 2H), 3.36 (t, 2H), 3.15 - 2.96 (m, 4H), 1.42 (s, 4H), 0.26 (s, 4H).

The compounds in the following table were prepared using conditions similar to those in Example 1.

| Compound No. | Structure | Name | $[M+H]^+$ | $^1H$-NMR data |
|---|---|---|---|---|
| 2 | 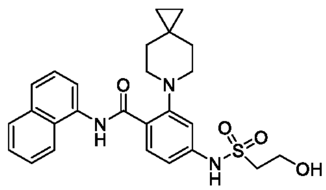 | 2-{6-azaspiro[2.5]octan-6-yl}-4-(2-hydroxyethanesulfonylamino) -*N*-(naphthalen-1-yl)benzamide | 480.20 | $^1H$ NMR (400 MHz, DMSO-$d_6$) δ 11.92 (s, 1H), 10.14 (s, 1H), 8.18 (t, 2H), 8.02 - 7.93 (m, 2H), 7.77 (d, 1H), 7.62 - 7.51 (m, 3H), 7.27 (d, 1H), 7.13 - 7.06 (m, 1H), 4.97 (s, 1H), 3.77 (d, 2H), 3.37 (d, 2H), 3.07 (s, 4H), 1.43 (s, 4H), 0.26 (s, 4H). |
| 3 | 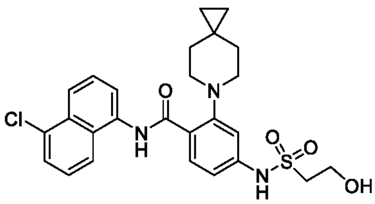 | 2-{6-azaspiro[2.5]octan-6-yl}-*N*-(5-chloronaphthalen-1-yl)-4-(2-hydroxyethanesulfonylamino) benzamide | 513.80 | $^1H$ NMR (400 MHz, DMSO-$d_6$) δ 12.01 (s, 1H), 10.15 (s, 1H), 8.26 (d, 1H), 8.21 (d, 1H), 8.05 (d, 1H), 7.95 (d, 1H), 7.79 (d, 1H), 7.74 (t, 1H), 7.61 - 7.51 (m, 1H), 7.26 (s, 1H), 7.09 (d, 1H), 4.98 (s, 1H), 3.78 (t, 2H), 3.37 (d, 2H), 3.07 (s, 4H), 1.41 (s, 4H), 0.26 (s, 4H). |
| 4 | 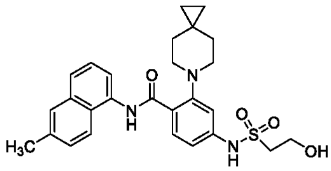 | 2-{6-azaspiro[2.5]octan-6-yl}-4-(2-hydroxyethanesulfonylamino) -*N*-(6-methylnaphthalen-1-yl)benzamide | 494.40 | $^1H$ NMR: (400 MHz, DMSO-$d_6$) δ 11.88 (s, 1H), 10.13 (s, 1H), 8.09 (dd, 2H), 7.95 (d, 1H), 7.75 (s, 1H), 7.66 (d, 1H), 7.50 (t, 1H), 7.41 (dd, 1H), 7.27 (d, 1H), 7.09 (dd, 1H), 4.96 (s, 1H), 3.78 (t, 2H), 3.32 (m, 2H), 3.07 (t, 4H), 2.50 (p, 3H), 1.43 (s, 4H), 0.07 (s, 4H). |

(continued)

| Compound No. | Structure | Name | $[M+H]^+$ | $^1$H-NMR data |
|---|---|---|---|---|
| 5 | 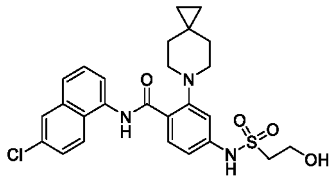 | {6-azaspiro[2.5]octan-6-yl}-*N*-(6-chloronaphthalen-1-yl)-4-(2-hydroxyethanesulfonylamino) benzamide | 513.80 | $^1$H NMR (400 MHz, DMSO-$d_6$) $\delta$ 11.88 (s, 1H), 10.12 (s, 1H), 8.19 (d, 1H), 8.17 - 8.09 (m, 2H), 7.92 (d, 1H), 7.77 (d, 1H), 7.61 (t, 1H), 7.58 - 7.53 (m, 1H), 7.26 (d, 1H), 7.08 (dd, 1H), 4.96 (s, 1H), 3.78 (t, 2H), 3.36 (t, 2H), 3.06 (t, 4H), 1.41 (s, 4H), 0.27 (s, 4H). |
| 6 | 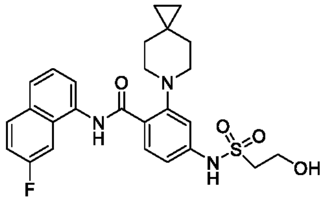 | 2-{6-azaspiro[2.5]octan-6-yl}-*N*-(7-fluoronaphthalen-1-yl)-4-(2-hydroxyethanesulfonylamino) benzamide | 497.85 | $^1$H NMR (400 MHz, DMSO-$d_6$) $\delta$ 11.65 (s, 1H), 10.11 (s, 1H), 8.17 (d, J = 7.6 Hz, 1H), 8.11 - 8.05 (m, 1H), 7.93 - 7.86 (m, 2H), 7.82 (d, 1H), 7.58 - 7.45 (m, 2H), 7.25 (d, 1H), 7.13 - 6.94 (m, 1H), 4.96 (s, 1H), 3.78 (t, 2H), 3.38 - 3.34 (m, 2H), 3.15 - 2.99 (m, 4H), 1.44 (s, 4H), 0.27 (s, 4H). |
| 7 | 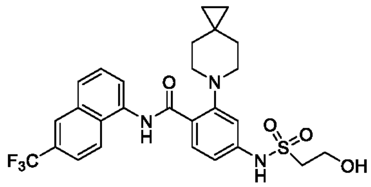 | 2-{6-azaspiro[2.5]octan-6-yl}-4-(2-hydroxyethanesulfonylamino) -*N*-(6-(trifluoromethyl)naphthalen-1-yl)benzamide | 547.85 | $^1$H NMR (400 MHz, DMSO-$d_6$) $\delta$ 11.94 (s, 1H), 10.14 (s, 1H), 8.50 (s, 1H), 8.37 (d, 1H), 8.25 (d, 1H), 8.02 (d, 1H), 7.93 (d, 1H), 7.79 (dd, 1H), 7.72 (t, 1H), 7.26 (s, 1H), 7.08 (dd, *J* = 8.6, 2.0 Hz, 1H), 4.98 (s, 1H), 3.78 (t, 2H), 3.39 - 3.34 (m, 2H), 3.08 (s, 4H), 1.42 (s, 4H), 0.26 (s, 4H). |

(continued)

| Compound No. | Structure | Name | [M+H]+ | 1H-NMR data |
|---|---|---|---|---|
| 8 | 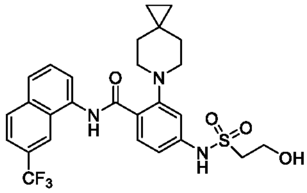 | 2-{6-azaspiro[2.5]octan-6-yl}-4-(2-hydroxyethanesulfonylamino) -*N*-(7-(trifluoromethyl)naphthalen-1-yl)benzamide | 547.85 | $^1$H NMR: (400 MHz, DMSO-d6) $\delta$ 11.80 (s, 1H), 10.06 (s, 1H), 8.50 (s, 1H), 8.23 (dd, 2H), 7.93 (d, 1H), 7.87 (d, 1H), 7.82 (dd, 1H), 7.75 (t, 1H), 7.23 (d, 1H), 7.06 (dd, *J* = 1H), 4.98 (s, 1H), 3.78 (t, 2H), 3.35 (t, 2H), 3.07 (s, 4H), 1.38 (d, 4H), 0.24 (s, 4H). |
| 95 | 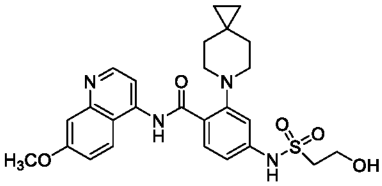 | 2-(6-azaspiro[2.5]octan-6-yl-4-(2-hydroxyethanesulfonylamino) -*N*-(7-methoxyquinolin-4-yl)benzamide | 511.05 | $^1$H NMR (400 MHz, DMSO-$d_6$) $\delta$ 11.96 (s, 1H), 10.16 (s, 1H), 8.75 (d, 1H), 8.33 - 8.22 (m, 2H), 7.91 (d, 1H), 7.42 (d, 1H), 7.28 (d, 1H), 7.25 (t, 1H), 7.11 - 7.04 (m, 1H), 4.97 (s, 1H), 3.94 (s, 3H), 3.78 (t, 2H), 3.37 (d, 2H), 3.03 (d, 4H), 1.40 (s, 4H), 0.24 (s, 4H). |

Example 2

2-{6-azaspiro[2.5]octan-6-yl}-4-(2-hydroxyethanesulfonylamino)-*N*-(6-methoxynaphthalen-1-yl)benzamide (compound 21)

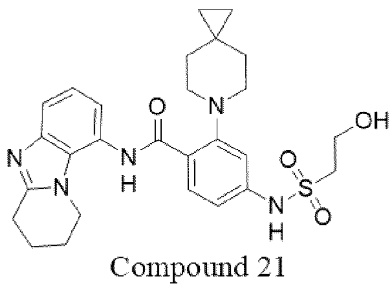

Compound 21

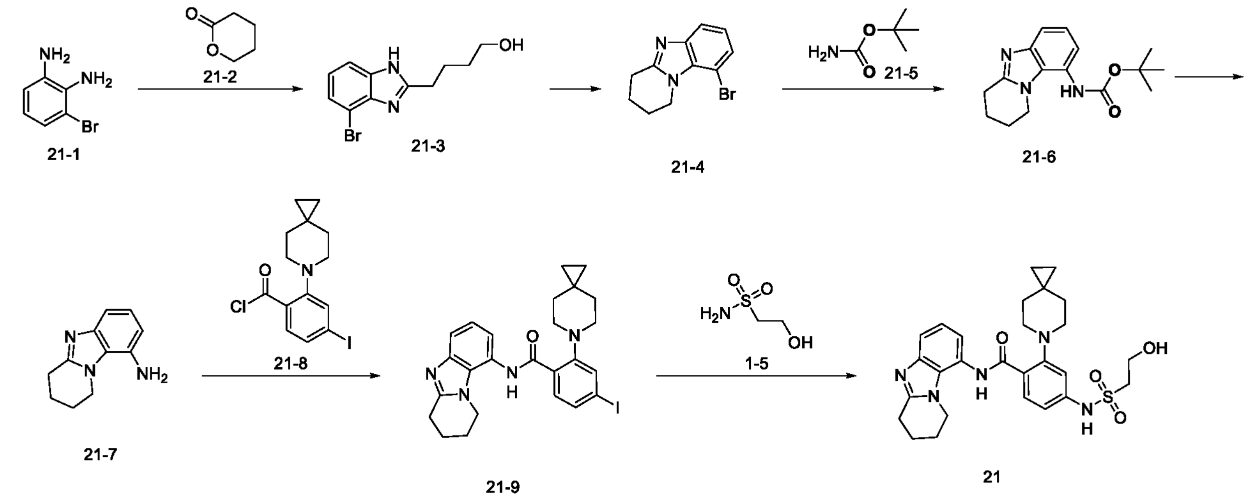


Step 1 Synthesis of 4-(4-bromo-1H-benzimidazol-2-yl)butan-1-ol (compound 21-3):

Under nitrogen protection and at 100°C, a solution of 3-bromobenzene-1,2-diamine (2 g, 10.693 mmol, 1 eq) and δ-valerolactone (4.28 g, 42.772 mmol, 4 eq) in hydrochloric acid (4M, 20 mL) was stirred for 2 h. The reaction mixture was cooled to room temperature and alkalinized to pH=11 with saturated aqueous potassium carbonate solution. The reaction mixture was extracted with ethyl acetate (3 × 50 mL). The organic phases were combined, washed with saturated brine (2 × 50 mL), and dried over anhydrous sodium sulfate. The resulting mixture was filtered, and then the filtrate was concentrated under reduced pressure. The resulting mixture was used directly without further purification.

MS(ESI, m/z): 269.20 $[M+H]^+$, RT(min):0.527

Step 2 Synthesis of 9-bromo-benzo[4,5]imidazo[1,2-a]piperidine (compound 21-4):

To a solution of 4-(4-bromo-1*H*-1,3-benzodiazol-2-yl)butan-1-ol (2 g, 7.431 mmol, 1 eq) and triphenylphosphine (2.92 g, 11.146 mmol, 1.5 eq) in toluene (20 mL) was added di-tert-butyl azodicarboxylate (2.57 g, 11.146 mmol, 1.5 eq) dropwise under nitrogen protection and ice bath. The reaction mixture was heated to 85°C and further stirred overnight. The reaction mixture was quenched with water (60 mL) at room temperature. The reaction mixture was extracted with ethyl acetate (3 × 50 mL). The organic phases were combined, washed with saturated brine (2 × 80 mL), and dried over anhydrous sodium sulfate. The resulting mixture was filtered, and then the filtrate was concentrated under reduced pressure. The resulting residue was purified by silica gel column chromatography with dichloromethane/methanol (10:1) to obtain 9-bromo-benzo[4,5]imidazo[1,2-a]piperidine (compound 21-4, 1 g, 53.59%).

MS(ESI, *m/z*): 250.90 $[M+H]^+$, RT(min):0.746

Step 3 Synthesis of tert-butyl benzo[4,5]imidazo[1,2-a]piperidin-9-ylcarbamate (compound 21-6):

Under nitrogen protection, to a solution of 9-bromo-benzo[4,5]imidazo[1,2-a]piperidine (500 mg, 1.991 mmol, 1 eq) and tert-butyl carbamate (466.49 mg, 3.982 mmol, 2 eq) in 1,4-dioxane (5 mL) were added bicyclohexyl(3-isopropoxy-2',4',6'-triisopropyl-[1,1'-biphenyl]-2-yl)phosphine (159.72 mg, 0.299 mmol, 0.15 eq), (methanesulfonic acid{bicyclohexyl(3-isopropoxy-2',4',6'-triisopropyl-[1,1'-biphenyl]-2-yl)phosphine}(2'-methylamino-1,1'-biphenyl-2-yl) palladium(II) (106.48 mg, 0.199 mmol, 0.1 eq) and cesium carbonate (1946.14 mg, 5.973 mmol, 3 eq) at room temperature. After the addition was completed, the system was further stirred at 90°C for 4 h. The reaction mixture was cooled to room temperature and the mixture was diluted with water (30 mL) at room temperature. The reaction mixture was extracted with ethyl acetate (3 × 30 mL). The organic phases were combined, washed with saturated brine (1 × 40 mL), and dried over anhydrous sodium sulfate. The resulting mixture was filtered, and then the filtrate was concentrated under reduced pressure. The resulting residue was purified by silica gel column chromatography with petroleum ether/ethyl acetate (3:1) to obtain tert-butyl benzo[4,5]imidazo[1,2-a]piperidin-9-ylcarbamate (compound 21-6, 400 mg, 69.91%).

MS(ESI, *m/z*): 288.40 $[M+H]^+$, RT(min):0.728

Step 4 Synthesis of benzo[4,5] imidazo[1,2-a]piperidin-9-amine (compound 21-7):

A solution of hydrochloric acid in 1,4-dioxane (4 mL) was added to a solution of tert-butyl benzo[4,5]imidazo[1,2-a]piperidin-9-ylcarbamate (400 mg, 1.392 mmol, 1 eq) in dichloromethane (4 mL) under nitrogen protection at room temperature. After the addition was completed, the system was further stirred at room temperature for 2 h. The target product was found as detected by LC-MS. The resulting residue was concentrated under reduced pressure. The resulting mixture was used directly without further purification.

MS(ESI, *m/z*): 188.05 $[M+H]^+$, RT(min):0.602

Step 5 Synthesis of 4-iodo-2-(6-azaspiro[2.5]octan-6-yl)-*N*-(benzo[4,5]imidazo[1,2-a]piperidin-9-yl)benzamide (compound 21-9):

Under nitrogen protection, potassium phosphate (340.08 mg, 1.602 mmol, 3 eq) was added to a solution of benzo[4,5]imidazo[1,2-a]piperidin-9-amine (240.73 mg, 0.641 mmol, 1.2 eq) in dichloromethane (2 mL) at room temperature and stirred for 1 min. Then, a solution of 4-iodo-2-(6-azaspiro[2.5]octan-6-yl)benzoyl chloride (100 mg, 0.534 mmol, 1 eq) and *N,N*-diisopropylethylamine (207.08 mg, 1.602 mmol, 3 eq) in dichloromethane (1 mL) was added at room temperature. The reaction mixture was allowed to react at room temperature for 2 h. At room temperature, the reaction mixture was quenched with water (20 mL). The reaction mixture was extracted with dichloromethane (3 × 20 mL). The organic phases were combined, washed with saturated brine (1 × 30 mL), and dried over anhydrous sodium sulfate. The resulting mixture was filtered, and then the filtrate was concentrated under reduced pressure. The resulting mixture was used directly without further purification.

MS(ESI, *m/z*): 526.80 $[M+H]^+$, RT(min):1.178

Step 6 Synthesis of 4-((2-hydroxyethyl)sulfonylamino)-2-(6-azaspiro[2.5]octan-6-yl)-*N*-(benzo[4,5]imidazo[1,2-a]piperidin-9-yl)benzamide (compound 21):

Under nitrogen protection, to a solution of 4-iodo-2-(6-azaspiro[2.5]octan-6-yl)-*N*-(benzo[4,5]imidazo[1,2-a]piperidin-9-yl)benzamide (80 mg, 0.152 mmol, 1 eq) in *N,N*-dimethylformamide (2 mL) were added 2-hydroxyethanesulfonamide (28.53 mg, 0.228 mmol, 1.5 eq), potassium carbonate (63.01 mg, 0.456 mmol, 3 eq), cuprous iodide (2.89 mg, 0.015 mmol, 0.1 eq) and 2-(methylamino)acetic acid (2.71 mg, 0.030 mmol, 0.2 eq) at room temperature. After the addition was completed, the system was further stirred at 120°C for 2 h. The reaction mixture was cooled to room temperature. The mixture was diluted with water (5 mL) and extracted with ethyl acetate (3 × 10 mL). The organic phases were combined, washed with saturated brine (1 × 10 mL), and dried over anhydrous sodium sulfate. The resulting mixture was filtered, and then the filtrate was concentrated under reduced pressure. The crude product was purified by HPLC under the following conditions (chromatographic column specifications: Kinetex EVO C18 Column, 30*150, 5um; mobile phase A: water (10 mmol/L ammonium bicarbonate), mobile phase B: acetonitrile; flow rate: 60 mL/min; elution gradient: 10% B to 50% B, 8 min, 50% B; detection wavelength: UV 220 nm; retention time (min): 7.47) to obtain 4-((2-hydroxyethyl)sulfonylamino)-2-(6-azaspiro[2.5]octan-6-yl)-*N*-(benzo[4,5]imidazo[1,2-a]piperidin-9-yl)benzamide (compound 21, 14.1 mg, 17.63%).

MS(ESI, *m/z*): 523.85 $[M+H]^+$, RT(min):1.157

$^1H$ NMR: (400 MHz, DMSO-$d_6$) δ 11.41 (s, 1H), 10.09 (s, 1H), 7.87 (d, 1H), 7.46 (s, 1H), 7.25 - 7.12 (m, 2H), 7.09 - 6.99 (m, 2H), 4.97 (s, 1H), 4.21 (t, 2H), 3.77 (t, 2H), 3.36 - 3.32 (m, 2H), 3.04 (t, 4H), 2.96 (s, 2H), 1.93 (d, 2H), 1.87 (s, 2H), 1.52 (t, 4H), 0.34 (s, 4H).

Example 3

4-(2-hydroxyethanesulfonylamino)-2-(6-azaspiro[2.5]octan-6-yl)-*N*-benzo[4,5]imidazo[1,2-a]piperidin-6-yl)benzamide (compound 22)

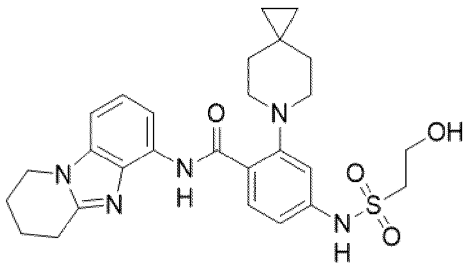

Compound 22

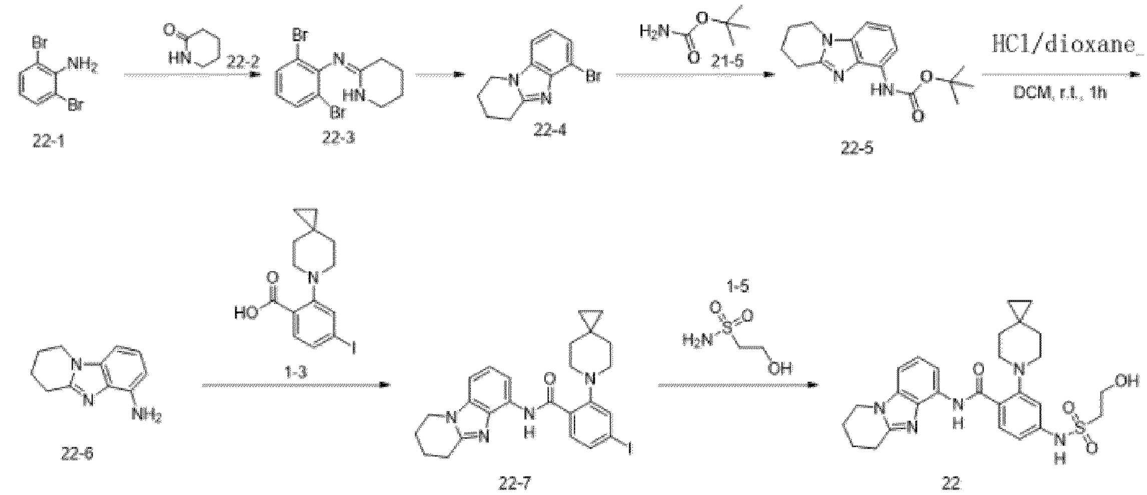

22-1 22-3 22-4 22-5 22-6 22-7 22

Step 1 Synthesis of (Z)-*N*-(2,6-dibromophenyl)piperidin-2-imine (compound 22-3):

Under nitrogen protection, phosphorus oxychloride (611.03 mg, 3.985 mmol, 1 eq) was added to a solution of 2-piperidone (790.15 mg, 7.971 mmol, 2.0 eq) in toluene at 0°C. After the reaction mixture was stirred at 0°C for 2 h, 2,6-dibromoaniline (1 g, 3.985 mmol, 1 eq) was added and the reaction mixture was stirred at 110°C for 16 h. The reaction mixture was cooled to room temperature. The resultant residue was concentrated under reduced pressure, and the reaction mixture was quenched with water (50 mL) at 0°C. The reaction mixture was alkalinized to pH = 10 with sodium hydroxide. The aqueous phase was extracted with dichloromethane (3 × 50 mL), the organic phases were combined, washed with saturated brine (1 × 80 mL), dried over anhydrous sodium sulfate, filtered, and the filtrate was concentrated under reduced pressure. The crude product was recrystallized with dichloromethane/petroleum ether (1:100) to obtain a white solid (*Z*)-*N*-(2,6-dibromophenyl)piperidin-2-imine (compound 22-3, 900 mg, 68.01%).

MS(ESI, *m*/*z*): 332.75 $[M+H]^+$, RT(min):0.731

Step 2 Synthesis of 6-bromo-benzo[4,5]imidazo[1,2-a]piperidine (compound 22-4):

Under nitrogen protection, cuprous iodide (1.43 mg, 0.008 mmol, 0.05 eq), potassium carbonate (20.81 mg, 0.151 mmol, 1 eq) and N,N-dimethylethylenediamine (1.33 mg, 0.015 mmol, 0.1 eq) were added to a solution of (*Z*)-*N*-(2,6-dibromophenyl)piperidin-2-imine (850 mg, 2.560 mmol, 1 eq) in acetonitrile (10 mL) at room temperature, and the reaction mixture was stirred at 100°C for 2 h. The reaction mixture was cooled to room temperature, diluted with water (30 mL), and extracted with ethyl acetate (3 × 30 mL). The organic phases were combined, washed with saturated brine (2 × 40 mL), dried over anhydrous sodium sulfate, filtered, and the filtrate was concentrated under reduced pressure. The resultant residue was concentrated under reduced pressure. The resulting residue was purified by silica gel column chromatography with petroleum ether/ethyl acetate (6:1) to obtain 6-bromo-benzo[4,5]imidazo[1,2-a]piperidine (compound 22-4, 360 mg, 56.00%).

MS(ESI, *m*/*z*): 250.85 $[M+H]^+$, RT(min):0.646

Step 3 Synthesis of tert-butyl (benzo[4,5]imidazo[1,2-a]piperidin-6-yl)carbamate (compound 22-5):

To a solution of 6-bromo-benzo[4,5]imidazo[1,2-a]piperidine (500 mg, 1.991 mmol, 1 eq) in N,N-dimethylformamide (15 mL) were added cesium carbonate (1946.14 mg, 5.973 mmol, 3 eq), tert-butyl carbamate (466.49 mg, 3.982 mmol, 2 eq), (methanesulfonic acid{biscyclohexyl(3-isopropoxy-2',4',6'-triisopropyl-[1,1'-biphenyl]-2-yl)phosphine}(2'-methylamino-1,1'-biphenyl-2-yl)palladium(II) (182.78 mg, 0.199 mmol, 0.1 eq) and dicyclohexyl(3-ethylpropoxy-2',4',6'-triisopropyl-[1,1'-diphenyl]-2-yl)phosphine; dicyclohexyl(3-isopropyl-2',4',6'-triisopropyl-[1,1'-biphenyl]-2-yl)phosphine (212.96 mg, 0.398 mmol, 0.2 eq) under nitrogen protection at room temperature, and the reaction mixture was stirred at 100°C for 1 h. The reaction mixture was cooled to room temperature, diluted with water (20 mL), and extracted with ethyl acetate (3 × 20 mL). The organic phases were combined, washed with saturated brine (1 × 20 mL), and dried over anhydrous sodium sulfate. The system was filtered, and the filtrate was concentrated under reduced pressure. The resulting residue was purified by silica gel column chromatography with petroleum ether/ethyl acetate (2:1) to obtain tert-butyl (benzo[4,5]imidazo[1,2-a]piperidin-6-yl)carbamate (compound 22-5, 490 mg, 85.64%).

MS (ESI, *m*/*z*): 288.35 $[M+H]^+$, RT(min):0.768

Step 4 Synthesis of benzo[4,5]imidazo[1,2-a]piperidin-6-amine (compound 22-6):

Under nitrogen protection, hydrogen chloride (gas) dissolved in 1,4-dioxane (5 mL) was added to a solution of tert-butyl (benzo[4,5]imidazo[1,2-a]piperidin-6-yl)carbamate (400 mg, 1.392 mmol, 1 eq) in dichloromethane (5 mL) at room temperature, and the system was stirred for 1 h. The resulting residue was concentrated under reduced pressure to give a crude product of benzo[4,5]imidazo[1,2-a]piperidin-6-amine, which was directly used in the next step without further purification.

MS (ESI, *m*/*z*): 188.35 $[M+H]^+$, RT(min):0.450

Step 5 Synthesis of 4-iodo-2-(6-azaspiro[2.5]octan-6-yl)-N-(benzo[4,5]imidazo[1,2-a]piperidin-6-yl)benzamide (compound 22-7):

Under nitrogen protection, thionyl chloride (279.54 mg, 2.350 mmol, 2 eq) was added to a solution of 4-iodo-2-(6-azaspiro[2.5]octan-6-yl)benzoic acid (629.51 mg, 1.763 mmol, 1.5 eq) in dichloromethane (8 mL) at room temperature, and the reaction mixture was stirred at room temperature for 1 h. The resulting residue was concentrated under reduced pressure. The resulting residue was dissolved in dichloromethane (4 mL), potassium phosphate (498.79 mg, 2.350 mmol, 2 eq) was added to the above system at room temperature, and the mixture was stirred at room temperature for 5 min. Then, a solution of benzo[4,5]imidazo[1,2-a]piperidin-6-amine (20 mg, 0.107 mmol, 1 eq) and *N,N*-diisopropylethylamine (455.57 mg, 3.525 mmol, 3 eq) in dichloromethane (4 mL) was added to the above system. After the addition was completed, the system was further stirred at room temperature for 2 h. The reaction mixture was quenched with water (20 mL) and extracted with ethyl acetate ($3 \times 20$ mL). The organic phases were combined, washed with saturated brine ($1 \times 20$ mL), and dried over anhydrous sodium sulfate. The resulting mixture was filtered, and then the filtrate was concentrated under reduced pressure. The resulting residue was purified by silica gel column chromatography with petroleum ether/ethyl acetate (5:1) to obtain 4-iodo-2-(6-azaspiro[2.5]octan-6-yl)-*N*-(benzo[4,5]imidazo[1,2-a]piperidin-6-yl)benzamide (compound 22-7, 530 mg, 85.69%).

MS(ESI, *m*/*z*): 527.35 $[M+H]^+$, RT(min):1.078

Step 6 Synthesis of 4-((2-hydroxyethanesulfonylamino)-2-(6-azaspiro[2.5]octan-6-yl)-*N*-(benzo[4,5]imidazo[1,2-a]piperidin-6-yl)benzamide (compound 22):

Under nitrogen protection, cuprous iodide (3.62 mg, 0.019 mmol, 0.1 eq), sarcosine (3.39 mg, 0.038 mmol, 0.2 eq), potassium carbonate (78.76 mg, 0.570 mmol, 3 eq) and 2-hydroxyethanesulfonamide (47.54 mg, 0.380 mmol, 2 eq) were added to a solution of 4-iodo-2-(6-azaspiro[2.5]octan-6-yl)-*N*-(benzo[4,5]imidazo[1,2-a]piperidin-6-yl)benzamide (100 mg, 0.190 mmol, 1 eq) in *N,N*-dimethylformamide (2 mL) at room temperature. The reaction mixture was stirred at 120°C for 1.5 h. The reaction mixture was cooled to room temperature, diluted with water (10 mL), and extracted with ethyl acetate ($3 \times 10$ mL). The organic phases were combined, washed with saturated brine ($1 \times 10$ mL) and dried over anhydrous sodium sulfate. The resulting mixture was filtered, and then the filtrate was concentrated under reduced pressure. The crude product was purified by HPLC under the following conditions (chromatographic column specifications: Xselect CSH C18 OBD Column 30*150mm 5μm, n; mobile phase A: water (0.1% formic acid), mobile phase B: acetonitrile; flow rate: 60 mL/min; elution gradient: 10% B to 37% B, 10 min, 37% B; detection wavelength: 220 nm; retention time (min): 9.03; number of runs: 0). 4-(2-hydroxyethanesulfonylamino)-2-(6-azaspiro[2.5]octan-6-yl)-*N*-(benzo [4,5]imidazo[1,2-a]piperidin-6-yl)benzamide (compound 22, 30.7 mg, 27.69%) was obtained.

MS (ESI, *m*/*z*): 523.90 $[M+H]^+$, RT(min):1.530

$^1H$ NMR (400 MHz, DMSO-$d_6$) δ 12.44 (s, 1H), 10.14 (s, 1H), 8.33 (d, 1H), 8.04 (d, 1H), 7.27 (d, 1H), 7.21 - 7.13 (m, 2H), 7.09 (dd, 1H), 4.93 (d, 1H), 4.13 (t, 2H), 3.76 (q, 2H), 3.32 (s, 2H), 3.00 (q, 6H), 2.11 - 2.03 (m, 2H), 2.02 - 1.92 (m, 2H), 1.83 (s, 4H), 0.31 (s, 4H).

Example 4

*N*-(2,3-dihydro-1*H*-benzo[d]pyrrolo[1,2-a]imidazol-5-yl)-4-(2-hydroxyethanesulfonylamino)-2-(6-azaspiro[2.5]octan-6-yl)benzamide (compound 36)

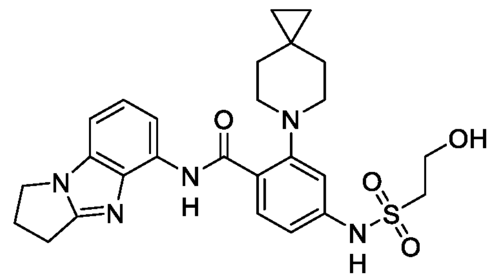

Compound 36

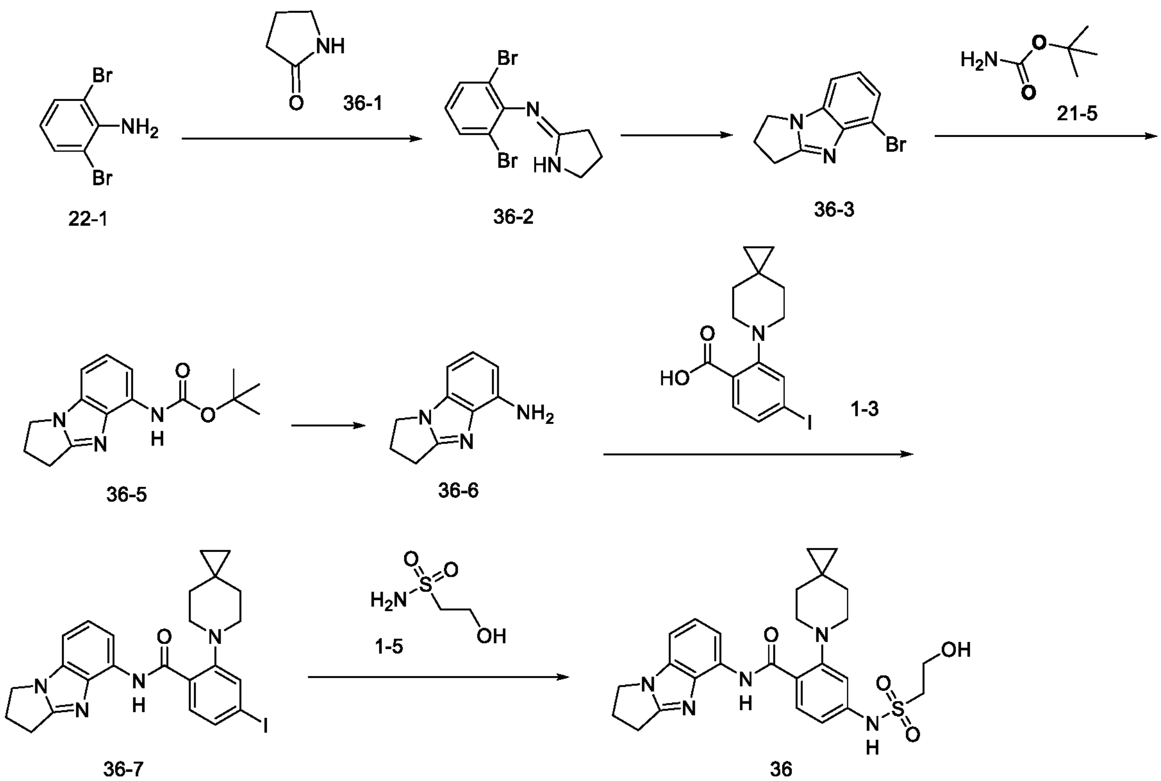


Step 1 Synthesis of (2*Z*)-*N*-(2,6-dibromophenyl)pyrrolidin-2-imine (compound 36-2):

Under nitrogen protection, phosphorus oxychloride (1.22 g, 85.106 mmol, 2eq) was added to a solution of pyrrolidone (1.36 g, 15.942 mmol, 2 eq) in toluene (10 mL) at 0°C. The reaction solution was allowed to react at 0°C for 2 h, and then 2,6-dibromoaniline (2 g, 7.971 mmol, 1 eq) was added. The reaction mixture was heated to 110°C and stirred for 16 h. The reaction mixture was cooled to room temperature, concentrated under reduced pressure, quenched with ice water (50 mL), adjusted to pH 10 with saturated sodium bicarbonate solution, and extracted with ethyl acetate (3 × 50 mL). The organic phases were combined, washed with saturated brine (1 × 50 mL), and dried over anhydrous sodium sulfate. The mixture was filtered and the filtrate was concentrated under reduced pressure. The resulting residue was purified by preparative chromatography using ethyl acetate/petroleum ether (1:10) to afford (2*Z*)-*N*-(2,6-dibromophenyl)pyrrolidin-2-imine (2.7 g, 27.65%).

MS (ESI, m/z):317.10 $[M+H]^+$, RT(min):0.919

Step 2 Synthesis of 5-bromo-2,3-dihydro-1*H*-benzo[d]pyrrolo[1,2-a]imidazole (compound 36-3):

Under nitrogen protection, *N*,*N*-dimethyl-1,2-ethylenediamine (74.84 mg, 0.849 mmol, 0.10 eq), cuprous iodide (80.85 mg, 0.425 mmol, 0.05 eq), and potassium carbonate (1.17 g, 8.490 mmol, 1 eq) were added to a solution of (2Z)-N-(2,6-dibromophenyl)pyrrolidin-2-imine (2.7 g, 8.490 mmol, 1 eq) in acetonitrile (3 mL) at room temperature. The reaction mixture was heated to 100°C and stirred for 2 h. The reaction mixture was cooled to room temperature, quenched

with water (80 mL) and extracted with ethyl acetate (3 × 60 mL). The organic phases were combined, washed with saturated brine (1 × 80 mL), and dried over anhydrous sodium sulfate. The mixture was filtered and the filtrate was concentrated under reduced pressure. 5-bromo-2,3-dihydro-1*H*-benzo[d]pyrrolo[1,2-a]imidazole (2 g, 74.07%) was obtained.

MS (ESI, m/z):237.15 $[M+H]^+$, RT(min):0.657

Step 3 Synthesis of tert-butyl (2,3-dihydro-1*H*-benzo[d]pyrrolo[1,2-a]imidazol-5-yl)carbamate (compound 36-5):

Under nitrogen protection, dicyclohexyl(3-isopropyl-2',4',6'-triisopropyl-[1,1'-biphenyl]-2-yl)phosphine (451.12 mg, 0.844 mmol, 0.1 eq), (methanesulfonic acid{bicycloethyl(3-isopropyl-2,4,6-triisopropyl-(1,1-biphenyl)-2-yl)phosphine})(2-methylamino-1,1-biphenyl-2-yl)palladium(II) (1.549 g, 1.688 mmol, 0.2 eq) and cesium carbonate (8.245 g, 25.308 mmol, 3 eq) were added to a solution of 5-bromo-2,3-dihydro-1*H*-benzo[d]pyrrolo[1,2-a]imidazole (2 g, 8.436 mmol, 1 eq) and tert-butyl carbamate (1.99 g, 16.87 mmol, 2 eq) in *N*,*N*-dimethylformamide (10 mL) at room temperature. The reaction mixture was heated to 100°C and stirred for 1 h. The reaction mixture was cooled to room temperature and quenched with water (80 mL). The system was extracted with ethyl acetate (3 × 60 mL). The organic phases were combined, washed with saturated brine (1 × 80 mL), and dried over anhydrous sodium sulfate. The mixture was filtered and the filtrate was concentrated under reduced pressure. The resulting residue was purified by preparative chromatography using ethyl acetate/petroleum ether (1:5) to afford tert-butyl (2,3-dihydro-1*H*-benzo[d]pyrrolo[1,2-a]imidazol-5-yl) carbamate (700 mg, 35.77%).

MS (ESI, *m*/*z*):274.35 $[M+H]^+$, RT(min):0.592

Step 4 Synthesis of 2,3-dihydro-1*H*-benzo[d]pyrrolo[1,2-a]imidazol-5-amine (compound 36-6):

A reaction mixture of tert-butyl (2,3-dihydro-1*H*-benzo[d]pyrrolo[1,2-a]imidazol-5-yl)carbamate (300 mg, 1.098 mmol, 1 eq), hydrochloric acid in 1,4-dioxane (2 mL), and dichloromethanol (2 mL) was stirred at room temperature for 30 min. The reaction mixture was concentrated under reduced pressure to give 2,3-dihydro-1*H*-benzo[d]pyrrolo[1,2-a] imidazol-5-amine (300 mg, 57.87%), which was directly used in the next step.

MS (ESI, *m*/*z*):174.35 $[M+H]^+$, RT(min):0.602

Step 5 Synthesis of 2-{6-azaspiro[2.5]octan-6-yl}-*N*-{2,3-dihydro-1H-benzo[d]pyrrolo[1,2-a]imidazol-5-yl}-4-iodobenzamide (compound 36-7):

Under nitrogen protection, tetramethylchloroformamidinium hexafluorophosphate (1.62 g, 5.772 mmol, 4 eq) and *N*-methylimidazole (1.18 g, 14.430 mmol, 10 eq) were added to a solution of 2,3-dihydro-1*H*-benzo[d]pyrrolo[1,2-a] imidazol-5-amine (250 mg, 1.443 mmol, 1 eq) and 2-{6-azaspiro[2.5]octan-6-yl}-4-iodobenzoic acid (618.62 mg, 1.732 mmol, 1.2 eq) in dichloromethane (5 mL) at room temperature, and the reaction mixture was stirred at room temperature for 2 h. The reaction mixture was diluted with water (20 mL) and extracted with ethyl acetate (3 × 20 mL). The organic phases were combined, washed with saturated brine (1 × 20 mL), and dried over anhydrous sodium sulfate. The mixture was filtered and the filtrate was concentrated under reduced pressure. The resulting residue was purified by preparative chromatography (ethyl acetate:petroleum ether = 1:5) to give 2-{6-azaspiro[2.5]octan-6-yl}-*N*-{2,3-dihydro-1*H*-benzo[d] pyrrolo[1,2-a]imidazol-5-yl}-4-iodobenzamide (100 mg, 40.00%).

MS (ESI, *m*/*z*):513.35 $[M+H]^+$, RT(min):1.406

Step 6 Synthesis of *N*-(2,3-dihydro-1*H*-benzo[d]pyrrolo[1,2-a]imidazol-5-yl)-4-(2-hydroxyethanesulfonylamino)-2-(6-azaspiro[2.5]octan-6-yl)benzamide (compound 36):

Under nitrogen protection, sarcosine (2.09mg, 0.023 mmol, 0.2 eq), cuprous iodide (2.23 mg, 0.012 mmol, 0.1 eq) and potassium carbonate (48.55 mg, 0.351 mmol, 3 eq) were added to a solution of *N*-(2,3-dihydro-1*H*-benzo[d]pyrrolo [1,2-a]imidazol-5-yl)-4-iodo-2-(6-azaspiro[2.5]octan-6-yl)benzamide (60 mg, 0.117 mmol, 1 eq) and 2-hydroxyethanesulfonamide (17.58 mg, 0.140 mmol, 1.2 eq) in *N,N*-dimethylformamide (2 mL) at room temperature. The reaction mixture was heated to 120°C and stirred for 1.5 h. The reaction mixture was cooled to room temperature, quenched with water, and extracted with ethyl acetate (3 × 10 mL). The organic phases were combined, washed with saturated aqueous brine solution (1 × 10 mL), and dried over anhydrous sodium sulfate. The resulting mixture was filtered, and then the filtrate was concentrated under reduced pressure. The resulting crude product was purified by preparative HPLC under the following conditions: chromatographic column specifications: Kinetex EVO C18 column, 30*150, 5μm; mobile phase A: water (10 mmol/L ammonium bicarbonate), mobile phase B: acetonitrile; flow rate: 60 mL/min; elution gradient: 30% B to 65% B in 8 min, 60% B; detection wavelength: UV 220 nm; retention time (min): 6.12, to obtain *N*-(2,3-dihydro-1*H*-benzo[d]pyrrolo [1,2-a]imidazol-5-yl)-4-(2-hydroxyethanesulfonylamino)-2-(6-azaspiro[2.5]octan-6-yl)benzamide (4.22 mg, 6.60%).

MS (ESI, *m*/*z*):510.15 $[M+H]^+$, RT(min):1.143

$^1H$ NMR: (400 MHz, DMSO-$d_6$) δ 12.34 (s, 1H), 10.14 (s, 1H), 8.30 (dd, 1H), 8.01 (d, 1H), 7.25 (d, 1H), 7.19 - 7.10 (m, 2H), 7.09 - 7.04 (m, 1H), 4.96 (s, 1H),4.15 (t, 2H), 3.76 (t, 2H), 3.36 (d, 2H), 2.97 (dt, 6H), 2.67 (q, 2H), 1.78 (s, 4H), 0.30 (s, 4H).

The compounds in the following table were prepared using conditions similar to those in Example 4.

| Compound No. | Structure | Name | $[M+H]^+$ | $^1$H-NMR data |
|---|---|---|---|---|
| 104 | 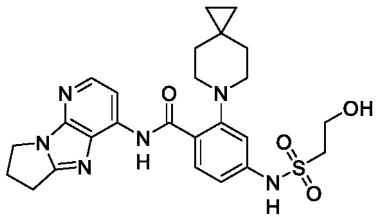 | *N*-(7,8-dihydro-6*H*-pyrrolo[2',1':2,3]imidazo[4,5-b]pyridin-4-yl)-4-(2-hydroxyethanesulfonylamino) -2-(6-azaspiro[2.5]octan-6-yl)benzamide | 511.00 | $^1$H NMR (400 MHz, DMSO-$d_6$) $\delta$ 12.80 (s, 1H), 10.18 (s, 1H), 8.26 (d, 1H), 8.15 (d, 1H), 8.04 (d, 1H), 7.29 (d, 1H), 7.15 -7.06 (m, 1H), 4.94 (s, 1H), 4.18 (t, 2H), 3.76 (t, 2H), 3.35 (t, 2H), 3.09 - 2.89 (m, 6H), 2.75 -2.63 (m, 2H), 1.86 (d, 4H), 0.32 (s, 4H) |
| 105 | 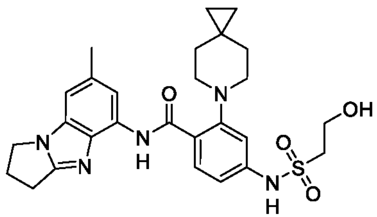 | 4-(2-hydroxyethanesulfonylamino) -*N*-(7-methyl-2,3-dihydro-1*H*-benzo[d]pyrrolo[1,2-a]imidazol-5-yl)-2-(6-azaspiro[2.5]octan-6-yl)benzamide | 524.40 | $^1$H NMR (400 MHz, DMSO-$d_6$) $\delta$ 12.24 (s, 1H), 10.10 (s, 1H), 8.17 (d, 1H), 7.99 (d, 1H), 7.24 (d, 1H), 7.14 - 7.01 (m, 1H), 6.97 (s, 1H), 4.92 (s, 1H), 4.10 (t, 2H), 3.76 (t, 2H), 3.34 (d, 2H), 2.98 (t, 4H), 2.92 (d, 2H), 2.72 - 2.61 (m, 2H), 2.42 (s, 3H), 1.77 (s, 4H), 0.29 (s, 4H) |

Example 5

2-{6-azaspiro[2.5]octan-6-yl}-*N*-{3',4'-dihydro-1'*H*-spiro[cyclopentane-1,2'-naphthalen]-5'-yl}-4-(2-hydroxyethanesulfonylamino)benzamide (compound 51)

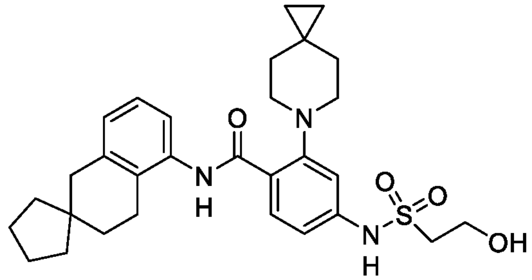

Compound 51

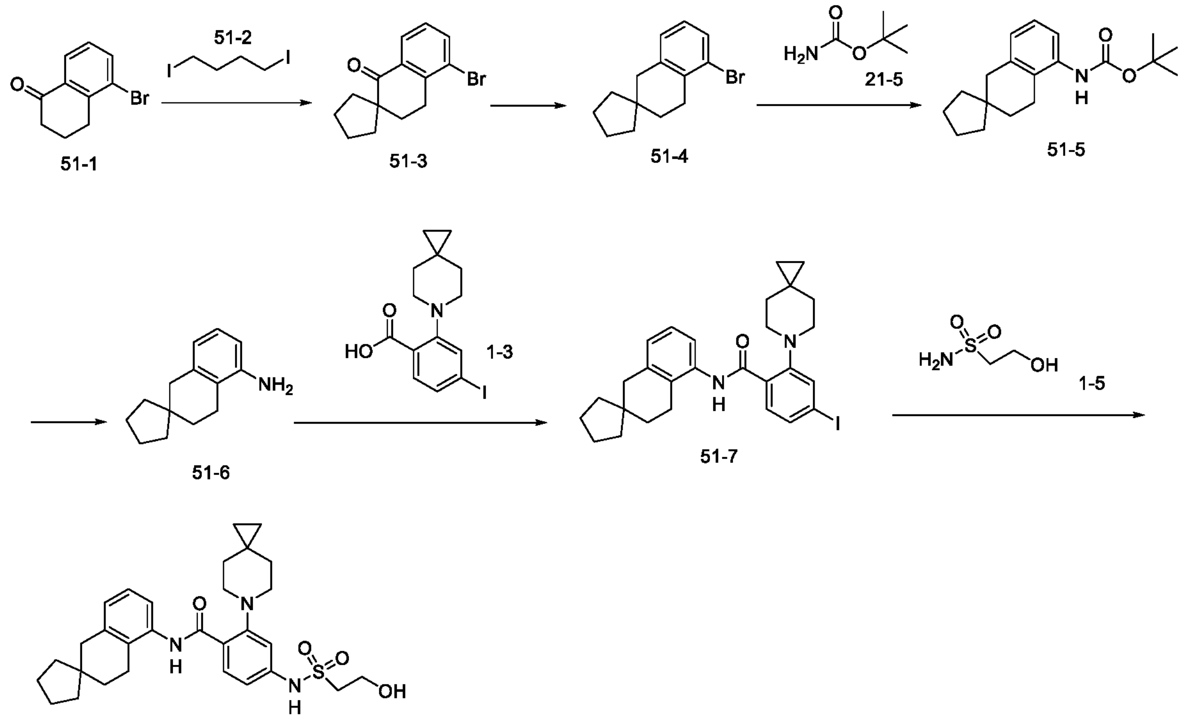


51-1 51-3 51-4 51-5 51-6 51-7 51

Step 1 Synthesis of 5'-bromo-3',4'-dihydrospiro[cyclopentane-1,2'-naphthalen]-1'-one (compound 51-3):

Under nitrogen protection, sodium hydride (0.71 g, 29.586 mmol, 3.33 eq) was added to a solution of 5-bromo-3,4-dihydro-2*H*-naphthalen-1-one (2 g, 8.886 mmol, 1 eq) in tetrahydrofuran (20 mL) at 0°C. The system was further stirred for 1 h, and then 1,4-diiodobutane (4.13 g, 13.329 mmol, 1.5 eq) was added dropwise at 0°C. The reaction mixture was heated to room temperature and stirred for 16 h. The reaction mixture was quenched with ice water at room temperature. The reaction mixture was extracted with ethyl acetate (3 × 80 mL). The organic phases were combined, washed with saturated aqueous brine solution (1 × 100 mL), and dried over anhydrous sodium sulfate. The resulting mixture was filtered, and then the filtrate was concentrated under reduced pressure. The resulting residue was purified by silica gel column chromatography with petroleum ether/ethyl acetate (10:1) to give 5'-bromo-3',4'-dihydrospiro[cyclopentane-1,2'-naphthalen]-1'-one (2.37 g, 80.56%).

MS:(ESI, *m*/*z*): 278.55 [M+H] $^+$, RT(min):1.325

Step 2 Synthesis of 5'-bromo-3',4'-dihydro-1'*H*-spiro[cyclopentane-1,2'-naphthalene] (compound 51-4):

Under nitrogen protection, triethylsilane (5.0 mL, 30.94 mmol, 4.35 eq) was added to a solution of 5'-bromo-3',4'-dihydrospiro[cyclopentane-1,2'-naphthalen]-1'-one (2 g, 7.11 mmol, 1 eq) in trifluoroacetic acid (15 mL) at 0°C. The reaction mixture was heated to 50°C and stirred for 1 h. The reaction mixture was cooled to room temperature and extracted with ethyl acetate (3 × 80 mL). The organic phases were combined, washed with saturated aqueous brine solution (1 × 80 mL), and dried over anhydrous sodium sulfate. The resulting mixture was filtered, and then the filtrate was concentrated under reduced pressure. The resulting residue was purified by silica gel column chromatography with petroleum ether/ethyl acetate (10:1) to give 5'-bromo-3',4'-dihydro-1'*H*-spiro[cyclopentane-1,2'-naphthalene] (1.43 g, 64.18%).

MS (EI, *m*/*z*): 264.1 [M], RT(min):8.694

Step 3 Synthesis of tert-butyl {3',4'-dihydro-1'*H*-spiro[cyclopentane-1,2'-naphthalen]-5'-yl}carbamate (compound 51-5):

Under nitrogen protection, cesium carbonate (3.7 g, 12.75 mmol, 3 eq), palladium acetate (84.5 mg, 0.35 mmol, 0.1 eq) and 2-dicyclohexylphosphino-2,4,6-triisopropylbiphenyl (359.5 mg, 0.75 mmol, 0.2 eq) were added to a solution of 5'-bromo-3',4'-dihydro-1'*H*-spiro[cyclopentane-1,2'-naphthalene] (1 g, 3.75 mmol, 1 eq) and tert-butyl carbamate (883.5 mg, 7.5 mmol, 2 eq) in 1,4-dioxane (10 mL) at room temperature. The reaction mixture was heated to 100°C and stirred for 1 h. The reaction mixture was cooled to room temperature, quenched with water, and extracted with ethyl acetate (3 × 50 mL). The organic phases were combined, washed with saturated aqueous brine solution (1 × 80 mL), and dried over anhydrous sodium sulfate. The resulting mixture was filtered, and then the filtrate was concentrated under reduced pressure. The resulting residue was purified by silica gel column chromatography with petroleum ether/ethyl acetate (10:1) to give {tert-butyl 3',4'-dihydro-1'*H*-spiro[cyclopentane-1,2'-naphthalen]-5'-yl}carbamate (800 mg, 70.79%).

MS (EI, *m*/*z*): 201.1 [M-100], RT(min):9.866

Step 4 Synthesis of 3',4'-dihydro-1'*H*-spiro[cyclopentane-1,2'-naphthalen]-5'-amine (compound 51-6):

To a solution of tert-butyl {3',4'-dihydro-1'*H*-spiro[cyclopentane-1,2'-naphthalen]-5'-yl}carbamate (200 mg, 0.66 mmol, 1 eq) in dichloromethane (2 mL) was added a solution of hydrochloric acid in 1,4-dioxane (2 mL) at room temperature, and the reaction mixture was stirred at room temperature for 30 min. The reaction mixture was concentrated under reduced pressure to obtain 3',4'-dihydro-1'H-spiro [cyclopentane-1,2'-naphthalen]-5'-amine (60 mg, 46.78%). The crude product was not further purified and was directly used in the next step.

MS:(ESI, *m*/*z*): 202.45 $[M+H]^+$, RT(min):1.041

Step 5 Synthesis of 2-{6-azaspiro[2.5]octan-6-yl}-*N*-{3',4'-dihydro-1'*H*-spiro[cyclopentane-1,2'-naphthalen]-5'-yl}-4-iodobenzamide (compound 51-7):

Under nitrogen protection, to a solution of 2-{6-azaspiro[2.5]octan-6-yl}-4-iodobenzoic acid (127.74 mg, 0.36 mmol, 1.2 eq) in dichloromethane (1 mL) were added *N,N,N,N*-tetramethylchloroformamidine hexafluorophosphate (334.5 mg, 1.12 mmol, 4 eq), *N*-methylimidazole (244.71 mg, 2.97 mmol, 10 eq) and 3',4'-dihydro-1'H-spiro[cyclopentane-1,2'-naphthalen]-5'-amine (60 mg, 0.300 mmol, 1 eq) at room temperature. The reaction mixture was allowed to react with stirring at room temperature for 1 h. The reaction mixture was extracted with ethyl acetate (3 × 20 mL). The organic phases were combined, washed with saturated aqueous brine solution (1 × 20 mL), and dried over anhydrous sodium sulfate. The resulting mixture was filtered, and then the filtrate was concentrated under reduced pressure. The resulting residue was purified by silica gel column chromatography with petroleum ether/ethyl acetate (10:1) to give 2-{6-azaspiro [2.5]octan-6-yl}-*N*-{3',4'-dihydro-1'*H*-spiro[cyclopentane-1,2'-naphthalen]-5'-yl}-4-iodobenzamide (100 mg, 90.34%).

MS(ESI, *m*/*z*): 541.35 $[M+H]^+$, RT(min):1.728

Step 6 Synthesis of 2-{6-azaspiro[2.5]octan-6-yl}-*N*-{3',4'-dihydro-1'*H*-spiro[cyclopentane-1,2'-naphthalen]-5'-yl}-4-(2-hydroxyethanesulfonylamino)benzamide (compound 51):

Under nitrogen protection, cuprous iodide (1.75 mg, 0.010 mmol, 0.1 eq), sarcosine (1.65 mg, 0.020 mmol, 0.20 eq) and potassium carbonate (38.35 mg, 0.285 mmol, 3 eq) were added to a solution of 2-{6-azaspiro[2.5]octan-6-yl}-*N*-{3',4'-dihydro-1'*H*-spiro[cyc1opentane-1,2'-naphthalen]-5'-yl}-4-iodobenzamide (50 mg, 0.095 mmol, 1 eq) and 2-hydroxyethanesulfonamide (13.9 mg, 0.115 mmol, 1.2 eq) in *N,N*-dimethylformamide (1 mL) at room temperature. The reaction mixture was heated to 120°C and stirred for 1 h. The reaction mixture was cooled to room temperature, quenched with water, and extracted with ethyl acetate (3 × 10 mL). The organic phases were combined, washed with saturated

aqueous brine solution (1 × 10 mL), and dried over anhydrous sodium sulfate. The resulting mixture was filtered, and then the filtrate was concentrated under reduced pressure. The resulting crude product was purified by preparative HPLC under the following conditions: chromatographic column specifications: Kinetex EVO C18 column, 30*150, 5μm; mobile phase A: water (10 mmol/L ammonium bicarbonate), mobile phase B: acetonitrile; flow rate: 60 mL/min; elution gradient: 45% B to 96% B in 8 min, 96% B; detection wavelength: UV 220 nm; retention time (min): 7.62, to obtain 2-{6-azaspiro[2.5]octan-6-yl}-*N*-{3',4'-dihydro-1'*H*-spiro[cyclopentane-1,2'-naphthalen]-5'-yl}-4-(2-hydroxyethanesulfonylamino)benzamide (10 mg, 20.06%).

MS (ESI, *m/z*): 538.10 $[M+H]^+$, RT(min):1.383

$^1H$ NMR: (400 MHz, DMSO-$d_6$) δ 10.87 (s, 1H), 10.07 (s, 1H), 7.85 (d, 1H), 7.58 (m, 1H), 7.19 (d, 1H), 7.17 (d, 1H), 7.03 (m, 2H), 4.93 (d, 1H), 3.76 (q, 2H), 2.99 (d, 4H), 2.78 (d, 2H), 2.07 (s, 2H), 1.89 (s, 1H), 1.78 (s, 1H), 1.70 (d, 6H), 1.46 - 1.36 (m, 4H), 1.30 - 1.13 (m, 4H), 0.34 (s, 4H).

Example 6

2-{6-azaspiro[2.5]octan-6-yl}-*N*-{3',4'-dihydro-1'*H*-spiro[cyclopropane-1,2'-naphthalen]-5'-yl}-4-(2-hydroxyethanesulfonylamino)benzamide (compound 56)

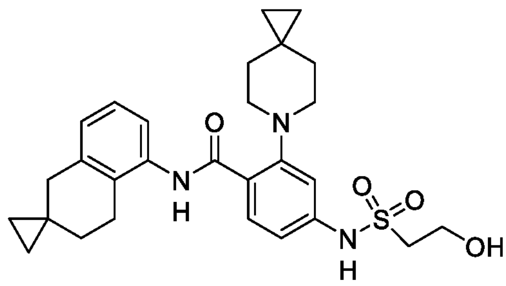

Compound 56

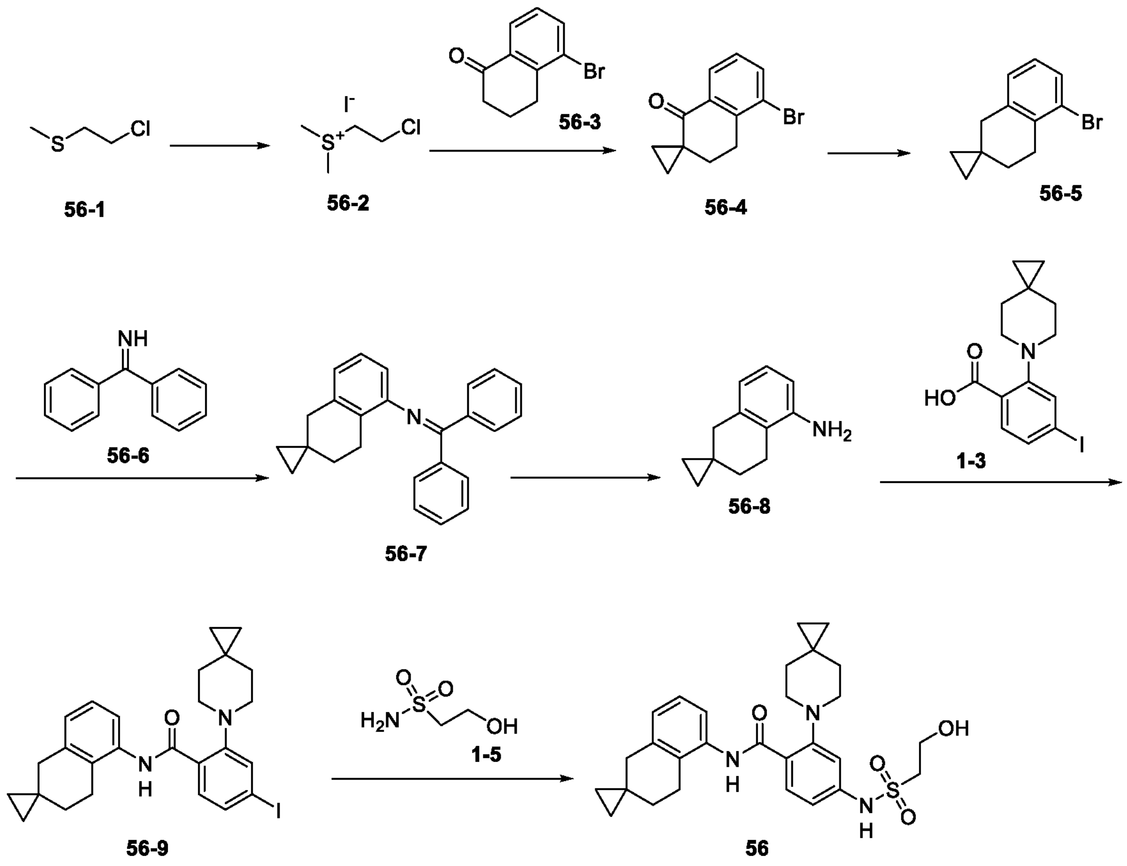

56-1 56-2 56-3 56-4 56-5

56-6 56-7 56-8 1-3

56-9 1-5 56

Step 1 Synthesis of (2-chloroethyl) dimethylsulfonium iodide (compound 56-2):

A solution of 1-chloro-2-(methylthio)ethane (8 g, 72.333 mmol, 1 eq) and iodomethane (27.02 mL, 433.998 mmol, 6 eq) was stirred at room temperature for 65 h. Solid in the mixture was precipitated out, the system was filtered, the filter cake was washed with acetone (3 × 30mL), and the solid was dried to obtain (2-chloroethyl) dimethylsulfonium iodide (9.5 g, 52.01%).

MS (ESI, *m*/*z*): 125.02 $[M]^+$. RT (min): 0.19.

Step 2 Synthesis of 5'-bromo-3',4'-dihydrospiro[cyclopropane-1,2'-naphthalen]-1'-one (compound 56-4):

To a solution of 5-bromo-3,4-dihydro-2H-naphthalen-1-one (2 g, 10.461 mmol, 1.00 eq) in tert-butanol (30 mL), sodium iodide (0.27 g, 1.777 mmol, 0.2 eq) and sodium hydride (0.71 g, 60%, dispersed in kerosene, 17.77 mmol, 2 eq) were added at 0°C. The mixture was stirred at room temperature for 20 min, and then (2-chloroethyl)dimethylsulfonium iodide (2.47 g, 9.775 mmol, 1.1 eq) was added portionwise. The mixture was stirred for 16 h. The reaction solution was quenched with water and extracted with ethyl acetate (3 x 50 mL). The organic phases were combined and washed with saturated brine (2 × 50 mL). The resulting mixture was filtered and the filtrate was concentrated under reduced pressure. The resulting residue was purified by silica gel column chromatography with petroleum ether/ethyl acetate (5:1) to give 5'-bromo-3',4'-dihydrospiro[cyclopropane-1,2'-naphthalen]-1'-one (1.8 g, 80.67%).

MS (EI, *m*/*z*): 250.1 $[M]^+$. RT (min): 8.46.

Step 3 Synthesis of 5'-bromo-3',4'-dihydro-1'*H*-spiro[cyclopropane-1,2'-naphthalene] (compound 56-5):

Under nitrogen protection at 0°C, triethylsilane (2.57 mL, 15.930 mmol, 5 eq) was added to a solution of 5'-bromo-3',4'-dihydrospiro[cyclopropane-1,2'-naphthalen]-1'-one (800 mg, 3.186 mmol, 1 eq) in dichloromethane (30 mL), and then boron trifluoride diethyl etherate solution (2.02 mL, 15.930 mmol, 5 eq) was added dropwise. The reaction mixture was stirred at 0°C for 4 h, then warmed to room temperature and stirred for 16 h. The reaction solution was quenched with saturated sodium bicarbonate solution and extracted with dichloromethane (3 × 50mL). The organic phases were combined, washed with saturated brine (1 × 60mL), and dried over anhydrous sodium sulfate. The system was filtered, and the filtrate was concentrated under reduced pressure. The resulting residue was purified by silica gel column

chromatography with petroleum ether/ethyl acetate (10:1) to give 5'-bromo-3',4'-dihydro-1'*H*-spiro[cyclopropane-1,2'-naphthalene] (550 mg, crude).

Step 4 Synthesis of *N*-{3',4'-dihydro-1'*H*-spiro[cyclopropane-1,2'-naphthalen]-5'-yl}-1,1-diphenylmethanimine (compound 56-7):

Under nitrogen protection, to a solution of 5'-bromo-3',4'-dihydro-1'*H*-spiro[cyclopropane-1,2'-naphthalene] (500 mg, 2.108 mmol, 1 eq), benzophenone imine (458.56 mg, 2.530 mmol, 1.2 eq), sodium tert-butoxide (303.95 mg, 3.162 mmol, 1.5 eq) in toluene (20 mL) were added 1,1'-binaphthyl-2,2'-bisdiphenylphosphine (131.29 mg, 0.211 mmol, 0.1 eq) and tris(dibenzylideneacetone)dipalladium (96.54 mg, 0.105 mmol, 0.05 eq) at room temperature. The reaction mixture was heated to 90°C and stirred for 16 h. The reaction mixture was cooled to room temperature and filtered. The filter cake was washed with ethyl acetate, and the mixture was extracted with ethyl acetate (3 × 50 mL). The organic phases were combined, washed with saturated brine (1 × 60 mL), dried over anhydrous sodium sulfate, filtered, and the filtrate was concentrated under reduced pressure to give a crude product *N*-{3',4'-dihydro-1'*H*-spiro[cyclopropane-1,2'-naphthalen]-5'-yl}-1,1-diphenylmethanimine (1.1 g, crude), which was directly used in the next step without further purification.
MS (ESI, *m/z*): 338.50 [M+H] $^+$. RT (min): 1.524.

Step 5 Synthesis of 3',4'-dihydro-1'*H*-spiro[cyclopropane-1,2'-naphthalen]-5'-amine (compound 56-8):

*N*-{3',4'-dihydro-1'*H*-spiro[cyclopropane-1,2'-naphthalen]-5'-yl}-1,1-diphenylmethaniline (1.1 g, crude) in 1 M hydrochloric acid (10 mL) was stirred at room temperature for 16 h. The reaction mixture was alkalinized to pH = 8 with saturated aqueous sodium bicarbonate solution. The reaction mixture was extracted with dichloromethane (3 × 50 mL). The organic phases were combined, washed with saturated brine (1 × 50 mL), and dried over anhydrous sodium sulfate. The resulting mixture was filtered and the filtrate was concentrated under reduced pressure. The resulting residue was purified by silica gel column chromatography with dichloromethane/methanol (20:1) to give 3',4'-dihydro-1'*H*-spiro [cyclopropane-1,2'-naphthalen]-5'-amine (100 mg).
MS (ESI, *m/z*): 174.45 [M+H] $^+$. RT (min): 0.892.

Step 6 Synthesis of 2-{6-azaspiro[2.5]octan-6-yl}-*N*-{3',4'-dihydro-1'*H*-spiro[cyclopropane-1,2'-naphthalen]-5'-yl}-4-iodobenzamide (compound 56-9):

Under nitrogen protection, to a solution of 3',4'-dihydro-1'H-spiro[cyclopropane-1,2'-naphthalen]-5'- amine (100 mg, 0.577 mmol, 1 eq), 2-{6-azaspiro[2.5]octan-6-yl}-4-iodobenzoic acid (247.39 mg, 0.692 mmol, 1.2 eq) and *N,N,N',N'*-tetramethylchloroformamidine hexafluorophosphate (647.77 mg, 2.308 mmol, 4 eq) in dichloromethane (4 mL) was added *N*-methylimidazole (473.89 mg, 5.770 mmol, 10 eq) at room temperature. The reaction mixture was heated to 80°C and allowed to react for 2 h. The reaction mixture was cooled to room temperature, extracted with dichloromethane (3 × 20 mL). The organic phases were combined, washed with saturated saline (1 × 30 mL), dried over anhydrous sulfuric acid, filtered, and the filtrate was concentrated under reduced pressure. The resulting residue was purified by silica gel column chromatography with petroleum ether/ethyl acetate (3:1) to give 2-{6-azaspiro[2.5]octan-6-yl}-*N*-{3',4'-dihydro-1'*H*-spiro [cyclopropane-1,2'-naphthalen]-5'-yl}-4-iodobenzamide (56 mg, 18.93%).
MS (ESI, *m/z*): 513.05 [M+H] $^+$. RT (min): 1.262.

Step 7 Synthesis of 2-{6-azaspiro[2.5]octan-6-yl}-*N*-{3',4'-dihydro-1'*H*-spiro[cyclopropane-1,2'-naphthalen]-5'-yl}-4-(2-hydroxyethanesulfonylamino)benzamide (compound 56):

Under nitrogen protection, cuprous iodide (1.86 mg, 0.010 mmol, 0.1 eq) and sarcosine (1.74 mg, 0.020 mmol, 0.2 eq) were added to a solution of 2-{6-azaspiro[2.5]octan-6-yl}-N-{3',4'-dihydro-1'*H*-spiro[cyclopropane-1,2'-naphthalen]-5'-yl}-4-iodobenzamide (50 mg, 0.098 mmol, 1 eq), 2-hydroxyethanesulfonamide (14.65 mg, 0.118 mmol, 1.2 eq) and potassium carbonate (40.46 mg, 0.294 mmol, 3 eq) in *N,N*-dimethylformamide (1 mL) at room temperature. The reaction mixture was heated to 120°C and allowed to react for 1 h. The reaction mixture was cooled to room temperature and extracted with ethyl acetate (3 × 20 mL). The organic phases were combined, washed with saturated brine (3 × 10 mL), dried over anhydrous sulfuric acid, filtered, and the filtrate was concentrated under reduced pressure. The crude product was purified by preparative HPLC under the following conditions (chromatographic column specifications: Xselect CSH C18 OBD column, 30*150mm 5μm; mobile phase A: water (0.1% formic acid), mobile phase B: acetonitrile; flow rate: 60 mL/min; elution gradient: 45% B to 75% B in 8 min; detection wavelength: UV 220 nm; retention time (min): 7.4) to obtain 2-{6-azaspiro[2.5]octan-6-yl}-*N*-{3',4'-dihydro-1'*H*-spiro[cyclopropane-1,2'-naphthalen]-5'-yl}-4-(2-hydroxyethanesulfonylamino)benzamide (12 mg, 24.01%).

MS (ESI, *m/z*): 510.55 [M+H] $^+$. RT (min): 1.926.

$^1$H NMR (400 MHz, DMSO-$d_6$) δ 10.93 (s, 1H), 10.08 (s, 1H), 7.86 (d, 1H), 7.65 (d, 1H), 7.18 (d, 1H), 7.12 (t, 1H), 7.02 (dd, 1H), 6.88 (d, 1H), 4.95 (s, 1H), 3.76 (t, 2H), 3.36 - 3.30 (m, 2H), 3.00 (t, 4H), 2.79 (t, 2H), 2.66 (s, 2H), 1.57 (t, 2H), 1.47 (s, 4H), 0.39 (s, 4H), 0.33 (s, 4H).

Example 7

*N*-(8,8-difluoro-4-methyl-pyrido[2,3-d]imidazo[1,2-a]piperidin-2-yl)-4-(2-hydroxyethanesulfonylamino)-2-(6-azaspiro [2.5]octan-6-yl)benzamide (compound 64)

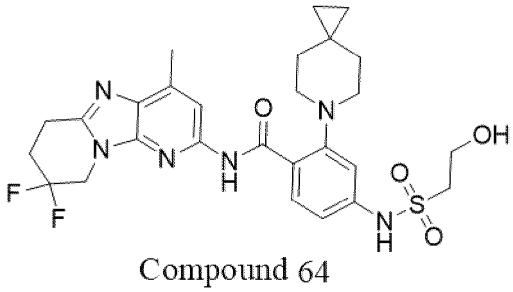

Compound 64

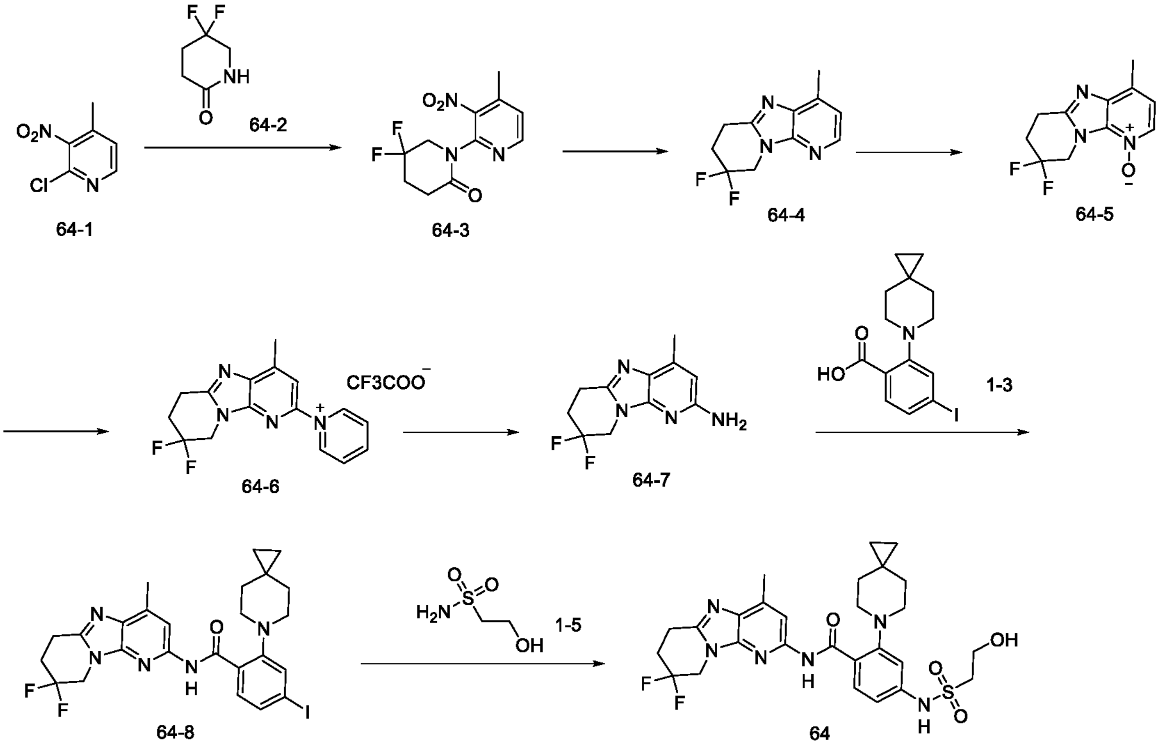


Step 1 Synthesis of 5,5-difluoro-1-(4-methyl-3-nitropyridin-2-yl)piperidin-2-one (compound 64-3):

Under nitrogen protection, palladium acetate (156.12 mg, 0.695 mmol, 0.05 eq), 4,5-bis(diphenylphosphino-9,9-dimethylxanthene) (804.73 mg, 1.391 mmol, 0.1 eq) and cesium carbonate (6.80 g, 20.861 mmol, 1.5 eq) were added to a solution of 2-chloro-4-methyl-3-nitropyridine (2.4 g, 13.907 mmol, 1 eq) and 5,5-difluoropiperidin-2-one (2.25 g, 16.688 mmol, 1.2 eq) in 1,4-dioxane (50 mL) at room temperature. The reaction mixture was heated to 100°C, and stirred for 16 h. The reaction mixture was cooled to room temperature and concentrated under reduced pressure. The resulting residue was purified by silica gel column chromatography with n-hexane/ethyl acetate (6:1) to obtain 5,5-difluoro-1-(4-methyl-3-nitropyridin-2-yl)piperidin-2-one (compound 64-3, 980 mg, 25.98%).

MS (ESI, *m/z*): 272.30 [M+H]$^+$. RT(min): 0.786

Step 2 Synthesis of 8,8-difluoro-4-methyl-pyrido[2,3-d]imidazo[1,2-a]piperidine (compound 64-4):

To a solution of 5,5-difluoro-1-(4-methyl-3-nitropyridin-2-yl)piperidin-2-one (1.06 g, 3.908 mmol, 1 eq) in ethanol and water (10 mL : 1 mL) were added iron powder (12.35 mg, 0.222 mmol, 3 eq) and ammonium chloride (0.13 g, 2.345 mmol, 0.6 eq) at room temperature. The reaction mixture was heated to 80°C and stirred for 16 h. The reaction mixture was cooled to room temperature, filtered, and the filtrate was concentrated to dryness. The resulting residue was purified by silica gel column chromatography with petroleum ether/ethyl acetate (4:1) to obtain 8,8-difluoro-4-methyl-pyrido[2,3-d]imidazo[1,2-a]piperidine (compound 64-4, 550 mg, 63.04%).

MS (ESI, *m/z*): 224.30 $[M+H]^+$. RT (min): 0.666

Step 3 Synthesis of 8,8-difluoro-4-methyl-pyrido[2,3-d]imidazo[1,2-a]piperidine-1-oxide (compound 64-5):

To a solution of 8,8-difluoro-4-methyl-pyrido[2,3-d]imidazo[1,2-a]piperidine (500 mg, 1.843 mmol, 1 eq) in dichloromethane (5 mL) was added meta-chloroperbenzoic acid (561.38 mg, 2.764 mmol, 1.5 eq, 85%) at room temperature. The mixture was stirred at room temperature for 16 h. The reaction mixture was directly concentrated under reduced pressure. The resulting residue was purified by silica gel column chromatography with petroleum ether/ethyl acetate (1:3) to give 8,8-difluoro-4-methyl-pyrido[2,3-d]imidazo[1,2-a]piperidine-1-oxide (compound 64-5, 220 mg, 53.46%).

MS (ESI, *m/z*): 240.30 $[M+H]^+$. RT (min): 0.585

Step 4 Synthesis of 1-(8,8-difluoro-4-methyl-pyrido[2,3-d]imidazo[1,2-a]piperidin-2-yl)pyridine-1-trifluoroacetate (compound 64-6):

Pyridine (0.37 mL, 4.600 mmol, 5 eq) was added to a solution of 8,8-difluoro-4-methyl-pyrido[2,3-d]imidazo[1,2-a]piperidine-1-oxide (220 mg, 0.920 mmol, 1 eq) in dichloromethane (4 mL) at room temperature. Trifluoroacetic anhydride (0.38 mL, 2.760 mmol, 3 eq) was added dropwise at 0°C. The reaction mixture was heated to room temperature and stirred for 16 h. The resulting mixture was used directly without further purification.

MS (ESI, *m/z*): 301.30 $[M-CF_3COO^-]^+$. RT (min): 0.501

Step 5 Synthesis of 8,8-difluoro-4-methylpyrido[2,3-d]imidazo[1,2-a]piperidin-2-amine (compound 64-7):

To a solution of 1-(8,8-difluoro-4-methyl-pyrido[2,3-d]imidazo[1,2-a]piperidin-2-yl)pyridine-1-trifluoroacetate (220 mg, 0.53 mmol, 1 eq) in ethanol (3 mL) was added ethanolamine (0.32 mL, 5.310 mmol, 10 eq) at room temperature. The reaction mixture was heated to 80°C and stirred for 16 h. The reaction mixture was concentrated under reduced pressure and the resulting residue was purified by silica gel column chromatography with petroleum ether/ethyl acetate (2:1) to give 8,8-difluoro-4-methylpyrido[2,3-d]imidazo[1,2-a]piperidin-2-amine (compound 64-7, 75 mg).

MS (ESI, *m/z*): 239.35 $[M+H]^+$. RT (min): 0.475.

Step 6 Synthesis of *N*-(8,8-difluoro-4-methyl-pyrido[2,3-d]imidazo[1,2-a]piperidin-2-yl)-4-iodo-2-(6-azaspiro[2.5]octan-6-yl)benzamide (compound 64-8):

Under nitrogen protection, *N*-methylimidazole (0.25 mL, 3.150 mmol, 10 eq) was added to a solution of 4-iodo-2-(6-azaspiro[2.5]octan-6-yl)benzoic acid (112.45 mg, 0.315 mmol, 1.00eq), 8,8-difluoro-4-methylpyrido[2,3-d]imidazo[1,2-a]piperidin-2-amine (75 mg, 0.315 mmol, 1.00 eq), and tetramethylchloroformamidine hexafluorophosphate (353.31 mg, 1.260 mmol, 4 eq) in dichloromethane (4 mL) at room temperature. The reaction mixture was heated to 60°C and allowed to react for 2 h. The reaction mixture was cooled to room temperature, diluted with water (10 mL), and extracted with ethyl acetate (3 × 10 mL). The organic phases were combined, washed with saturated brine (2 × 10 mL), dried over anhydrous sodium sulfate, filtered, and the filtrate was concentrated under reduced pressure. The resulting residue was purified by preparative chromatography with petroleum ether/ethyl acetate (6:1) to give *N*-(8,8-difluoro-4-methyl-pyrido[2,3-d]imidazo[1,2-a]piperidin-2-yl)-4-iodo-2-(6-azaspiro[2.5]octan-6-yl)benzamide (compound 64-8, 30 mg, 16.50%).

MS (ESI, *m/z*): 578.30 $[M+H]^+$. RT (min): 1.432

Step 7 Synthesis of *N*-(8,8-difluoro-4-methyl-pyrido[2,3-d]imidazo[1,2-a]piperidin-2-yl)-4-(2-hydroxyethanesulfonylamino)-2-(6-azaspiro[2.5]octan-6-yl)benzamide (compound 64):

Under nitrogen protection, cuprous iodide (0.99 mg, 0.005 mmol, 0.1 eq) was added to a solution of *N*-(8,8-difluoro-4-methylpyrido[2,3-d]imidazo[1,2-a]piperidin-2-yl)-4-iodo-2-(6-azaspiro[2.5]octan-6-yl)benzamide (30 mg,

0.052 mmol, 1 eq), 2-hydroxyethylsulfonamide (7.80 mg, 0.062 mmol, 1.2 eq), sarcosine (0.93 mg, 0.010 mmol, 0.2 eq) and potassium carbonate (21.54 mg, 0.156 mmol, 3 eq) in dimethylformamide (0.8 mL) at room temperature. The reaction mixture was heated to 120°C and allowed to react for 1 h. The reaction mixture was cooled to room temperature, diluted with water (10 mL), extracted with ethyl acetate (3 × 10 mL). The organic phases were combined, washed with saturated brine (2 × 10 mL), dried over anhydrous sodium sulfate, filtered, and the filtrate was concentrated under reduced pressure. The resultant residue was purified by HPLC under the following conditions (chromatographic column specifications: Kinetex EVO prep C18, 30*150, 5μm; mobile phase A: water (10 mmol/L ammonium bicarbonate); mobile phase B: acetonitrile; flow rate: 60 mL/min; elution gradient: 20% B to 55% B, 8 min, 55% B; detection wavelength: 220 nm; retention time (min): 7.57). N-(8,8-difluoro-4-methylpyrido[2,3-d]imidazo[1,2-a]piperidin-2-yl)-4-(2-hydroxyethanesulfonylamino)-2-(6-azaspiro[2.5]octan-6-yl)benzamide (compound 64, 2.24 mg, 7.46%) was obtained.

MS (ESI, *m/z*): 575.35 $[M+H]^+$. RT (min): 2.150

$^1H$ NMR (400 MHz, DMSO-$d_6$) δ 13.57 (s, 1H), 8.11 - 8.02 (m, 2H), 7.25 (s, 1H), 7.11 (d, 1H), 4.47 (t, 2H), 3.76 (m, 4H), 3.33(m, 2H), 3.19 (m, 2H), 2.99 (t, 4H), 2.61 (m, 2H), 2.56 (s, 3H), 1.81 (s, 4H), 0.40 (s, 4H).

Example 8

*N*-(3,3-difluoro-2,3-dihydro-1*H*-benzo[d]pyrrolo[1,2-a]imidazol-5-yl)-4-(2-hydroxyethanesulfonylamino)-2-(6-azaspiro[2.5]octan-6-yl)benzamide (compound 94)

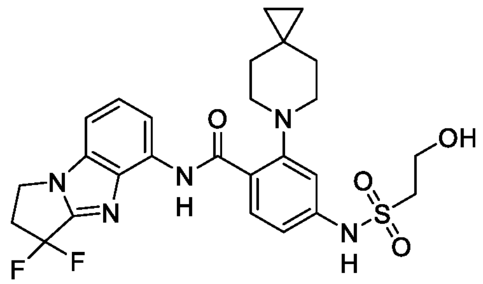

Compound 94

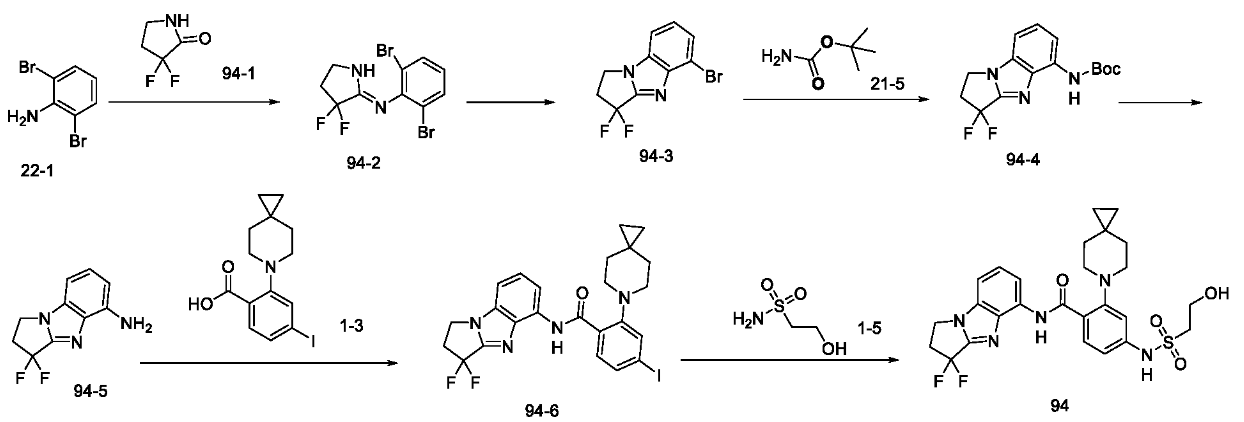


Step 1 Synthesis of (2*Z*)-*N*-(2,6-dibromophenyl)-3,3-difluoropyrrolidin-2-imine (compound 94-2):

Under nitrogen protection, $POCl_3$ (837.11 mg, 5.460 mmol, 1 eq) was added to a solution of 3,3-difluoropyrrolidin-2-one (0.99 g, 8.176 mmol, 1.50 eq) in dry toluene (15 mL) at 0°C, and the reaction mixture was stirred at room temperature for 2 h. To the above solution was added a solution of 2,6-dibromoaniline (1.37 g, 5.460 mmol, 1 eq) in toluene (5 mL), and the reaction mixture was heated to 110°C and stirred for 16 h. The reaction mixture was cooled to room temperature and quenched with water. The reaction mixture was extracted with dichloromethane (3 × 60 mL). The organic phases were combined, washed with saturated sodium chloride solution (1 × 80mL), and dried over anhydrous sodium sulfate. The system was filtered, and the filtrate was concentrated under reduced pressure. The resulting residue was

recrystallized with petroleum ether/dichloromethane (100:1) to give (2Z)-N-(2,6-dibromophenyl)-3,3-difluoropyrrolidin-2-imine (1.2 g, 62.09%).

MS (ESI, m/z): 352.85 $[M+H]^+$, RT(min):0.674

Step 2 Synthesis of 5-bromo-3,3-difluoro-2,3-dihydro-1*H*-benzo[d]pyrrolo[1,2-a]imidazole (compound 94-3):

Under nitrogen protection, to a solution of (2*Z*)-*N*-(2,6-dibromophenyl)-3,3-difluoropyrrolidin-2-imine (500 mg, 1.412 mmol, 1 eq) in *N,N*-dimethylformamide (10.00 mL) were added *N,N*-dimethylethylenediamine (12.45 mg, 0.141 mmol, 0.1 eq), anhydrous potassium carbonate (195.21 mg, 1.412 mmol, 1 eq) and cuprous iodide (13.45 mg, 0.071 mmol, 0.05 eq) at room temperature. The reaction mixture was heated to 100°C, and stirred for 1 h. The reaction mixture was cooled to room temperature, quenched with water, and extracted with dichloromethane (3 × 50 mL). The organic phases were combined, washed with saturated aqueous brine solution (1 × 50 mL), and dried over anhydrous sodium sulfate. The mixture was filtered and the filtrate was concentrated under reduced pressure. The resulting residue was purified by silica gel column chromatography with petroleum ether/ethyl acetate (2:1) to give 5-bromo-3,3-difluoro-2,3-dihydro-1*H*-benzo[d]pyrrolo[1,2-a]imidazole (240 mg, 62.22%).

MS (ESI, *m*/*z*):272.90 $[M+H]^+$, RT(min):0.837

Step 3 Synthesis of tert-butyl (3,3-difluoro-2,3-dihydro-1*H*-benzo[d]pyrrolo[1,2-a]imidazol-5-yl)carbamate (compound 94-4):

Under nitrogen protection, to a solution of 5-bromo-3,3-difluoro-2,3-dihydro-1H-benzo[d]pyrrolo[1,2-a]imidazole (230 mg, 0.842 mmol, 1 eq) in *N,N*-dimethylformamide (5.00 mL) were added tert-butyl carbamate (197.33 mg, 1.684 mmol, 2 eq), (methanesulfonic acid{biscyclohexyl(3-isopropoxy-2',4',6'-triisopropyl-[1,1'-biphenyl]-2-yl)phosphine}(2'-methylamino-1,1'-biphenyl-2-yl)palladium(II) (154.73 mg, 0.168 mmol, 0.20 eq), dicyclohexyl(3-isopropyl-2',4',6'-triisopropyl-[1,1'-biphenyl]-2-yl)phosphine (45.04 mg, 0.084 mmol, 0.10 eq), cesium carbonate (823.25 mg, 2.526 mmol, 3.00 eq) at room temperature. The reaction mixture was heated to 100°C and stirred for 1 h. The reaction was complete as monitored by LC-MS. The reaction mixture was extracted with ethyl acetate (3 × 50 mL). The organic phases were combined, washed with saturated sodium chloride solution (1 × 50 mL), and dried over anhydrous sodium sulfate. The mixture was filtered and the filtrate was concentrated under reduced pressure. The resulting residue was purified by silica gel column chromatography with petroleum ether/ethyl acetate (2:1) to give tert-butyl (3,3-difluoro-2,3-dihydro-1*H*-benzo[d]pyrrolo[1,2-a]imidazol-5-yl)carbamate (189 mg, 72.55%).

MS (ESI, m/z): 310.05 $[M+H]^+$,RT(min):1.004

Step 4 Synthesis of 3,3-difluoro-2,3-dihydro-1*H*-benzo[d]pyrrolo[1,2-a]imidazol-5-amine (compound 94-5):

Under nitrogen protection, a solution of hydrochloric acid in 1,4-dioxane (2 mL) was added to a solution of tert-butyl (3,3-difluoro-2,3-dihydro-1*H*-benzo[d]pyrrolo[1,2-a]imidazol-5-yl)carbamate (189 mg, 0.611 mmol, 1 eq) in dichloromethane (2 mL) at room temperature, and the reaction mixture was stirred at room temperature for 1 h. The reaction mixture was concentrated under reduced pressure. 3,3-difluoro-2,3-dihydro-1*H*-benzo[d]pyrrolo[1,2-a]imidazol-5-amine (127 mg, 99.35%) was obtained.

MS (ESI, *m*/*z*):210.10 $[M+H]^+$,RT(min):0.193

Step 5 Synthesis of *N*-(3,3-difluoro-2,3-dihydro-1*H*-benzo[d]pyrrolo[1,2-a]imidazol-5-yl)-4-iodo-2-(6-azaspiro[2.5]octan-6-yl)benzamide (compound 94-6):

Under nitrogen protection, 3,3-difluoro-2,3-dihydro-1H-benzo[d]pyrrolo[1,2-a]imidazol-5-amine (100 mg, 0.478 mmol, 1 eq), tetramethylchloroformamidine hexafluorophosphate (536.48 mg, 1.912 mmol, 4 eq) and *N*-methylimidazole (392.48 mg, 4.780 mmol, 10 eq) were added to a solution of 2-{6-azaspiro[2.5]octan-6-yl}-4-iodobenzoic acid (204.89 mg, 0.574 mmol, 1.2 eq) in dichloromethane (10.00 mL) at room temperature. The reaction mixture was heated to 50°C and stirred for 1 h. The reaction mixture was cooled to room temperature, diluted with water, and extracted with dichloromethane (3 × 50 mL). The organic phases were combined, washed with saturated saline (1 × 50 mL), dried over anhydrous sodium sulfate, filtered, and the filtrate was concentrated under reduced pressure. The resulting residue was purified by silica gel column chromatography with petroleum ether/ethyl acetate (4:1) to give *N*-(3,3-difluoro-2,3-dihydro-1*H*-benzo[d]pyrrolo[1,2-a]imidazol-5-yl)-4-iodo-2-(6-azaspiro[2.5]octan-6-yl)benzamide (150 mg, 57.22%).

MS (ESI, *m*/*z*):549.05 $[M+H]^+$,RT(min):1.311

Step 6 Synthesis of *N*-(3,3-difluoro-2,3-dihydro-1*H*-benzo[d]pyrrolo[1,2-a]imidazol-5-yl)-4-(2-hydroxyethanesulfonylamino)-2-(6-azaspiro[2.5]octan-6-yl)benzamide (compound 94):

Under nitrogen protection, to a solution of *N*-(3,3-difluoro-2,3-dihydro-1*H*-benzo[d]pyrrolo[1,2-a]imidazol-5-yl)-4-iodo-2-(6-azaspiro[2.5]octan-6-yl)benzamide (100 mg, 0.182 mmol, 1 eq) in *N,N*-dimethylformamide (20.00 mL) were added 2-hydroxyethanesulfonamide (27.38 mg, 0.218 mmol, 1.2 eq), sarcosine (3.25 mg, 0.036 mmol, 0.2 eq), cuprous iodide (3.47 mg, 0.018 mmol, 0.1 eq) and potassium carbonate (75.61 mg, 0.55 mmol, 3 eq) at room temperature. The reaction mixture was heated to 140°C and stirred for 1 h. The reaction mixture was cooled to room temperature, quenched with water, and extracted with ethyl acetate (3 × 30 mL). The organic phases were combined, washed with saturated aqueous brine solution (1 × 30 mL), and dried over anhydrous sodium sulfate. The mixture was filtered and the filtrate was concentrated under reduced pressure. The resulting residue was purified by preparative HPLC under the following conditions (chromatographic column specifications: Kinetex EVO prep C18, 30*150, 5μm; mobile phase A: water (10 mmol/L sodium bicarbonate), mobile phase B: acetonitrile; flow rate: 60 mL/min; elution gradient: 20% B to 65% B in 8 min, 65% B; detection wavelength: 220 nm; retention time (min): 7.45). *N*-(3,3-difluoro-2,3-dihydro-1*H*-benzo[d]pyrrolo[1,2-a]imidazol-5-yl)-4-(2-hydroxyethylsulfonylamino)-2-(6-azaspiro[2.5]octan-6-yl)benzamide (23.00 mg, 23.12%) was obtained.

MS (ESI, *m/z*):546.30 $[M+H]^+$,RT(min):1.621

$^1H$ NMR (400 MHz, DMSO-$d_6$) δ 12.55 (s, 1H), 10.16 (s, 1H), 8.47 (m, 1H), 8.04 (d, 1H), 7.45 - 7.33 (m, 2H), 7.28 (d, 1H), 7.09 (m, 1H), 4.92 (s, 1H), 4.66 - 4.34 (m, 2H), 3.76 (t, 2H), 3.43 - 3.32 (m, 2H), 3.01 (d, 4H), 1.77 (s, 4H), 1.23 (s, 2H), 0.27 (s, 4H).

The compounds in the following table were prepared using conditions similar to those in Example 8.

| Compound No. | Structure | Name | $[M+H]^+$ | $^1H$-NMR data |
|---|---|---|---|---|
| 106 | 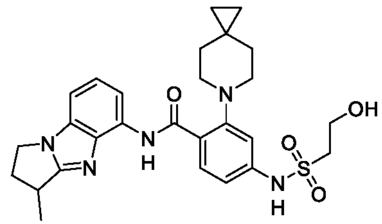 | 4-(2-hydroxyethanesulfonylamino) -*N*-(3-methyl-2,3-dihydro-1H-benzo [d]pyrrolo[1,2-a]imidazol-5-yl)-2-(6-azaspiro[2.5]octan-6-yl)benzamide | 524.45 | $^1H$ NMR (400 MHz, DMSO-$d_6$) $\delta$ 12.29 (s, 1H), 10.13 (s, 1H), 8.40 - 8.32 (m, 1H), 8.03 (d, 1H), 7.26 (d, 1H), 7.19 - 7.11 (m, 2H), 7.11 - 7.05 (m, 1H), 4.93 (s, 1H), 4.28 - 4.16 (m, 1H), 4.12 - 3.98 (m, 1H), 3.76 (t, 2H), 3.35 (t, 3H), 3.09 - 2.94 (m, 4H), 2.92 - 2.82 (m, 1H), 2.33 - 2.20 (m, 1H), 1.79 (s, 4H), 1.42 (d, 3H), 0.29 (d, 4H) |
| 107 | 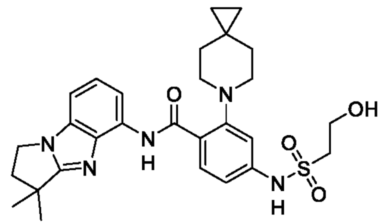 | *N*-(3,3-dimethyl-2,3-dihydro-1H-benzo[d]pyrrolo[1,2-a]imidazol-5-yl) 4-(2-hydroxyethanesulfonylamino) -2-(6-azaspiro[2.5]octan-6-yl)benzamide | 538.50 | $^1H$ NMR: (400 MHz, DMSO-$d_6$) $\delta$ 12.28 (s, 1H), 10.12 (s, 1H), 8.37 (dd, 1H), 8.03 (d, 1H), 7.26 (d, 1H), 7.20 - 7.10 (m, 2H), 7.07 (dd, 1H), 4.92 (s, 1H), 4.16 (t, 2H), 3.76 (t, 2H), 3.31 (s, 2H), 3.01 (s, 4H), 2.50 (s, 2H), 1.91 (s, 4H), 1.43 (s, 6H), 0.30 (s, 4H) |

Example 9

4-(2-hydroxyethanesulfonylamino)-2-(6-azaspiro[2.5]octan-6-yl)-*N*-(1,2,3,4-tetrahydro-1,4-methylenebenzo[4,5]imidazo[1,2-a]pyridin-6-yl)benzamide (compound 96)

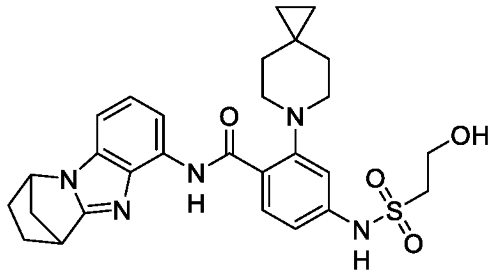

Compound 96

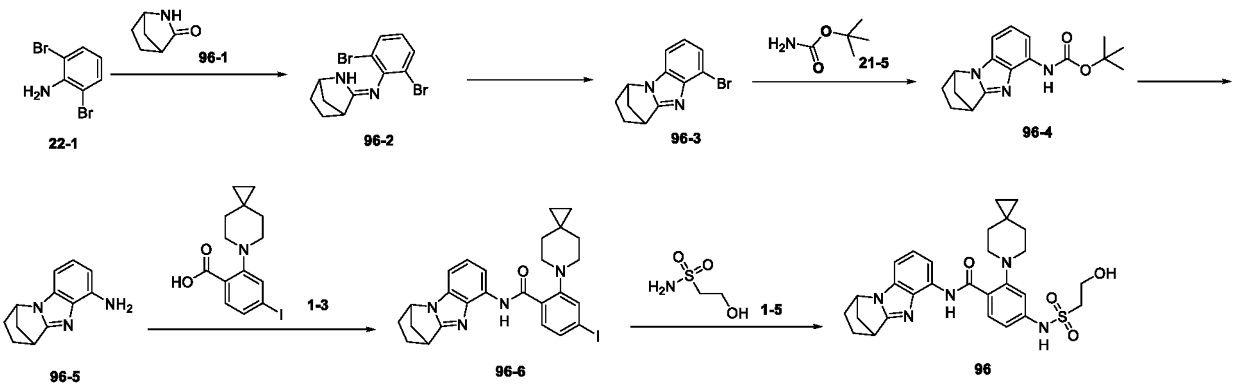


Step 1 Synthesis of (3*Z*)-*N*-(2,6-dibromophenyl)-2-azabicyclo[2.2.1]heptan-3-imine (compound 96-2):

Under nitrogen protection, phosphorus oxychloride (1833.09 mg, 11.955 mmol, 3 eq) was added to a solution of 2-azabicyclo[2.2.1]heptan-3-one (885.89 mg, 7.970 mmol, 2 eq) in anhydrous toluene (15 mL) at 0°C, and the reaction mixture was stirred at room temperature for 2 h. To the above solution was added a solution of 2,6-dibromoaniline (1 g, 3.985 mmol, 1 eq) in toluene (5 mL), and the reaction mixture was heated to 100°C and stirred for 16 h. The reaction mixture was cooled to room temperature, and quenched with water (30 mL). The reaction mixture was extracted with ethyl acetate (3 × 80 mL). The organic phases were combined, washed with saturated sodium chloride solution (1 × 80mL), dried over anhydrous sodium sulfate, and filtered, and the filtrate was concentrated under reduced pressure. The resulting residue was purified by silica gel column chromatography with petroleum ether/ethyl acetate (3:1) to give (3Z)-N-(2,6-dibromophenyl)-2-azabicyclo[2.2.1]heptan-3-imine (190 mg, 13.86%).

MS (ESI, *m/z):* 344.80 $[M+H]^+$, RT(min):0.592.

Step 2 Synthesis of 6-bromo-1,2,3,4-tetrahydro-1,4-methylenebenzo[4,5]imidazo[1,2-a]pyridine (compound 96-3):

Under nitrogen protection, to a solution of (3Z)-N-(2,6-dibromophenyl)-2-azabicyclo[2.2.1]heptan-3-imine (190 mg, 0.553 mmol, 1 eq) in acetonitrile (5.0 mL) were added N,N-dimethylethylenediamine (4.87 mg, 0.055 mmol, 0.1 eq), anhydrous potassium carbonate (76.22 mg, 0.553 mmol, 1 eq) and cuprous iodide (5.26 mg, 0.028 mmol, 0.05 eq) at room temperature. The reaction mixture was heated to 100°C and stirred for 1 h. The reaction mixture was cooled to room temperature, quenched with water, and extracted with ethyl acetate (3 × 50 mL). The organic phases were combined, washed with saturated aqueous brine solution (1 × 50 mL) and dried over anhydrous sodium sulfate. The mixture was filtered and the filtrate was concentrated under reduced pressure. 6-bromo-1,2,3,4-tetrahydro-1,4-methylenebenzo[4,5]imidazo[1,2-a]pyridine (140 mg, 96.55%) was obtained.

MS (ESI, *m/z*):263.00 $[M+H]^+$, RT(min):0.602.

Step 3 Synthesis of tert-butyl (1,2,3,4-tetrahydro-1,4-methylenebenzo[4,5]imidazo[1,2-a]pyridin-6-yl)carbamate (compound 96-4):

Under nitrogen protection, to a solution of 6-bromo-1,2,3,4-tetrahydro-1,4-methylenebenzo[4,5]imidazo[1,2-a]pyridine (130 mg, 0.494 mmol, 1 eq) in *N,N*-dimethylformamide (5.0 mL) were added tert-butyl carbamate (115.75 mg, 0.988 mmol, 2 eq), (methanesulfonic acid{biscyclohexyl(3-isopropoxy-2',4',6'-triisopropyl-[1,1'-biphenyl]-2-yl)phosphine}(2'-methylamino-1,1'-biphenyl-2-yl)palladium(II) (45.38 mg, 0.049 mmol, 0.1 eq), dicyclohexyl(3-isopropyl-2',4',6'-triisopropyl-[1,1'-biphenyl]-2-yl)phosphine (52.84 mg, 0.099 mmol, 0.2 eq), cesium carbonate (482.9 mg, 1.482 mmol, 3 eq) at room temperature. The reaction mixture was heated to 100°C and stirred for 1 h. The reaction mixture was cooled to room temperature and extracted with ethyl acetate (3 × 50 mL). The organic phases were combined, washed with saturated sodium chloride solution (1 × 50 mL), and dried over anhydrous sodium sulfate. The mixture was filtered and the filtrate was concentrated under reduced pressure. The resulting residue was purified by silica gel column chromatography with petroleum ether/ethyl acetate (5:4) to give tert-butyl (1,2,3,4-tetrahydro-1,4-methylenebenzo[4,5]imidazo[1,2-a]pyridin-6-yl)carbamate (94 mg, 63.56%).

MS (ESI, *m/z):* 300.10 $[M+H]^+$, RT(min):0.730.

Step 4 Synthesis of 1,2,3,4-tetrahydro-1,4-methylenebenzo[4,5]imidazo[1,2-a]pyridin-6-amine (compound 96-5):

To a solution of tert-butyl (1,2,3,4-tetrahydro-1,4-methylenebenzo[4,5]imidazo[1,2-a]pyridin-6-yl)carbamate (94 mg, 0.314 mmol, 1 eq) in dichloromethane (2 mL) was added a solution of hydrochloric acid in 1,4-dioxane (2 mL) at room temperature, and the reaction mixture was stirred at room temperature for 1 h. The reaction mixture was concentrated under reduced pressure. 1,2,3,4-tetrahydro-1,4-methylenebenzo[4,5]imidazo[1,2-a]pyridin-6-amine (62 mg, 99.10%) was obtained.

MS (ESI, *m/z*):200.00 $[M+H]^+$, RT(min):0.166

Step 5 Synthesis of 4-iodo-2-(6-azaspiro[2.5]octan-6-yl)-*N*-(1,2,3,4-tetrahydro-1,4-methylenebenzo[4,5]imidazo[1,2-a]pyridin-6-yl)benzamide (compound 96-6):

Under nitrogen protection, tetramethylchloroformamidine hexafluorophosphate (337.95 mg, 1.204 mmol, 4 eq) and *N*-methylimidazole (247.24 mg, 3.010 mmol, 10 eq) were added to a solution of 1,2,3,4-tetrahydro-1,4-methylenebenzo[4,5]imidazo[1,2-a]pyridin-6-amine (60 mg, 0.301 mmol, 1 eq) and 2-{6-azaspiro[2.5]octan-6-yl}-4-iodobenzoic acid (129.07 mg, 0.361 mmol, 1.20 eq) in dichloromethane (10.0 mL) at room temperature, and the reaction mixture was stirred at room temperature for 1 h. The reaction mixture was extracted with dichloromethane (3 × 50 mL). The organic phases were combined, washed with saturated saline (1 × 50 mL), dried over anhydrous sulfuric acid, filtered, and the filtrate was concentrated under reduced pressure. The resulting residue was purified by silica gel column chromatography with petroleum ether/ethyl acetate (5:2) to obtain 4-iodo-2-(6-azaspiro[2.5]octan-6-yl)-*N*-(1,2,3,4-tetrahydro-1,4-methylenebenzo[4,5]imidazo[1,2-a]pyridin-6-yl)benzamide (70 mg, 43.17%).

MS (ESI, *m/z*):539.35 $[M+H]^+$,RT(min):1.495.

Step 6 Synthesis of 4-(2-hydroxyethanesulfonylamino)-2-(6-azaspiro[2.5]octan-6-yl)-*N*-(1,2,3,4-tetrahydro-1,4-methylenebenzo[4,5]imidazo[1,2-a]pyridin-6-yl)benzamide (compound 96):

Under nitrogen protection, to a solution of 4-iodo-2-(6-azaspiro[2.5]octan-6-yl)-*N*-(1,2,3,4-tetrahydro-1,4-methylenebenzo[4,5]imidazo[1,2-a]pyridin-6-yl)benzamide (65 mg, 0.121 mmol, 1 eq), 2-hydroxyethanesulfonamide (30.21 mg, 0.242 mmol, 2.0 eq) and potassium carbonate (50.05 mg, 0.363 mmol, 3 eq) in N,N-dimethylformamide (5.0 mL) were added sarcosine (2.16 mg, 0.024 mmol, 0.2 eq) and cuprous iodide (2.3 mg, 0.012 mmol, 0.1 eq) at room temperature. The reaction mixture was heated to 120°C and stirred for 1 h. The reaction mixture was cooled to room temperature, quenched with water and extracted with ethyl acetate (3 × 30 mL). The organic phases were combined, washed with saturated aqueous brine solution (1 × 30 mL) and dried over anhydrous sodium sulfate. The mixture was filtered and the filtrate was concentrated under reduced pressure. The resulting residue was purified by preparative HPLC under the following conditions (chromatographic column specifications: Kinetex EVO prep C18, 30*150, 5μm; mobile phase A: water (10 mmol/L sodium bicarbonate), mobile phase B: acetonitrile; flow rate: 60 mL/min; elution gradient: 20% B to 65% B in 8 min, 65% B; detection wavelength: UV 220 nm; retention time (min): 7.45). 4-(2-hydroxyethanesulfonylamino)-2-(6-azaspiro[2.5]octan-6-yl)-*N*-(1,2,3,4-tetrahydro-1,4-methylenebenzo[4,5]imidazo[1,2-a]pyridin-6-yl)benzamide (16.99 mg, 25.96%) was obtained.

MS (ESI, *m/z*):536.00 $[M+H]^+$, RT(min): 1.488.

$^1$H NMR (400 MHz, DMSO-$d_6$) δ 12.32 (s, 1H), 10.13 (s, 1H), 8.28 (d, 1H), 8.00 (d, *J* = 8.6 Hz, 1H), 7.31 - 7.18 (m, 2H), 7.12 (t, 1H), 7.06 (m, 1H), 5.15 (s, 1H), 4.94 (s, 1H), 3.76 (t, 2H), 3.59 (d, 1H), 3.35 (d, 2H), 3.08 - 2.83 (m, 4H),

2.25-2.23 (m, 1H), 2.16-2.10 (m, 1H), 2.02-1.95 (m, 2H), 1.75 (s, 4H), 1.19-1.13 (m, 2H), 0.29 (s, 4H).

The compounds in the following table were prepared using conditions similar to those in Example 9.

| Compound No. | Structure | Name | [M+H]+ | 1H-NMR data |
|---|---|---|---|---|
| 96a | 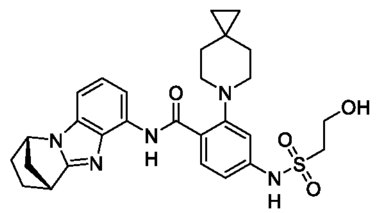 | 4-(2-hydroxyethanesulfonylamino)-2-(6-azaspiro[2.5]octan-6-yl)-*N*-((1*S*,4*R*)-1,2,3,4-tetrahydro-1,4-methylenebenzo[4,5]imidazo [1,2-a]pyridin-6-yl)benzamide | 536.55 | $^1$H NMR (400 MHz, DMSO-$d_6$) $\delta$ 12.31 (s, 1H), 10.13 (s, 1H), 8.28 (d, 1H), 8.00 (d, 1H), 7.30 - 7.21 (m, 2H), 7.12 (t, 1H), 7.07 (dd, 1H), 5.14 (s, 1H), 4.93 (s, 1H), 3.76 (t, 2H), 3.59 (d, 1H), 3.34 (t, 2H), 2.98 (q, 4H), 2.24 (d, 1H), 2.13 (td, 1H), 1.98 (dd, 2H), 1.82 (s, 4H), 1.16 (t, 2H), 0.29 (s, 4H) |
| 96b | 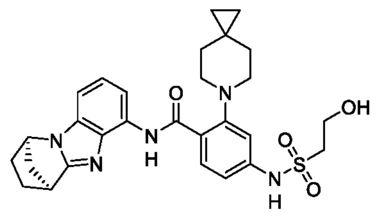 | 4-(2-hydroxyethanesulfonylamino)-2-(6-azaspiro[2.5]octan-6-yl)-*N*-((1*R*,4*S*)-1,2,3,4-tetrahydro-1,4-methylenebenzo[4,5]imidazo [1,2-a]pyridin-6-yl)benzamide | 536.45 | $^1$H NMR (400 MHz, DMSO-$d_6$) $\delta$ 12.32 (s, 1H), 10.14 (s, 1H), 8.28 (m, 1H), 8.01 (d, 1H), 7.28 - 7.21 (m, 2H), 7.13 (t, 2H), 7.07 (m, 1H), 5.15 (d, 1H), 4.94 (s, 1H), 3.76 (t, 2H), 3.59 (d, 1H), 3.36 (t, 2H), 2.98 (m, 4H), 2.24 (d, 1H), 2.13 (m, 1H), 1.98 (m, 2H), 1.74 (s, 4H), 1.20 - 1.02 (m, 2H), 0.29 (s, 4H) |

Example 10

4-(2-hydroxyethanesulfonylamino)-2-(6-azaspiro[2.5]octan-6-yl)-*N*-(7,8,9,10-tetrahydro-6*H*-benzo[4,5]imidazo[1,2-a]azepin-4-yl)benzamide (compound 97)

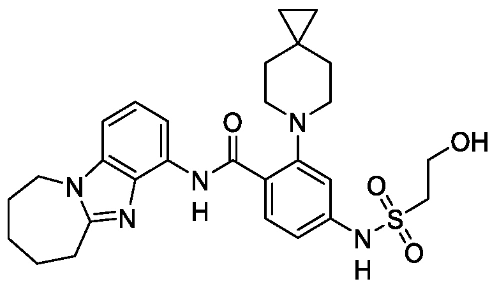

Compound 97

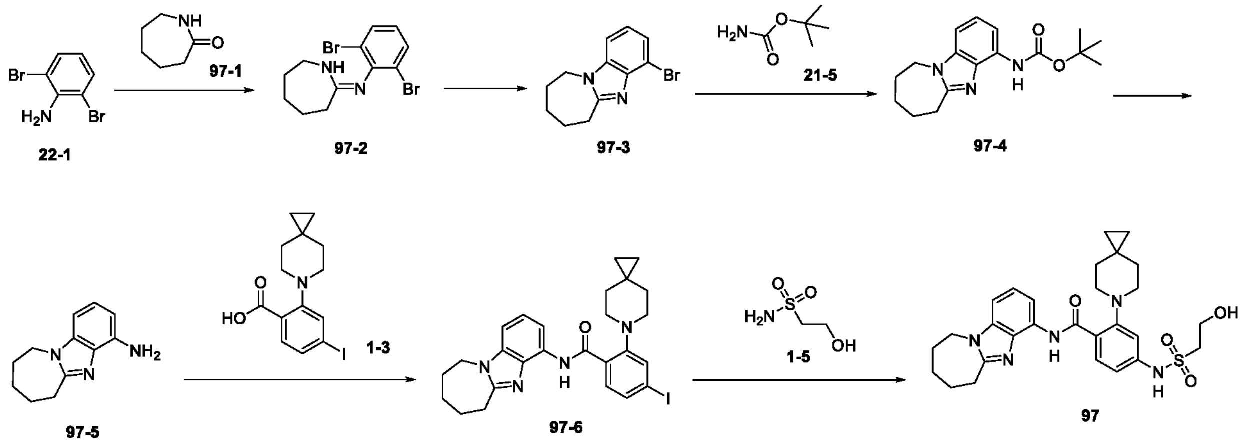


Step 1 Synthesis of (2*Z*)-*N*-(2,6-dibromoanilino)azepin-2-imine (compound 97-2):

Under nitrogen protection, phosphorus oxychloride (6.1 g, 39.85 mmol, 5 eq) was added dropwise to a solution of caprolactam (1.36 g, 15.94 mmol, 2 eq) in toluene (10 mL) at 0°C, and the reaction mixture was allowed to react at 0°C for 2 h. Then, a solution of 2,6-dibromoaniline (2 g, 7.97 mmol, 1 eq) in toluene (5 mL) was added, and the reaction mixture was heated to 110°C and stirred for 16 h. The reaction mixture was cooled to room temperature, concentrated under reduced pressure, quenched with ice water (50 mL), adjusted to pH 10 with saturated sodium bicarbonate solution, and the mixture was extracted with ethyl acetate (3 × 60 mL). The organic phases were combined, washed with saturated brine (1 × 50 mL) and dried over anhydrous sodium sulfate. The mixture was filtered and the filtrate was concentrated under reduced pressure. The resulting residue was purified by silica gel column chromatography with petroleum ether/ethyl acetate (10:1) to give (2*Z*)-*N*-(2,6-dibromoanilino)azepin-2-imine (2.7 g, 27.65%).

MS:(ESI, *m/z*): 345.10 $[M+H]^+$, RT(min): 0.595.

Step 2 Synthesis of 4-bromo-7,8,9,10-tetrahydro-6*H*-benzo[4,5]imidazo[1,2-a]azepine (compound 97-3):

Under nitrogen protection, *N,N*-dimethylethylenediamine (25.47 mg, 0.289 mmol, 0.10 eq), cuprous iodide (27.52mg, 0.145 mmol, 0.05 eq), and potassium carbonate (599.04 mg, 4.335 mmol, 3 eq) were added to a solution of (2Z)-*N*-(2,6-dibromoanilino)azepin-2-imine (500 mg, 1.445 mmol, 1 eq) in acetonitrile (3 mL) at room temperature. The reaction mixture was heated to 100°C and stirred for 1 h. The reaction mixture was cooled to room temperature, diluted with water (40 mL), and extracted with ethyl acetate (3 × 30 mL). The organic phases were combined, washed with saturated brine (1 × 40 mL) and dried over anhydrous sodium sulfate. The mixture was filtered and the filtrate was concentrated under reduced pressure. 4-bromo-7,8,9,10-tetrahydro-6H-benzo[4,5]imidazo[1,2-a]azepine (250 mg, 65.26%) was

obtained.

MS:(ESI, *m/z*):264.90 $[M+H]^+$, RT(min):1.151.

Step 3 Synthesis of tert-butyl (7,8,9,10-tetrahydro-6H-benzo[4,5]imidazo[1,2-a]azepin-4-yl)carbamate (compound 97-4):

Under nitrogen protection, tert-butyl carbamate (220.91 mg, 1.886 mmol, 2 eq), dicyclohexyl(3-isopropyl-2',4',6'-triisopropyl-[1,1'-biphenyl]-2-yl)phosphine (50.42 mg, 0.094 mmol, 0.1 eq), (methanesulfonic acid{bicycloethyl(3-isopropyl-2,4,6-triisopropyl-(1,1-biphenyl)-2-yl)phosphine})(2-methylamino-1,1-biphenyl-2-yl)palladium(II) (173.21 mg, 0.189 mmol, 0.2 eq) and cesium carbonate (921.59 mg, 2.829 mmol, 3 eq) were added to a solution of 4-bromo-7,8,9,10-tetrahydro-6H-benzo[4,5]imidazo[1,2-a]azepine (250 mg, 0.943 mmol, 1 eq) in *N,N*-dimethylformamide (10 mL) at room temperature. The reaction mixture was heated to 100°C and stirred for 1 h. The reaction was cooled to room temperature and extracted with ethyl acetate (3 × 50 mL). The organic phases were combined, washed with saturated brine (1 × 50 mL) and dried over anhydrous sodium sulfate. The mixture was filtered and the filtrate was concentrated under reduced pressure. The resulting residue was purified by silica gel column chromatography with petroleum ether/ethyl acetate (5:1) to give tert-butyl (7,8,9,10-tetrahydro-6*H*-benzo[4,5]imidazo[1,2-a]azepin-4-yl)carbamate (150 mg, 52.79%).

MS:(ESI, *m/z*):302.35 $[M+H]^+$, RT(min):1.106.

Step 4 Synthesis of 7,8,9,10-tetrahydro-6*H*-benzo[4,5]imidazo[1,2-a]azepin-4-amine (compound 97-5):

To a solution of tert-butyl (7,8,9,10-tetrahydro-6*H*-benzo[4,5]imidazo[1,2-a]azepin-4-yl)carbamate (150 mg, 0.498 mmol, 1 eq) in dichloromethane (2 mL) was added a solution of hydrochloric acid in 1,4-dioxane (4 M, 2 mL) at room temperature. The reaction mixture was stirred at room temperature for 1 h. The reaction mixture was concentrated under reduced pressure to give 7,8,9,10-tetrahydro-6*H*-benzo[4,5]imidazo[1,2-a]azepin-4-amine (150 mg, 66.67%). The product was directly used in the next step without purification.

MS:(ESI, *m/z*):202.40 $[M+H]^+$, RT(min):0.544.

Step 5 Synthesis of 4-iodo-2-(6-azaspiro[2.5]octan-6-yl)-N-(7,8,9,10-tetrahydro-6H-benzo[4,5]imidazo[1,2-a]azepin-4-yl)benzamide (compound 97-6):

Under nitrogen protection, N-methylimidazole (611.90 mg, 7.450 mmol, 10 eq) was added to a solution of 7,8,9,10-tetrahydro-6H-benzo[4,5]imidazo[1,2-a]azepin-4-amine (150 mg, 1.443 mmol, 1 eq), 2-{6-azaspiro[2.5]octan-6-yl}-4-iodobenzoic acid (319.44 mg, 0.894 mmol, 1.2 eq) and N,N,N,N-tetramethylchloroformamidine hexafluorophosphate (836.42 mg, 2.980 mmol, 4eq) in dichloromethane (5 mL) at room temperature. The reaction mixture was stirred at room temperature for 2 h. The system was extracted with ethyl acetate (3 × 20 mL). The organic phases were combined, washed with saturated brine (1 × 20 mL), and dried over anhydrous sodium sulfate. The mixture was filtered and the filtrate was concentrated under reduced pressure. The resulting residue was purified by silica gel column chromatography with petroleum ether/ethyl acetate (5:1) to obtain 4-iodo-2-(6-azaspiro[2.5]octan-6-yl)-N-(7,8,9,10-tetrahydro-6H-benzo[4,5]imidazo-1,2-a] azepin-4-yl)benzamide (60 mg, 14.90%).

MS:(ESI, *m/z*):541.35 $[M+H]^+$, RT(min):1.398.

Step 6 Synthesis of 4-(2-hydroxyethanesulfonylamino)-2-(6-azaspiro[2.5]octan-6-yl)-*N*-(7,8,9,10-tetrahydro-6*H*-benzo [4,5]imidazo[1,2-a]azepin-4-yl)benzamide (compound 97):

Under nitrogen protection, sarcosine (2.09mg, 0.023 mmol, 0.2 eq), cuprous iodide (2.23 mg, 0.012 mmol, 0.1 eq) and potassium carbonate (48.55 mg, 0.351 mmol, 3 eq) were added to a solution of 4-iodo-2-(6-azaspiro[2.5]octan-6-yl)-*N*-(7,8,9,10-tetrahydro-6H-benzo[4,5]imidazo[1,2-a]azepin-4-yl)benzamide (60 mg, 0.117 mmol, 1 eq) and 2-hydroxyethanesulfonamide (17.58 mg, 0.140 mmol, 1.2 eq) in N,N-dimethylformamide (2 mL) at room temperature. The reaction mixture was heated to 120°C and stirred for 1 h. The reaction mixture was cooled to room temperature and extracted with ethyl acetate (3 × 20 mL). The organic phases were combined, washed with saturated aqueous brine solution (1 × 10 mL) and dried over anhydrous sodium sulfate. The resulting mixture was filtered and the filtrate was concentrated under reduced pressure. The resulting crude product was purified by preparative HPLC to give 4-(2-hydroxyethanesulfonylamino)-2-(6-azaspiro[2.5]octan-6-yl)-*N*-(7,8,9,10-tetrahydro-6*H*-benzo[4,5]imidazo[1,2-a]azepin-4-yl)benzamide (12.53 mg, 22.81%).

MS:(ESI, *m/z*): 538.00 $[M+H]^+$, RT(min): 1.588.

$^1$H NMR (400 MHz, DMSO-$d_6$) δ 12.47 (s, 1H), 10.15 (s, 1H), 8.30 (d, 1H), 8.03 (s, 1H), 7.27 (d, 1H), 7.23 (d, 1H), 7.16 (d, 1H), 7.09 (s, 1H), 4.94 (s, 1H), 4.27 (d, 2H), 3.76 (s, 2H), 3.37 (s, 2H), 3.09 (s, 2H), 3.01-2.93 (m, 4H), 1.90 (s, 4H),

1.73 (s, 6H), 0.30 (s, 4H).

Example 11

4-(2-hydroxyethanesulfonylamino)-*N*-(4-methyl-1,2,3,4-tetrahydrobenzo[4,5]imidazo[1,2-a]pyridin-6-yl)-2-(6-azaspiro [2.5]octan-6-yl)benzamide (compound 98)

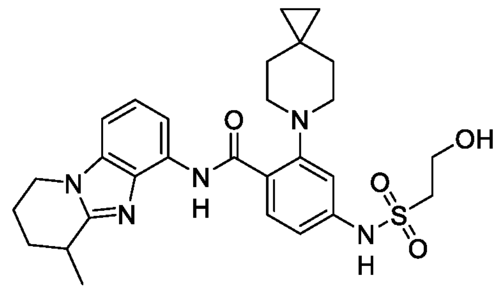

Compound 98

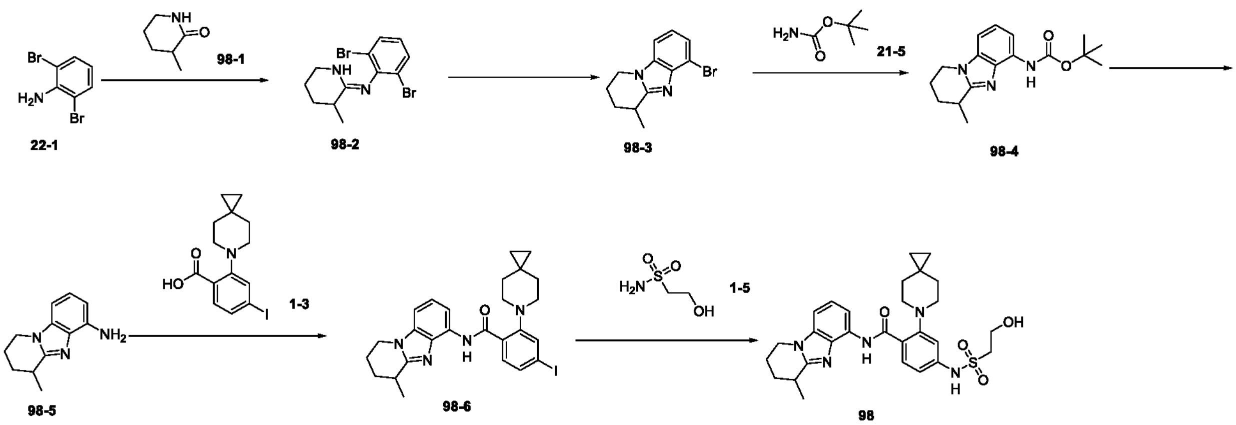


Step 1 Synthesis of (2*Z*)-*N*-(2,6-dibromophenyl)-3-methylpiperidin-2-imine (compound 98-2):

Under nitrogen protection, phosphorus oxychloride (3.67 g, 23.912 mmol, 2 eq) was added to a solution of 3-methylpiperidin-2-one (1.62 g, 14.347 mmol, 1.2 eq) in toluene (20 mL) at 0°C. After the addition was completed, the reaction system was further stirred at 0°C for 2 h. Then, 2,6-dibromoaniline (3 g, 11.956 mmol, 1 eq) was added. After the addition was completed, the reaction system was further stirred at 110°C for 1 h. The reaction mixture was cooled to room temperature and quenched with water (20 mL). The reaction mixture was extracted with ethyl acetate (3 × 60 mL). The organic phases were combined, washed with saturated sodium chloride solution (1 × 60 mL), and dried over anhydrous sodium sulfate. The resulting mixture was filtered and the filtrate was concentrated under reduced pressure. The resulting residue was purified by silica gel column chromatography with petroleum ether/ethyl acetate (5:1) to give (2Z)-N-(2,6-dibromophenyl)-3-methylpiperidin-2-imine (2.3 g, 55.59%).

MS (ESI, *m/z*):346.85 $[M+H]^+$, RT(min):0.620

Step 2 Synthesis of 6-bromo-4-methyl-1,2,3,4-tetrahydrobenzo[4,5]imidazo[1,2-a]pyridine (compound 98-3):

Under nitrogen protection, cuprous iodide (71.54 mg, 0.376 mmol, 0.1 eq) and *N,N*-dimethylethylenediamine (66.23 mg, 0.751 mmol, 0.2 eq) were added to a solution of *(2Z)-N-(2,6*-dibromophenyl)-3-methylpiperidin-2-imine (1.3 g, 3.757 mmol, 1 eq) and potassium carbonate (1038.34 mg, 7.514 mmol, 2 eq) in acetonitrile (2 mL) at room temperature. After the addition was completed, the reaction system was heated to 100°C and further stirred for 1 h. The reaction mixture was cooled to room temperature and quenched with water (10 mL). The reaction mixture was extracted with ethyl acetate (1 × 20 mL). The organic phases were combined, washed with saturated sodium chloride solution (1 × 20 mL), and dried

over anhydrous sodium sulfate. The resulting mixture was filtered, and then the filtrate was concentrated under reduced pressure. The crude product/resulting mixture was used directly in the next step without further purification.

MS(ESI, *m/z*):264.90 $[M+H]^+$, RT(min):0.561

Step 3 Synthesis of tert-butyl (4-methyl-1,2,3,4-tetrahydrobenzo[4,5]imidazo[1,2-a]pyridin-6-yl)carbamate (compound 98-4):

Under nitrogen protection, cesium carbonate (3.50 g, 10.749 mmol, 3 eq), bicyclohexyl(3-isopropoxy-2',4',6'-triisopropyl-[1,1'-biphenyl]-2-yl)phosphine (383.23 mg, 0.717 mmol, 0.2 eq) and methanesulfonic acid{dicyclohexyl(3-isopropoxy-2',4',6'-triisopropyl-[1,1'-biphenyl]-2-yl)phosphine}(2'-methylamino-1,1'-biphenyl-2-yl)palladium(II) (329.11 mg, 0.358 mmol, 0.1 eq) were added to a solution of 6-bromo-4-methyl-1,2,3,4-tetrahydrobenzo[4,5]imidazo[1,2-a]pyridine (950 mg, 3.583 mmol, 1 eq) and tert-butyl carbamate (503.66 mg, 4.300 mmol, 1.2 eq) in N,N-dimethylformamide (10 mL) at room temperature. After the addition was completed, the reaction system was heated to 100°C and further stirred for 1 h. The reaction mixture was cooled to room temperature. The reaction mixture was extracted with ethyl acetate ($3 \times 30$ mL). The organic phases were combined, washed with saturated sodium chloride solution ($1 \times 40$ mL), and dried over anhydrous sodium sulfate. The resulting mixture was filtered and then the filtrate was concentrated under reduced pressure. The resulting residue was purified by silica gel column chromatography with petroleum ether/ethyl acetate (5:1) to give tert-butyl (4-methyl-1,2,3,4-tetrahydrobenzo[4,5]imidazo[1,2-a]pyridin-6-yl)carbamate (950 mg, 87.98%).

MS (ESI, *m/z*):302.30 $[M+H]^+$, RT(min):1.128

Step 4 Synthesis of 4-methyl-1,2,3,4-tetrahydrobenzo[4,5]imidazo[1,2-a]pyridin-6-amine (compound 98-5):

Under nitrogen protection, to a solution of tert-butyl (4-methyl-1,2,3,4-tetrahydrobenzo[4,5]imidazo[1,2-a]pyridin-6-yl)carbamate (700 mg, 2.323 mmol, 1 eq) in dichloromethane (7 mL) was added a solution of hydrogen chloride in 1,4-dioxane (7.5 mL, 4 mol/L) at room temperature, and the reaction mixture was further stirred at room temperature for 1 h. The reaction mixture was concentrated under reduced pressure. The resulting residue was used directly in the next step without further purification.

MS (ESI, *m/z*):202.25 $[M+H]^+$, RT(min):0.777

Step 5 Synthesis of 4-iodo-N-(4-methyl-1,2,3,4-tetrahydrobenzo[4,5]imidazo[1,2-a]pyridin-6-yl)-2-(6-azaspiro[2.5]octan-6-yl)-benzamide (compound 98-6):

Under nitrogen protection, to a solution of 4-methyl-1,2,3,4-tetrahydrobenzo[4,5]imidazo[1,2-a]pyridin-6-amine (520 mg, 2.584 mmol, 1 eq) and 2-(6-azaspiro[2.5]octan-6-yl-4-iodobenzoic acid (1.11 g, 3.101 mmol, 1.2 eq) in dichloromethane (10 mL) were added *N*,*N*,*N*',*N*'-tetramethylchloroformamidine hexafluorophosphate (2.90 g, 10.336 mmol, 4 eq) and N-methylimidazole (2.12 g, 25.840 mmol, 10 eq) at room temperature. After the addition was completed, the reaction system was further stirred at room temperature for 1 h. The reaction mixture was extracted with ethyl acetate ($3 \times 40$ mL). The organic phases were combined, washed with saturated sodium chloride solution ($1 \times 50$ mL) and dried over anhydrous sodium sulfate. The resulting mixture was filtered, and then the filtrate was concentrated under reduced pressure. The resulting residue was purified by silica gel column chromatography with petroleum ether/ethyl acetate (4:1) to obtain 4-iodo-N-(4-methyl-1,2,3,4-tetrahydrobenzo[4,5]imidazo[1,2-a]pyridin-6-yl)-2-(6-azaspiro[2.5]octan-6-yl)-benzamide (620 mg, 44.40%).

MS (ESI, *m/z*):541.25 $[M+H]^+$, RT(min):1.585

Step 6 Synthesis of 4-(2-hydroxyethanesulfonylamino)-*N*-(4-methyl-1,2,3,4-tetrahydrobenzo[4,5]imidazo[1,2-a]pyridin-6-yl)-2-(6-azaspiro[2.5]octan-6-yl)benzamide (compound 98):

Under nitrogen protection, to a solution of 4-iodo-N-(4-methyl-1,2,3,4-tetrahydrobenzo[4,5]imidazo[1,2-a]pyridin-6-yl)-2-(6-azaspiro[2.5]octan-6-yl)benzamide (100 mg, 0.185 mmol, 1 eq) and 2-hydroxyethanesulfonamide (27.79 mg, 0.222 mmol, 1.2 eq) in *N,N*-dimethylformamide (2 mL) were added 2-(methylamino)acetic acid (13.19 mg, 0.148 mmol, 0.8 eq), cuprous iodide (14.1 mg, 0.074 mmol, 0.4 eq) and potassium phosphate (117.83 mg, 0.555 mmol, 3 eq) at room temperature. After the addition was completed, the reaction system was heated to 120°C and further stirred for 1.5 h. The reaction mixture was cooled to room temperature and extracted with ethyl acetate ($3 \times 10$ mL). The organic phases were combined, washed with saturated sodium chloride solution ($1 \times 20$ mL), and dried over anhydrous sodium sulfate. The resulting mixture was filtered and the filtrate was concentrated under reduced pressure. The crude product was purified by preparative HPLC under the following conditions: chromatographic column specifications: Kinetex 5 μm EVO C18, 30mm * 150 mm; mobile phase A: water (10mmol/L sodium bicarbonate), mobile phase B: acetonitrile; flow rate: 60ml/min; elution gradient: 10% B to 37% B in 10 min; detection wavelength: 254nm/220nm; retention time (min): 9.03, to

obtain 4-(2-hydroxyethanesulfonylamino)-*N*-(4-methyl-1,2,3,4-tetrahydrobenzo[4,5]imidazo[1,2-a]pyridin-6-yl)-2-(6-azaspiro[2.5]octan-6-yl)benzamide (35.6 mg, 35.39%).

MS (ESI, *m*/*z*):538.40 $[M+H]^+$, RT(min):1.536

$^1$H NMR (400 MHz, DMSO-$d_6$) δ 12.07 (s, 1H), 10.12 (s, 1H), 8.39 (dd, 1H), 8.01 (d, 1H), 7.25 (d, 1H), 7.21 - 7.10 (m, 2H), 7.07 (dd, 1H), 5.11 (d, 1H), 4.25 - 4.11 (m, 1H), 4.10 - 3.93 (m, 1H), 3.76 (t, 2H), 3.35 (t, 2H), 3.20 - 3.07 (m, 1H), 3.02 (s, 4H), 2.24 - 2.07 (m, 2H), 2.08 - 1.94 (m, 1H), 1.93 - 1.65 (m, 4H), 1.68 - 1.56 (m, 1H), 1.48 (d, 3H), 0.30 (s, 4H).

The compounds in the following table were prepared using conditions similar to those in Example 11.

| Compound No. | Structure | Name | $[M+H]^+$ | $^1$H-NMR data |
|---|---|---|---|---|
| 34 | 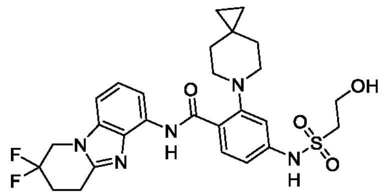 | *N*-(2,2-difluoro-1,2,3,4-tetrahydrobenzo[4,5]imidazo [1,2-a]pyridin-6-yl)-4-(2-hydroxyethanesulfonylamino) --2-(6-azaspiro[2.5]octan-6-yl) benzamide | 560.40 | $^1$H NMR: (400 MHz, DMSO-$d_6$) $\delta$ 12.51 (s, 1H), 10.14 (s, 1H), 8.38 (dd, 1H), 8.05 (d, 1H), 7.28-7.26 (m, 1H), 7.24-7.19 (m, 2H), 7.09 (dd, 1H), 4.93 (s, 1H), 4.68 (t, 2H), 3.76 (t, 2H), 3.35 (t, 2H), 3.21 (t, *J* = 6.9 Hz, 2H), 2.99 (s, 4H), 2.62 (dt, 2H), 1.83 (s, 4H), 0.32 (s, 4H). |
| 44 | 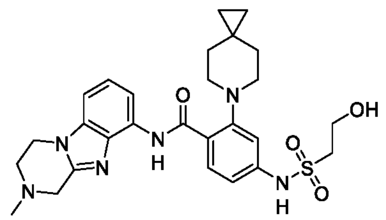 | 4-(2-hydroxyethanesulfonylamino) -*N*-(2-methyl-1,2,3,4-tetrahydrobenzo[4,5]imidazo [1,2-a]pyrazin-9-yl)-2-(6-azaspiro[2.5]octan-6-yl) benzamide | 539.45 | $^1$H NMR: (400 MHz, DMSO-$d_6$) $\delta$ 12.44 (s, 1H), 10.13 (s, 1H), 8.35 (dd, 1H), 8.04 (d, 1H), 7.27 (d, 1H), 7.24 - 7.15 (m, 2H), 7.09 (dd, 1H), 4.92 (t, 1H), 4.16 (d, 2H), 3.84 - 3.70 (m, 4H), 3.35 (t, 2H), 2.97 (d, 6H), 2.47 (s, 3H), 1.80 (s, 4H), 0.32 (s, 4H) |
| 101 | 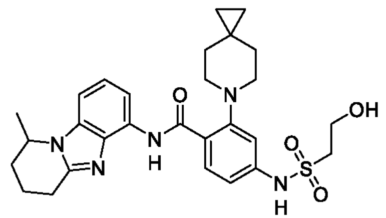 | 4-(2-hydroxyethanesulfonylamino) -*N*-(1-methyl-1,2,3,4-tetrahydrobenzo[4,5]imidazo [1,2-a]pyridin-6-yl)-2-(6-azaspiro[2.5]octan-6-yl) benzamide | 538.35 | $^1$H NMR: (400 MHz, DMSO-$d_6$) $\delta$ 12.49 (s, 1H), 10.15 (s, 1H), 8.32 (d, 1H), 8.04 (d, 1H), 7.27 (d, 1H), 7.23 (d, 1H), 7.14 (t, 1H), 7.08 (dd, 1H), 4.95 (s, 1H), 4.68 (d, 1H), 3.76 (t, 2H), 3.35 (s, 2H), 3.00 (d, 6H), 2.25 - 2.11 (m, 1H), 2.04 (dd, 1H), 1.92 (d, 4H), 1.84 (s, 2H), 1.46 (d, 3H), 0.32 (s, 4H) |
| 102 | 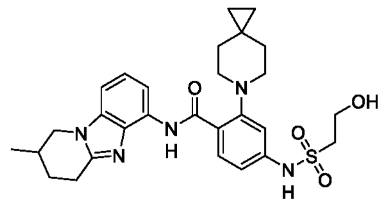 | 4-(2-hydroxyethanesulfonylamino) -*N*-(2-methyl-1,2,3,4-tetrahydrobenzo[4,5]imidazo [1,2-a]pyridin-6-yl)-2-(6-azaspiro[2.5]octan-6-yl) benzamide | 538.00 | $^1$H NMR (400 MHz, DMSO-$d_6$) $\delta$ 12.45 (s, 1H), 10.14 (s, 1H), 8.33 (dd, 1H), 8.04 (d, 1H), 7.27 (d, *J* = 2.2 Hz, 1H), 7.21 - 7.12 (m, 2H), 7.15 - 7.05 (m, 1H), 4.94 (s, 1H), 4.29 (dd, 1H), 3.76 (t, 2H), 3.65 - 3.55 (m, 1H), 3.35 (t, 2H), 3.14 - 2.99 (m, 2H), 2.98 (s, 4H), 2.23 (s, 1H), 2.10 - 2.01 (m, 1H), 1.83 (s, 4H), 1.73 - 1.59 (m, 1H), 1.14 (d, 3H), 0.31 (s, 4H) |
| 103 | 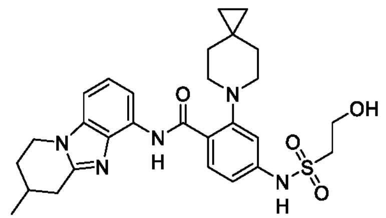 | 4-(2-hydroxyethanesulfonylamino) -*N*-(3-methyl-1,2,3,4-tetrahydrobenzo[4,5]imidazo [1,2-a]pyridin-6-yl)-2-(6-azaspiro[2.5]octan-6-yl) benzamide | 538.05 | $^1$H NMR (400 MHz, DMSO-$d_6$) $\delta$ 12.44 (s, 1H), 10.12 (s, 1H), 8.35 - 8.28 (m, 1H), 8.03 (d, 1H), 7.26 (d, 1H), 7.20 - 7.12 (m, 2H), 7.12 -7.06 (m, 1H), 4.94 (s, 1H), 4.31 - 4.22 (m, 1H), 4.07 - 3.97 (m, 1H), 3.76 (t, 2H), 3.36 (d, 2H), 3.14 - 3.07 (m, 1H), 2.98 (d, 4H), 2.66 - 2.55 (m, 1H), 2.13 (d, 2H), 2.02 - 1.61 (m, 5H), 1.14 (d, 3H), 0.31 (s, 4H) |

Example 12

4-(2-hydroxyethanesulfonylamino)-*N*-((1*S*,4*R*)-8-methyl-1,2,3,4-tetrahydro-1,4-methylenebenzo[4,5]imidazo[1,2-a] pyridin-6-yl)-2-(6-azaspiro[2.5]octan-6-yl)benzamide (compound 99)

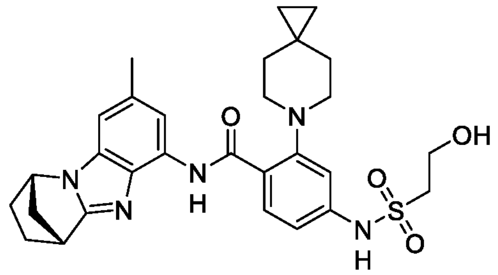

Compound 99

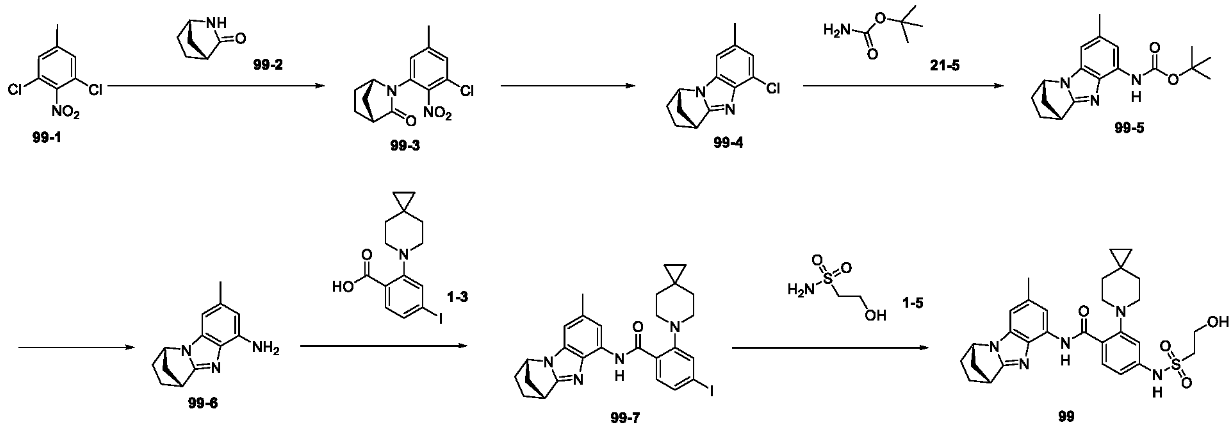


Step 1 Synthesis of (1S,4R)-2-(3-chloro-5-methyl-2-nitrophenyl)-2-azabicyclo[2.2.1]heptan-3-one (compound 99-3):

Under nitrogen protection, palladium acetate (108.97 mg, 0.485 mmol, 0.1 eq) and 1,1'-bis(diphenylphosphino) ferrocene (536.23 mg, 0.971 mmol, 0.2 eq) were added to a solution of 1,3-dichloro-5-methyl-2-nitrobenzene (1g, 4.854 mmol, 1 eq), potassium phosphate (3.09 g, 14.562 mmol, 3 eq) and (1*S*,4*R*)-2-azabicyclo[2.2.1]heptan-3-one (539.48 mg, 4.854 mmol, 1 eq) in 1,4-dioxane (10 mL) at room temperature. The reaction mixture was heated to 100°C and stirred for 1 h. The reaction mixture was cooled to room temperature and filtered. The filter cake was washed with ethyl acetate (3 × 10 mL). The reaction mixture was extracted with ethyl acetate (3 × 50 mL). The organic phases were combined, washed with saturated brine (2 × 50 mL), and dried over anhydrous sodium sulfate. The resulting mixture was filtered and then the filtrate was concentrated under reduced pressure. The resulting residue was purified by silica gel column chromatography with petroleum ether/ethyl acetate (1:1) to give (1S,4R)-2-(3-chloro-5-methyl-2-nitrophenyl)-2-azabicyclo[2.2.1]heptan-3-one (800 mg, 58.71%).

MS:(ESI, *m/z*): 281.30 $[M+H]^+$, RT(min): 1.079

Step 2 Synthesis of (1S,4R)-6-chloro-8-methyl-1,2,3,4-tetrahydro-1,4-methylenebenzo[4,5]imidazo[1,2-a]pyridine (compound 99-4):

Under nitrogen protection, reduced iron powder (785.82 mg, 14.070 mmol, 5 eq) and acetic acid (4 mL) were added to a solution of (1S,4R)-2-(3-chloro-5-methyl-2-nitrophenyl)-2-azabicyclo[2.2.1]heptan-3-one (790 mg, 2.814 mmol, 1 eq) in ethanol (10 mL) at room temperature. The reaction mixture was heated to 100°C and further stirred for 2 h. The reaction mixture was cooled to room temperature and concentrated under reduced pressure. The crude product was diluted with water (50 mL) and then the mixture was extracted with ethyl acetate (3 × 40 mL). The organic phases were combined, washed with saturated brine (2 × 30 mL), and dried over anhydrous sodium sulfate. The resulting mixture was

filtered and the filtrate was concentrated under reduced pressure. The resulting residue was purified by silica gel column chromatography with petroleum ether/ethyl acetate (1:1) to give (1S,4R)-6-chloro-8-methyl-1,2,3,4-tetrahydro-1,4-methylenebenzo[4,5]imidazo[1,2-a]pyridine (560 mg, 85.51%).

MS:(ESI, *m*/*z*): 233.35 $[M+H]^+$, RT(min): 0.814

Step 3 Synthesis of tert-butyl ((1S,4R)-8-methyl-1,2,3,4-tetrahydro-1,4-methylenebenzo[4,5]imidazo[1,2-a]pyridin-6-yl)carbamate (compound 99-5):

Under nitrogen protection, to a solution of (1S,4R)-6-chloro-8-methyl-1,2,3,4-tetrahydro-1,4-methylenebenzo [4,5]imidazo[1,2-a]pyridine (550 mg, 2.363 mmol, 1 eq), cesium carbonate (2.31 g, 7.089 mmol, 3 eq) and tert-butyl carbamate (553.75 mg, 4.726 mmol, 2 eq) in *N,N*-dimethylformamide (6 mL) were added (methanesulfonic acid {biscyclohexyl(3-isopropoxy-2',4',6'-triisopropyl-[1,1'-biphenyl]-2-yl)phosphine}(2'-methylamino-1,1'-biphenyl-2-yl)palladium(II) (217.10 mg, 0.236 mmol, 0.1 eq) and dicyclohexyl(3-isopropyl-2',4',6'-triisopropyl-[1,1'-biphenyl]-2-yl)phosphine (252.80 mg, 0.473 mmol, 0.2 eq) at room temperature. The reaction mixture was heated to 100°C and stirred for 16 h. The reaction mixture was cooled to room temperature and extracted with ethyl acetate (3 × 40 mL). The organic phases were combined, washed with saturated brine (2 × 30 mL) and dried over anhydrous sodium sulfate. The resulting mixture was filtered and the filtrate was concentrated under reduced pressure. The resulting residue was purified by silica gel column chromatography with petroleum ether/ethyl acetate (1:1) to give tert-butyl ((1*S*,4*R*)-8-methyl-1,2,3,4-tetrahydro-1,4-methylenebenzo[4,5]imidazo[1,2-a]pyridin-6-yl)carbamate (650 mg, 87.75%).

MS:(ESI, *m*/*z*): 314.45 $[M+H]^+$, RT(min): 0.871

Step 4 Synthesis of (1S,4R)-8-methyl-1,2,3,4-tetrahydro-1,4-methylenebenzo[4,5]imidazo[1,2-a]pyridin-6-amine (compound 99-6):

To a solution of tert-butyl ((1S,4R)-8-methyl-1,2,3,4-tetrahydro-1,4-methylenebenzo[4,5]imidazo[1,2-a]pyridin-6-yl)carbamate (650 mg, 2.074 mmol, 1 eq) in dichloromethane (10 mL) was added a solution of hydrogen chloride in 1,4-dioxane (10 mL) at room temperature, and the mixture was stirred for 1 h. The reaction was complete as monitored by LC-MS, and the mixture was concentrated in vacuum to give (1S,4R)-8-methyl-1,2,3,4-tetrahydro-1,4-methylenebenzo[4,5]imidazo[1,2-a]pyridin-6-amine as a crude product (700 mg). The crude product was directly used in the next step without further purification.

MS: (ESI, *m*/*z*): 214.40 $[M+H]^+$, RT(min): 0.230

Step 5 Synthesis of 4-iodo-N-((1S,4R)-8-methyl-1,2,3,4-tetrahydro-1,4-methylenebenzo[4,5]imidazo[1,2-a]pyridin-6-yl)-2-(6-azaspiro[2.5]octan-6-yl)benzamide (compound 99-7):

Under nitrogen protection, N-methylimidazole (344.80 mg, 4.200 mmol, 10 eq) was added to a solution of (1*S*,4*R*)-8-methyl-1,2,3,4-tetrahydro-1,4-methylenebenzo[4,5]imidazo[1,2-a]pyridin-6-amine (107.48 mg, 0.504 mmol, 1.2 eq), 4-iodo-2-(6-azaspiro[2.5]octan-6-yl)benzoic acid (150 mg, 0.420 mmol, 1.0 eq) and *N*,*N*,*N'*,*N'*-tetramethylchloroformamidine hexafluorophosphate (471.31 mg, 1.680 mmol, 4 eq) in dichloromethane (5 mL) at room temperature. The reaction mixture was further stirred at room temperature for 3 h. The reaction mixture was extracted with dichloromethane (3 × 30 mL). The organic phases were combined, washed with saturated brine (2 × 30 mL), and dried over anhydrous sodium sulfate. The resulting mixture was filtered and the filtrate was concentrated under reduced pressure. The resulting residue was purified by silica gel column chromatography with petroleum ether/ethyl acetate (2:1) to obtain 4-iodo-*N*-((1*S*,4R)-8-methyl-1,2,3,4-tetrahydro-1,4-methylenebenzo[4,5]imidazo[1,2-a]pyridin-6-yl)-2-(6-azaspiro[2.5]octan-6-yl)benzamide (110 mg, 47.41%).

MS:(ESI, *m*/*z*): 553.10 $[M+H]^+$,RT(min): 1.118

Step 6 Synthesis of 4-(2-hydroxyethanesulfonylamino)-*N*-((1*S*,4*R*)-8-methyl-1,2,3,4-tetrahydro-1,4-methylenebenzo [4,5]imidazo[1,2-a]pyridin-6-yl)-2-(6-azaspiro[2.5]octan-6-yl)benzamide (compound 99):

Under nitrogen protection, cuprous iodide (3.45 mg, 0.018 mmol, 0.1 eq) and sarcosine (3.23 mg, 0.036 mmol, 0.2 eq) were added to a solution of 4-iodo-*N*-((1*S*,4*R*)-8-methyl-1,2,3,4-tetrahydro-1,4-methylenebenzo[4,5]imidazo[1,2-a]pyridin-6-yl)-2-(6-azaspiro[2.5]octan-6-yl)benzamide (100 mg, 0.181 mmol, 1 eq), potassium carbonate (75.05 mg, 0.543 mmol, 3 eq) and 2-hydroxyethane-1-sulfonamide (45.30 mg, 0.362 mmol, 2 eq) in *N,N*-dimethylformamide (2 mL) at room temperature. The reaction mixture was heated to 120°C and stirred for 2 h. The reaction mixture was cooled to room temperature and filtered. The filter cake was washed with ethyl acetate (3 × 5 mL). The mixture was extracted with ethyl acetate (3 × 20 mL). The organic phases were combined, washed with saturated brine (2 × 30 mL) and dried over anhydrous sodium sulfate. The resulting mixture was filtered and the filtrate was concentrated under reduced pressure.

The crude product was purified by preparative HPLC under the following conditions (chromatographic column specifications: Kinetex 5 μm EVO C18, 30mm × 150 mm; mobile phase A: water (10mmol/L sodium bicarbonate), mobile phase B: acetonitrile; flow rate: 60ml/min; elution gradient: 27% B to 62% B in 8 min; detection wavelength: 254nm/220nm; retention time (min): 7.92) to obtain 4-(2-hydroxyethanesulfonylamino)-*N*-((1*S*,4*R*)-8-methyl-1,2,3,4-tetrahydro-1,4-methylenebenzo[4,5]imidazo[1,2-a]pyridin-6-yl)-2-(6-azaspiro[2.5]octan-6-yl)benzamide (38.29 mg, 38.33%).

MS:(ESI, *m*/*z*): 550.45 $[M+H]^+$, RT(min): 1.525

$^1H$ NMR (400 MHz, DMSO-$d_6$) $\delta$ 12.22 (s, 1H), 10.13 (s, 1H), 8.15 (d, 1H), 7.98 (d, 1H), 7.23 (d, 1H), 7.10 - 7.01 (m, 2H), 5.09 (s, 1H), 4.94 (s, 1H), 3.76 (t, 2H), 3.55 (d, 1H), 3.35 (d, 2H), 2.97 (d, 4H), 2.41 (s, 3H), 2.21 (d, 1H), 2.14 - 2.05 (m, 1H), 2.00 - 1.90 (m, 2H), 1.72 (s, 4H), 1.20 - 1.07 (m, 2H), 0.28 (s, 4H).

The compounds in the following table were prepared using conditions similar to those in Example 12.

| Compound No. | Structure | Name | $[M+H]^+$ | $^1$H-NMR data |
|---|---|---|---|---|
| 113a | 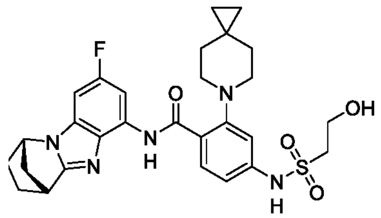 | *N*-((1S,4R)-8-fluoro-1,2,3,4-tetrahydro-1,4-methylenebenzo [4,5]imidazo [1,2-a]pyridin-6-yl) 4-(2-hydroxyethanesulfonylamino) -2-(6-azaspiro[2.5]octan-6-yl)benzamide | 553.80 | $^1$H NMR (400 MHz, DMSO-$d_6$) $\delta$ 12.55 (d, 1H), 10.11 (s, 1H), 8.17 - 8.09 (m, 1H), 8.01 (d, 1H), 7.27 (d, 1H), 7.19 - 7.12 (m, 1H), 7.12 - 7.05 (m, 1H), 5.14 (s, 1H), 4.94 (s, 1H), 3.77 (t, 2H), 3.57 (d, 1H), 3.35 (t, 2H), 3.05 - 2.93 (m, 4H), 2.28 - 2.20 (m, 1H), 2.18 - 2.09 (m, 1H), 2.05 - 1.93 (m, 2H), 1.76 (s, 4H), 1.25 - 1.12 (m, 2H), 0.30 (s, 4H). |

Example 13

4-(2-hydroxyethanesulfonylamino)-2-(6-azaspiro[2.5]octan-6-yl)-*N*-(1,2,3,4-tetrahydro-1,4-ethylbenzo[4,5]imidazo[1,2-a]pyridin-6-yl)benzamide (compound 100)

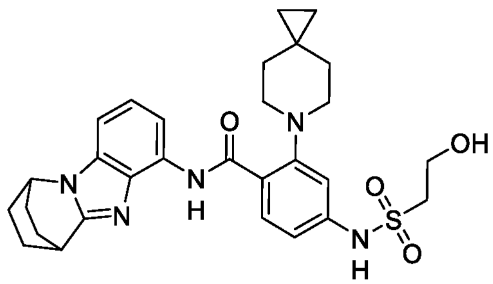

Compound 100

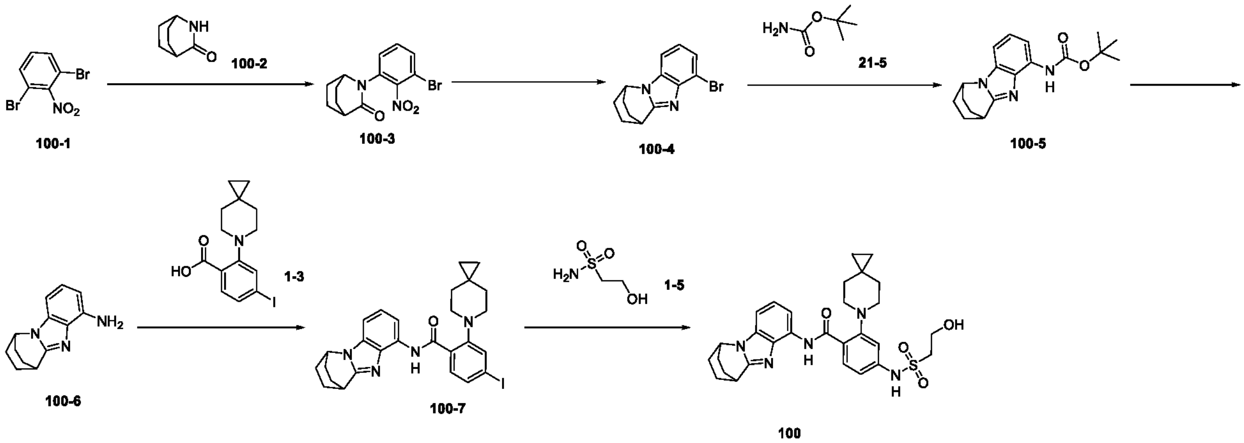


Step 1 Synthesis of 2-(3-bromo-2-nitrophenyl)-2-azabicyclo[2.2.2]octan-3-one (compound 100-3):

Under nitrogen protection, tris(dibenzylideneacetone)dipalladium (326 mg, 0.356 mmol, 0.1 eq) and 4,5-bis(diphenylphosphino-9,9-dimethyloxanthene) (412 mg, 0.712 mmol, 0.2 eq) were added to a solution of 1,3-dibromo-2-nitrobenzene (1 g, 3.56 mmol, 1 eq), 2-azabicyclo[2.2.2]octan-3-one (534 mg, 4.3 mmol, 1.2 eq) and cesium carbonate (3.5 g, 10.7 mmol, 3 eq) in 1,4-dioxane (10 mL) at room temperature. After the addition was completed, the reaction mixture was heated to 80°C and stirred for 3 h. The reaction mixture was cooled to room temperature and filtered. The filter cake was washed with ethyl acetate (3 × 10 mL). The mixture was extracted with ethyl acetate (3 × 50 mL). The organic phases were combined and washed with saturated brine (2 × 50 mL), dried over anhydrous sodium sulfate, filtered, and the filtrate was concentrated under reduced pressure. The resulting residue was purified by silica gel column chromatography with petroleum ether/ethyl acetate (1:1) to give 2-(3-bromo-2-nitrophenyl)-2-azabicyclo[2.2.2]octan-3-one (603 mg, 52%).

MS:(ESI, *m/z):* 325.30 $[M+H]^+$, RT(min):1.021

Step 2 Synthesis of 6-bromo-1,2,3,4-tetrahydro-1,4-ethylbenzo[4,5]imidazo[1,2-a]pyridine (compound 100-4):

Under nitrogen protection, tetrahydroxydiboron (480 mg, 5.4 mmol, 3 eq) and 4,4'-bipyridine (1.4 mg, 0.009 mmol, 0.005 eq) were added to a solution of 2-(3-bromo-2-nitrophenyl)-2-azabicyclo[2.2.2]octan-3-one (580 mg, 1.8 mmol, 1 eq) in N,N-dimethylformamide (1 mL) at room temperature. After the addition was completed, the mixture was stirred at room temperature for 16 h. The reaction mixture was quenched with water (10 mL) at room temperature and extracted with ethyl acetate (3 × 40 mL). The organic phases were combined and washed with saturated brine (2 × 10 mL), dried over anhydrous sodium sulfate, filtered, and the filtrate was concentrated under reduced pressure. The resulting

residue was purified by silica gel column chromatography with petroleum ether/ethyl acetate (1:1) to give 6-bromo-1,2,3,4-tetrahydro-1,4-ethylbenzo[4,5]imidazo[1,2-a]pyridine (380 mg, 76%).

MS:(ESI, *m*/*z*):277.20 $[M+H]^+$, RT(min):0.683

Step 3 Synthesis of tert-butyl (1,2,3,4-tetrahydro-1,4-ethylbenzo[4,5]imidazo[1,2-a]pyridin-6-yl)carbamate (compound 100-5):

Under nitrogen protection, to a solution of 6-bromo-1,2,3,4-tetrahydro-1,4-ethylbenzo[4,5]imidazo[1,2-a]pyridine (100 mg, 0.36 mmol, 1 eq) in 1,4-dioxane (2 mL) were added tert-butyl carbamate (51 mg, 0.43 mmol, 1.2 eq), (methanesulfonic acid{biscyclohexyl(3-isopropoxy-2',4',6'-triisopropyl-[1,1'-biphenyl]-2-yl)phosphine}(2'-methylamino-1,1'-biphenyl-2-yl)palladium(II) (33 mg, 0.036 mmol, 0.1 eq), dicyclohexyl(3-isopropoxy-2',4',6'-triisopropyl-[1,1'-biphenyl]-2-yl)phosphine (39 mg, 0.072 mmol, 0.2 eq), cesium carbonate (588 mg, 1.8 mmol, 5 eq) at room temperature. After the addition was completed, the system was heated to 100°C and stirred for 16 h. The reaction mixture was cooled to room temperature, filtered, and the filter cake was washed with ethyl acetate (3 × 5 mL). The mixture was extracted with ethyl acetate (3 × 20 mL). The organic phases were combined and washed with saturated brine (2 × 30 mL), dried over anhydrous sodium sulfate, filtered, and the filtrate was concentrated under reduced pressure. The resulting residue was purified by silica gel column chromatography with petroleum ether/ethyl acetate (1:1) to obtain tert-butyl (1,2,3,4-tetrahydro-1,4-ethylbenzo[4,5]imidazo[1,2-a]pyridin-6-yl)carbamate (90 mg, 79%).

MS: (ESI, *m*/*z*): 314.40 $[M+H]^+$, RT (min): 0.768

Step 4 Synthesis of 1,2,3,4-tetrahydro-1,4-ethylbenzo[4,5]imidazo[1,2-a]pyridin-6-amine (compound 100-6):

Under nitrogen protection, to a solution of tert-butyl (1,2,3,4-tetrahydro-1,4-ethylbenzo[4,5]imidazo[1,2-a]pyridin-6-yl)carbamate (85 mg, 0.27 mmol, 1 eq) in dichloromethane (1 mL) was added a solution of hydrogen chloride in 1,4-dioxane (1 mL) at room temperature, and after the addition was completed, the reaction mixture was stirred at room temperature for 1 h. The reaction mixture was concentrated under reduced pressure to give 1,2,3,4-tetrahydro-1,4-ethylbenzo[4,5]imidazo[1,2-a]pyridin-6-amine (57 mg, 98%).

MS:(ESI, *m*/*z*):214.4 $[M+H]^+$, RT(min):0.196

Step 5 Synthesis of 4-iodo-2-(6-azaspiro[2.5]octan-6-yl)-N-(1,2,3,4-tetrahydro-1,4-ethylbenzo[4,5]imidazo[1,2-a]pyridin-6-yl)benzamide (compound 100-7):

Under nitrogen protection, 2-{6-azaspiro[2.5]octan-6-yl}-4-iodobenzoic acid (104 mg, 0.23 mmol, 1.2 eq), N,N,N,N-tetramethylchloroformamidine hexafluorophosphate (342 mg, 1.2 mmol, 5 eq) and N-methylimidazole (200 mg, 2.4 mmol, 10eq) were added to a solution of 1,2,3,4-tetrahydro-1,4-ethylbenzo[4,5]imidazo[1,2-a]pyridin-6-amine (52 mg, 0.24 mmol, 1 eq) in dichloromethane (1 mL) at room temperature. After the addition was completed, the mixture was stirred at room temperature for 1 h. The reaction mixture was extracted with ethyl acetate (3 × 30 mL), and the organic phases were combined, washed with saturated brine (2 × 20 mL), and dried over anhydrous sodium sulfate. The mixture was filtered, and the filtrate was concentrated under reduced pressure. The resulting residue was purified by silica gel column chromatography with petroleum ether/ethyl acetate (3:1) to obtain 4-iodo-2-(6-azaspiro[2.5]octan-6-yl)-N-(1,2,3,4-tetrahydro-1,4-ethylbenzo[4,5]imidazo[1,2-a]pyridin-6-yl)benzamide (110 mg, 82%).

MS:(ESI, *m*/*z*): 553.5 $[M+H]^+$, RT(min):1.235

Step 6 Synthesis of 4-(2--hydroxyethanesulfonylamino)-2-(6-azaspiro[2.5]octan-6-yl)-*N*-(1,2,3,4-tetrahydro-1,4-ethylbenzo[4,5]imidazo[1,2-a]pyridin-6-yl)benzamide (compound 100):

Under nitrogen protection, to a solution of 4-iodo-2-(6-azaspiro[2.5]octan-6-yl)-N-(1,2,3,4-tetrahydro-1,4-ethylbenzo[4,5]imidazo[1,2-a]pyridin-6-yl)benzamide (100 mg, 0.18 mmol, 1 eq) in *N',N'*-dimethylformamide were added 2-hydroxyethanesulfonamide (27 mg, 0.2 mmol, 1.2 eq), cuprous iodide (3.5 mg, 0.018 mmol, 0.1 eq), sarcosine (3.2 mg, 0.036 mmol, 0.2 eq) and potassium carbonate (75 mg, 0.5 mmol, 3 eq) at room temperature. The reaction mixture was stirred at 120°C for 1.5 h. The reaction mixture was cooled to room temperature and filtered. The filter cake was washed with ethyl acetate (3 × 5 mL). The mixture was extracted with ethyl acetate (3 × 30 mL). The organic phases were combined and washed with saturated brine (2 × 20 mL), dried over anhydrous sodium sulfate. The mixture was filtered, and the filtrate was concentrated under reduced pressure. The resulting residue was purified by preparative HPLC under the following conditions: chromatographic column specifications: Kinetex 5 μm EVO C18, 30mm × 150 mm; mobile phase A: water (10mmol/L sodium bicarbonate), mobile phase B: acetonitrile; flow rate: 60ml/min; elution gradient: 30% B to 60% B in 8 min; detection wavelength: 254nm/220nm; retention time (min): 7.65, to obtain 4-(2-hydroxyethanesulfonylamino)-2-(6-azaspiro[2.5]octan-6-yl)-*N*-(1,2,3,4-tetrahydro-1,4-ethylbenzo[4,5]imidazo[1,2-a]pyridin-6-yl)benzamide

(28.84 mg, 28%).

MS:(ESI, *m/z):* 550.10 [M+H]+, RT(min):1.548

$^1$H NMR: (400 MHz, $CDCl_3$) $\delta$ 12.28 (s, 1H), 8.34 (d, 1H), 8.01 (d, 1H), 7.85 (d, 1H), 7.64 - 7.28 (m, 1H), 7.27 (s, 1H), 7.15 (d, 1H), 6.92 (dd, 1H), 4.83 (s, 1H), 4.05 (t, 2H), 3.52 (s, 2H), 3.27 (t, 2H), 3.08 (t, 4H), 2.02 (dt, 4H), 1.88 - 1.66 (m, 8H), 0.24 (s, 4H).

Example 14

*N*-(3,3-difluoro-2,3-dihydro-1*H*-benzo[d]pyrrolo[1,2-a]imidazol-5-yl)-4-(2-hydroxyethanesulfonylamino)-2-spiro[2.5]oct-5-en-6-yl)benzamide (compound 90)

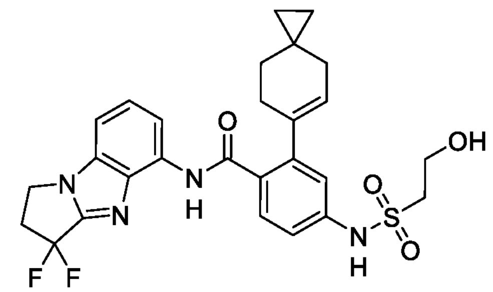

Compound 90

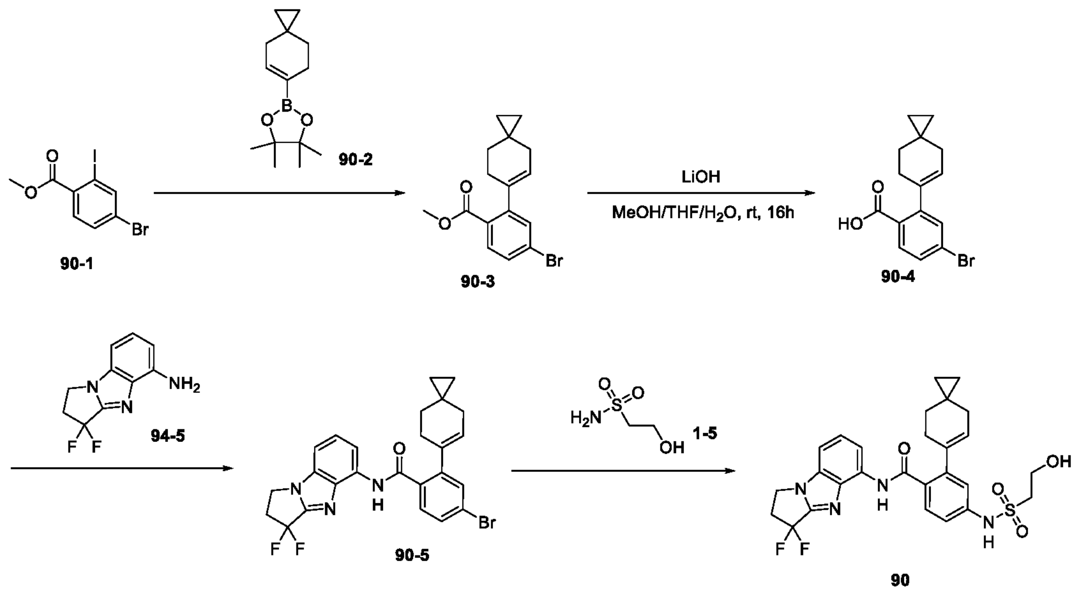


Step 1 synthesis of methyl 4-bromo-2-(spiro[2.5]oct-5-en-6-yl)benzoate (compound 90-3):

Under nitrogen protection, to a solution of methyl 4-bromo-2-iodobenzoate (700 mg, 2.053 mmol, 1 eq), sodium carbonate (326.41 mg, 3.079 mmol, 1.5 eq) and 4,4,5,5-tetramethyl-2-{spiro[2.5]oct-5-en-6-yl}-1,3,2-dioxaborolane (480.74 mg, 2.053 mmol, 1 eq) in 1,4-dioxane (8 mL) and water (1.6 mL) was added [1,1-bis(diphenylphosphino) ferrocene]dichloropalladium dichloromethane complex (167.25 mg, 0.205 mmol, 0.1 eq) at room temperature. The reaction mixture was heated to 80°C and further stirred for 1 h. The reaction mixture was cooled to room temperature. The reaction mixture was filtered and the filter cake was washed with ethyl acetate (3 × 50 mL). The filtrate was diluted with water (30 mL). The mixture was extracted with ethyl acetate (2 × 50 mL), and the organic phases were combined, washed with saturated brine (1 × 50 mL), and dried over anhydrous sodium sulfate. The mixture was filtered and the filtrate was concentrated under reduced pressure. The resulting residue was purified by a reverse phase column under the following conditions: chromatographic column specifications, C18; mobile phase: acetonitrile in water, gradient 10% to 70% in 10

min; detection wavelength: UV 254 nm. Methyl 4-bromo-2-(spiro[2.5]oct-5-en-6-yl)benzoate (320 mg, 48.52%) was obtained.

MS:(ESI, *m/z):* 320.95 $[M+H]^+$,RT(min):1.942

Step 2 synthesis of 4-bromo-2-(spiro[2.5]oct-5-en-6-yl)benzoic acid (compound 90-4):

To a solution of methyl 4-bromo-2-(spiro[2.5]oct-5-en-6-yl)benzoate (300 mg, 0.934 mmol, 1 eq) in methanol (2 mL), tetrahydrofuran (2 mL) and water (2 mL) was added lithium hydroxide (111.84 mg, 4.670 mmol, 5 eq) at room temperature. The reaction mixture was stirred at room temperature for 16 h. The reaction mixture was diluted with water (15 mL), the mixture was extracted with ethyl acetate (2 × 20 mL), the aqueous phase was collected and acidified to pH 4-5 with 1 mol/L dilute hydrochloric acid, and the aqueous phase was extracted with ethyl acetate (3 × 30 mL). The organic phases were combined and collected, washed with saturated brine (2 × 20 mL) and dried over anhydrous sodium sulfate. The mixture was filtered and the filtrate was concentrated under reduced pressure to give 4-bromo-2-{spiro[2.5]oct-5-en-6-yl}benzoic acid (250 mg, 87.14%).

MS:(ESI,*m*/*z*):308.90 $[M+H]^+$, RT(min):1.323

Step 3 Synthesis of 4-bromo-*N*-(3,3-difluoro-2,3-dihydro-1*H*-benzo[*d*]pyrrolo[1,2-*a*]imidazol-5-yl)-2-(spiro[2.5]oct-5-en-6-yl)benzamide (compound 90-5):

Under nitrogen protection, to a solution of 3,3-difluoro-2,3-dihydro-1H-benzo[d]pyrrolo[1,2-*a*]imidazol-5-amine (112.37 mg, 0.537 mmol, 1.1 eq), N,N,N',N'-tetramethylchloroformamidine hexafluorophosphate (548.03 mg, 1.952 mmol, 4 eq) and 4-bromo-2-(spiro[2.5]oct-5-en-6-yl)benzoic acid (150 mg, 0.488 mmol, 1.00 eq) in dichloromethane (5 mL) was added N-methylimidazole (400.93 mg, 4.880 mmol, 10 eq) dropwise at room temperature. After the addition was completed, the system was further stirred at 60°C for 2 h. The reaction mixture was cooled to room temperature and extracted with ethyl acetate (3 × 30 mL). The organic phases were combined, washed with saturated brine (2 × 20 mL) and dried over anhydrous sodium sulfate. The resulting mixture was filtered, and then the filtrate was concentrated under reduced pressure. The resulting residue was purified by silica gel column chromatography with petroleum ether/ethyl acetate (2:1) to give 4-bromo-*N*-(3,3-difluoro-2,3-dihydro-1*H*-benzo[*d*]pyrrolo[1,2-*a*]imidazol-5-yl)-2-(spiro[2.5]oct-5-en-6-yl)benzamide (64 mg, 26.30%).

MS:(ESI, *m*/*z*):498.40 $[M+H]^+$, RT(min):1.449

Step 4 Synthesis of 4-bromo-*N*-(3,3-difluoro-2,3-dihydro-1*H*-benzo[*d*]pyrrolo[1,2-*a*]imidazol-5-yl)-2-(spiro[2.5]oct-5-en-6-yl)benzamide (compound 90):

Under nitrogen protection, to a solution of 4-bromo-*N*-(3,3-difluoro-2,3-dihydro-1*H*-benzo[*d*]pyrrolo[1,2-*a*]imidazol-5-yl)-2-(spiro[2.5]oct-5-en-6-yl)benzamide (50 mg, 0.100 mmol, 1 eq), cesium carbonate (98.06 mg, 0.300 mmol, 3 eq) and 2-hydroxyethane-1-sulfonamide (25.11 mg, 0.200 mmol, 2 eq) in N,N-dimethylformamide (2 mL) were added (methanesulfonic acid{biscyclohexyl(3-isopropoxy-2',4',6'-triisopropyl-[1,1'-biphenyl]-2-yl)phosphine}(2'-methylamino-1,1'-biphenyl-2-yl)palladium(II) (9.22 mg, 0.010 mmol, 0.1 eq) and bicyclohexyl(3-isopropoxy-2',4',6'-triisopropyl-[1,1'-biphenyl]-2-yl)phosphine (10.73 mg, 0.020 mmol, 0.2 eq) at room temperature. The reaction mixture was heated to 100°C and stirred for 1 h. The reaction mixture was cooled to room temperature, extracted with ethyl acetate (3 × 30 mL), the organic phases were combined, washed with saturated brine (1 × 30 mL), and dried over anhydrous sodium sulfate. The resulting mixture was filtered, and then the filtrate was concentrated under reduced pressure. The crude product was purified by HPLC under the following conditions (chromatographic column specifications: Kinetex 5 m EVO C18, 30mm * 150 mm; mobile phase A: water (10 mmol/L ammonium bicarbonate), mobile phase B: acetonitrile; flow rate: 60 ml/min; elution gradient: 23% B to 60% B for 8 min; detection wavelength: UV 254nm/220nm; retention time (min): 7.6). *N*-(3,3-difluoro-2,3-dihydro-1*H*-benzo[*d*]pyrrolo[1,2-*a*]imidazol-5-yl)-4-(2-hydroxyethanesulfonylamino)-2-(spiro[2.5]oct-5-en-6-yl)benzamide (10.84 mg, 19.83%) was obtained.

MS:(ESI, *m*/*z*):543.50 $[M+H]^+$, RT(min):1.650

$^1$H NMR (400 MHz, DMSO-$d_6$) $\delta$ 9.92 (s, 1H), 9.48 (s, 1H), 8.13 (d, 1H), 7.59 (d, 1H), 7.34 - 7.29 (m, 1H), 7.26 (t, 1H), 7.14 (dd, 1H), 6.99 (d, 1H), 5.88 (s, 1H), 4.84 (s, 1H), 4.34 (d, 2H), 3.66 (t, 2H), 3.16 (d, 4H), 2.16 (s, 2H), 2.08 - 1.98 (m, 2H), 1.16 (t, 2H), 0.00 (d, 4H).

The compounds in the following table were prepared using conditions similar to those in Example 14.

| Compound No. | Structure | Name | $[M+H]^+$ | $^1$H-NMR data |
|---|---|---|---|---|
| 108 | 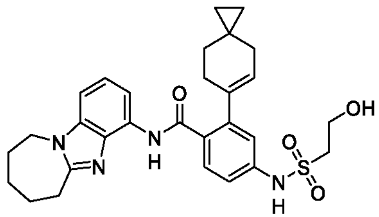 | 4-(2-hydroxyethanesulfonylamino)-2-(spiro[2.5]oct-5-en-6-yl)-*N*-(7,8,9,10-tetrahydro-6*H*-benzo[4,5]imidazo[1,2-a]azepin-4-yl)benzamide | 535.45 | $^1$H NMR (400 MHz, DMSO-$d_6$) $\delta$ 10.05 (s, 1H), 9.40 (s, 1H), 8.07 (d, 1H), 7.70 (d, 1H), 7.26 (d, 1H), 7.21 (d, 1H), 7.15 (t, 1H), 7.07 (s, 1H), 5.96 (s, 1H), 4.96 (s, 1H), 4.30 - 4.23 (m, 2H), 3.76 (t, 2H), 3.30 (s, 2H), 3.04 (d, 2H), 2.24 (s, 2H), 2.15 (s, 2H), 1.88 (s, 2H), 1.70 (d, 4H), 1.29 (s, 2H), 0.13 (s, 4H). |
| 109a | 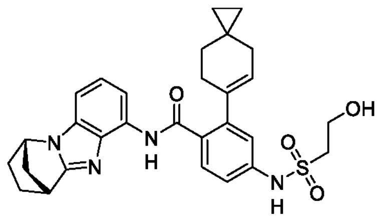 | 4-(2-hydroxyethanesulfonylamino)-2-(spiro[2.5]oct-5-en-6-yl)-*N*-((1*S*,4*S*)-1,2,3,4-tetrahydro-1,4-methylenebenzo[4,5]imidazo [1,2-a] pyridin-6-yl)benzamide | 533.40 | $^1$H NMR: (400 MHz, DMSO-$d_6$) $\delta$ 10.06 (s, 1H), 9.32 (s, 1H), 8.05 (d, 1H), 7.67 (d, 1H), 7.25 (ddd, 2H), 7.19 - 7.02 (m, 2H), 5.94 (s, 1H), 5.14 (s, 1H), 4.94 (s, 1H), 3.76 (t, 2H), 3.60 (d, 1H), 3.34 (s, 2H), 2.21 (d, 4H), 2.16 - 2.05 (m, 1H), 1.98 (dd, 8.9 Hz, 3H), 1.38 - 1.24 (m, 1H), 1.16 (dt, 3H), 0.13 - 0.03 (m, 4H) |
| 110 | 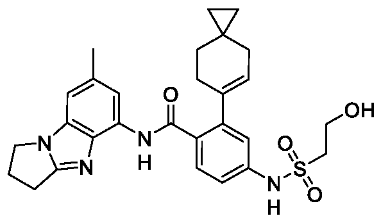 | 4-(2-hydroxyethanesulfonylamino)-*N*-(7-methyl-2,3-dihydro-1*H*-benzo[d]pyrrolo[1,2-a]imidazol-5-yl)-2-(6-azaspiro[2.5]octan-6-yl)benzamide | 524.40 | $^1$H NMR (400 MHz, DMSO-$d_6$) $\delta$ 12.24 (s, 1H), 10.10 (s, 1H), 8.17 (d, 1H), 7.99 (d, 1H), 7.24 (d, 1H), 7.14 - 7.01 (m, 1H), 6.97 (s, 1H), 4.92 (s, 1H), 4.10 (t, 2H), 3.76 (t, 2H), 3.34 (d, 2H), 2.98 (t, 4H), 2.92 (d, 2H), 2.72 - 2.61 (m, 2H), 2.42 (s, 3H), 1.77 (s, 4H), 0.29 (s, 4H) |

Example 15

*N*-(dibenzo[b,d]furan-4-yl)-4-(2-hydroxyethanesulfonylamino)-2-(6-azaspiro[2.5]octan-6-yl)benzamide (compound 111)

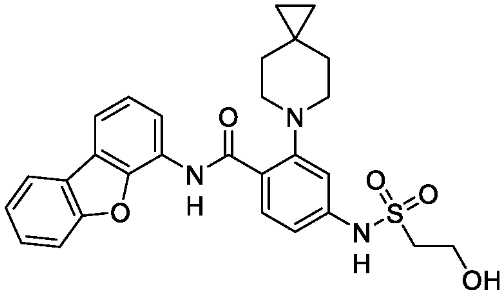

Compound 111

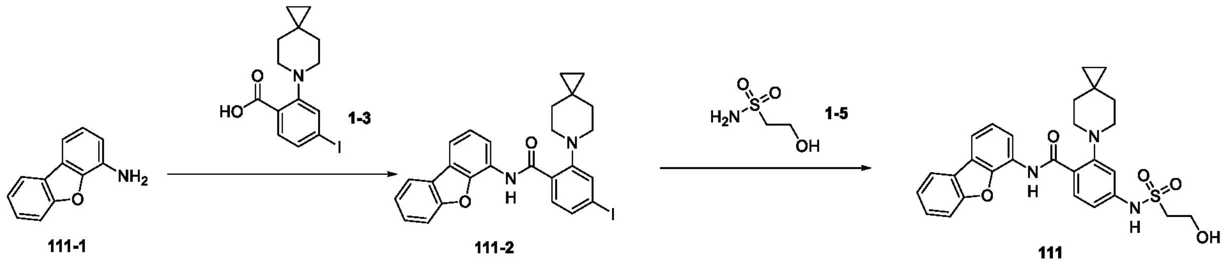


Step 1 Synthesis of N-(dibenzo[b,d]furan-4-yl)-4-iodo-2-(6-azaspiro[2.5]octan-6-yl)benzamide (compound 111-2):

Under nitrogen protection, 4-aminodibenzofuran (61.55 mg, 0.336 mmol, 1.2 eq), *N,N,N',N'*-tetramethylchloroformamidine hexafluorophosphate (314.21 mg, 1.120 mmol, 4 eq) and N-methylimidazole (229.87 mg, 2.800 mmol, 10 eq) were added to a solution of 4-iodo-2-(6-azaspiro[2.5]octan-6-yl)benzoic acid (100 mg, 0.280 mmol, 1 eq) in dichloromethane (2 mL) at room temperature. The reaction mixture was heated to 80°C and stirred for 1 h. The reaction mixture was cooled to room temperature and quenched with water (20 mL). The reaction mixture was extracted with ethyl acetate (3 × 30 mL). The organic phases were combined, washed with saturated brine (3 × 20 mL) and dried over anhydrous sodium sulfate. The resulting mixture was filtered and the filtrate was concentrated under reduced pressure. The resulting residue was purified by silica gel column chromatography with petroleum ether/ethyl acetate (10:1) to give N-(dibenzo[b,d]furan-4-yl)-4-iodo-2-(6-azaspiro[2.5]octan-6-yl)benzamide (140 mg, 95.73%).

MS: (ESI, m/z): 523.30 $[M+H]^+$, RT(min): 1.722

Step 2 Synthesis of N-(dibenzo[b,d]furan-4-yl)-4-((2-hydroxyethyl)sulfonylamino)-2-(6-azaspiro[2.5]octan-6-yl) benzamide (compound 111)

Under nitrogen protection, 2-hydroxyethane-1-sulfonamide (32.34 mg, 0.258 mmol, 1.5 eq), cuprous iodide (8.2 mg, 0.043 mmol, 0.25 eq), potassium carbonate (71.43 mg, 0.516 mmol, 3 eq) and 2-(methylamino)acetic acid (6.14 mg, 0.069 mmol, 0.4 eq) were added to a solution of *N*-(dibenzo[b,d]furan-4-yl)-4-iodo-2-(6-azaspiro[2.5]octan-6-yl)benzamide (90 mg, 0.172 mmol, 1 eq) in N,N-dimethylformamide (2 mL) at room temperature. The reaction mixture was heated to 120°C and stirred for 1 h. The reaction mixture was cooled to room temperature, and quenched with water. The reaction mixture was extracted with ethyl acetate (3 × 20 mL). The organic phases were combined, washed with saturated brine (2 × 20 mL) and dried over anhydrous sodium sulfate. The resulting mixture was filtered, and the filtrate was concentrated under reduced pressure. The crude product was purified by HPLC under the following conditions: chromatographic column specifications: Kinetex EVO prep C18, 30*150, 5μm; mobile phase A: water (10 mmol/L sodium bicarbonate), mobile phase B: acetonitrile; flow rate: 60 mL/min; elution gradient: 45% B to 70% B in 8 min; detection wavelength: UV 220 nm; retention time (min): 7.03. N-(dibenzo[b,d]furan-4-yl)-4-(2-hydroxyethanesulfonylamino)-2-(6-azaspiro[2.5]octan-6-yl)benzamide (25.9 mg, 27.89%) was obtained.

$MS^+$:(ESI, *m/z*):519.90 $[M+H]^+$, RT(min):1.784.

$^1H$ NMR: (400 MHz, DMSO-$d_6$) $\delta$ 12.85 (s, 1H), 10.21 (s, 1H), 8.74 -8.62 (m, 1H), 8.26 - 8.18 (m, 1H), 8.17 - 8.07

(m, 1H), 7.95 -7.84 (m, 1H), 7.73 (d, 1H), 7.67 -7.57 (m, 1H), 7.51 -7.45(m, 1H), 7.44 - 7.35 (m, 2H), 7.23 -7.13 (m, 1H), 4.95 (s, 1H), 3.84 -3.72 (m, 2H), 3.40 -3.35 (m, 2H), 3.14 - 2.97 (m, 4H), 1.83 (s, 4H), 0.43 (d, 4H).

Example 16

2-{6-azaspiro[2.5]octan-6-yl}-*N*-(7-cyclopropylquinolin-4-yl)-4-(2-hydroxyethylsulfonylamino)benzamide (compound 112)

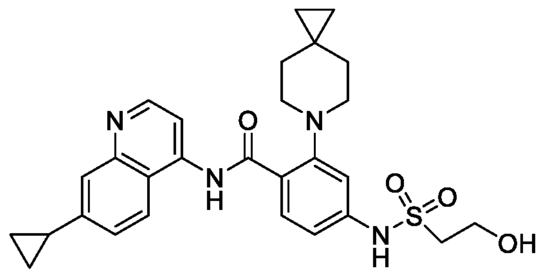

Compound 112

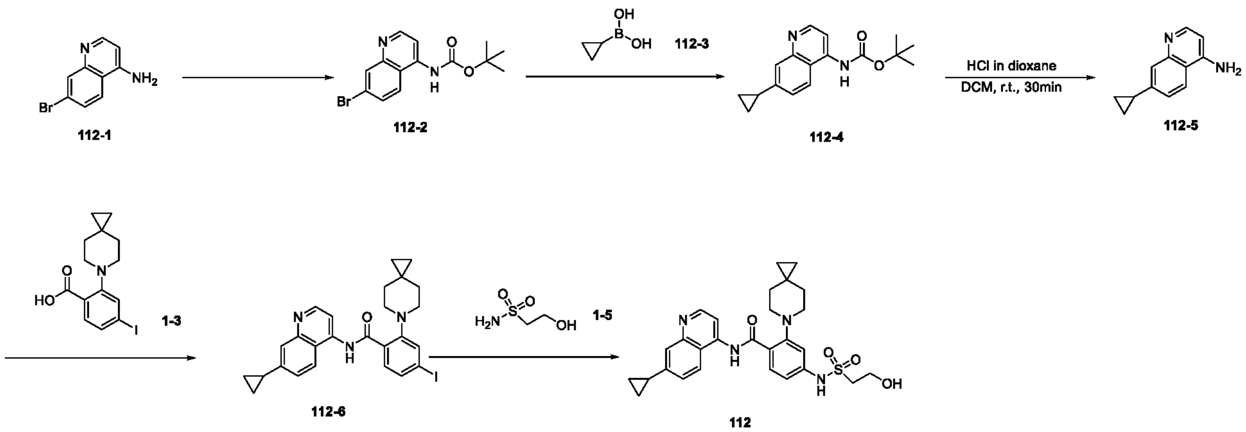


Step 1 Synthesis of tert-butyl *N*-(7-bromoquinolin-4-yl)carbamate (compound 112-2):

At room temperature, triethylamine (1.36 g, 13.449 mmol, 3 eq) and di-tert-butyl dicarbonate (978.37 mg, 4.483 mmol, 1 eq) were added to a solution of 7-bromoquinolin-4-amine (1.0 g, 4.483 mmol, 1 eq) in dichloromethane (15.0 mL). The reaction mixture was stirred at room temperature for 17 h. There was still some raw material left, and the reaction system was heated to 50°C to react for 1 h. The reaction mixture was cooled to room temperature, and extracted with dichloromethane (3 × 60 mL). The organic phases were combined, washed with saturated brine (2 × 60 mL) and dried over anhydrous sodium sulfate. The resulting mixture was filtered and the filtrate was concentrated under reduced pressure. The resulting residue was purified by silica gel column chromatography with petroleum ether/ethyl acetate (5:1) to give tert-butyl N-(7-bromoquinolin-4-yl)carbamate (1.16 g, 80.07%).

MS:(ESI, *m/z*): 323.05 $[M+H]^+$, RT(min):1.427

Step 2 Synthesis of tert-butyl N-(7-bromoquinolin-4-yl)carbamate (compound 112-4):

Under nitrogen protection, cyclopropylboronic acid (558.15 mg, 6.498 mmol, 3 eq), [1,1'-bis(diphenylphosphino) ferrocene]dichloropalladium dichloromethane complex (176.44 mg, 0.217 mmol, 0.1 eq) and cesium carbonate (2117.08 mg, 6.498 mmol, 3 eq) were added to a solution of tert-butyl N-(7-bromoquinolin-4-yl)carbamate (700 mg, 2.166 mmol, 1 eq) in 1,4-dioxane (2 mL) and water (0.5 mL) at room temperature. The reaction mixture was heated to 80°C and stirred for one hour. The reaction mixture was cooled to room temperature and extracted with ethyl acetate (3 × 50 mL). The organic phases were combined, washed with saturated brine (2 × 40 mL) and dried over anhydrous sodium sulfate. The resulting

mixture was filtered and the filtrate was concentrated under reduced pressure. The resulting residue was purified by silica gel column chromatography with petroleum ether/ethyl acetate (1:1) to give tert-butyl N-(7-cycloproylquinolin-4-yl) carbamate (530 mg, 86.05%).

MS:(ESI, *m/z*): 285.30 $[M+H]^+$, RT(min):1.054

Step 3 Synthesis of 7-cyclopropylquinolin-4-amine (compound 112-5):

To a solution of tert-butyl *N*-(7-cyclopropylquinolin-4-yl)carbamate (500 mg, 1.758 mmol, 1 eq) in dichloromethane (5 mL) was added 4 mol/L hydrogen chloride in 1,4-dioxane (2.5 mL) at room temperature and the mixture was stirred for 30 min. The resulting residue was concentrated under reduced pressure to obtain the product 7-cyclopropylquinolin-4-amine (240 mg, 74.08%).

MS:(ESI, *m/z*): 185.20 $[M+H]^+$, RT(min):0.672

Step 4 Synthesis of 2-(6-azaspiro[2.5]octan-6-yl-*N*-(7-cyclopropylquinolin-4-yl)-4-iodobenzamide (compound 112-6):

Under nitrogen protection, *N*,*N*,*N'*,*N'*-tetramethylchloroformamidine hexafluorophosphate (609.16 mg, 2.172 mmol, 4 eq) and N-methylimidazole (445.64 mg, 5.430 mmol, 10 eq) were added to a solution of 7-cyclopropylquinolin-4-amine (100 mg, 0.543 mmol, 1 eq) and 2-(6-azaspiro[2.5]octan-6-yl-4-iodobenzoic acid (290.81 mg, 0.815 mmol, 1.5 eq) in dichloromethane(4 mL) at room temperature. The reaction mixture was stirred at room temperature for one hour. The reaction mixture was quenched with water (3 mL). The reaction mixture was extracted with dichloromethane (2 × 40 mL). The organic phases were combined, washed with saturated brine (2 × 20 mL), and dried over anhydrous sodium sulfate. The resulting mixture was filtered and the filtrate was concentrated under reduced pressure. The resulting residue was purified by silica gel column chromatography with petroleum ether/ethyl acetate (5:1) to give 2-(6-azaspiro[2.5]octan-6-yl-*N*-(7-cyclopropylquinolin-4-yl)-4-iodobenzamide (70 mg, 24.64%).

MS:(ESI, *m/z*): 524.45 $[M+H]^+$, RT(min):1.029

Step 5 Synthesis of 2-{6-azaspiro[2.5]octan-6-yl}-*N*-(7-cyclopropylquinolin-4-yl)-4-(2-hydroxyethylsulfonylamino)benzamide (compound 112):

Under nitrogen protection, to a solution of 2-{6-azaspiro[2.5]octan-6-yl}-N-(7-cyclopropylquinolin-4-yl)-4-iodobenzamide (60 mg, 0.115 mmol, 1 eq) in *N,N*-dimethylformamide (1 mL) were added 2-(methylamino)acetic acid (2.04 mg, 0.023 mmol, 0.2 eq), potassium carbonate (47.53 mg, 0.345 mmol, 3 eq), cuprous iodide (2.18 mg, 0.012 mmol, 0.1 eq) and 2-hydroxyethane-1-sulfonamide (17.21 mg, 0.138 mmol, 1.2 eq) at room temperature. The reaction mixture was heated to 120°C and stirred for 1.5 h. The reaction mixture was cooled to room temperature and extracted with ethyl acetate (2 × 30 mL). The organic phases were combined, washed with saturated brine (2 × 20 mL) and dried over anhydrous sodium sulfate. The resulting mixture was filtered and the filtrate was concentrated under reduced pressure. The crude product was purified by HPLC to give 2-{6-azaspiro[2.5]octan-6-yl}-*N*-(7-cyclopropylquinolin-4-yl)-4-(2-hydroxyethylsulfonylamino)benzamide (18.96 mg, 31.55%).

MS: (ESI, *m/z*):521.40 $[M+H]^+$, RT(min):1.352

$^1H$ NMR (400 MHz, DMSO- $d_6$) δ 11.99(s, 1H), 10.17(s, 1H), 8.76(d, 1H), 8.33(d, 1H), 8.23(d, 1H), 7.90(d, 1H), 7.69(d, 1H), 7.39(dd, 1H), 7.23(s, 1H), 7.06(d, 1H), 4.96(s, 1H), 3.78(t, 2H), 3.35(t, 2H), 3.03(d, 4H), 2.18(ddd, 1H), 1.41(s, 4H), 1.15 - 1.06(m, 2H), 0.91 - 0.83(m, 2H), 0.25(s, 4H).

**Biological Evaluation**

**Test Example 1**

Test name: imaging-based nuclear count analysis (NCA) in OVCAR-3 cells

Day 0 Compound dilution and treatment

a) The final test concentrations of AM-5308: 10000, 3333.3, 1111.1, 370.3, 123.4, 41.1, 13.7, 4.5, 1.5, 0.5 nM.
b) The final test concentrations of the test compound: 10000, 3333.3, 1111.1, 370.3, 123.4, 41.1, 13.7, 4.5, 1.5, 0.5 nM.
c) The cells were cultured in a 37°C, 5% $CO_2$ incubator for 4 days.
d) DMSO concentration was 0.1%.

Day 1 Seeding the cells into a 384-well cell culture plate

a) When the cell confluence reached 80%-90%, the cells were treated.
b) The cells were resuspended in a medium and then counted and diluted at a desired density.
c) A cell suspension containing appropriate cells was added to the 384-well plate at 30 μL/well: 600 cells/well.

Day 4 Testing

a) 30 μL of 8% fixative (final concentration 4%) was added and the plate was incubated at room temperature for 30 min.
b) The plate was centrifuged at 1000RPM for 30s.
c) The plate was washed twice with 60 μl/well PBS.
d) After immobilization, the cells were permeabilized and stained in 60 μL of a wash buffer containing 2 μg/mL Hoechst 33342 DNA dye (1% BSA, 0.2% Triton X-100, 1X PBS).
e) The plate was sealed and incubated at room temperature in the dark for 1 h.
f) It was washed with PBS 3 times.
g) 50μLPBS/well was added and the plate was scanned using HCS.
h) Data acquisition and detection

Data analysis

Inhibition rate (%) = 100 - (compound well readings - low-reading control well readings)/(high- reading control well readings - low-reading control well readings) * 100

High-reading control wells: cells + 30 nL DMSO; low-reading control wells: 10 μM AM-5308.
$IC_{50}$ (nM) was calculated using GraphPad Prism 8 software and the effect-dose curves of the compound were plotted.

Table 1 Bioactivity data of the compound of the present application

| Compound No. | NCA (OVCAR-3) $IC_{50}$ (nM) |
|---|---|
| 1 | 163.4 |
| 2 | 568.5 |
| 4 | 477.6 |
| 5 | 369 |
| 6 | 976 |
| 7 | 457.6 |
| 22 | 143 |
| 36 | 72.2 |
| 51 | 258 |
| 56 | 73.09 |
| 64 | 763.3 |
| 90 | 71.19 |
| 94 | 36.12 |
| 95 | 98.91 |
| 96 | 89.67 |

(continued)

| Compound No. | NCA (OVCAR-3) $IC_{50}$ (nM) |
|---|---|
| 96a | 60.9 |
| 97 | 58.8 |
| 98 | 169.5 |
| 99 | 109.4 |
| 100 | 110.5 |
| 106 | 124.9 |
| 108 | 182.9 |
| 111 | 160.7 |
| 112 | 116.9 |
| 113a | 97.89 |

**Test Example 2**

Test name: KIF18A enzyme activity test

Steps:

1) Compound dilution and treatment: The final concentrations of AM-5308: 10000, 3333.3, 1111.1, 370.3, 123.4, 41.1, 13.7, 4.5, 1.5, 0.5 nM, the final testing concentrations of the testing compound: 10000, 3333.3, 1111.1, 370.3, 123.4, 41.1, 13.7, 4.5, 1.5 and 0.5 nM.
2) To each well of the reaction plate, 100 nL of the diluted compound stock solution was transferred using Echo 655. The final concentration of DMSO was 1%.
3) The reaction plate was sealed with a sealing membrane and centrifuged at 1000g for 1 min.
4) A 2× enzyme solution was prepared with 1× reaction buffer.
5) To each well of the reaction plate, 5 μL of 2×enzyme solution was added. The plate was sealed with a sealing membrane, centrifuged at 1000g for 1 min and left at room temperature for 15 min.
6) A 2× ATP solution was prepared with 1× reaction buffer.
7) To the reaction plate, 5 μL of 2×ATP solution was added, the plate was centrifuged at 1000g for 1 min, and the reaction was started.
8) The system was allowed to react at room temperature for 60 min.
9) 10 μL of ADP Glo reagent was added. The system was centrifuged at 1000 g for 1 min and incubated at room temperature for 60 min.
10) 20 μL of kinase detection reagent was added. The system was centrifuged at 1000 g for 1 min, and incubated at room temperature for 60 min.
11) The system was centrifuged at 1000 g for 1 min.
12) The luminescence signals were read on Envision 2104.

Data analysis:

The inhibition percentage is calculated as follows:

$$\%inhibition = 100- (Signal_{cmpd}-Signal_{Ave_PC})/(Signal_{Ave_VC}-Signal_{Ave_PC}) \times 100$$

$Signal_{cmpd}$: Average value of the test compound on the reaction plate.
$Signal_{Ave_PC}$: Average value of the positive control (AM-5308) on the reaction plate.
$Signal_{Ave_VC}$: Average value of the negative control (DMSO) on the reaction plate.

$IC_{50}$ was calculated and the dose-effect curve was fit:
The $IC_{50}$ of the compound was obtained by non-linear fitting formula with GraphPad 8.0.

3) Quality control

Z factor > 0.5; S/B > 2.

Table 2 Enzyme activity data for the compound of the present application

| Compound No. | KIF18A $IC_{50}$ (nM) |
|---|---|
| 1 | 21.24 |
| 22 | 21.29 |
| 36 | 13.75 |
| 94 | 15.11 |
| 96 | 8.97 |
| 96a | 5.35 |
| 97 | 9.09 |
| 98 | 26.45 |

The embodiments of the technical solution of the present invention is described in an illustrative manner above. It is to be understood that the scope of protection of the present invention is not limited to the above-mentioned embodiments. Any modifications, equivalent substitutions, improvements, etc. made by those skilled in the art within the spirit and principles of the present invention should be included in the scope of protection of the claims of the present application.

## Claims

1. A compound represented by formula (I), a racemate, stereoisomer, tautomer, isotope-labeled counterpart, solvate, polymorph, pharmaceutically acceptable salt thereof or a prodrug compound thereof:

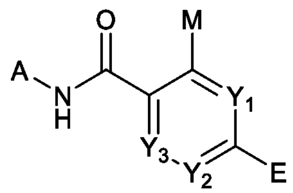

(I)

wherein, A is selected from fused ring groups which are unsubstituted or optionally substituted by one, two or more $R_a$ groups; the fused ring group comprises two, three or four rings independently selected from a saturated or partially unsaturated $C_{3-14}$ carbon ring, a $C_{6-14}$ aromatic ring, a 5- to 14-membered heteroaromatic ring, or a 3- to 14-membered heterocyclic ring;
the $R_a$ groups are each identical or different and are independently selected from H, OH, halogen, cyano, $NH_2$, $NO_2$, the following groups which are unsubstituted or optionally substituted by one, two or more $R_{a1}$ groups: $C_{1-12}$ alkyl, $C_{1-12}$ alkoxy, $C_{3-12}$ cycloalkyl; or two $R_a$ groups attached to the same ring carbon atom together with the carbon atom to which they are attached form a saturated or partially unsaturated $C_{3-14}$ carbon ring; or, two non-adjacent $R_a$ groups are linked at their end groups to form $C_{1-3}$ alkylene; the $R_{a1}$ groups are each identical or different and are independently selected from H, OH, halogen, cyano, $NH_2$, $NO_2$, $C_{1-12}$ alkyl, $C_{1-12}$ alkoxy, $C_{3-12}$ cycloalkyl;
E is selected from the following groups which are unsubstituted or optionally substituted by one, two or more $R_e$ groups: $-NH-S(=O)_2-R_{e1}$, $-S(=O)_2-NH-R_{e2}$, $-S(=O)(=NH)-R_{e3}$, $-N(R_{e4})(R_{e5})$, 3- to 14-membered heterocyclic groups; the $R_e$ groups are each identical or different and are independently selected from OH, halogen, cyano,

$C_{1-12}$ alkyl, $C_{1-12}$ alkoxy, halo-$C_{1-12}$ alkyl, halo-$C_{1-12}$ alkoxy, cyano-$C_{1-12}$ alkyl, cyano-$C_{1-12}$ alkoxy, -N($R_{e6}$)($R_{e7}$); the $R_{e1}$, $R_{e2}$, $R_{e3}$, $R_{e4}$, $R_{e5}$, $R_{e6}$, $R_{e7}$ groups are each identical or different and are independently selected from H, $C_{1-12}$ alkyl, hydroxy-$C_{1-12}$ alkyl, halo-$C_{1-12}$ alkyl, halo-$C_{1-12}$ alkoxy, cyano-$C_{1-12}$ alkyl, cyano-$C_{1-12}$ alkoxy, $C_{3-12}$ cycloalkyl, 3- to 14-membered heterocyclic groups, $C_{1-12}$ alkoxy-$C_{1-12}$ alkyl;

M is selected from $C_{3-12}$ cycloalkyl, $C_{3-12}$ cycloalkenyl or nitrogen-containing 3- to 14-membered heterocyclic groups, which are unsubstituted or optionally substituted by one, two or more $R_m$ groups; the $R_m$ groups are each identical or different and are independently selected from H, halogen, cyano, $C_{1-12}$ alkyl, halo-$C_{1-12}$ alkyl, cyano-$C_{1-12}$ alkyl, $C_{1-12}$ alkoxy, cyano-$C_{1-12}$ alkoxy; or, two $R_m$ groups attached to the same ring carbon atom together with the carbon atom to which they are attached form a saturated or partially unsaturated $C_{3-14}$ carbon ring;

$Y_1$, $Y_2$, $Y_3$ are identical or different and are independently selected from N or CH.

**2.** The compound, racemate, stereoisomer, tautomer, isotope-labeled counterpart, solvate, polymorph, pharmaceutically acceptable salt thereof or prodrug compound thereof according to claim 1, wherein A is selected from fused ring groups which are unsubstituted or optionally substituted by one, two or more $R_a$ groups; the fused ring group comprises two, three or four rings independently selected from a saturated or partially unsaturated $C_{3-8}$ carbon ring, a $C_{6-10}$ aromatic ring, a 5- to 10-membered heteroaromatic ring, a 3- to 8-membered heterocyclic ring;

the $R_a$ groups are each identical or different and are independently selected from H, OH, halogen, cyano, $NH_2$, $NO_2$, $C_{1-6}$ alkyl, $C_{1-6}$ alkoxy, $C_{3-8}$ cycloalkyl, halo-$C_{1-6}$ alkyl, halo-$C_{1-6}$ alkoxy, cyano-$C_{1-6}$ alkyl, cyano-$C_{1-6}$ alkoxy, $C_{3-8}$ cycloalkyl-$C_{1-6}$ alkoxy; or, two $R_a$ groups attached to the same ring carbon atom together with the carbon atom to which they are attached form a saturated or partially unsaturated $C_{3-8}$ carbon ring; or, two non-adjacent $R_a$ groups are linked at their end groups to form $C_{1-3}$ alkylene;

preferably, A is selected from fused ring groups which are unsubstituted or optionally substituted by one, two, or more $R_a$ groups; the fused ring group is selected from

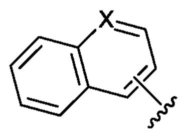,

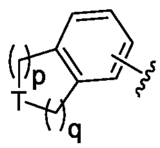, 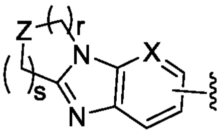;

wherein, T is selected from $CH_2$, CH, NH, $NR_a$ or O;

Z is selected from $CH_2$, CH, NH, $NR_a$ or O;

X is selected from N or CH;

p and q are independently selected from 0, 1, 2, and 3, and p and q are not 0 at the same time;

r and s are independently selected from 0, 1, 2, and 3, and r and s are not 0 at the same time;

preferably, A is selected from fused ring groups which are unsubstituted or optionally substituted by one, two, or more $R_a$ groups; the fused ring group is selected from

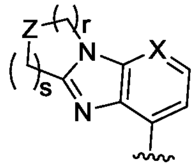,

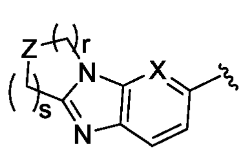 or 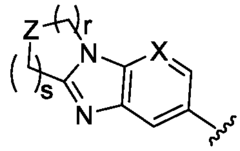;

wherein, Z is selected from $CH_2$, CH, NH, $NR_a$ or O;
X is selected from N or CH;
r and s are independently selected from 0, 1, 2, and 3, and r and s are not 0 at the same time;
preferably, A is selected from

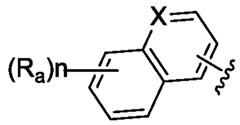
,
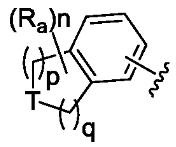
,

and

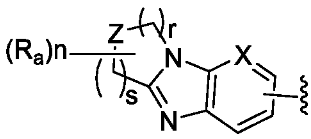
;

wherein, T is selected from $CH_2$, CH, NH, $NR_a$ or O;
Z is selected from $CH_2$, CH, NH, $NR_a$ or O;
X is selected from N or CH;
n is selected from 0, 1, 2, 3, 4, or 5;
p and q are independently selected from 0, 1, 2, and 3, and p and q are not 0 at the same time;
r and s are independently selected from 0, 1, 2, and 3, and r and s are not 0 at the same time;
preferably, A is selected from fused ring groups which are unsubstituted or optionally substituted by one, two or more $R_a$ groups; the fused ring group is formed by fusing two, three, four or more rings selected from a benzene ring, a pyridine ring, an imidazole ring, a piperazine ring, a piperidine ring, a tetrahydropyrrole ring, a tetrahydropyran ring, a tetrahydrofuran ring, a furan ring, a morpholine ring, a cyclopropane ring, a cyclobutane ring, a cyclopentane ring, a cyclohexane ring, a cycloheptane ring, and a cycloheximide ring;
preferably, A is selected from

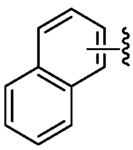
,
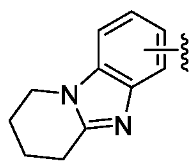
,
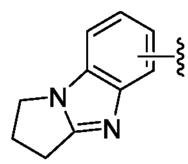
,
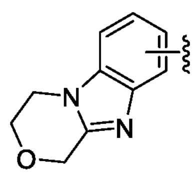
,
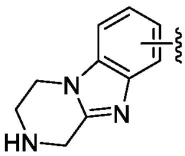
,
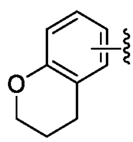
,
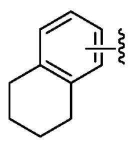
,
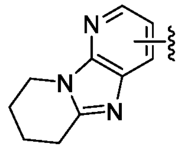
,
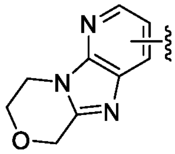
,
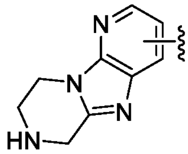
,
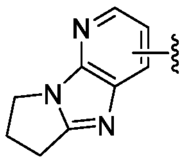
,
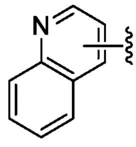
,
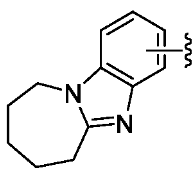
,
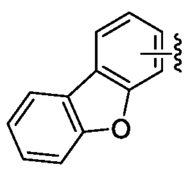
,
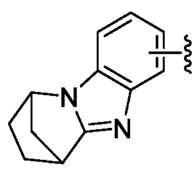

which are unsubstituted or optionally substituted by one, two, or more $R_a$ groups;
preferably, the $R_a$ groups are each identical or different and are independently selected from H, OH, F, Cl, cyano, methyl, ethyl, isopropyl, methoxy, ethoxy, trifluoromethyl, difluoromethoxy, trifluoromethoxy, cyclopropyl, cyclopropylmethoxy; or, two $R_a$ groups attached to the same ring carbon atom together with the carbon atom to which they are attached form a cyclopropane ring, a cyclobutane ring, a cyclopentane ring, a cyclohexane ring, a cycloheptane ring, or two non-adjacent $R_a$ groups are linked at their end groups to form methylene or ethylene;

preferably, A is selected from

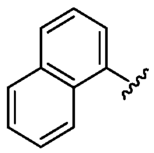 , 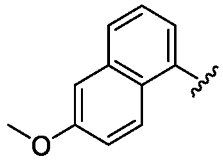 , 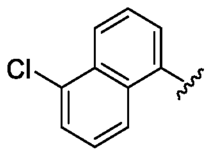 ,

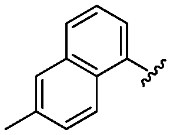 , 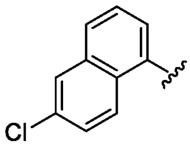 , 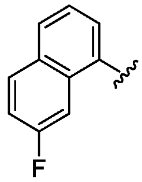 , 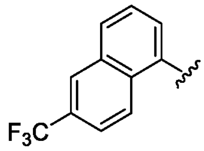 , 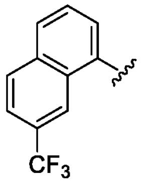 , 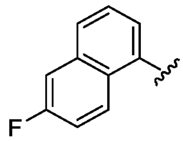 ,

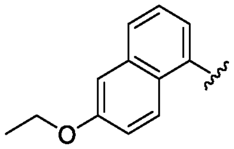 , 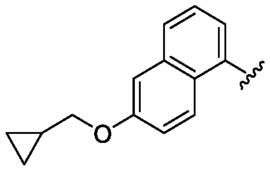 , 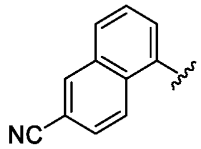 , 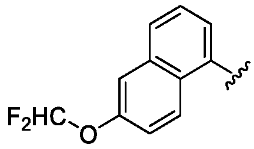 ,

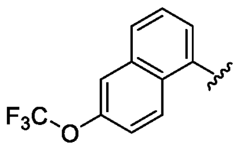 , 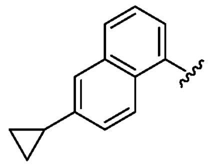 , 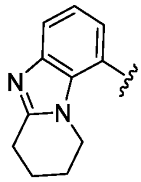 , 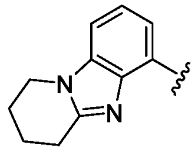 , 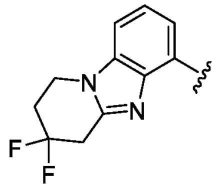 ,

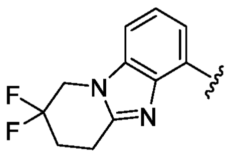 , 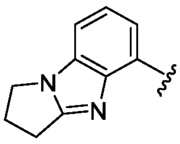 , 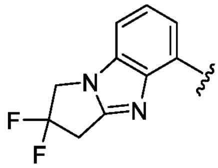 , 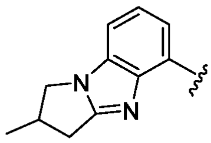 , 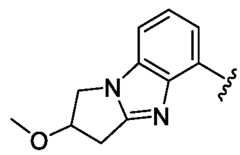 ,

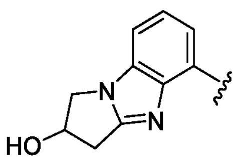 , 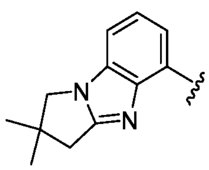 , 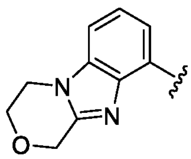 , 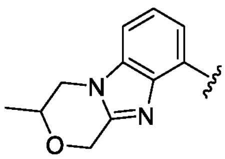 , 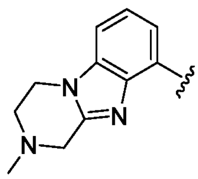 ,

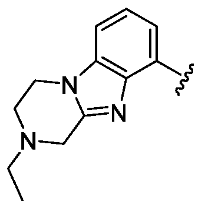 , 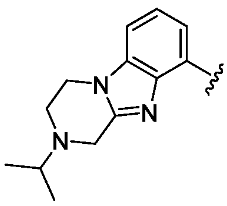 , 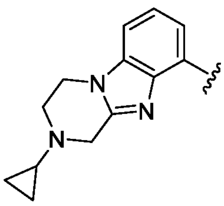 , 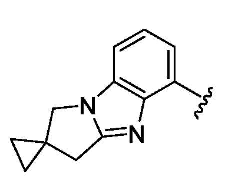 ,

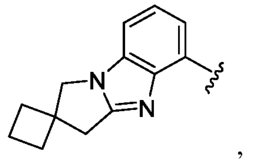,
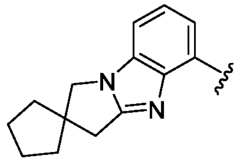,
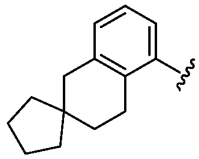,
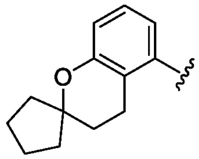,
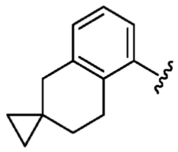,

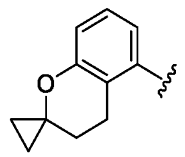,
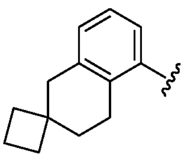,
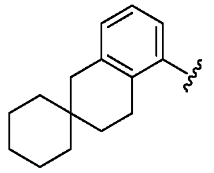,
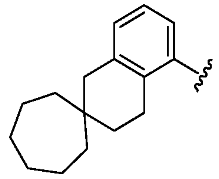,
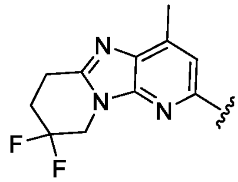,

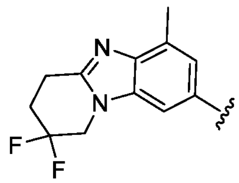,
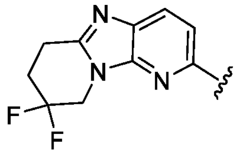,
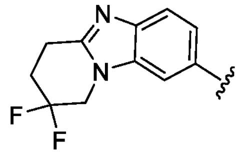,
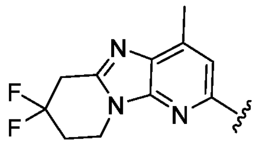,

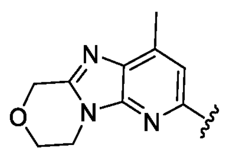,
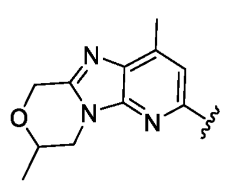,
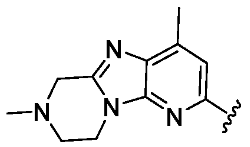,
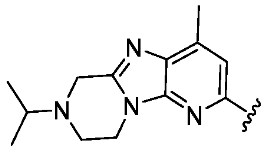,

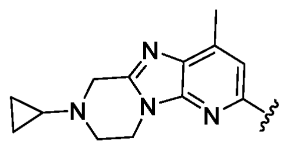,
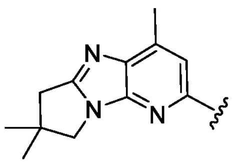,
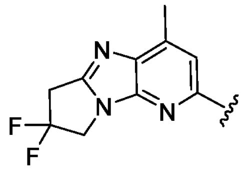,
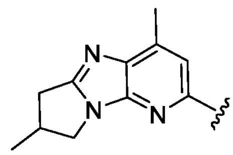,

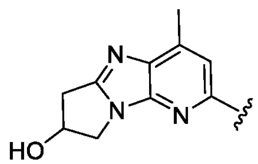,
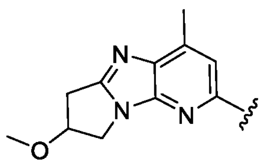,
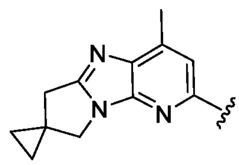,
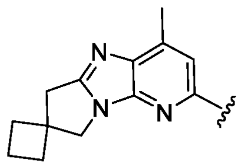,

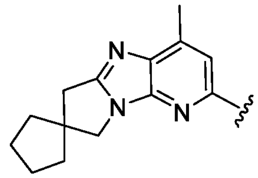,
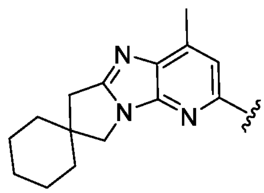,
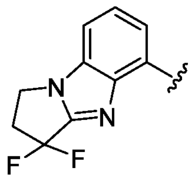,
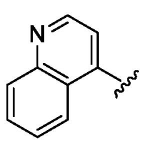,
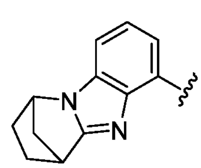,

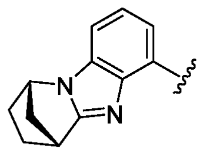 , 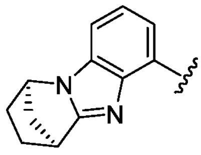 , 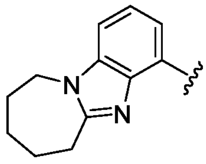 , 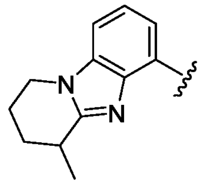 , 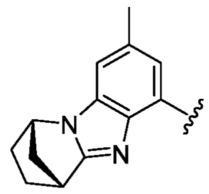 ,

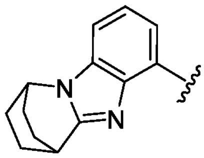 , 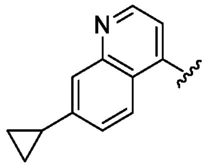 , 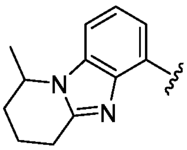 , 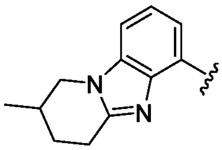 , 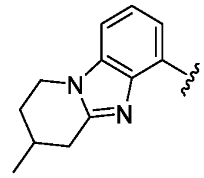 ,

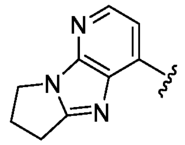 , 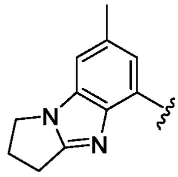 , 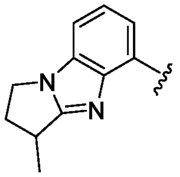 , 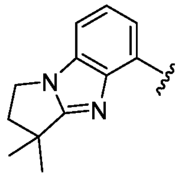 , 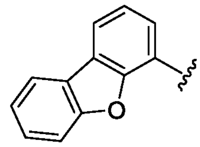 ,

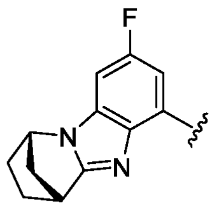 .

**3.** The compound, racemate, stereoisomer, tautomer, isotope-labeled counterpart, solvate, polymorph, pharmaceutically acceptable salt thereof or prodrug compound thereof according to claim 1 or 2, wherein E is selected from the following groups which are unsubstituted or optionally substituted by one or two $R_e$ groups: -NH-S(=O)$_2$-$R_{e1}$, -S(=O)$_2$-NH-$R_{e2}$, -S(=O)(=NH)-$R_{e3}$; the $R_{e1}$, $R_{e2}$, $R_{e3}$ groups are each identical or different and are independently selected from H, $C_{1-6}$ alkyl, hydroxy-$C_{1-6}$ alkyl, halo-$C_{1-6}$ alkyl, halo-$C_{1-6}$ alkoxy, cyano-$C_{1-6}$ alkyl, cyano-$C_{1-6}$ alkoxy, $C_{3-8}$ cycloalkyl, 3- to 8-membered heterocyclic groups, $C_{1-6}$ alkoxy-$C_{1-6}$ alkyl; the $R_e$ groups are each identical or different and are independently selected from OH, halogen, cyano, $C_{1-6}$ alkyl, $C_{1-6}$ alkoxy, halo-$C_{1-6}$ alkyl, halo-$C_{1-6}$ alkoxy, cyano-$C_{1-6}$ alkyl, cyano-$C_{1-6}$ alkoxy;
preferably, E is selected from

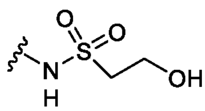 .

**4.** The compound, racemate, stereoisomer, tautomer, isotope-labeled counterpart, solvate, polymorph, pharmaceutically acceptable salt thereof or prodrug compound thereof according to any one of claims 1-3, wherein M is selected from $C_{3-8}$ cycloalkyl, $C_{3-8}$ cycloalkenyl or nitrogen-containing 3- to 8-membered heterocyclic groups, which are unsubstituted or optionally substituted by one, two or more $R_m$ groups; the $R_m$ groups are each identical or different and are independently selected from H, halogen, cyano, $C_{1-6}$ alkyl, halo-$C_{1-6}$ alkyl, cyano-$C_{1-6}$ alkyl, $C_{1-6}$ alkoxy and cyano-$C_{1-6}$ alkoxy; or, two $R_m$ groups attached to the same ring carbon atom together with the carbon atom to which they are attached form a saturated or partially unsaturated $C_{3-8}$ carbon ring;
preferably, M is selected from

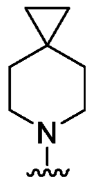 , 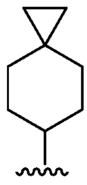 or 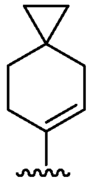 .

**5.** The compound, racemate, stereoisomer, tautomer, isotope-labeled counterpart, solvate, polymorph, pharmaceutically acceptable salt thereof or prodrug compound thereof according to any one of claims 1-4, wherein $Y_1$, $Y_2$, and $Y_3$ are not N at the same time;
preferably, when $Y_1$ is CH, $Y_2$ is CH, $Y_3$ is N or CH; when $Y_1$ is CH, $Y_2$ is N, $Y_3$ is N or CH; when $Y_1$ is N, $Y_2$ is CH, $Y_3$ is N or CH; when $Y_1$ is N, $Y_2$ is N, $Y_3$ is CH.

**6.** The compound, racemate, stereoisomer, tautomer, isotope-labeled counterpart, solvate, polymorph, pharmaceutically acceptable salt thereof or prodrug compound thereof according to any one of claims 1-5, wherein the compound has the following structure:

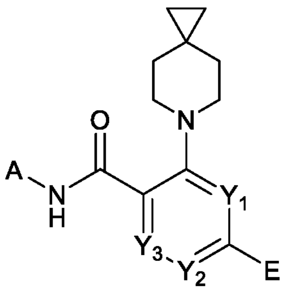

IA-1

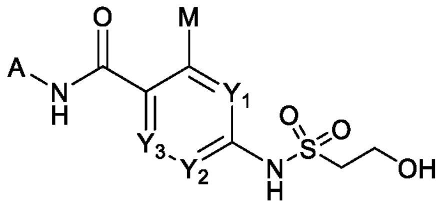

IA-2

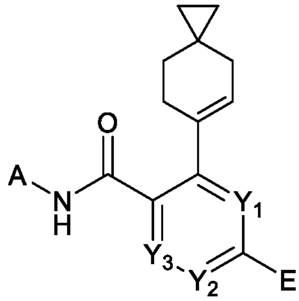

IA-3

wherein, A, M, E, $Y_1$, $Y_2$, and $Y_3$ are independently as defined in any one of claims 1-5;
preferably, the compound represented by formula (I) has the following structure:

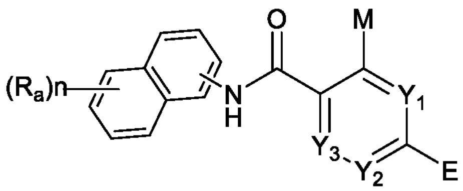

IB-1

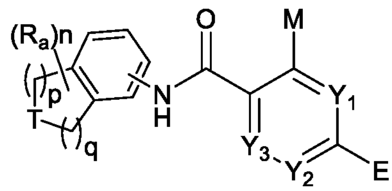

IB-2

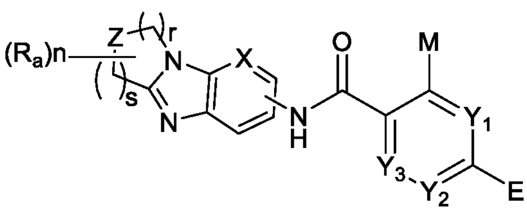

IB-3

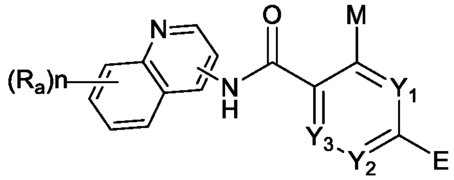

IB-4

wherein, M, E, $Y_1$, $Y_2$, $Y_3$, $R_a$, X, Z, T, n, p, q, r, s are independently as defined in any one of claims 1-5;
preferably, the compound represented by formula (I) has the following structure:

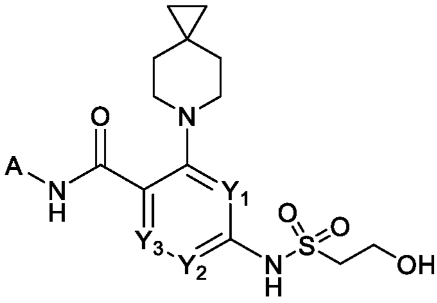

IC-1

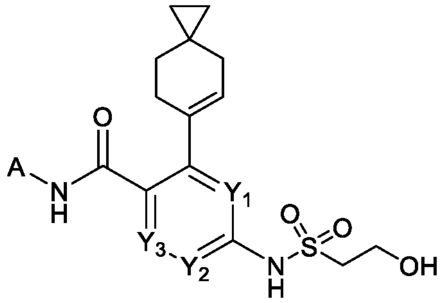

IC-2

wherein, A, $Y_1$, $Y_2$, and $Y_3$ are independently as defined in any one of claims 1-5;
preferably, the compound represented by formula (I) has the following structure:

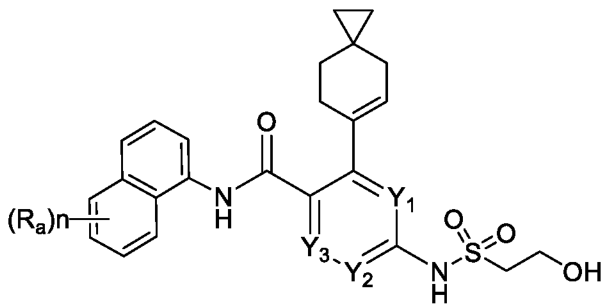

ID-1

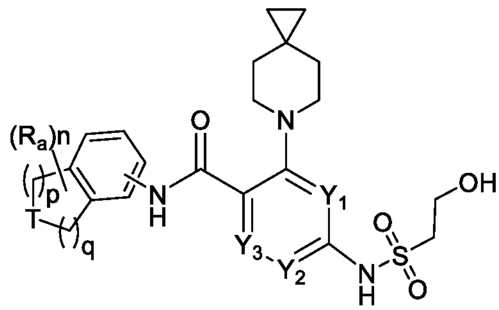

ID-2

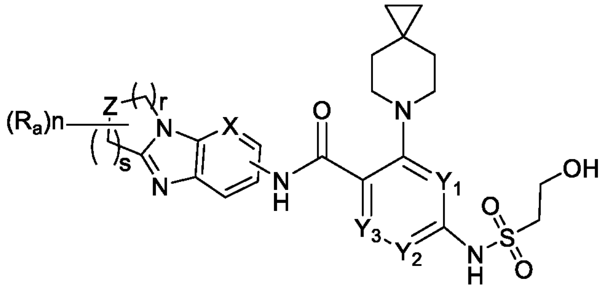

ID-3

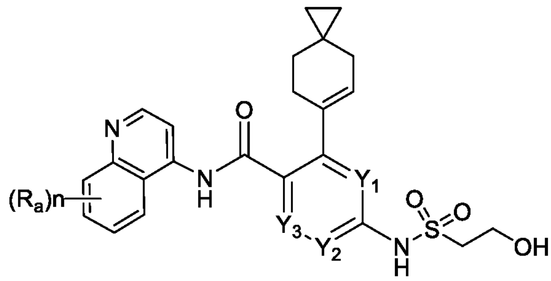

ID-4

wherein, $Y_1$, $Y_2$, $Y_3$, $R_a$, X, Z, T, n, p, q, r, s are independently as defined in any one of claims 1-5;
preferably, the compound represented by formula (I) has the following structure:

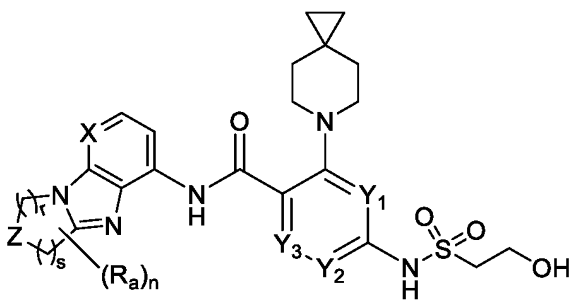

IE-1

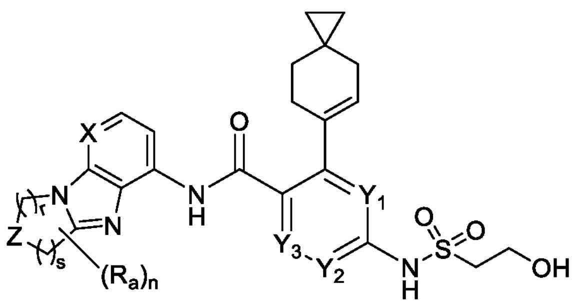

IE-2

wherein, $Y_1$, $Y_2$, $Y_3$, $R_a$, X, Z, n, r, s are independently as defined in any one of claims 1-5.

**7.** The compound, racemate, stereoisomer, tautomer, isotope-labeled counterpart, solvate, polymorph, pharmaceutically acceptable salt thereof or prodrug compound thereof according to any one of claims 1-6, wherein the compound is selected from the following structures:

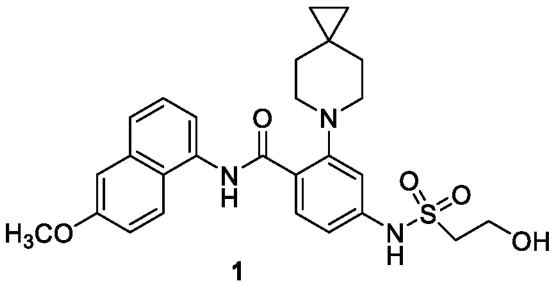
1
,
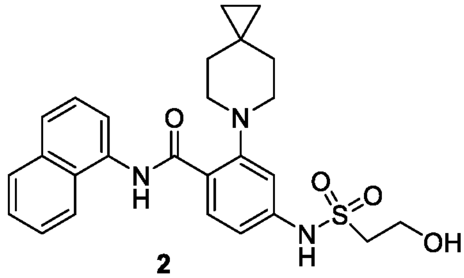
2
,

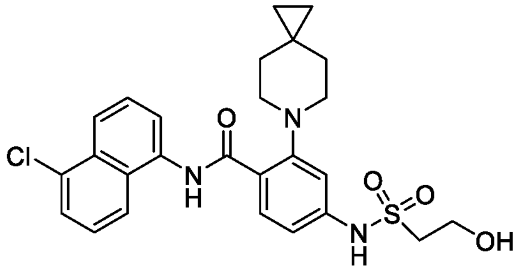
3
,
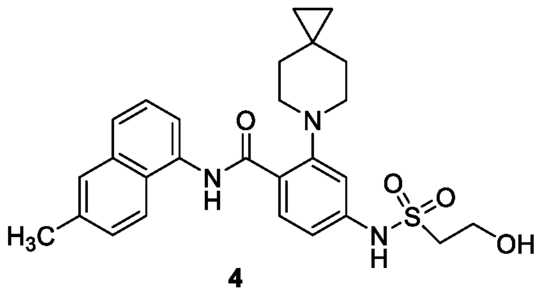
4
,

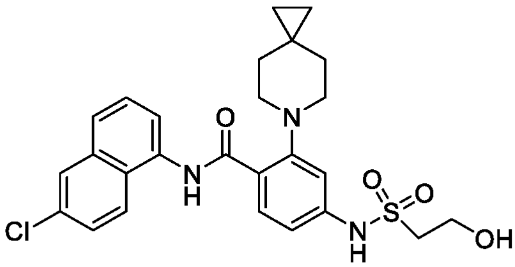
5
,
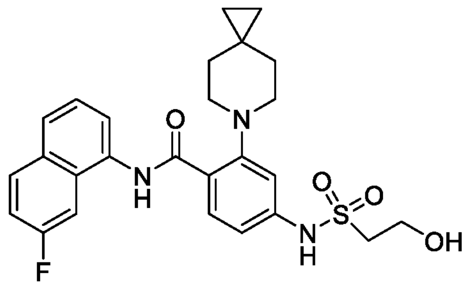
6
,

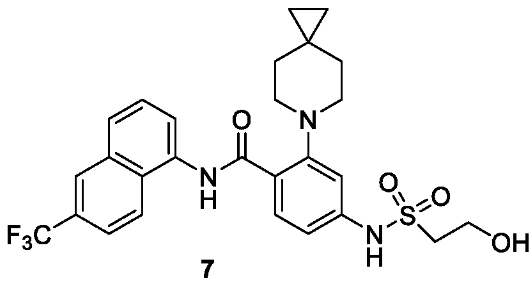
7
,
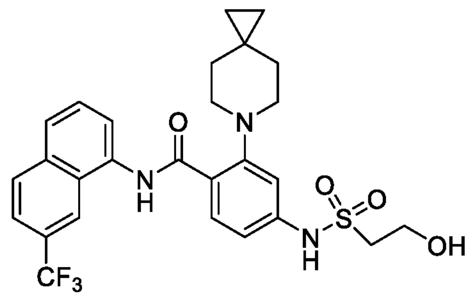
8
,

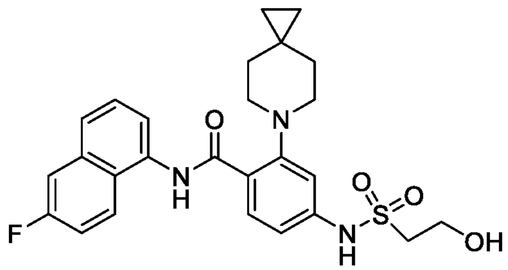

9

,

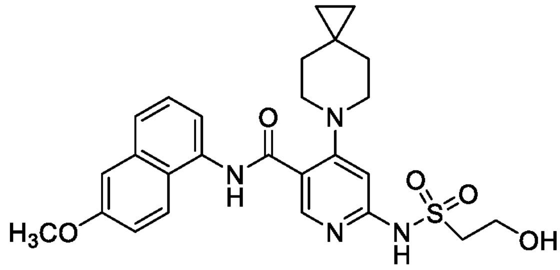

10

,

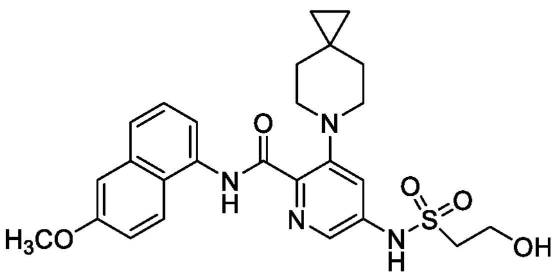

11

,

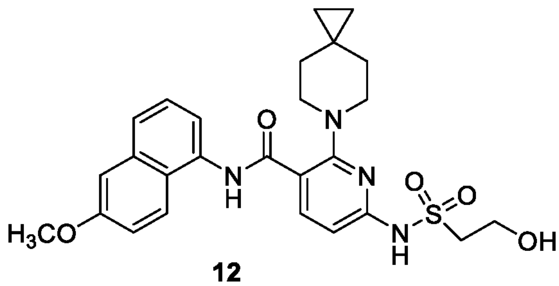

12

,

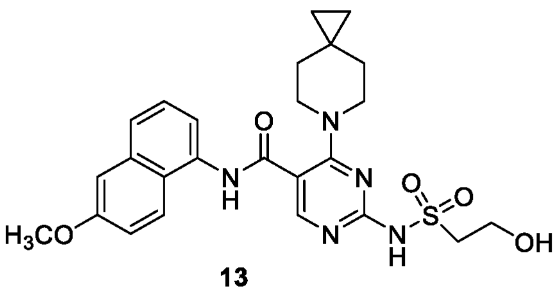

13

,

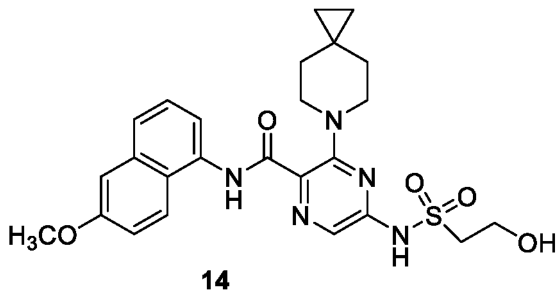

14

,

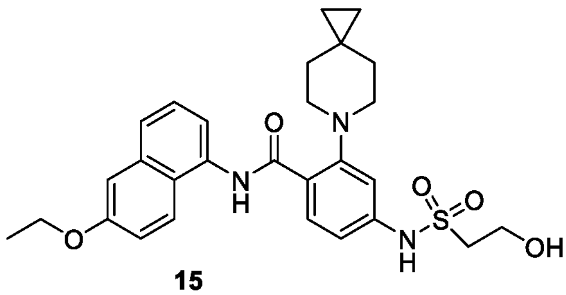

15

,

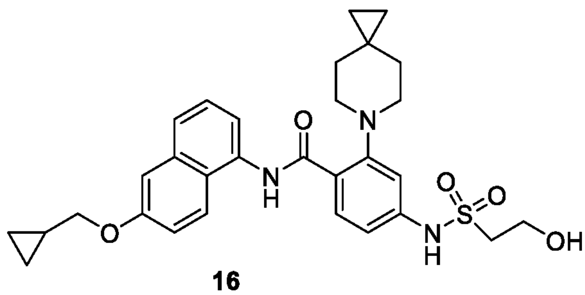

16

,

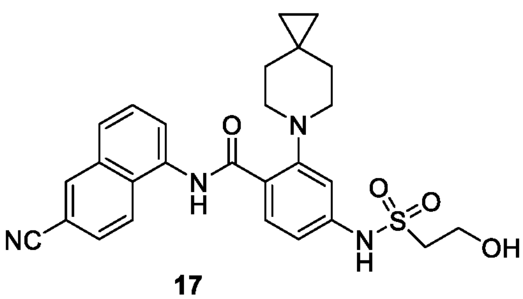

17

,

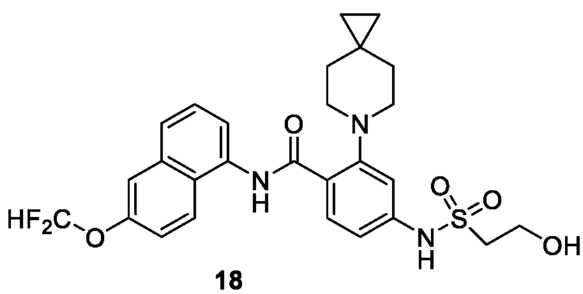

18

,

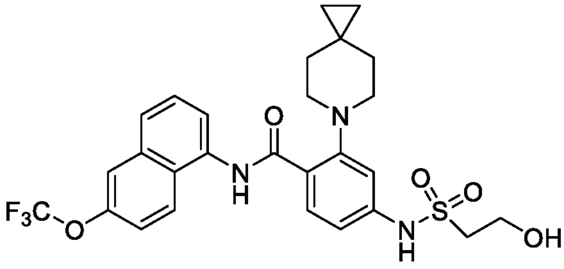
19 ,

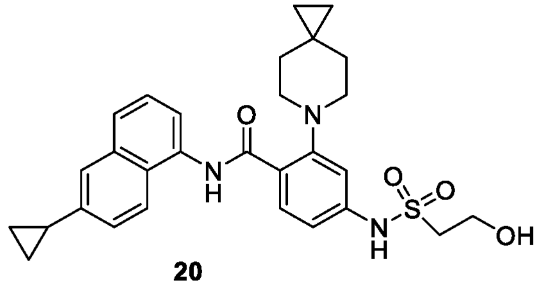
20 ,

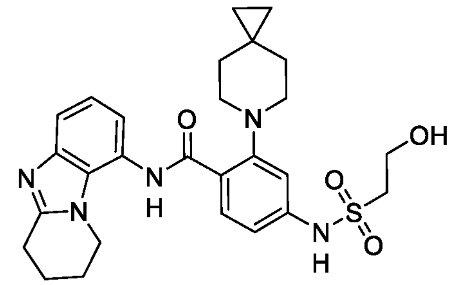
21 ,

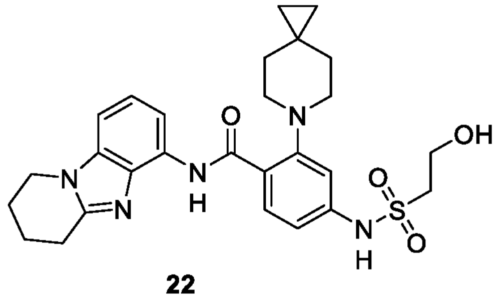
22 ,

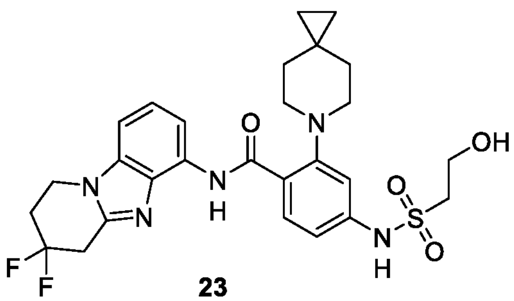
23 ,

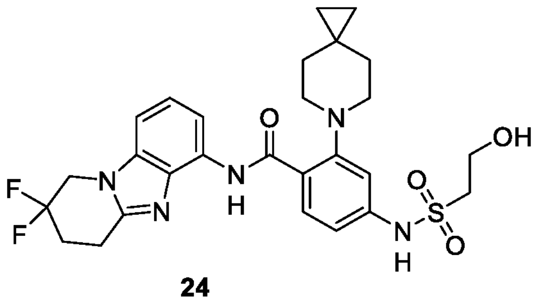
24 ,

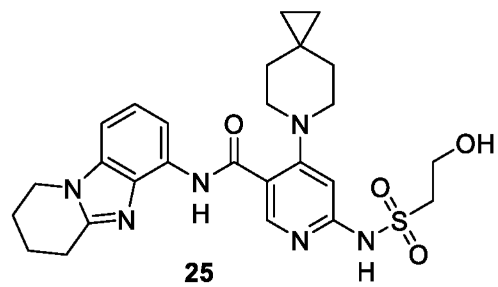
25 ,

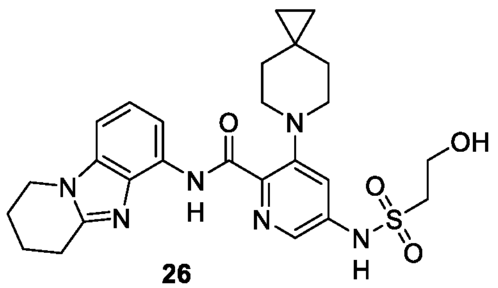
26 ,

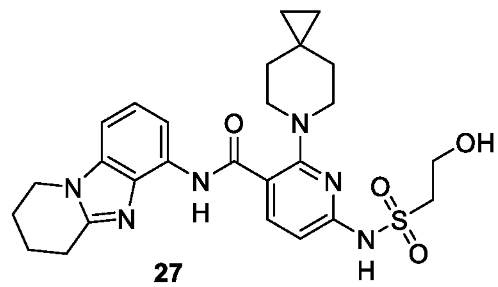
27 ,

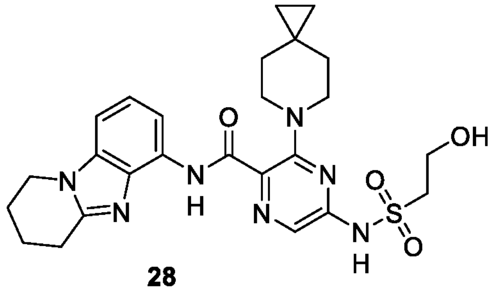
28 ,

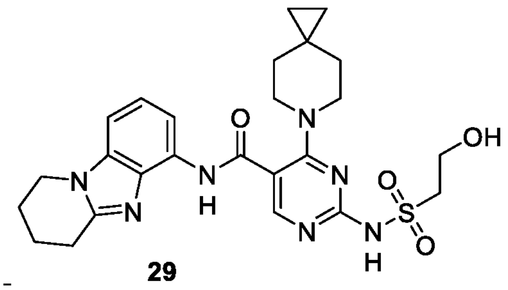

**29**,

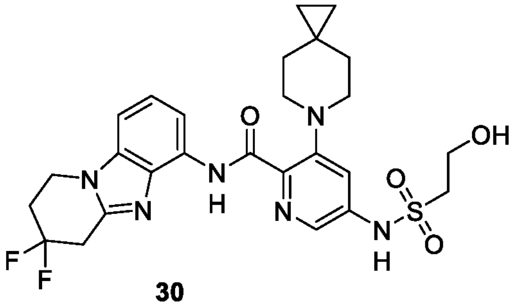

**30**,

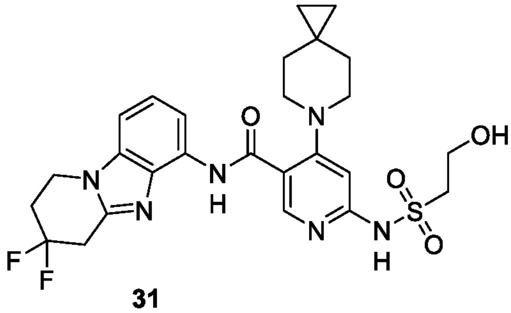

**31**,

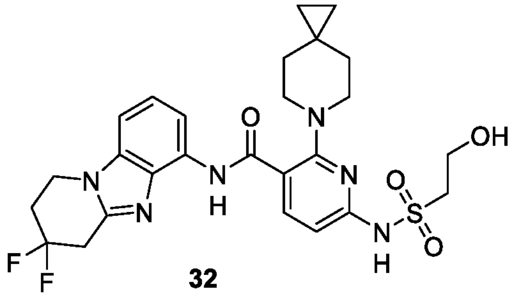

**32**,

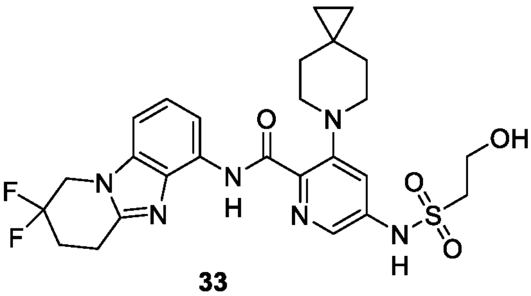

**33**,

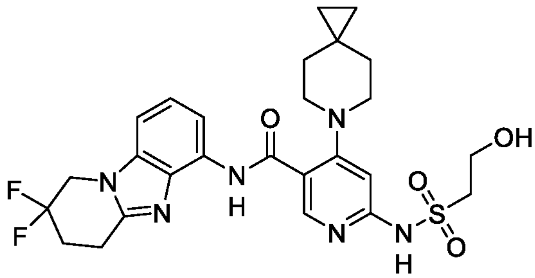

**34**,

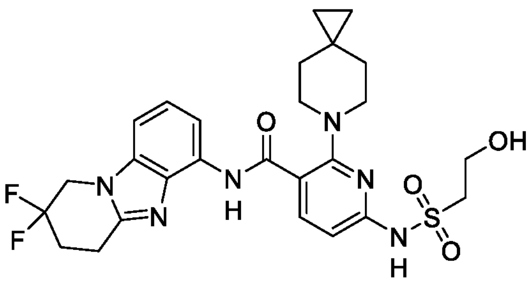

**35**,

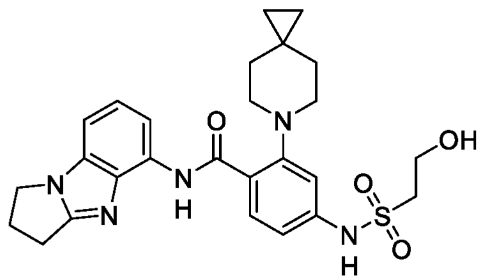

**36**,

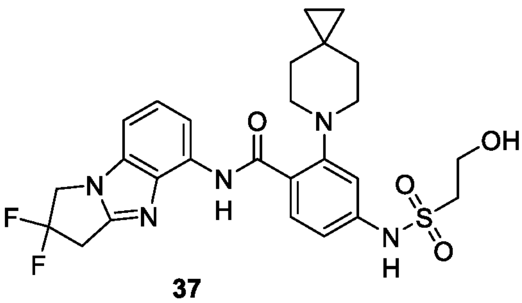

**37**,

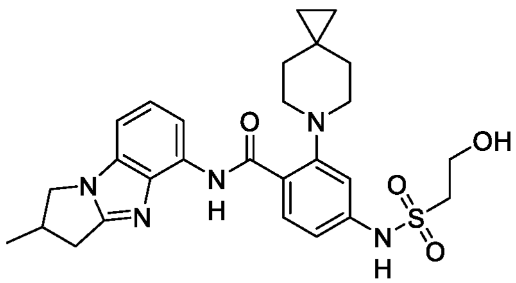

**38**,

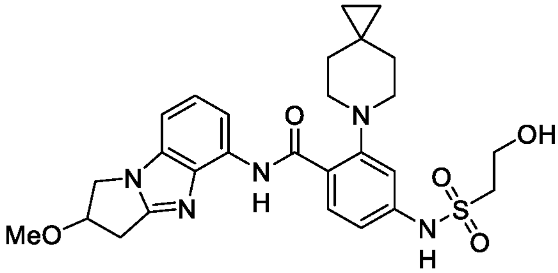

**39**

,

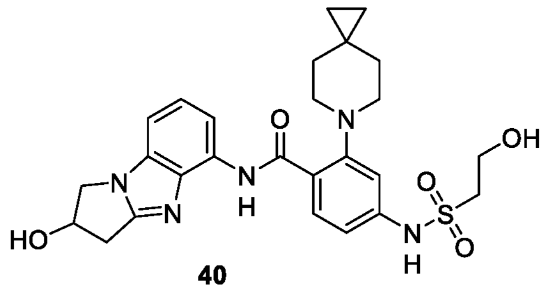

**40**

,

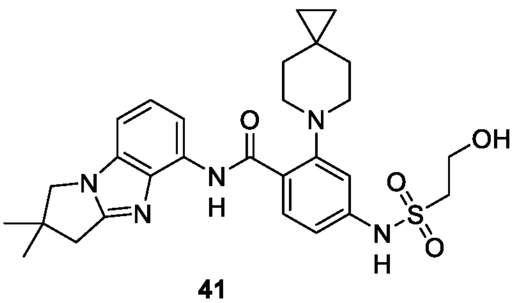

**41**

,

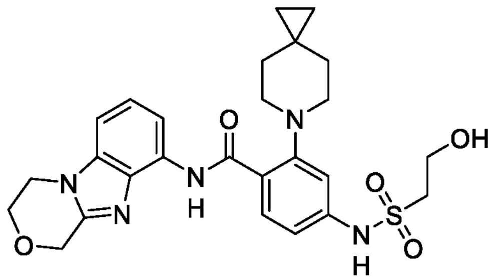

**42**

,

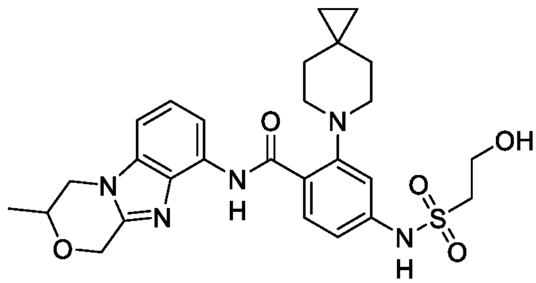

**43**

,

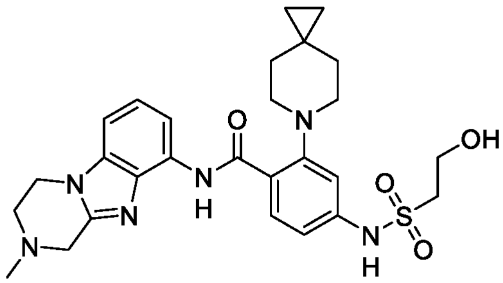

**44**

,

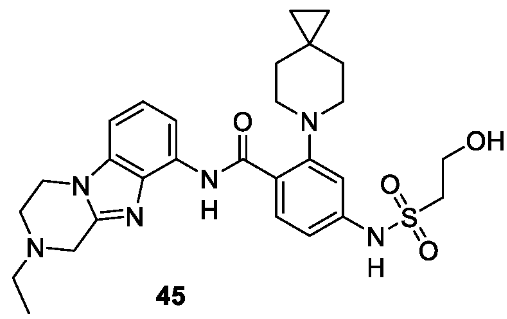

**45**

,

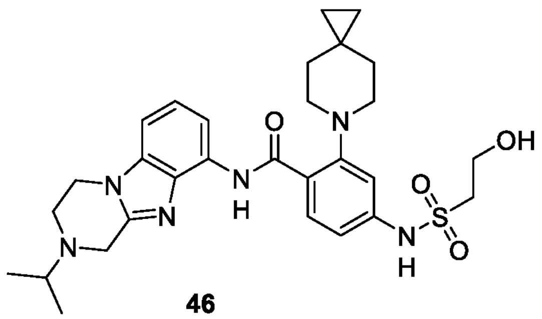

**46**

,

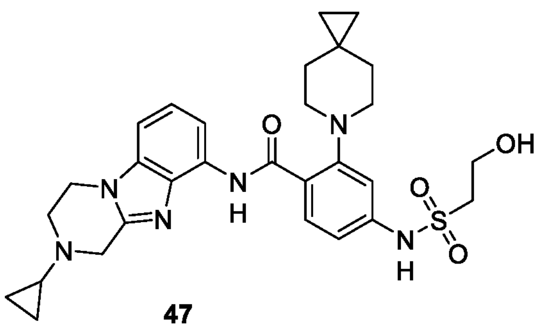

**47**

,

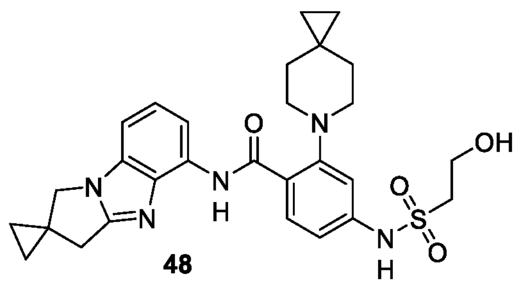

**48**

,

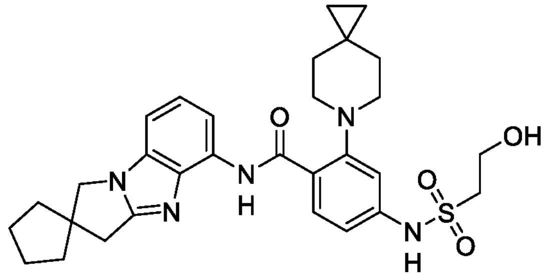

**49** ,

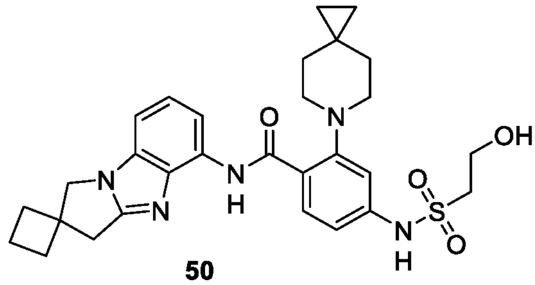

**50** ,

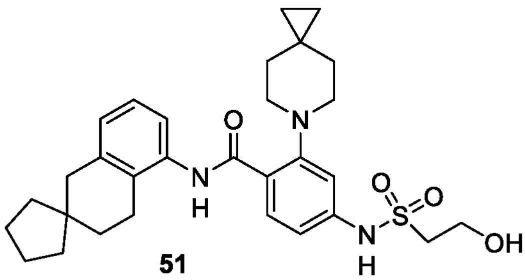

**51** ,

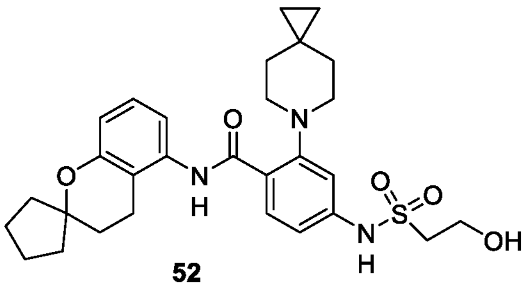

**52** ,

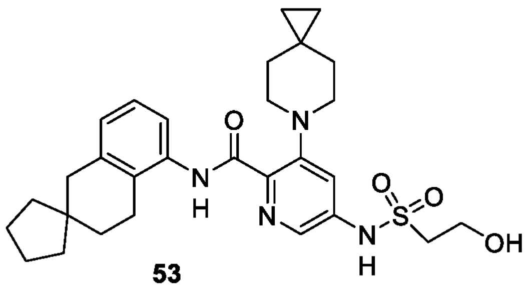

**53** ,

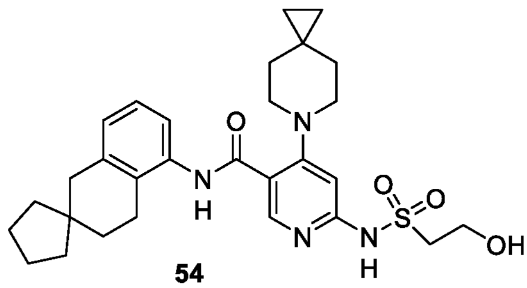

**54** ,

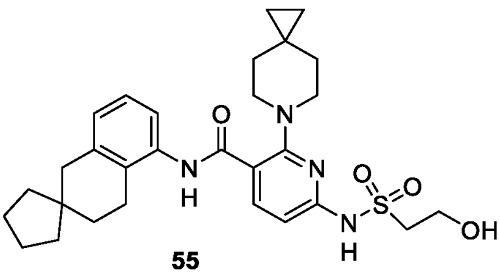

**55** ,

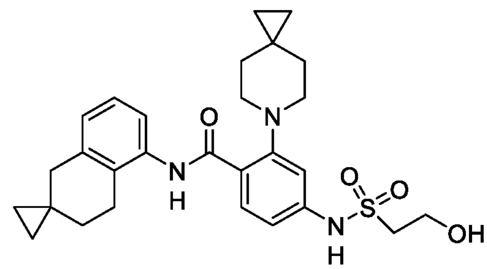

**56** ,

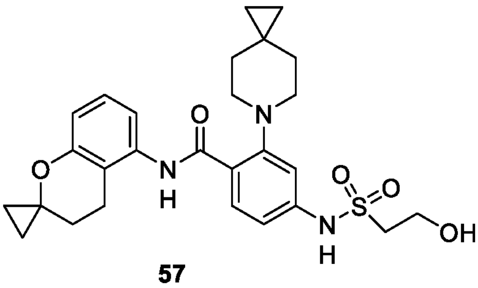

**57** ,

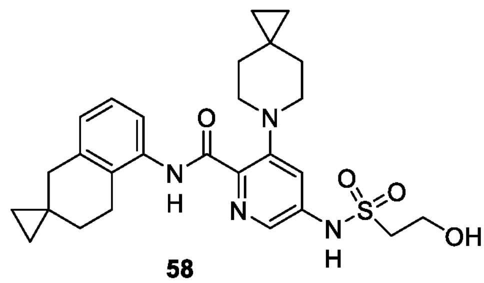

**58** ,

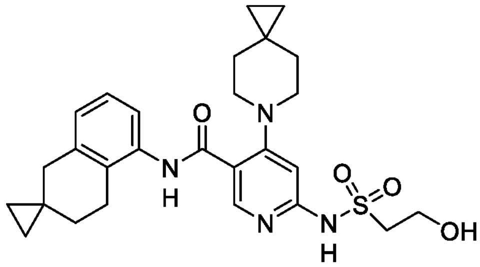

59

,

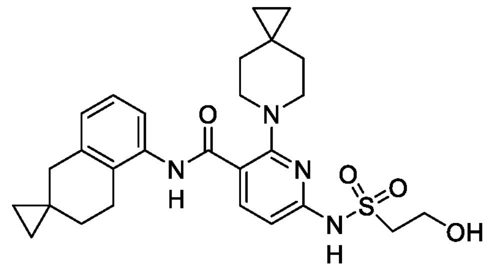

60

,

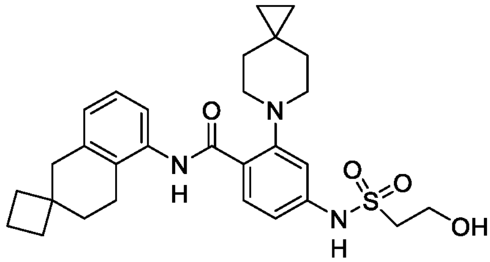

61

,

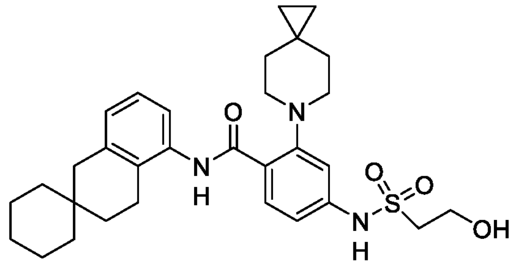

62

,

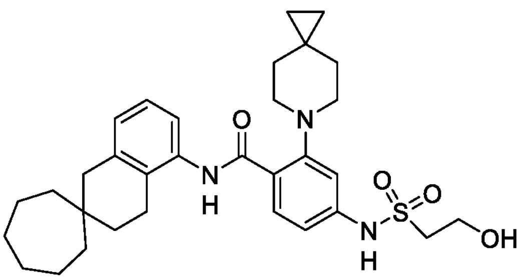

63

,

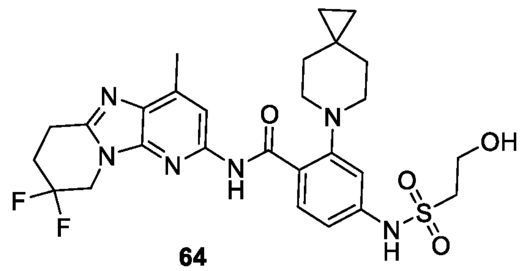

64

,

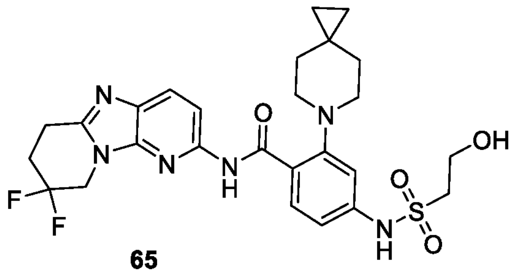

65

,

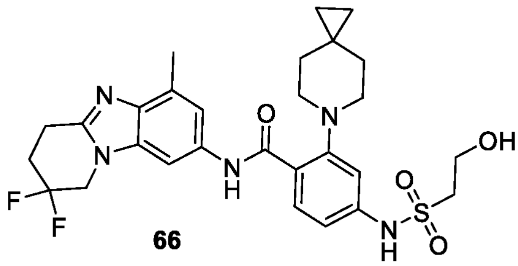

66

,

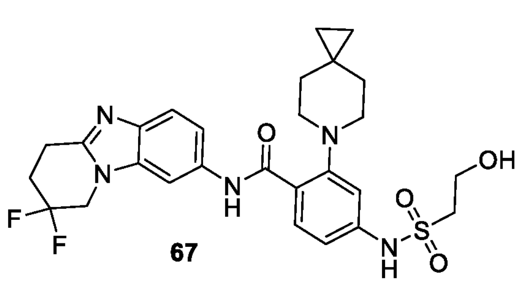

67

,

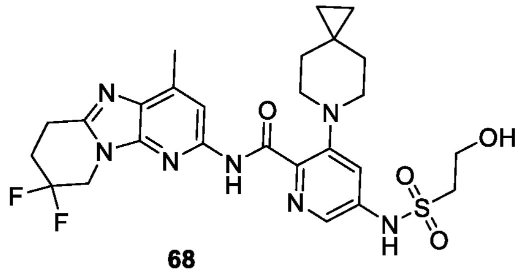

68

,

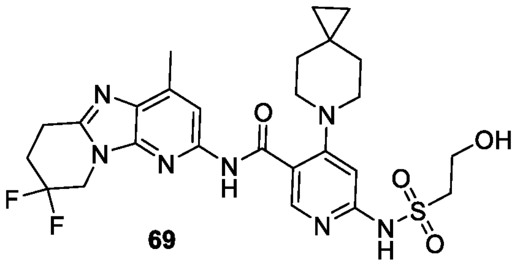

69

,

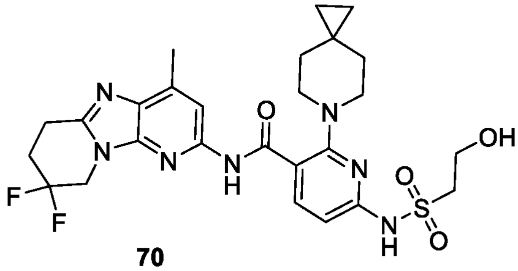

70

,

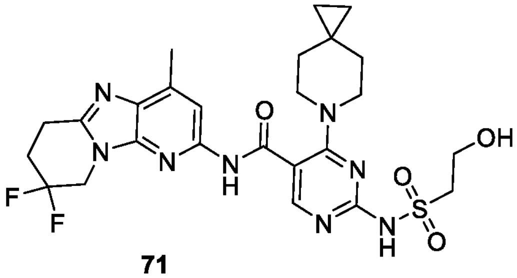

71

,

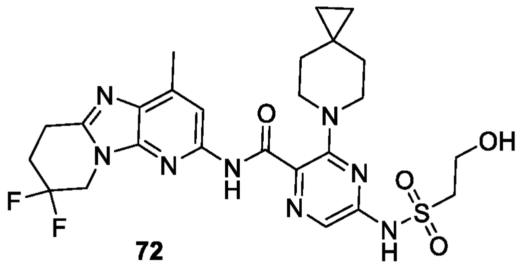

72

,

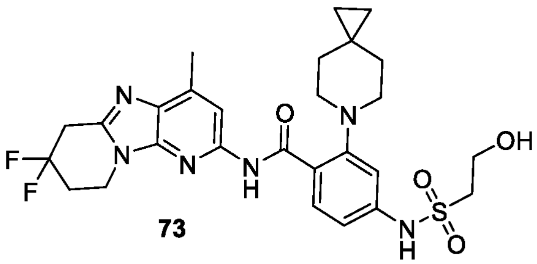

73

,

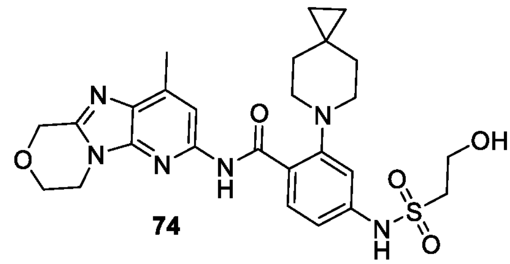

74

,

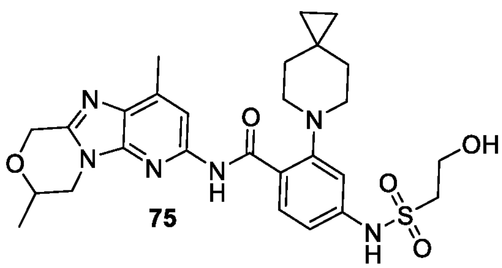

75

,

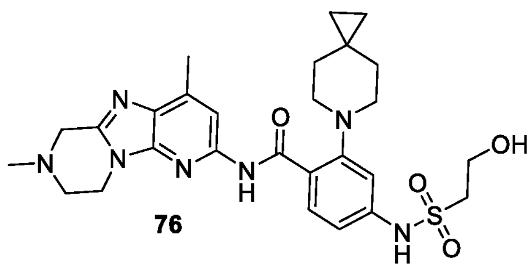

76

,

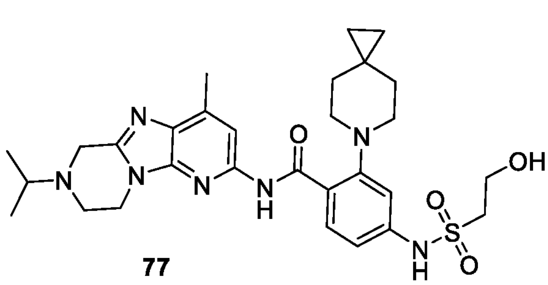

77

,

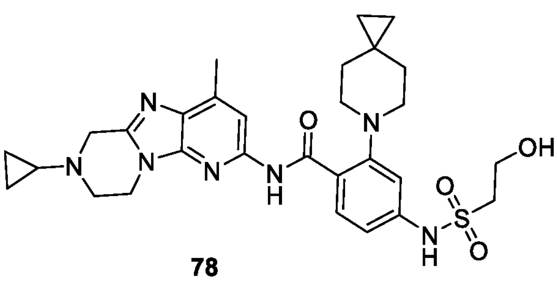

78

,

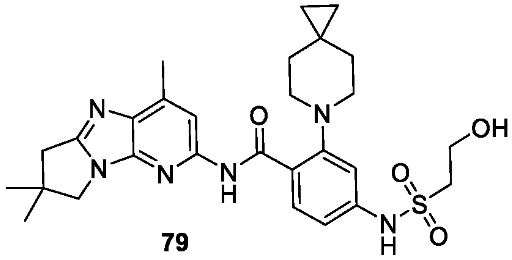

79

,

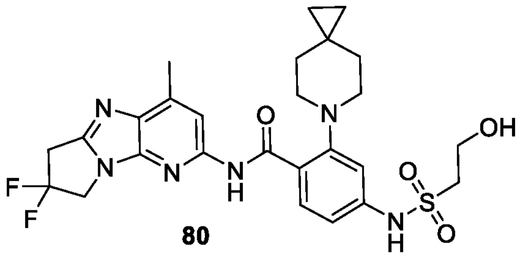

80

,

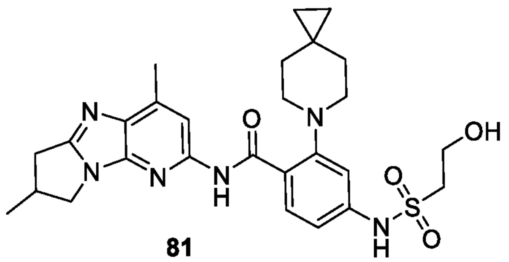

81

,

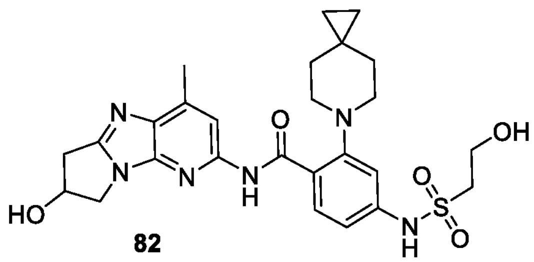

82

,

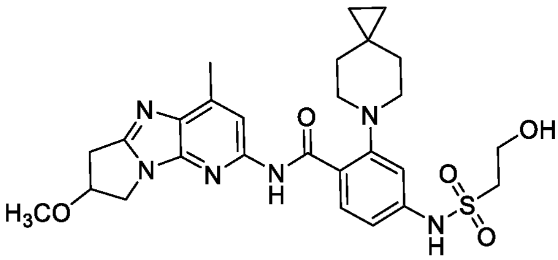

83

,

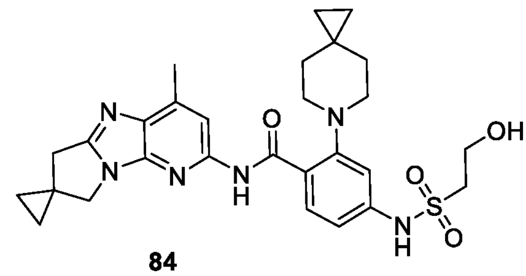

84

,

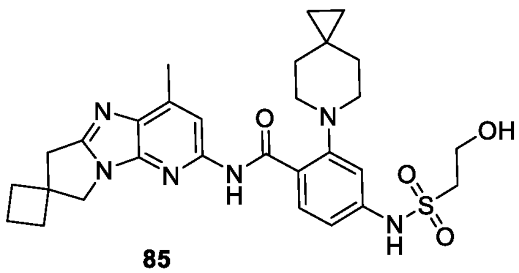

85

,

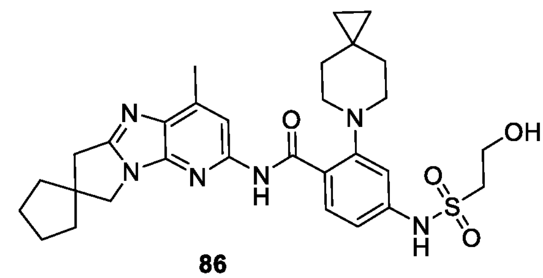

86

,

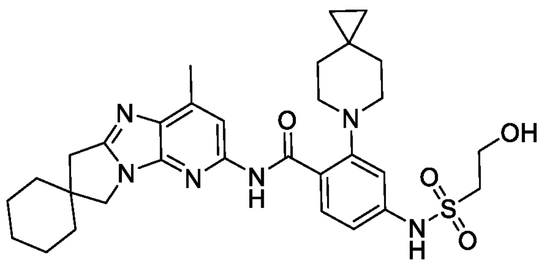

87

,

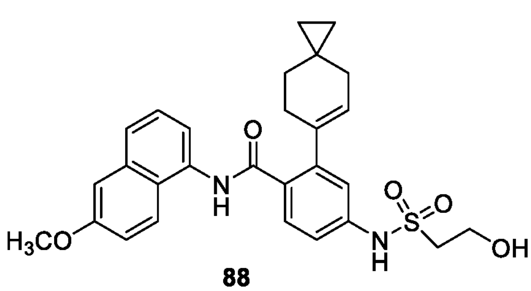

88

,

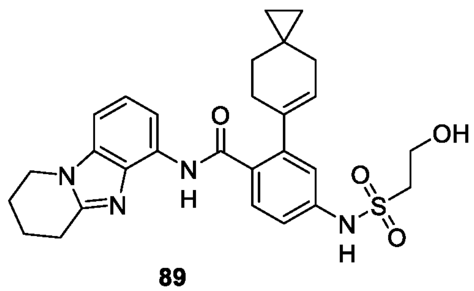

**89**

,

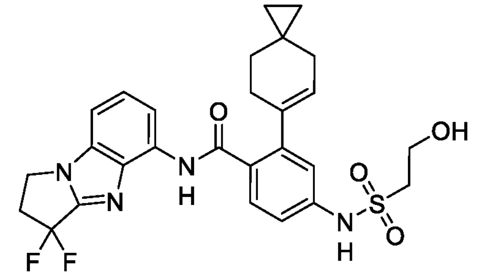

**90**

,

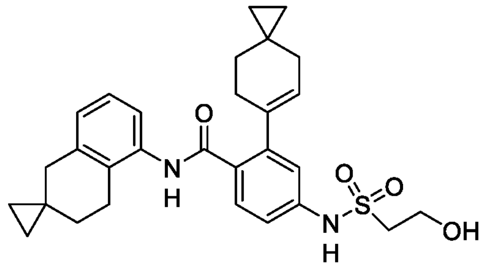

**91**

,

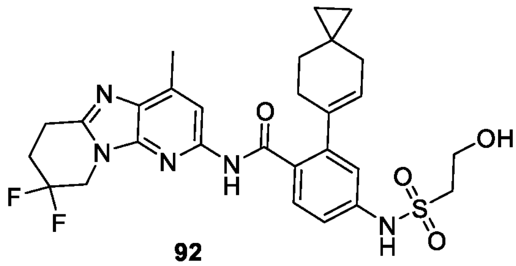

**92**

,

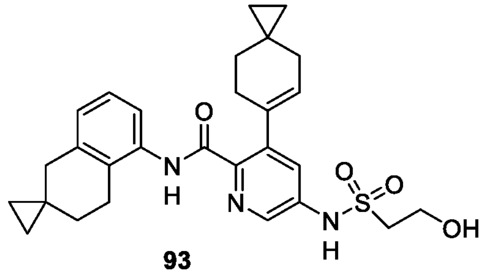

**93**

,

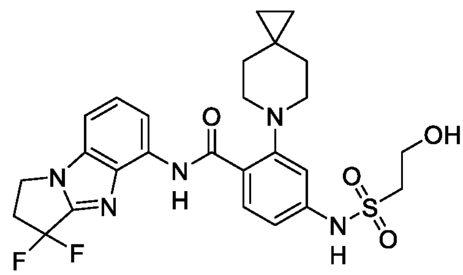

**94**

,

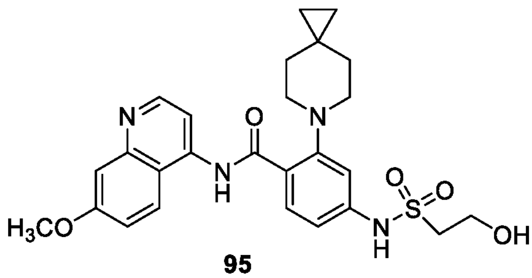

**95**

,

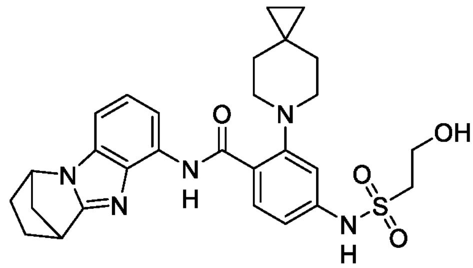

**96**

,

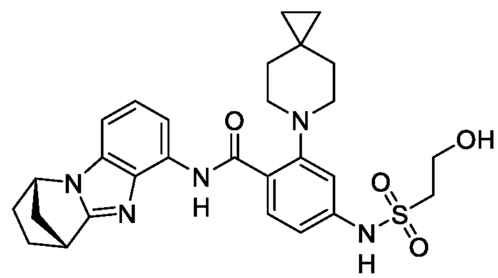

**96a**

,

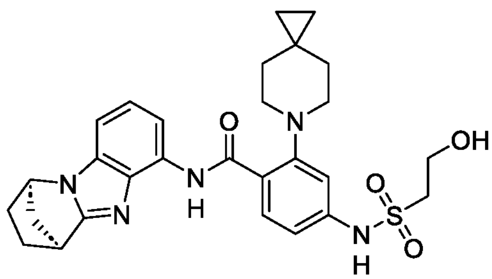

**96b**

,

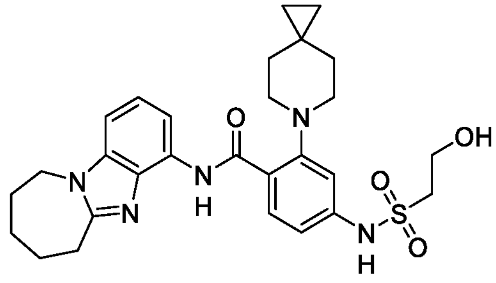

97 ,

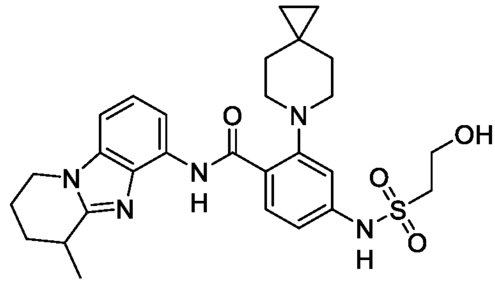

98 ,

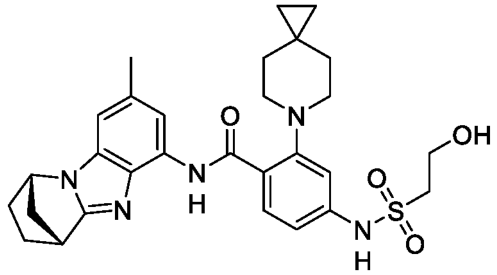

99 ,

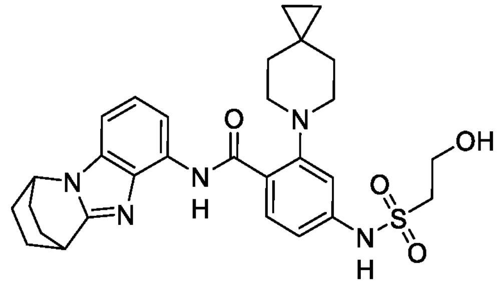

100 ,

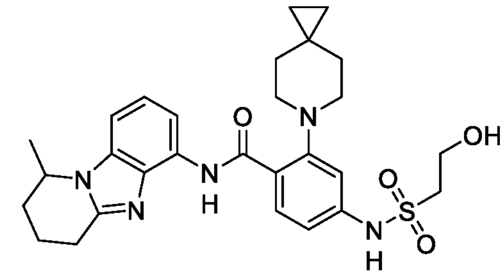

101 ,

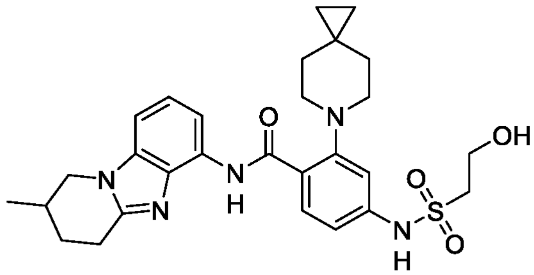

102 ,

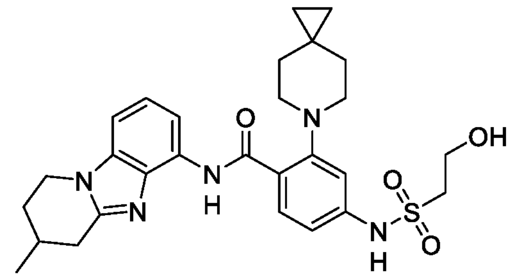

103 ,

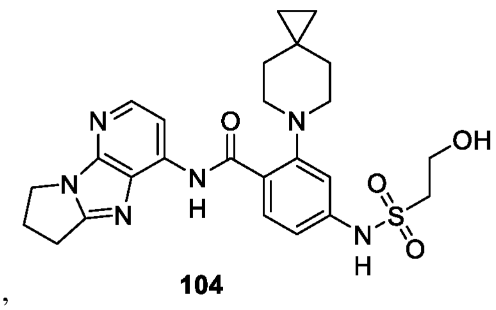

104 ,

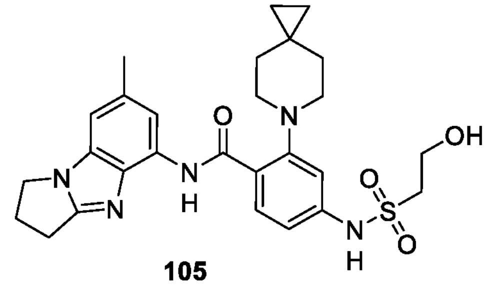

**105** ,

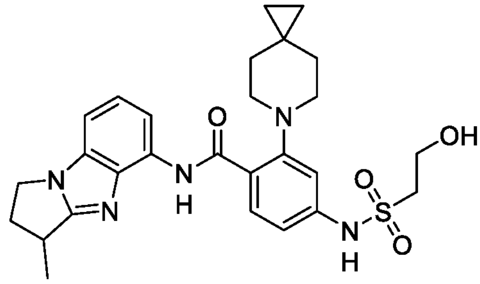

**106** ,

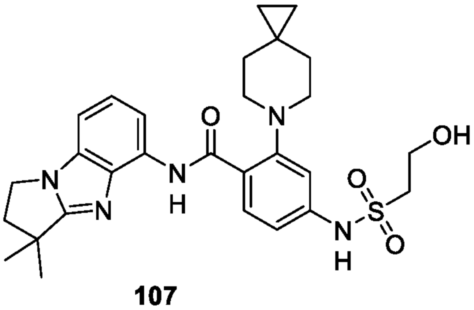

**107** ,

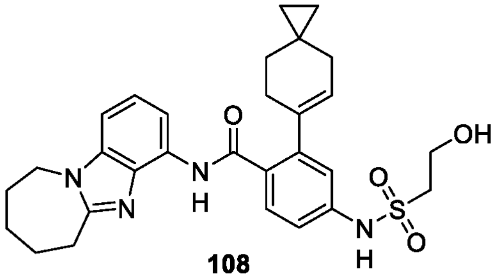

**108** ,

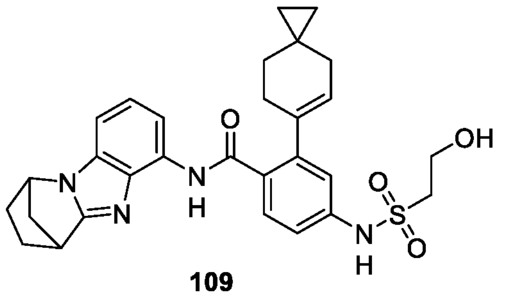

**109** ,

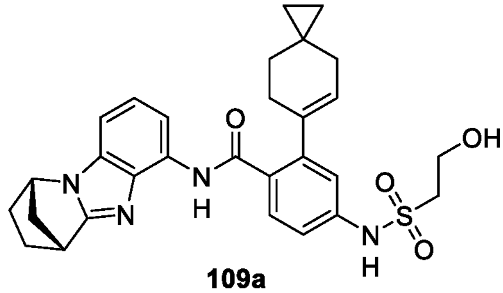

**109a** ,

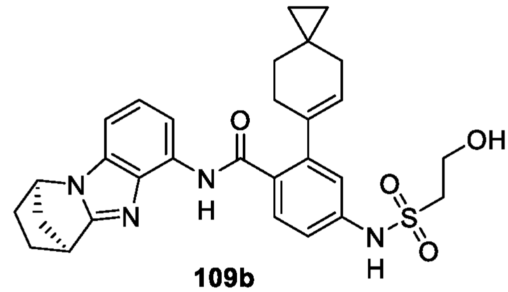

**109b** ,

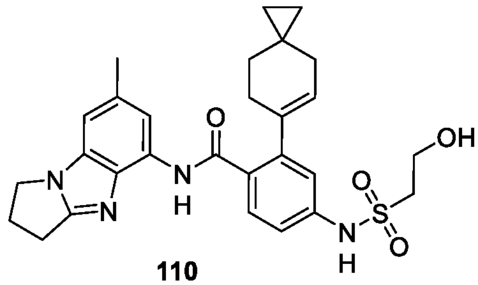

**110** ,

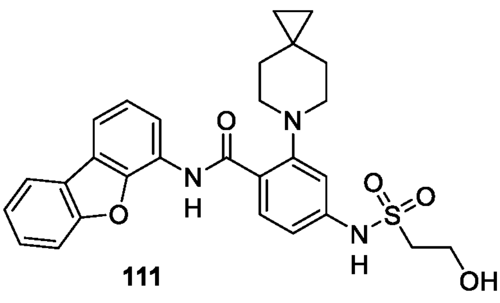

**111** ,

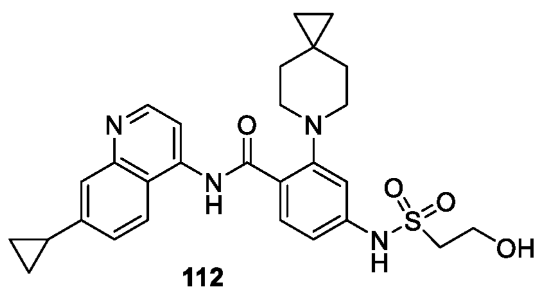

**112** ,

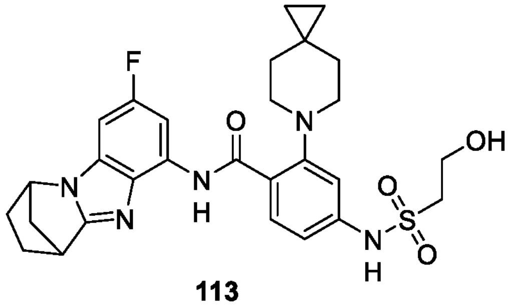

113 ,

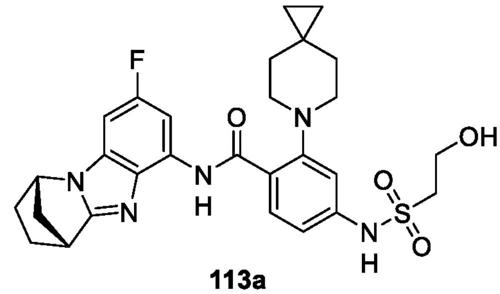

113a ,

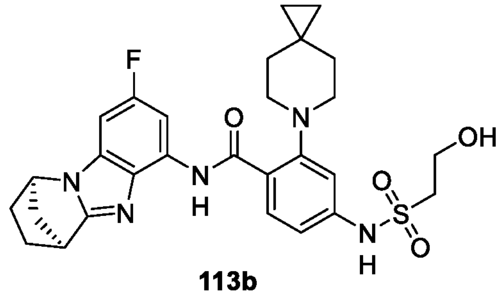

113b .

8. A method for preparing the compound, racemate, stereoisomer, tautomer, isotope-labeled counterpart, solvate, polymorph, pharmaceutically acceptable salt thereof or prodrug compound thereof according to any one of claims 1-7, comprising steps of:

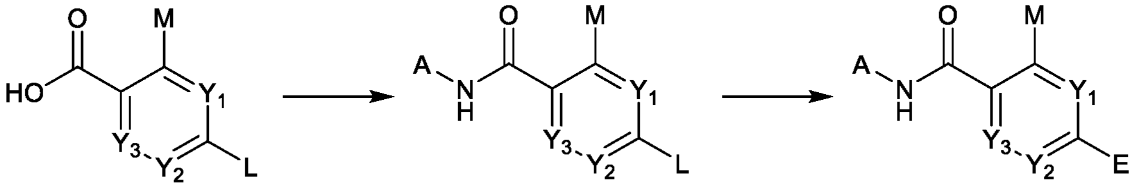

a b (I)

(1) reacting compound a with compound A-$NH_2$ to obtain compound b;
(2) reacting compound b with compound E-H to obtain the compound represented by formula (I);

wherein, A, E, M, $Y_1$, $Y_2$, $Y_3$ are independently as defined in any one of claims 1-7; L is selected from halogens, such as Cl, Br, I.

9. A pharmaceutical composition comprising a therapeutically effective amount of at least one of the compound, racemate, stereoisomer, tautomer, isotope-labeled counterpart, solvate, polymorph, pharmaceutically acceptable salt thereof or prodrug compound thereof according to any one of claims 1-7.

10. Use of at least one of the compound, racemate, stereoisomer, tautomer, isotope-labeled counterpart, solvate, polymorph, pharmaceutically acceptable salt thereof or prodrug compound thereof according to any one of claims 1-7 in the preparation of a drug;

preferably, the use is for use in the preparation of a drug for the treatment of a KIF18A mediated condition and/or disease, such as in the preparation of a KIF18A inhibitor drug;
preferably, the disease is for example cancer, including intestinal cancer, breast cancer, lung cancer, pancreatic cancer, prostate cancer, bladder cancer, head and neck cancer, cervical cancer or ovarian cancer.

**INTERNATIONAL SEARCH REPORT**

International application No.
**PCT/CN2023/117322**

**A. CLASSIFICATION OF SUBJECT MATTER**

C07D 401/12(2006.01)i; C07D 401/14(2006.01)i; A61K 31/438(2006.01)i; A61K 31/4427(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; CNABS; ENTXT; ENTXTC; VEN; CJFD; ISI-Web of Science; CNKI; 万方, WANFANG; STN: 长春金赛药业, 陆标, 杨方龙, 张辰, 王思勤, 金磊, KIF18A, 抑制, 癌, 肿瘤, 酰胺, 磺酰, 氮杂螺, 辛, +cancer?, inhibitory, inhibitor?, tumor, tumour, azaspiro+, +octan+, +sulfon+, 式(I)结构式

**C. DOCUMENTS CONSIDERED TO BE RELEVANT**

| Category* | Citation of document, with indication, where appropriate, of the relevant passages | Relevant to claim No. |
|---|---|---|
| E | WO 2023174175 A1 (WIGEN BIOMEDICINE TECHNOLOGY (SHANGHAI) CO., LTD.) 21 September 2023 (2023-09-21)<br>description, pages 8-24, 33, and 42 | 1-6, 9, 10 |
| X | CN 107398300 A (INSTITUTE OF CHEMISTRY, CHINESE ACADEMY OF SCIENCES) 28 November 2017 (2017-11-28)<br>embodiment 7 | 1, 2, 5 |
| X | ROANE, James et al. "A General Method for Aminoquinoline-Directed, Copper-Catalyzed sp2 C-H Bond Amination"<br>*J. Am. Chem. Soc.*, Vol. 138, No. 13, 18 March 2016 (2016-03-18), 4601-4607<br>ISSN: 0002-7863,<br>Table 2 | 1, 2, 5 |
| X | CAS. "2726653-65-0, 1798670-41-3, 1798670-34-4 et al."<br>*STN-REGISTRY*, 08 November 2021 (2021-11-08),<br>compound structure formula | 1, 2, 5 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 November 2023** | **28 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/117322**

**C. DOCUMENTS CONSIDERED TO BE RELEVANT**

| Category* | Citation of document, with indication, where appropriate, of the relevant passages | Relevant to claim No. |
|---|---|---|
| X | CN 114302880 A (AMGEN INC.) 08 April 2022 (2022-04-08) description, paragraphs [0008]-[0078], [0155]-[0160], [0306]-[0324], and [0478]-[0484], and tables 7-16 and A | 1-10 |
| Y | CN 114302880 A (AMGEN INC.) 08 April 2022 (2022-04-08) description, paragraphs [0008]-[0078], [0155]-[0160], [0306]-[0324], and [0478]-[0484], and tables 7-16 and A | 1-10 |
| Y | WO 2021026099 A1 (AMGEN INC.) 11 February 2021 (2021-02-11) description, paragraphs [0007]-[0033] | 1-10 |
| A | CN 113226473 A (AMGEN INC.) 06 August 2021 (2021-08-06) entire document | 1-10 |

Form PCT/ISA/210 (second sheet) (July 2022)

**INTERNATIONAL SEARCH REPORT**
**Information on patent family members**

International application No.
**PCT/CN2023/117322**

| Patent document cited in search report | | | Publication date (day/month/year) | Patent family member(s) | | | Publication date (day/month/year) |
|---|---|---|---|---|---|---|---|
| WO | 2023174175 | A1 | 21 September 2023 | None | | | |
| CN | 107398300 | A | 28 November 2017 | CN | 107398300 | B | 21 August 2020 |
| CN | 114302880 | A | 08 April 2022 | CA | 3146693 | A1 | 11 February 2021 |
| | | | | US | 2022289724 | A1 | 15 September 2022 |
| | | | | EP | 4007752 | A1 | 08 June 2022 |
| | | | | JP | 2022542967 | A | 07 October 2022 |
| | | | | WO | 2021026098 | A1 | 11 February 2021 |
| | | | | AU | 2020326627 | A1 | 17 March 2022 |
| WO | 2021026099 | A1 | 11 February 2021 | JP | 2022542319 | A | 30 September 2022 |
| | | | | AU | 2020324406 | A1 | 17 March 2022 |
| | | | | CA | 3147272 | A1 | 11 February 2021 |
| | | | | EP | 4007753 | A1 | 08 June 2022 |
| | | | | CN | 114401953 | A | 26 April 2022 |
| CN | 113226473 | A | 06 August 2021 | CA | 3123871 | A1 | 25 June 2020 |
| | | | | US | 2020239441 | A1 | 30 July 2020 |
| | | | | US | 11236069 | B2 | 01 February 2022 |
| | | | | US | 2022106293 | A1 | 07 April 2022 |
| | | | | TW | 202034924 | A | 01 October 2020 |
| | | | | JP | 2022513972 | A | 09 February 2022 |
| | | | | AU | 2019403486 | A1 | 24 June 2021 |
| | | | | BR | 112021011989 | A2 | 08 September 2021 |
| | | | | WO | 2020132648 | A1 | 25 June 2020 |
| | | | | KR | 20210106474 | A | 30 August 2021 |
| | | | | EP | 3897852 | A1 | 27 October 2021 |
| | | | | AR | 117490 | A1 | 11 August 2021 |
| | | | | SG | 11202106520 | VA | 29 July 2021 |
| | | | | IN | 202117031747 | A | 10 December 2021 |
| | | | | VN | 82664 | A | 27 December 2021 |
| | | | | HK | 40062253 | A0 | 10 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022110982256
- CN 2022114366075
- CN 2023100987012
- CN 2023111078615